(19) Europäisches Patentamt
European Patent Office
Office européen des brevets

(11) Veröffentlichungsnummer : **0 618 222 A2**

(12) # EUROPÄISCHE PATENTANMELDUNG

(21) Anmeldenummer : **94810133.2**

(22) Anmeldetag : **02.03.94**

(51) Int. Cl.[5] : **C07K 5/02,** C07K 7/02, A61K 37/64

(30) Priorität : **11.03.93 CH 772/93**

(43) Veröffentlichungstag der Anmeldung : **05.10.94 Patentblatt 94/40**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Bold, Guido, Dr.**
**Bleumatthöhe 16**
**CH-5264 Gipf-Oberfrick (CH)**
Erfinder : **Lang, Marc, Dr.**
**Rue de Valdoie 24**
**F-68200 Mulhouse (FR)**
Erfinder : **Fässler, Alexander, Dr.**
**Hallenstrasse 10**
**CH-4104 Oberwil (CH)**
Erfinder : **Capraro, Hans-Georg, Dr.**
**Habsburgerstrasse 60**
**CH-4310 Rheinfelden (CH)**
Erfinder : **Bhagwat, Shripad, Dr.**
**321 Victor Street**
**Scotch Plains, NJ 07076 (US)**

(54) **5-Amino-4-Hydroxy-Hexansäuredipeptid Derivate.**

(57) Beschrieben werden Verbindungen der Formel I',

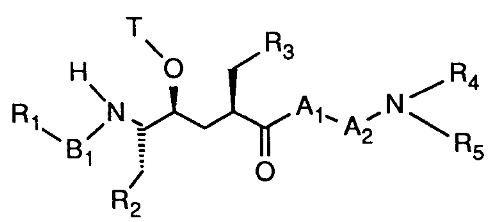

(I'),

worin T ein Acylradikal der Formel Z,

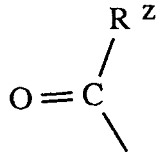

(Z)

worin

$R^Z$ unsubstituiertes oder substituiertes Hydrocarbyl, in dem mindestens ein Kohlenstoffatom durch ein Heteroatom ersetzt ist mit der Massgabe, dass ein Heteroatom nicht direkt an das Carbonyl gebunden ist, an das der Rest $R^Z$ gebunden ist, Alkyl mit 2 oder mehr Kohlenstoffatomen, Niederalkenyl, Niederalkinyl, Aryl oder unsubstituiertes oder substituiertes Amino bedeutet, und worin die Reste $R_1$, $B_1$, $R_2$, $R_3$, $A_1$, $A_2$ und $NR_4R_5$ die in der Beschreibung genannten Bedeutungen haben, und Vorstufen davon. Die erbindungen sind pharmazeutisch wirksam, etwa zur Behandlung von AIDS.

EP 0 618 222 A2

Jouve, 18, rue Saint-Denis, 75001 PARIS

Die Erfindung betrifft neue chemische Verbindungen, bei denen es sich um Derivate von nicht hydrolysierbaren Analoga für durch Aspartatproteasen spaltbare Peptide, nämlich acylierte 5-Amino-4-hydroxy-hexansäurederivate und neue Vorstufen davon, handelt, Verfahren zur Herstellung dieser Verbindungen, pharmazeutische Präparate, die diese Peptidanaloga enthalten, und deren Verwendung als Arzneimittel oder zur Herstellung pharmazeutischer Präparate zur Bekämpfung retroviral bedingter Erkrankungen.

Seit die Erkrankung erstmals zu Beginn der achtziger Jahre aufgetreten ist, ist AIDS (Aquired Immunodeficiency Disease Syndrome) mit seinen verheerenden Folgen Gegenstand zahlreicher wissenschaftlicher und sonstiger Publikationen. Mit Ausnahme kleinerer Gegenmeinungen gilt das Virus HIV (Human Immunodeficiency Virus) als Ursache dieser Erkrankung, von dem bisher zwei Formen (HIV-1 und HIV-2) näher beschrieben wurden. Zur Zeit werden vor allem Hemmer der Reversen Transcriptase, eines virusspezifischen Enzyms, zur Therapie dieser Erkrankung eingesetzt, beispielsweise 3'-Azidothymidin (AZT), obwohl diese starke toxische Nebenwirkungen haben. Auf der anderen Seite wurde versucht, den T4-Zellrezeptor, welcher auf bestimmten Zellen des Abwehrsystems im menschlichen Körper vorliegt und für die Verankerung und Einschleusung von infektiösen Viruspartikeln in diese Zellen verantwortlich ist und damit für deren Infektion, beispielsweise als rekombinantes Molekül oder Molekülbruchstück in den Körper einzuschleusen. Die Konsequenz wäre, dass Bindungsstellen an den Viruspartikeln wegtitriert würden und die Virionen so nicht mehr an die Zellen binden könnten. Zwar scheint die Behandlung mit CD4 und Derivaten den Vorteil geringer Toxizität zu haben, aber Daten über die therapeutische Wirksamkeit sind noch nicht bekannt.

HIV hat eine Genomorganisation, welche mit der anderer Retroviren vergleichbar ist: Das Genom ist in die Abschnitte gag/pol/env organisiert. Es konnte gezeigt werden, dass in HIV und anderen Retroviren die proteolytische Reifung der gag und gag/pol-Fusionsproteine durch eine Protease, die HIV-Protease, bewirkt wird, welche selbst durch die pol-Region des viralen Genoms kodiert wird. Ohne diese proteolytische Spaltung können keine infektiösen Viruspartikel entstehen, da beispielsweise die sogenannten "Core-Proteine", das sind Strukturproteine des Viruskerns, nicht aus ihren Vorläufern freigesetzt werden können.

Zur Zeit sind nach Schätzungen der WHO etwa 10 Millionen durch HIV infiziert. Die Erkrankung ist praktisch stets tödlich.

HIV-1 und HIV-2 weisen in ihrem Genom Bereiche auf, die für die HIV-Protease kodieren, welche aus einem Vorläuferprotein entsteht, das vermutlich autoprotolytisch gespalten wird.

Die Protease hat in ihrem katalytischen Zentrum einen Aspartatrest und ist homolog mit anderen Aspartatproteasen. Im Falle von HIV besteht sie aus 99 Aminosäuren. Die auch als rekombinantes und chemisch synthetisiertes Molekül inzwischen erhaltene HIV-Protease wurde mit verschiedenen Inhibitoren behandelt, wobei gezeigt werden konnte, dass auch sie als Aspartatprotease funktioniert. Röntgenstrukturanalyse der HIV-Protease und eines verwandten Enzyms aus Rous-Sarkom-Virus untermauert, dass das Enzym, welches als Dimeres in der aktiven Form vorliegt, als Aspartatprotease wirkt.

Aufgrund der zentralen Rolle der HIV-Protease bei der Prozessierung der genannten "Core-Proteine" wird davon ausgegangen, dass eine wirksame Inhibierung dieses Enzyms in vivo den Zusammenbau reifer Virionen unterbindet, so dass entsprechende Inhibitoren therapeutisch eingesetzt werden können.

Voraussetzungen für die therapeutische Wirksamkeit in vivo sind das Erreichen einer guten Hemmung der Virusvermehrung in Zellversuchen und eine gute Bioverfügbarkeit, z.B. ein hoher Blutspiegel, welcher ausreicht, um so im Körper an infizierten Zellen ausreichend hohe Konzentrationen zu erreichen.

Das Ziel der vorliegenden Erfindung ist, neue Verbindungen bereitzustellen, welche vorteilhafte pharmakologische Eigenschaften zeigen.

Überraschend wurde nun gefunden, dass die erfindungsgemässen Verbindungen in der Lage sind, dieses Ziel zu erreichen.

Bei den erfindungsgemässen Verbindungen handelt es sich in erster Linie um Verbindungen der Formel I',

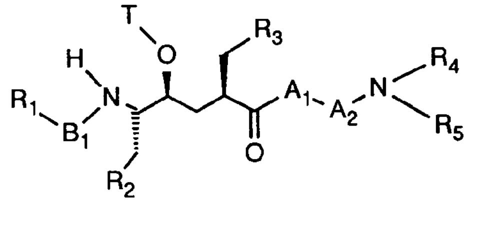

(I'),

worin T ein Acylradikal der Formel Z

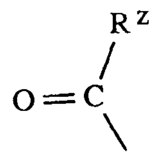

(Z)

worin

$R^z$ unsubstituiertes oder substituiertes Hydrocarbyl, in dem mindestens ein Kohlenstoffatom durch ein Heteroatom ersetzt ist mit der Massgabe, dass ein Heteroatom nicht direkt an das Carbonyl gebunden ist, an das der Rest $R^z$ gebunden ist, Alkyl mit 2 oder mehr Kohlenstoffatomen, Niederalkenyl, Niederalkinyl, Aryl oder unsubstituiertes oder substituiertes Amino bedeutet, und worin

$R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet,

$B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist,

$R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten,

$A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist,

$A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und

$R_4$ und $R_5$ gemeinsam mit dem bindenden Stickstoffatom unsubstituiertes oder substituiertes Thiomorpholino oder Morpholino bedeuten, oder Salze dieser Verbindungen, sofern salzbildende Gruppen vorliegen.

In der Beschreibung der vorliegenden Erfindung bedeutet der bei der Definition von Gruppen oder Resten, z. B. Niederalkyl, Niederalkoxycarbonyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Gegebenenfalls vorhandene asymmetrische Kohlenstoffatome in den Substituenten T, $R_1$, $B_1$, $R_2$, $R_3$, $A_1$und/oder $A_2$, sowie in aus $R_4$ und $R_5$ gemeinsam mit dem bindenden Stickstoffatom gebildetem substituiertem Thiomorpholino oder Morpholino, können in der (R)-, (S)- oder (R,S)-Konfiguration vorliegen, vorzugsweise in der (R)- oder (S)-Konfiguration. Somit können die vorliegenden Verbindungen als Isomerengemische oder als reine Isomeren, insbesondere als Diastereomerengemische, Enantiomerenpaare oder vorzugsweise reine Enantiomere vorliegen.

Die in der Beschreibung der vorliegenden Erfindung verwendeten allgemeinen Ausdrücke und Bezeichnungen haben vorzugsweise die folgenden Bedeutungen, wobei auf den verschiedenen Definitionsebenen von den vor- oder nachstehend aufgeführten Resten beliebige Kombinationen oder Einzelreste anstelle der allgemeinen Definitionen verwendet werden können:

Unsubstituiertes oder substituiertes Hydrocarbyl $R^z$, in dem mindestens ein Kohlenstoffatom durch ein Heteroatom ersetzt ist mit der Massgabe, dass ein Heteroatom nicht direkt an das Carbonyl gebunden ist, an das der Rest $R^z$ gebunden ist, ist ein gesättigter, teilweise gesättigter oder ungesättigter Kohlenwasserstoffrest mit maximal 30 Kohlenstoffatomen, vorzugsweise mit bis zu 22 Kohlenstoffatomen, enthält anstelle von mindestens einem Kohlenstoffatom, vorzugsweise anstelle von einem bis vier Kohlenstoffatomen, pro ersetztes Kohlenstoffatom ein Heteroatom ausgewählt aus Stickstoff, Sauerstoff oder Schwefel und ist unsubstituiert oder substituiert durch einen oder mehrere Reste, vorzugsweise bis zu drei Substituenten, insbesondere Hydroxy, Niederalkoxy, wie Methoxy, Niederalkoxy-niederalkoxy, wie 2-Methoxyethoxy, Niederalkoxy-niederalkoxy-niederalkyoxy, wie 2-(2-Methoxyethoxy)ethoxy, Phenyl- oder Napthylniederalkoxy-niederalkoxy, worin Phenyl oder Naphthyl unsubstituiert oder durch einen oder mehrere, vorzugsweise einen der Reste Halogen, wie Fluor, Chlor oder Brom, Niederalkyl, wie Methyl, Halogenniederalkyl, wie Trifluormethyl, Hydroxy, Niederalkoxy, wie Methoxy, Niederalkanoyloxy, Carboxy, Niederalkoxycarbonyl, Phenylniederalkoxycarbonyl,

Cyano, Carbamoyl, Mono- oder Diniederalkylcarbamoyl, Mono- oder Di-hydroxyniederalkyl-carbamoyl, Heterocyclylniederalkyl, worin Heterocyclyl einen gesättigten, teilweise gesättigten oder ungesättigten einfachen Ring bedeutet, welcher 3 bis 7, vorzugsweise 5 bis 7 Ringatome und bis zu zwei Heteroatome ausgewählt aus Stickstoff, Schwefel, Sauerstoff und durch Niederalkyl, Benzyl, Diphenylmethyl, Triphenylmethyl oder Niederalkanoyl substituiertem Stickstoff enthält, z.B. Piperidinomethyl, Piperazin-1-ylmethyl, 4-Niederalkyl-piperazin-1-yl-methyl, wie 4-Methyl- oder 4-Ethylpiperazin-1-ylmethyl, Morpholinomethyl oder Thiomorpholinomethyl, oder Nitro, welche unabhängig voneinander vorliegen können, substituiert ist, Phenylniederalkanoyloxy, wie Benzyloxy, Halogen, wie Fluor, Chlor oder Brom, Halogenniederalkyl, wie Trifluormethyl oder Chlormethyl, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, $C_3$-$C_8$-Cycloakyl, wie Cyclobutyl, Cyclopentyl oder Cyclohexyl, oder Aryl, vorzugsweise $C_6$-$C_{14}$-Aryl, wie Phenyl, Naphthyl, wie 1- oder 2-Naphthyl, oder Fluorenyl, wie Fluoren-9-yl, wobei im Falle des Vorliegens mehrerer Substituenten diese unabhängig voneinander sind; wobei ein Heteroatom vorzugsweise direkt dem im Rest Hydrocarbyl $R^z$ enthaltenen, an die Carbonylgruppe, welche $R^z$ in Formel I' bindet, bindenden Kohlenstoffatom benachbart ist (d.h. in 3-Stellung ab der in die Zählung einbezogenen $R^Z$ bindenden Carbonylgruppe vorliegt); und ist vor allem

- über ein Ringkohlenstoffatom gebundenes Heterocyclyl, welches vorzugsweise einen gesättigten, teilweise gesättigten oder ungesättigten Ring bedeutet, der 3 bis 7, insbesondere 5 bis 7 Ringatome enthält, und ein oder mehrere, insbesondere bis zu höchstens vier, in erster Linie bis zu zwei Heteroatome, die unabhängig voneinander aus Stickstoff, Schwefel und Sauerstoff ausgewählt sind, enthält; wobei der Ring entweder als solcher vorliegt oder bis zu zweifach, insbesondere einfach, benzanneliert, cyclopenta-, cyclohexa- oder cyclohepta-anneliert sein kann; wobei das Ringsystem durch bis zu drei unabhängig voneinander aus Niederalkyl, Phenylniederalkyl, Diphenylniederalkyl, Triphenylniederalkyl, wie Triphenylmethyl, Niederalkanoyl, Hydroxy, Niederalkoxy, Phenylniederalkoxy, wie Benzyloxy, Diphenylmethoxy oder Triphenylmethoxy, Hydroxyniederalkyl, wie Hydroxymethyl, Halogen, wie Fluor, Chlor oder Brom, Cyano, Niederalkoxycarbonyl, wie Methoxy- oder tert-Butoxycarbonyl, Phenylniederalkoxycarbonyl, wie Benzyloxycarbonyl und Haloniederalkyl, wie Chlormethyl oder Trifluormethyl, ausgewählte Reste substituiert oder vorzugsweise unsubstituiert sein kann; in erster Linie ausgewählt aus Pyrrolyl, 2,5-Dihydropyrrolyl, Indolyl, Indolizinyl, Isoindolyl, Pyrrolidinyl, wie Pyrrolidin-3-yl oder insbesondere Pyrrolidin-2-yl (in der (R,S)- oder vorzugsweise der (R)- oder (S)-Konfiguration), Hydroxypyrrolidinyl, wie 3- oder insbesondere 4-Hydroxypyrrolidinyl, Furyl, wie Furan-3-yl oder insbesondere Furan-2-yl, Tetrahydrofuryl, Thienyl, Cyclohepta[b]pyrrolyl, Imidazolyl, wie Imidazolyl-2-yl, Imidazolyl-3-yl oder insbesondere Imidazolyl-5-yl, N-Triphenylniederalkyl-imidazolyl, wie N-Triphenylmethyl-imidazolyl, Pyrazolyl, insbesondere Pyrazol-3-yl, Oxazolyl, Isoxazolyl, wie Isoxazol-3-yl oder -5-yl, Thiazolyl, Isothiazolyl, wie Isothiazol-3-yl oder -5-yl, Triazolyl, wie 1,2,3-Triazoly-4- oder -5-yl oder 1,2,4-Triazol-5-yl, Tetrazolyl, Pyridyl, wie Pyridin-4-yl oder -3-yl oder insbesondere Pyridin-2-yl, Chinolyl, wie Chinolin-2-yl, Isochinolyl, insbesondere Isochinolin-1-yl oder -3-yl, Piperidyl, insbesondere Piperidin-2-yl, γ-Pyranyl, 4, 5-Dihydropyranyl, 4H-Chromenyl, Chromanyl, γ-Thiopyranyl, Pyridazinyl, Cinnolyl, Phthalazinyl, Chinazolinyl, Chinoxalinyl, Pyrimidinyl, Pyrazinyl, Phenyzinyl, Phenoxazinyl, Phenothiazinyl, Morpholinyl und Thiazinyl, welche über ein Ringkohlenstoffatom gebunden sind; wobei diejenigen der genannten Reste, die ein Ringheteroatom direkt in Nachbarstellung zum bindenden Ringkohlenstoffatom enthalten, ganz besonders bevorzugt sind. Sehr bevorzugt sind die Reste Furan-2-yl, (S)- oder (R)-Pyrrolidin-2-yl und Imidazol-4-yl, Pyridin-2-yl, -3-yl oder -4-yl, oder Isochinolin-3-yl; sowie ferner Tetrahydropyranyl, wie 4-Tetrahydropyranyl;

- durch mindestens einen aus verethertem oder verestertem Hydroxy oder (unoxidiertem oder durch 1 oder 2 Oxogruppen oxidiertem) Mercapto, unsubstituiertem oder substituiertem Amino und Heterocyclyl ausgewählte Reste, insbesondere einen der genannten Reste, substituiertes Niederalkyl (insbesondere Methyl, Ethyl, n-Propyl oder n-Butyl), welches einen weiteren Substituenten Aryl, der wie unten am Ende dieses Abschnittes definiert ist, tragen kann, worin

verethertes Hydroxy in erster Linie Niederalkoxy, wie Methoxy, Ethoxy oder n-Butoxy, oder durch einen oder zwei Substituenten, insbesondere Aryl, vor allem Phenyl oder Napthyl, Niederalkoxy, wie Ethoxy oder Methoxy, Niederalkylthio, wie Methylthio oder Ethylthio, Niederalkoxyniederalkoxy, wie 2-Methoxyethoxy, Niederalkylthioniederalkoxy, wie 2-Methylthioethoxy, Aryloxy oder Arylthio, worin Aryl die unten genannten Bedeutungen hat, insbesondere Phenyl oder o-, m- oder p-Chlorphenyl, z.B. p-Chlorphenyloxy, Amino, N-Niederalkylamino oder N,N-Diniederalkylamino, wie 2-Amino, 2-(N-Niederalkyl)amino oder 2-(N,N-Diniederalkyl) amino, z.B. 2-Dimethylamino, Heterocyclyl, welches wie oben für über ein Ringkohlenstoffatom gebundenes Heterocyclyl $R^z$ definiert ist, insbesondere 2-, 3- oder 4-Pyridyl, oder (ferner oder vorzugsweise) durch Niederalkoxycarbonyl, wie Methoxycarbonyl, Niederalkanoyloxyniederalkoxycarbonyl, wie Pivaloyloxymethoxycarbonyl oder Acetyloxymethoxycarbonyl, Carboxy, Phenylniederalkoxycarbonylamino-niederalkoxy, wie 2-(Benzyloxycarbonylamino)-ethoxy, Aminoniederalkoxy, wie 2-Aminoethoxy, Diniederalkylaminoniederalkoxy,

wie 2-(Dimethylamino)-ethoxy, oder N,N-Diniederalkylaminoniederalkoxy, wie 2-(Dimethylamino)-ethoxy, substituiertes Niederalkoxy, wie Methoxy, Ethoxy oder n-Butoxy, oder Aryloxy, insbesondere Phenyloxy, oder (ferner oder insbesondere) Tetrahydropyranyloxy, wie 4-Tetrahydropyranyloxy, bedeutet;

verestertes Hydroxy in erster Linie Niederalkanoyloxy, wie Acetyloxy, Benzoyloxy oder Phenylniederalkanoyloxy, wie Phenylacetyloxy, bedeutet;

verethertes Mercapto in erster Linie Niederalkylthio, wie Methylthio, Ethylthio oder n-Butylthio, oder durch einen oder zwei Substituenten, insbesondere Aryl, vor allem Phenyl oder Napthyl, Niederalkoxy, wie Ethoxy oder Methoxy, Niederalkylthio, wie Methylthio oder Ethylthio, Niederalkoxyniederalkoxy, wie 2-Methoxyethoxy, Niederalkylthioniederalkoxy, wie 2-Methylthioethoxy, Aryloxy oder Arylthio, worin Aryl die unten genannten Bedeutungen hat, insbesondere Phenyl oder o-, m- oder p-Chlorphenyl, z.B. p-Chlorphenyloxy, Amino, N-Niederalkylamino oder N,N-Diniederalkylamino, wie 2-Amino, 2-(N-Niederalkyl)amino oder 2-(N,N-Diniederalkyl)amino, z.B. 2-Dimethylamino, Heterocyclyl, welches wie oben für über ein Ringkohlenstoffatom gebundenes Heterocyclyl $R^z$ definiert ist, insbesondere 2-, 3- oder 4-Pyridyl, oder (ferner oder insbesondere) Niederalkoxycarbonyl, wie Methoxycarbonyl, substituiertes Niederalkylthio, wie Methylthio, Ethylthio oder n-Butylthio, oder Arylthio, wie Phenylthio, bedeutet; wobei der Mercapto-Schwefel ferner oder insbesondere durch eine oder vorzugsweise 2 Oxo-Gruppen oxidiert sein kann, vor allem in Niederalkoxycarbonyl-niederalkylsulfo, wie Methoxycarbonyl-methylsulfo;

verestertes Mercapto in erster Linie Niederalkanoylthio, Benzoylthio oder Phenylniederalkanoylthio, wie Phenacetylthio, bedeutet;

unsubstituiertes oder substituiertes Amino in erster Linie Amino oder durch einen oder zwei Reste ausgewählt aus Niederalkyl, wie Methyl, Heterocyclylniederalkyl, worin Heterocyclyl wie für über ein Ringkohlenstoffatom gebundenes Heterocyclyl $R^z$ definiert ist, insbesondere Heterocyclylmethyl, wie über einen Ringkohlenstoff gebundenes Imidazolylmethyl, z.B. 4-Imidazolylmethyl, oder Pyridylmethyl, z.B. 2-, 3- oder 4-Pyridylmethyl, Arylniederalkyl, wie Phenyl- oder Napthylniederalkyl, z.B. Phenyl- oder Naphthylmethyl, Niederalkanoyl, wie Acetyl, Niederalkoxycarbonyl, wie tert-Butoxycarbonyl, Arylniederalkoxycarbonyl, wie Phenylniederalkoxycarbonyl, z.B. Benzyloxycarbonyl, substituiertes Amino bedeutet, wobei in allererster Linie einer der Aminosubstituenten Niederalkyl, insbesondere Methyl ist, und der andere Wasserstoff oder einen der zuvor als Aminosubstituenten genannten Reste bedeutet; und

Heterocyclyl in erster Linie wie oben für über ein Ringkohlenstoffatom gebundenes Heterocyclyl $R^z$, welches vorzugsweise einen gesättigten, teilweise gesättigten oder ungesättigten Ring bedeutet und auch wie oben anneliert oder substituiert sein kann, definiert ist, insbesondere als Pyridin-2-yl, -3-yl oder -4-yl;

oder

- Heterocyclylniederalkyl, worin Niederalkyl vorzugsweise Methyl, 1- oder 2-Ethyl oder 3-Propyl bedeutet und worin Heterocyclyl wie oben für über ein Ringkohlenstoffatom gebundenes Heterocyclyl $R^z$, welches vorzugsweise einen gesättigten, teilweise gesättigten oder ungesättigten Ring bedeutet und auch wie oben anneliert oder substituiert sein kann, definiert ist, jedoch auch über ein Ringstickstoffatom gebunden sein kann, insbesondere Imidazol-1-yl, Imidazol-2-yl, Imidazol-5-yl oder vor allem Imidazol-4-yl, N-Triphenylniederalkylimidazolyl, wie N-Triphenylmethyl-imidazol-5-yl oder insbesondere -4-yl, oder Pyrazolyl, wie Pyrazol-1-yl, -3-yl, -4-yl oder -5-yl.

Alkyl $R^z$ mit zwei oder mehr Kohlenstoffatomen ist insbesondere Ethyl, n-Propyl, Isopropyl, n-Butyl oder 1,1-Dimethylethyl, oder vor allem $C_7$-$C_{20}$-Alkyl, so dass T z.B. Propionyl, Butyryl, Methylpropionyl, Valeroyl oder Pivaloyl, oder vor allem Octanoyl, Decanoyl oder Palmitoyl bedeutet.

Niederalkenyl $R^z$ ist in erster Linie $C_2$-$C_7$-, vor allem $C_2$-$C_3$-Alkenyl, worin die Doppelbindung vorzugsweise in 1-Stellung vorliegt, so dass T z.B. Acryloyl, Crotonoyl, Isocrotonoyl oder Methacryloyl bedeutet.

Niederalkinyl $R^z$ ist in erster Linie $C_2$-$C_7$-, vor allem $C_2$-$C_3$Alkinyl, worin die Dreifachbindung vorzugsweise in 1-Stellung vorliegt, so dass T z.B. Propinoyl bedeutet.

Aryl $R^z$ selbst oder Aryl als Substituent in den oben genannten Resten $R^z$ ausser Aryl selbst ist in erster Linie $C_6$-$C_{14}$-Aryl, vor allem Phenyl, Napthtyl, wie 1- oder 2-Napthtyl, oder Fluorenyl, wie 9-Fluorenyl, und ist unsubstituiert oder substituiert durch bis zu drei unabhängig voneinander aus Niederalkyl, Niederalkanoyl, Hydroxy, Niederalkoxy, Phenylniederalkoxy, wie Benzyloxy, Diphenylmethoxy oder Triphenylmethoxy, Hydroxyniederalkyl, wie Hydroxymethyl, Halogen, wie Fluor, Chlor oder Brom, Cyano, Niederalkoxycarbonyl, wie Methoxy- oder tert-Butoxycarbonyl, Phenylniederalkoxycarbonyl, wie Benzyloxycarbonyl, Haloniederalkyl, wie Chlormethyl oder Trifluormethyl, Heterocyclylniederalkyl, worin Heterocyclyl einen gesättigten, teilweise gesättigten oder ungesättigten einfachen Ring bedeutet, welcher 3 bis 7, vorzugsweise 5 bis 7 Ringatome und bis zu zwei Heteroatome ausgewählt aus Stickstoff, Schwefel, Sauerstoff und durch Niederalkyl, Benzyl, Diphenylmethyl, Triphenylmethyl oder Niederalkanoyl substituiertem Stickstoff enthält, z.B. Piperidinomethyl, Piperazin-1-ylmethyl, 4-Niederalkylpiperazin-1-yl-methyl, wie 4-Methyl- oder 4-Ethylpiperazin-1-ylmethyl, Morpholinomethyl oder Thiomorpholinomethyl, und Nitro ausgewählte Reste, welche unabhängig voneinander

vorliegen können, substituiert ist, insbesondere entsprechend substituiertes Phenyl. Besonders bevorzugt ist o-, m- oder p-Chlorphenyl, Chlorniederalkylphenyl, wie p-Chlormethylphenyl, p-(Morpholinoniederalkyl)phenyl, wie p-Morpholinomethylphenyl, oder p-(Thiomorpholinoniederalkyl)phenyl, wie p-Thiomorpholinomethylphenyl, oder ferner Phenyl.

Unsubstituiertes oder substituiertes Amino $R^z$ trägt am Stickstoff 1 bis 2 Substituenten, die unabhängig voneinander aus unsubstituiertem oder substituiertem Niederalkyl, worin die Substituenten von Niederalkyl vorzugsweise aus Hydroxy, Niederalkoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Cyano, Oxo und Phenyl oder Naphthyl, welche unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Halogenniederalkyl, wie Trifluormethyl, Cyano und/oder Nitro ein oder mehrfach, vorzugsweise einfach, substituiert sind, insbesondere Phenyl, welches durch einen der genannten Reste in p-Stellung substituiert ist, ausgewählt sind; insbesondere aus unsubstituiertem Niederalkyl, wie Methyl oder Ethyl; und Aryl, welches vorzugsweise 6 bis 14 Kohlenstoffatome hat und unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Haloniederalkyl, wie Trifluormethyl, Cyano und/oder Nitro ein oder mehrfach, vorzugsweise einfach, substituiert ist; ausgewählt sind, wobei der Stickstoff der resultierenden N-substituierten Carbamoylgruppe T nicht mehr als einen Arylrest trägt; insbesondere handelt es sich um Amino, Mono- oder Diniederalkylamino, wie N-Methyl-, N-Ethyl-, N,N-Dimethyl- oder N,N-Diethylamino, oder Phenylniederalkylamino, worin Phenyl unsubstituiert oder durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, substituiert ist, wie in N-Benzyl-, N-(4-Fluorbenzyl)-, N-(4-Chlorbenzyl)-, N-(4-Trifluormethylbenzyl)- oder N-(4-Cyanobenzyl)-amino; besonders bevorzugt ist durch nur einen Rest am Stickstoff substituiertes Amino, z.B. N-Niederalkylamino, wie N-Methyl- oder N-Ethyl-amino, oder Phenylniederalkylamino, worin Phenyl unsubstituiert oder durch Niederalkyl, wie Methyl, Halogenniederalkyl, wie Chlor- oder Brommethyl oder Trifluormethyl, Halogen, wie Fluor oder Chlor, Hydroxy, Niederalkoxy, wie Methoxy, Carboxy, und/oder Cyano substituiert ist, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-amino. Die jeweils unter die Definition von unsubstituiertem oder substituiertem Amino $R^z$ fallenden Definitionen und der Rest Aminocarbonyloxy $R_5$ können vorzugsweise bei allen vor- und nachstehend genannten Definitionen von Verbindungen der Formel I' weggelassen werden.

Besonders bevorzugt sind für $R^z$ die genannten Bedeutungen ausser Alkyl mit mehr als 2 Kohlenstoffatomen, Niederalkenyl und Niederalkinyl; ferner auch ausser Aryl.

Insbesondere sind bei allen Definitionen solche Reste $R^z$ bevorzugt, bei denen in 2-Stellung oder ferner in einer höheren Stellung, wie 3- oder 4-Stellung, ein Heteroatom ausgewählt aus Stickstoff, Sauerstoff oder Schwefel vorliegt, insbesondere Stickstoff (besonders wertvolle Eigenschaften, z.B. besonders gute Abspaltbarkeit von T, liegen hier vor).

T ist in erster Linie Pyrrolidin-2-ylcarbonyl oder -3-ylcarbonyl, wie (R)- oder (S)-Pyrrolidin-2-ylcarbonyl ((D)- oder (L)-Prolyl), Furan-3- oder insbesondere Furan-2-ylcarbonyl, Pyridyl-4-, Pyridyl-3- oder insbesondere Pyridyl-2-ylcarbonyl, Isochinolin- 1- oder insbesondere Isochinolin-3-ylcarbonyl, Pyrazin-2-ylcarbonyl, Niederalkoxyniederalkylcarbonyl, wie Methoxyacetyl, n-Butoxyacetyl oder 3-Methoxypropionyl, Phenyl- oder Naphthylniederalkoxyniederalkylcarbonyl, wie Benzyloxyacetyl, $\alpha$-Niederalkoxy-$\alpha$-phenylniede ralkylcarbonyl, wie (R)- oder (S)-$\alpha$-Methoxy-$\alpha$-phenylacetyl, Niederalkoxyniederalkoxyniederalkylcarbonyl, wie 2-(Methoxyethoxy)acetyl, Niederalkoxyniederalkoxyniederalkoxyniederalkylcarbonyl, wie 2-(2-(Methoxyethoxy)ethoxy)acetyl, o-, m- oder p-Chlorphenyloxy-niederalkoxy-niederalkylcarbonyl, N,N-Diniederalkylaminoniederalkoxyniederalkylcarbonyl, wie 2-(N,N-Dimethylamino)-ethyloxyacetyl oder -3-propionyl, 2-, 3- oder 4-Pyridylniederalkyloxyniederalkylcarbonyl, wie Pyridin-2-ylmethoxyacetyl, Phenyloxyniederalkylcarbonyl, wie Phenoxyacetyl, Niederalkylthioniederalkylcarbonyl, wie Methylthioacetyl, Phenylniederalkylthioniederalkylcarbonyl, wie Benzylthioacetyl, N,N-Diniederalkylaminoniederalkylcarbonyl, wie N,N-Dimethylaminoacetyl, -3-propionyl oder -4-butyryl, N-Niederalkylaminoniederalkylcarbonyl, wie N-Methylaminoacetyl oder -3-propionyl, N-Imidazol(-2-, -4- oder -5-)ylmethyl-N-niederalkylaminoniederalkylcarbonyl, wie N-(Imidazol-4-ylmethyl)-N-methylaminoacetyl, N-Pyridin(-2-,-3- oder -4-)ylmethyl-N-niederalkylaminoniederalkylcarbonyl, wie N-Pyridin-2-ylmethyl-N-methylaminoacetyl, N-Phenylniederalkoxycarbonyl-N-niederalkylaminoniederalkylcarbonyl, wie N-Benzyloxycarbonyl-N-methylaminoacetyl, Imidazol(-1-, -2-, -4- oder -5-)ylniederalkylcarbonyl,

wie 3-(Imidazol-4-yl)propionyl, oder N-Triphenylniederalkylimidazol(-4- oder -5-)ylniederalkylcarbonyl, wie 3-(N-Triphenylmethylimidazol-4-yl)propionyl, oder Pyrazol(-1-, -3-, -4- oder -5-)ylniederalkylcarbonyl, wie Pyrazol-1-ylacetyl, oder ferner Haloniederalkylbenzoyl, wie p-Chlormethylbenzoyl, p-(Morpholino- oder Thiomorpholinomethyl)benzoyl oder Benzoyl, und (ferner oder insbesondere) Niederalkanoyloxy-niederalkylcarbonyl, wie Acetyloxyacetyl, Niederalkoxycarbonylniederalkoxy-niederalkylcarbonyl, wie Methoxycarbonylmethoxyacetyl, Niederalkoxycarbonylniederalkylthio-niederalkylcarbonyl, wie Methoxycarbonylmethylthio-acetyl, Niederal- koxycarbonylniederalkylsulfo-niederalkylcarbonyl, wie Methoxycarbonylmethylsulfo-acetyl, Niederalkanoy- loxyniederalkoxycarbonylniederalkoxy-niederalkylcarbonyl, wie Pivaloyloxymethoxycarbonylmethoxyacetyl oder Acetyloxymethoxycarbonylmethoxy-acetyl, Carboxyniederalkoxyniederalkylcarbonyl, wie Carboxyme- thoxyacetyl, N-Phenylniederalkoxycarbonylamino-niederalkylcarbonyl, wie 3-Benzyloxycarbonylamino-propionyl, Aminoniederalkylcarbonyl, wie 3-Aminopropionyl, Diniederalkylaminonie- deralkoxyniederalkoxy-niederalkylcarbonyl, wie [2-(2-Dimethylaminoethoxy)-ethoxy]-acetyl, N-Phenylniederalkoxycarbonyl-aminoniederalkoxyniederalkoxy-niederalkylcarbonyl, wie 2-(2-N-Benzyloxycarbonylaminoethoxy)ethoxy-acetyl, Aminoniederalkoxyniederalkoxy-niederalkylcarbonyl, wie 2-(2-Aminoethoxy)ethoxy-acetyl, oder Tetrahydro- furanyloxy-niederalkylcarbonyl, wie 2-(4-Tetrahydrofuranyloxy)-acetyl oder 2(R)-, 2(S)- oder ferner 2(R,S)-(4-Tetrahydrofuranyloxy)-propionyl.

Niederalkoxycarbonyl $R_1$ enthält vorzugsweise einen verzweigten Niederalkylrest, insbesondere einen sec- oder tert-Niederalkylrest, und ist z. B. Butoxycarbonyl, wie tert-Butoxycarbonyl oder Isobutoxycarbonyl oder (ferner oder insbesondere) Ethoxycarbonyl. Besonders bevorzugt ist tert-Butoxycarbonyl.

Heterocyclylcarbonyl $R_1$ enthält insbesondere einen 5- oder 6-gliedrigen Heterocyclus, der 1 bis 3 Heteroatome, die unabhängig voneinander aus S, O oder N ausgewählt sind, enthält, ungesättigt oder ganz oder teilweise gesättigt ist und ein- oder bis zu dreifach benzanelliert, cyclopenta-, cyclohexa- oder cycloheptaanelliert ist, wobei die genannten anellierten Ringe ein weiteres Stickstoffatom als Heteroatom enthalten können, beispielsweise einen Heterocyclylrest ausgewählt aus Pyrrolyl, Furanyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, vorzugsweise teilweise gesättigt, oder ist ausgewählt aus Pyridylcarbonyl, z. B. Pyridyl-3-carbonyl, Morpholinylcarbonyl, z. B. Morpholinocarbonyl, und Benzofuranoyl, z.B. 3-Benzofuranoyl, sowie alternativ oder ergänzend hierzu Tetrahydroisochinolylcarbonyl, z. B. Tetrahydroisochinolyl-3-carbonyl, vorzugsweise Tetrahydroisochinolyl-3(S)-carbonyl.

Benzyloxycarbonyl $R_1$ ist unsubstituiert oder durch bis zu drei unabhängig voneinander aus Fluor, Haloniederalkyl, z. B. Trifluormethyl oder Pentafluorethyl, Niederalkanoyl, wie Acetyl, Propanoyl, Butyryl oder Pivaloyl, Sulfo, Niederalkylsulfonyl, z. B. Methylsulfonyl, Ethylsulfonyl, n-Propylsulfonyl oder iso-Propylsulfonyl, und Cyano ausgewählte Reste substituiert. Bevorzugt ist unsubstituiertes oder durch einen aus Fluor, Trifluormethyl, Sulfo, Methylsulfonyl, Ethylsulfonyl und Cyano ausgewählten Rest im Phenylring o-, m- oder p-substituiertes, insbesondere p-substituiertes Benzyloxycarbonyl, z. B. Benzyloxycarbonyl, Fluorphenylmethoxycarbonyl, wie p-Fluorphenylmethoxycarbonyl, Trifluormethylphenylmethoxycarbonyl, wie p-Trifluormethylphenylmethoxycarbonyl, Methylsulfonylphenylmethoxycarbonyl, wie p-Methylsulfonylphenylmethoxycarbonyl, oder Cyanophenylmethoxycarbonyl, wie p-Cyanophenylmethoxycarbonyl.

Heterocyclyloxycarbonyl $R_1$ enthält als Heterocyclyl insbesondere einen 5- oder 6-gliedrigen Heterocyclus, der 1 bis 3 Heteroatome, die unabhängig voneinander aus S, O oder N ausgewählt sind, enthält, ungesättigt oder ganz oder teilweise gesättigt ist und ein- oder bis zu dreifach benzanelliert, cyclopenta-, cyclohexa- oder cyclohepta-anelliert ist, wobei die genannten anellierten Ringe ein weiteres Stickstoffatom als Heteroatom enthalten können, beispielsweise einen Rest ausgewählt aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl, oder ferner oder insbesondere γ-Pyranyl oder Furanyl; und einem benzanellierten, cyclopenta-, cyclohexa- oder cycloheptaanellierten Derivat dieser Reste, die auch ganz oder teilweise gesättigt vorliegen können, wobei die Heterocyclylreste über ein Ringkohlenstoffatom an den Sauerstoff des zugehörigen Oxycarbonylrestes gebunden sind, vorzugsweise ausgewählt aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem ganz oder teilweise gesättigten Derivat dieser Reste, z. B. einem teilweise gesättigten Derivat dieser Reste oder Indol3-yl-oxycarbonyl, Benzthiazol-6-yloxycarbonyl oder Chinol-8-yl-oxycarbonyl, ferner oder insbesondere Tetrahydropyranyloxycarbonyl, wie 4-Tetrahydropyranyloxycarbonyl, oder Tetrahydrofuranyloxycarbonyl, wie 3(R)-, 3(S)- oder ferner 3(R,S).Tetrahydrofuranyloxycarbonyl. In einer ganz besonders bevorzugten Variante der Definition von $R_1$ sind die unter die Definition der Substituenten Heterocyclyloxycarbonyl fallenden Reste auf allen Definitionsebenen nicht umfasst.

In den genannten Resten kann die bindende Carbonyl- auch durch eine Thiocarbonylgruppe ersetzt sein. Bevorzugt ist eine Carbonylgruppe.

Niederalkylsulfonyl (= Niederalkyl-$SO_2$-) $R_1$ ist vorzugsweise Methylsulfonyl, Ethylsulfonyl, n-Propylsulfonyl oder iso-Propylsulfonyl. Die Verbindungen der Formel I', worin $R_1$ Niederalkylsulfonyl bedeutet und die übrigen Reste die genannten Bedeutungen haben, können bei der Definition der Verbindungen der Formel I' vorzugsweise wegfallen, oder sie sind besonders bevorzugt.

Heterocyclylsulfonyl enthält als Heterocyclyl vorzugsweise einen der unter Heterocyclylcarbonyl $R_1$ genannten Heterocyclen, der unsubstituiert oder durch Niederalkyl, wie Methyl oder Ethyl, substituiert ist, wobei Heterocyclen bevorzugt sind, die wenigstens ein Stickstoffatom enthalten, welches an den Schwefel der Sulfonylgruppe gebunden ist, und ist insbesondere Piperidinosulfonyl, unsubstituiertes oder am nicht an den Sulfonyl-Schwefel gebundenen Stickstoff durch Niederalkyl, wie Methyl, substituiertes Piperazin-1-yl-sulfonyl, Pyrrolidin-1-yl-sulfonyl, Imidazolidin-1-yl-sulfonyl, Pyrimidin-1-yl-sulfonyl, Chinolin-1-ylsulfonyl, Morpholinosulfonyl oder Thiomorpholinosulfonyl, vor allem Thiomorpholinosulfonyl oder Morpholinosulfonyl. Die Verbindungen der Formel I', worin $R_1$ Heterocyclylsulfonyl bedeutet und die übrigen Reste die genannten Bedeutungen haben, können bei der Definition der Verbindungen der Formel I' vorzugsweise wegfallen, oder sie sind besonders bevorzugt.

N-(Heterocylylniederalkyl)-N-niederalkyl-aminocarbonyl $R_1$ enthält als Heterocyclyl vorzugsweise einen der unter Heterocyclylcarbonyl $R_1$ genannten Heterocyclen, insbesondere Pyridyl, wie 2-, 3- oder 4-Pyridyl, Pyrazinyl, Pyrimidinyl, Morpholinyl, wie Morpholino, Thiomorpholinyl, wie Thiomorpholino, oder Chinolyl, wie 2- oder 3-Chinolyl, und ist insbesondere N-(Heterocyclylmethyl)-N-methyl-aminocarbonyl, z.B. N-(Pyridylmethyl)-N-methyl-aminocarbonyl, wie N-(2-Pyridylmethyl)-N-methylaminocarbonyl. Die Verbindungen der Formel I', worin $R_1$ N-(Heterocylylniederalkyl)-N-niederalkyl-aminocarbonyl bedeutet und die übrigen Reste die genannten Bedeutungen haben, können bei der Definition der Verbindungen der Formel I' vorzugsweise wegfallen, oder sie sind besonders bevorzugt.

Ein bivalentes Radikal $B_1$ einer $\alpha$-Aminosäure, welches N-terminal mit $R_1$ und C-terminal mit der Aminogruppe am $R_2$-$CH_2$- tragenden Kohlenstoffatom verbunden ist, ist vorzugsweise ausgewählt aus Glycin (H-Gly-OH), Alanin (H-Ala-OH), Valin (H-Val-OH), Norvalin ($\alpha$-Aminovaleriansäure), Leucin, (H-Leu-OH), Isoleucin (H-Ile-OH), Norleucin ($\alpha$-Aminohexansäure, H-Nle-OH), Serin (H-Ser-OH), Homoserin ($\alpha$-Amino-$\gamma$-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), p-Fluorphenylalanin (H-(p-F-Phe)-OH), Tyrosin (H-Tyr-OH), p-Niederalkoxy-phenylalanin, worin Niederalkoxy unverzweigt oder verzweigt sein kann, wie p-Methoxy-phenylalanin (H-(p-$CH_3O$-Phe)-OH), oder ferner 4-Isobutyloxyphenylalanin (H-(4-Isobutyloxy-Phe)--OH) oder (insbesondere) 4-(n-Butyloxy)-phenylalanin (H-(4-n-Butyloxy-Phe)-OH), p-Phenylniederalkoxy-phenylalanin, wie p-Benzyloxyphenylalanin (H-(p-BzlOPhe)-OH), 4-Aminophenylalanin, 4-Chlorphenylalanin, 4-Carboxyphenylalanin, $\beta$-Phenylserin ($\beta$-Hydroxyphenylalanin), Phenylglycin, $\alpha$-Naphthylalanin (H-Nal-OH), Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Aminomalonsäure, Aminomalonsäure-monoamid, Asparaginsäure (H-Asp-OH), Asparagin (H-Asn-OH), Glutaminsäure (H-Glu-OH), Glutamin (H-Gln-OH), Histidin (H-His-OH), Arginin (H-Arg-OH), Lysin (H-Lys-OH), $\delta$-Hydroxylysin, Ornithin ($\alpha$,$\delta$-Diaminovaleriansäure), $\alpha$,$\gamma$-Diaminobuttersäure und $\alpha$,$\beta$-Diaminopropionsäure, oder alternativ und ergänzend hierzu 4-Cyano-phenylalanin (H-(p-CN-Phe)-OH), besonders bevorzugt der Rest einer hydrophoben Aminosäure, z.B. Prolin, Phenylalanin, p-Fluorphenylalanin, p-Methoxy-phenylalanin, p-Benzyloxy-phenylalanin, Tyrosin, Phenylglycin, $\alpha$-Naphthylalanin, Cyclohexylalanin, Cyclohexylglycin, oder ferner 4-Isobutyloxy- oder insbesondere 4-n-Butyloxy-phenylalanin, oder eine aliphatische $\alpha$-Aminosäure ausgewählt aus Glycin, Valin, Norvalin, Alanin, Leucin, Norleucin und Isoleucin, insbesondere Valin, wobei jede der genannten $\alpha$-Aminosäuren in der D-, L- oder (D,L)-, vorzugsweise in der L-Form vorliegt, und insbesondere mit Resten $R_1$ ausgewählt aus Niederalkoxycarbonyl, z. B. tert-Butoxycarbonyl, oder Heterocyclylcarbonyl, z. B. Morpholinocarbonyl, verknüpft ist.

Wenn $B_1$ eine Bindung bedeutet, ist $R_1$ direkt mit dem Aminostickstoff verbunden, den das den Rest $R_2$-$CH_2$- tragende Kohlenstoffatom in Formel I' bindet.

Phenyl oder Cyclohexyl $R_2$ oder $R_3$ ist unsubstituiert oder durch bis zu drei unabhängig aus Hydroxy, Niederalkoxy, worin Niederalkoxy unverzweigt oder verzweigt sein kann, wie Methoxy oder Ethoxy oder (ferner oder insbesondere) Isobutyloxy oder n-Butyloxy (bevorzugt), Phenylniederalkoxy, wie Benzyloxy, Halo, z. B. Fluor, Haloniederalkyl, z. B. Trifluormethyl, Sulfo, Niederalkylsulfonyl, z. B. Methyl- oder Ethylsulfonyl, Cyano und Nitro ausgewählte Reste substituiert, vorzugsweise durch ein bis zwei dieser Reste, besonders bevorzugt ausgewählt aus Hydroxy, Methoxy, Benzyloxy, Fluor, Trifluormethyl, Sulfo, Niederalkylsulfonyl, z. B. Methyl- oder Ethylsulfonyl, und Cyano; für Phenyl ganz besonders bevorzugt aus Fluor und Cyano, für Cyclohexyl ganz besonders bevorzugt aus Fluor, Trifluormethyl, Sulfo oder Niederalkylsulfonyl, in erster Linie Fluor; wobei die genannten Substituenten in 2-, 3- oder 4- Stellung des Phenyl- oder Cyclohexylringes, insbesondere in 4-Stellung, gebunden sind, wie in Phenyl, Cyclohexyl, 2-, 3- oder 4-Fluor- oder 2-, 3- oder 4-Cyanophenyl oder 4-Fluorcyclohexyl, insbesondere in Phenyl, Cyclohexyl, 4-Cyano-phenyl oder 4-Fluorphenyl.

Besonders bevorzugt ist $R_2$ ausgewählt aus Phenyl, 4-Hydroxyphenyl, 4-Methoxyphenyl, 4-Benzyloxyphenyl, 4-Fluorphenyl, Cyclohexyl und 4-Trifluormethylphenyl, während $R_3$ ausgewählt ist aus Phenyl, 4-Hydroxyphenyl, 4-Methoxyphenyl, Cyclohexyl, 2-, 3- oder 4-Fluorphenyl, 4-Trifluormethylphenyl und 2-, 3- oder 4-Cyanophenyl.

In erster Linie ist $R_2$ ausgewählt aus Phenyl, 4-Fluorphenyl und Cyclohexyl, während $R_3$ ausgewählt ist aus Phenyl, Cyclohexyl, 4-Fluorphenyl und 4-Cyanophenyl.

In allererster Linie bevorzugt sind die Kombinationen: $R_2$ Phenyl und $R_3$ Phenyl; $R_2$ Cyclohexyl und $R_3$ 4-Cyanophenyl; $R_2$ Cyclohexyl und $R_3$ 4-Fluorphenyl; und $R_2$ und $R_3$ jeweils Cyclohexyl. Alternativ oder ergänzend hierzu sind auch die Kombinationen $R_2$ Phenyl und $R_3$ 4-Fluorphenyl; $R_2$ Phenyl und $R_3$ 4-Cyanophenyl; $R_2$ 4-Fluorphenyl und $R_3$ 4-Fluorphenyl; $R_2$ 4-Fluorphenyl und $R_3$ 4-Trifluormethylphenyl; $R_2$ 4-Trifluormethylphenyl und $R_3$ Phenyl; $R_2$ 4-Trifluormethylphenyl und $R_3$ 4-Fluorphenyl; $R_2$ 4-Trifluormethylphenyl und $R_3$ 4-Trifluormethylphenyl; $R_2$ Hydroxyphenyl und $R_3$ Phenyl; $R_2$ Phenyl und $R_3$ Hydroxyphenyl; $R_2$ Phenyl und $R_3$ p-Trifluormethylphenyl; $R_2$ Cyclohexyl und $R_3$ p-Methoxyphenyl; $R_2$ Cyclohexyl und $R_3$ p-Trifluormethylphenyl; $R_2$ Phenyl und $R_3$ p-Benzyloxyphenyl; $R_2$ p-Methoxyphenyl und $R_3$ p-Benzyloxyphenyl; $R_2$ p-Benzyloxyphenyl und $R_3$ Phenyl; $R_2$ p-Benzyloxyphenyl und $R_3$ p-Benzyloxyphenyl; $R_2$ Phenyl und $R_3$ o-Fluorphenyl; $R_2$ Phenyl und $R_3$ m-Fluorphenyl; $R_2$ Phenyl und $R_3$ p-Methoxyphenyl; $R_2$ p-Methoxyphenyl und $R_3$ p-Hydroxyphenyl; $R_2$ p-Methoxyphenyl und $R_3$ Phenyl; $R_2$ Phenyl und $R_3$ o-Methoxyphenyl; $R_2$ Phenyl und $R_3$ m-Methoxyphenyl; $R_2$ p-Methoxyphenyl und $R_3$ p-Methoxyphenyl; $R_2$ p-Methoxyphenyl und R3 m-Methoxyphenyl; $R_2$ p-Methoxyphenyl und $R_3$ o-Methoxyphenyl; $R_2$ Phenyl und $R_3$ m-Cyanophenyl; $R_2$ Phenyl und $R_3$ o-Cyanophenyl; oder $R_2$ 4-Hydroxyphenyl und $R_3$ 4-Hydroxyphenyl in allererster Linie bevorzugt; und (ferner oder insbesondere) $R_2$ = Phenyl und $R_3$ = 2,4-Difluorphenyl; $R_2$ = Phenyl und $R_3$ = 4-Isobutyloxyphenyl; $R_2$ = Cyclohexyl und $R_3$ = 4-Benzzyloxyphenyl; $R_2$ = Cyclohexyl und $R_3$ = 4-Hydroxyphenyl; $R_2$ = Phenyl und $R_3$ = 3,4-Dimethoxyphenyl; $R_2$ = Phenyl und $R_3$ = 3,4,5-Trimethoxyphenyl; oder $R_2$ = Phenyl und $R_3$ = 2,3,4-Trimethoxyphenyl.

Stark bevorzugt sind die Verbindungen, worin einer der Reste $R_2$ und $R_3$ p-Methoxyphenyl bedeutet und der andere eine der genannten Bedeutungen, insbesondere p-Methoxyphenyl, p-Fluorphenyl, Phenyl oder p-Trifluormethylphenyl hat.

Ein bivalentes Radikal einer $\alpha$-Aminosäure $A_1$, welches N-terminal mit der Gruppe -C=O und C-terminal mit $A_2$ verbunden ist, ist beispielsweise eine der oben für $B_1$ genannten $\alpha$-Aminosäuren, wobei diese Aminosäuren in der (D)-, (L)- oder (D,L)-Form, vorzugsweise der (D)- oder (L)-, insbesondere der (L)-Form, vorliegen können. Bevorzugt sind die unter $B_1$ genannten hydrophoben $\alpha$-Aminosäuren, insbesondere die dort genannten aliphatischen hydrophoben $\alpha$-Aminosäuren, z. B. Glycin, Valin oder Isoleucin, oder ferner oder insbesondere Leucin oder Phenylglycin. In den genannten $\alpha$-Aminosäuren ist die an $A_2$ bindende Carboxygruppe nicht reduziert oder ferner reduziert, insbesondere zu einer Methylengruppe, z. B. in den genannten hydrophoben $\alpha$-Aminosäuren, wie in den reduzierten Aminosäureradikalen Gly(red), Val(red) oder Ile(red), insbesondere in Val(red), wobei der Zusatz (red) die Reduktion der Carbonylgruppe des entsprechenden Aminosäureradikals zur Methylengruppe anzeigt.

Bedeutet $A_1$ eine Bindung, so ist $A_2$ direkt mit der Carbonylgruppe am Kohlenstoffatom, der den Rest $R_3$-$CH_2$- trägt, verbunden.

Ein bivalentes Radikal einer $\alpha$-Aminosäure $A_2$, welches N-terminal mit $A_1$ und C-terminal mit der Gruppe $NR_4R_5$ verbunden ist, ist beispielsweise eine der oben für $B_1$ genannten $\alpha$-Aminosäuren, wobei diese Aminosäuren in der (D)-, (L)- oder (D,L)-Form, vorzugsweise der (D)- oder (L)-, insbesondere der (L)-Form, vorliegen können. Bevorzugt sind die unter $B_1$ genannten hydrophoben $\alpha$-Aminosäuren, z. B. Glycin, Alanin, Valin, Leucin, Isoleucin, Phenylalanin, p-Fluorphenylalanin, Tyrosin, p-Methoxy-phenylalanin, p-Benzyloxyphenylalanin, Phenylglycin, $\alpha$-Naphthylalanin, Cyclohexylalanin oder Cyclohexylglycin, vorzugsweise Glycin, Alanin, Valin, Leucin, Phenylalanin, p-Fluorphenylalanin, p-Methoxy-phenylalanin, p-Benzyloxyphenylalanin oder Cyclohexylalanin, oder ferner 4-Isobutyloxyphenylalanin oder (insbesondere) 4-n-Butyloxyphenylalanin, wobei die genannten Reste in der (D)- oder (L)-Form, vorzugsweise, ausser Phenylalanin, das in der (L)- oder der (D)-Form vorliegt, in der (L)-Form vorliegen.

Ein aus $A_1$ und $A_2$ gebildetes bivalentes Radikal eines Dipeptides, dessen zentrale Peptidbindung reduziert ist und das N-terminal mit der Gruppe -C=O und C-terminal mit der Gruppe $NR_4R_5$ verbunden ist, besteht vorzugsweise aus 2 der oben genannten hydrophoben $\alpha$-Aminosäuren, insbesondere aus einem N-terminalen Aminosäureradikal ausgewählt aus Gly(red), Val(red) und Ile(red) und einer C-terminalen Aminosäure ausgewählt aus Glycin, Phenylalanin, Tyrosin, p-Methoxyphenylalanin, p-Benzyloxyphenylalanin, Cyclohexylalanin und p-Fluorphenylalanin, ferner aus 4-Isobutyloxyphenylalanin und (insbesondere) 4-n-Butyloxyphenylalanin.

Besonders bevorzugt bilden $A_1$ und $A_2$ zusammen ein bivalentes Radikal eines Dipeptides der Formel Val-Phe, Ile-Phe, Val-Cha, Ile-Cha, Val-Gly, Val-(p-F-Phe), Val-(p-$CH_3O$-Phe), Gly-(p-F-Phe); und alternativ oder zusätzlich das Radikal eines Dipeptides der Formel Val-Tyr, Ile-Tyr, Gly-Tyr, Ile-Gly, Val-(p-BzlOPhe); Val-Ile; Val-Ala, Val-Leu oder Val-Val, oder (ferner oder insbesondere) Phenylglycyl-(p-$CH_3O$-Phe), Val-(4-isobutyloxy-Phe) oder -Val-(4-n-butyloxy-Phe); worin die Aminosäuren in der (D)- oder (L)-, insbesondere der (L)-Form vorliegen mit Ausnahme von (L)-Val-Phe, in dem Phe in der (L)- oder (D)-Form vorliegt, oder eines Derivates hiervon mit reduzierter zentraler Amidbindung, z. B. mit der Formel Val(red)-Phe, das N-terminal mit der Gruppe -C=O und C-terminal mit der Gruppe $NR_4R_5$ verbunden ist.

Eine bevorzugte Ausführungsform der Erfindung bezieht sich entweder auf Verbindungen der Formel I', in denen $B_1$ eines der genannten bivalenten Radikale einer $\alpha$-Aminosäure bedeutet und einer der Reste $A_1$ oder $A_2$ eine Bindung bedeutet und der andere eine der genannten $\alpha$-Aminosäuren bedeutet, oder auf Verbindungen der Formel I', worin $B_1$ eine Bindung bedeutet und $A_1$ und $A_2$ jeweils eines der genannten bivalenten Radikale einer $\alpha$-Aminosäure oder gemeinsam eines der genannten bivalenten Radikale eines Dipeptides mit reduzierter zentraler Amidbindung bedeuten.

Aus $R_4$ und $R_5$ gemeinsam mit dem bindenden Stickstoffatom gebildetes Thiomorpholino oder Morpholino ist unsubstituiert oder substituiert an einem oder mehreren der Kohlenstoffatome, bevorzugt einem oder zwei Kohlenstoffatomen, z.B. einem Kohlenstoffatom, durch Niederalkyl, wie Ethyl, Propyl, Butyl, iso-Butyl, tert-Butyl oder insbesondere Methyl, durch Phenyl- oder Naphthylniederalkyl, wie Benzyl, 1- oder 2-Naphthylmethyl oder Phenyl-1- oder Phenyl-2-ethyl, insbesondere Phenyl-1- oder Phenyl-2-ethyl, durch Hydroxy, durch Niederalkoxy, wie Methoxy, Ethoxy oder tert-Butoxy, durch Amino, durch Niederalkylamino, wie Methyl- oder Ethylamino, oder durch Diniederalkylamino, wie Dimethylamino oder Diethylamino, durch Niederalkanoyl, wie Acetyl oder Propionyl, durch Phenyl- oder Naphthyl-niederalkanoyl, wie Phenylacetyl oder 1-oder 2-Naphthylacetyl, durch Carboxy, durch Niederalkoxycarbonyl, wie iso-Propoxycarbonyl oder tert-Butoxycarbonyl, durch Phenyl-, Naphthyl- oder Fluorenylniederalkoxycarbonyl, wie Benzyloxycarbonyl, 1- oder 2-Naphthylmethoxycarbonyl oder 9-Fluorenylmethoxycarbonyl, durch Carbamoyl, durch Mono- oder Diniederalkylcarbamoyl, wie Dimethylcarbamoyl, durch Mono- oder Di-hydroxyniederalkyl-carbamoyl, wie Dihydroxymethyl-carbamoyl, durch Sulfo, durch Niederalkylsulfonyl, wie Methylsulfonyl oder Ethylsulfonyl, durch Phenyl- oder Naphthylsulfonyl, wobei Phenyl durch Niederalkyl, z. B. Methyl oder Ethyl, substituiert sein kann, z. B. Phenylsulfonyl oder Toluolsulfonyl, durch Sulfamoyl, durch Halogen, z. B. Fluor oder Chlor, durch Cyano, durch Nitro und/oder durch Oxo.

Sehr bevorzugt bilden $R_4$ und $R_5$ zusammen mit dem bindenden Stickstoffatom unsubstituiertes Thiomorpholino oder Morpholino, vor allem unsubstituiertes Morpholino.

Salze von Verbindungen der Formel I', worin salzbildende Gruppen vorliegen, d.h. eine oder mehrere salzbildende Gruppen, sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, bei dosisgerechter Anwendung nichttoxischen Salze von Verbindungen der Formel I'.

Solche Salze werden beispielsweise von Verbindungen der Formel I' mit einer oder mehreren sauren Gruppen, z. B. einer Carboxygruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumverbindungen gebildet werden, z. B. mit N-Methyl-N-ethylamin, Diethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tert-butylamin oder Tris(hydroxymethyl)-methylamin, N,N-Diniederalkyl-N-(hydroxyniederalkyl)-aminen, wie N,N-Dimethyl-N-(2-hydroxyethyl)-amin oder Tris-(2-hydroxyethyl)-amin, N-Methyl-D-glucamin oder quaternären Ammoniumsalzen, wie Tetrabutylammoniumsalzen. Die Verbindungen der Formel I' mit einer oder mehreren basischen Gruppen, z.B. einer Amino- oder Iminogruppe, können Säureadditionssalze bilden, beispielsweise mit anorganischen Säuren, z. B. Halogenwasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie, z. B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z. B. Glutaminsäure oder Asparaginsäure, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I' mit sauren und basischen Gruppen können auch innere Salze bilden.

Zur Isolierung oder Reinigung können auch pharmazeutisch ungeeignete Salze Verwendung finden.

Die Verbindungen der Formel I' haben wertvolle pharmakologische Eigenschaften. Insbesondere sind sie gegen retrovirale Erkrankungen wirksam, insbesondere gegen AIDS, welches durch HIV, vor allem HIV-1 oder HIV-2, verursacht wird. So dienen sie als metabolische Vorstufen für Verbindungen der Formel I,

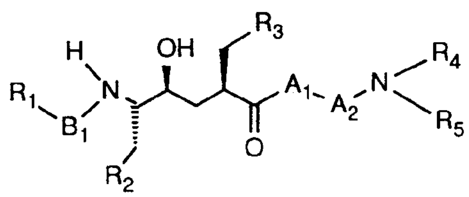

(I)

worin anstelle des Restes T in Verbindungen der Formel I' ein Wasserstoffatom steht, während die übrigen Definitionen die für Verbindungen der Formel I' genannten Bedeutungen haben; welche antiretroviral wirksam sind, insbesondere zur Behandlung von AIDS als Hemmstoffe der Aspartatproteasen von HIV-1 und/oder HIV-2 (und gegebenenfalls weiteren Retroviren, die AIDS-analoge Symptome hervorrufen) geeignet sind. Daher sind die Verbindungen der Formel I' therapeutisch gegen retrovirale Erkrankungen, insbesondere AIDS, einsetzbar.

Verbindungen der Formel I sind in der europäischen Patentanmeldung mit der Publikationsnummer EP 0 532 466 (publiziert am 17.03.1993) genannt und beschrieben.

Die Verbindungen der Formel I werden dabei im Körper des zu behandelnden Tieres, vorzugsweise eines Warmblüters einschliesslich eines Menschen, aus den Verbindungen der Formel I' freigesetzt.

Mit Hilfe der Verbindungen der Formel I' ist es beispielsweise möglich, insbesondere auch bei enteraler, vorzugsweise oraler, Verabreichung der Verbindungen eine andere, vorteilhafte Pharmakokinetik zu erzielen im Vergleich zur bei Verwendung der Verbindungen der Formel I selbst erzielten.

Die pharmakodynamischen Eigenschaften der Verbindungen der Formel I' können beispielsweise wie folgt belegt werden:

Die zu untersuchenden Verbindungen der Formel I' oder (beispielsweise als Kontrolle) die entsprechende Verbindung der Formel I werden in einem organischen Lösungsmittel, wie Dimethylsulfoxid (DMSO), in einer Konzentration von 240 mg/ml gelöst. Die resultierenden Lösungen werden mit 20% (w/v) Hydroxypropyl-β-cyclodextrin (HPβCD) verdünnt, um eine Konzentration der Testsubstanz von 12 mg/ml zu erhalten. Diese Lösung wird Mäusen oral durch künstliche Sonderernährung in einer Dosis von 120 mg/kg verabreicht. 30, 60, 90 und 120 min nach der Verabreichung werden die Tiere getötet und Blut entnommen. Pro Zeitpunkt werden drei bis vier Tiere untersucht. Das Blut wird heparinisiert und für die Analyse durch eine der folgenden beiden Methoden vorbereitet: Nach der ersten Methode wird Vollblut deproteinisiert durch Mischen eines Volumenteils Blut mit einem Volumenteil Acetonitril; nach Zentrifugation (10 000 g, 5 min) wird der Überstand durch Reversed-Phase-HPLC untersucht. Nach der zweiten Methode werden 2 µl einer 1 mM Lösung eines internen Standards (wie einer anderen Verbindung der Formel I) zu 0,5 ml heparinisiertem Blut zugegeben. Das Blut wird zentrifugiert (10 000 g, 5 min), und das Plasma mit demselben Volumen Acetonitril vermischt. Das präzipitierte Protein wird durch Zentrifugation (10 000 g, 5 min) entfernt und der Überstand im Vakuum eingedampft. Der Rückstand wird in 0,1 ml Phthalat-Puffer (0,05 M, pH 3) und 20 µl 3M NaCl aufgenommen. Die Mischung wird mit 1 ml und dann erneut mit 0,2 ml Diisopropylether (=DIPE; Merck, Darmstadt) extrahiert. Die DIPE-Fraktionen werden vereint und unter Vakuum eingedampft. Der Rückstand wird in 50% Wasser (Baker) und 50% Acetonitril (v/v) vor der Analyse durch Reversed-Phase-HPLC aufgelöst.

Die Analyse mittels Reversed-Phase-HPLC wird mit einer 125 x 4,6 mm Nucleosil® $C_{18}$-Säule (Reversed-Phase-Material der Firma Macherey-Nagel, Düren, Bundesrep. Deutschland auf der Basis von mit Kohlenwasserstoffresten von 18 Kohlenstoffatomen derivatisiertem Kieselgel) durchgeführt, welche äquilibriert ist mit einer mobilen Phase von Acetonitril in Wasser/0,1% Trifluoressigsäure. Der Volumenanteil des Acetonitrils liegt je nach Verbindung in zweckmässiger und sinnvoller Weise beispielsweise zwischen 35 und 60 Volumenprozent. Die Flussrate ist 1 ml/min. Detektion erfolgt bei 215 nm. Standards für die Verbindungen in Blut werden analog den Blutproben aufgearbeitet und zur Erstellung von Standardkurven verwendet, aufgrund derer die in-vivo-Konzentrationen ermittelt werden.

Für die Verbindungen der Formel I' oder der Formel I liegt die Konzentration der Komponente der Formel I im Blut von Mäusen nach oraler Gabe nach 60 min vorzugsweise bei bis zu $10^{-4}$ M, vorzugsweise zwischen $5 \times 10^{-8}$ M und $5 \times 10^{-5}$M.

Die Verbindungen der Formel I zeigen Hemmwirkung auf retrovirale Aspartatproteasen, insbesondere HIV-Protease-hemmende Wirkungen. In erster Linie hemmen sie in den nachfolgend beschriebenen Tests in Konzentrationen von $10^{-6}$ bis $10^{-9}$ M, z.B. mit einer $IC_{50}$ von 10 bis 1000 nM, insbesondere von 10 bis 100 nM,

die Wirkung der HIV-Protease von HIV-1. Daher sind sie und damit auch die Prodrugs der Formel I' geeignete Mittel gegen durch dieses oder verwandte Retroviren verursachte Krankheiten (retrovirale Erkrankungen), wie gegen AIDS oder auch dessen Vorstufen.

Die Fähigkeit der Verbindungen der Formel I, die proteolytische Aktivität von z. B. HIV-1-Protease zu inhibieren, lässt sich beispielsweise gemäss dem von A. D. Richards et al., J. Biol. Chem. 265(14), 7733-7736 (1990), beschriebenen Verfahren demonstrieren. Hierbei wird als Substrat für eine rekombinante HIV-1-Protease (Herstellung gemäss Billich, S., et al., J. Biol. Chem. 263(34), 17905 - 17908 (1990)) ein synthetisches chromophores Peptid (z. B. HKARVL[$NO_2$]FEANleS (Bachem, Schweiz; vgl. M. W. Pennington et al., Peptides 1990, ed.: E. Girault and D. Andrew (1991), ESCOM Sci. Publ. B.V., S. 787-789) oder ein Icosapeptid wie RRSNQVSQNYPIVQNIQGRR (hergestellt durch Peptidsynthese nach bekannten Verfahren: J. Schneider et al., Cell 54, 363-368 (1988)) eingesetzt, das einer der Spaltstellen des gag-Vorläuferproteins entspricht. Dieses Substrat und Spaltprodukte davon können durch Hochdruckflüssig-Chromatographie (HPLC) gemessen werden.

Hierzu wird ein zu testender Hemmstoff der Formel I in Dimethylsulfoxid gelöst; der Enzymtest wird durchgeführt, indem geeignete Verdünnungen des Hemmstoffes in 20 mM β-Morpholinoethansulfonsäure (MES)-Puffer pH 6,0 zum Assay-Mix aus 67,2 μM des oben genannten chromophoren Peptides in 0,3 M Natriumacetat, 0,1 M NaCl pH 7,4; oder 122 μM des oben genannten Icosapeptids in 20 mM MES-Puffer pH 6,0 zugegeben werden. Die Grösse der Ansätze beträgt 100 μl. Die Reaktion wird gestartet durch Zugabe von im ersten Fall 2 μl, im zweiten Fall 10 μl HIV-I-Protease und im ersten Fall nach 15 min durch Zugabe von 100 μl 0,3 M $HClO_4$, im zweiten Fall nach einer Stunde Inkubation bei 37 °C durch Zugabe von 10 μl 0,3 M $HCLO_4$ gestoppt. Die Reaktionsprodukte werden nach Abzentrifugieren der Probe für 5 min bei 10 000 x g in 100 μl (Ansatz mit chromophorem Peptid) bzw. 20 μl (Icosapeptid-Ansatz) des erhaltenen Überstandes und nach Auftragen auf eine 125 x 4,6 mm Nucleosil® C18-5μ-HPLC-Säule (Macherey & Nagel, Düren) und Elution quantifiziert anhand der Peak-Höhe des Spaltproduktes bei 280 (Ansatz mit chromophorem Peptid) oder bei 215 nm (Ansatz mit Icosapeptid), Gradient: 100 % E1.1 -> 50 % E1.1 /50 % E1.2 (E1.1: 75 % Acetonitril, 90 % $H_2O$, 0,1 % Trifluoressigsäure (TFA); E1.2: 75 % Acetonitril, 25 % $H_2O$, 0,08 % TFA) innerhalb von 15 min; Durchflussrate 1 ml/min (El. = Eluens).

Hierbei werden für Verbindungen der Formel I beispielsweise $IC_{50}$-Werte ($IC_{50}$ = Konzentration, welche die Aktivität der HIV-1-Protease gegenüber einer Kontrolle ohne Hemmstoff um 50 % senkt) von etwa 50 x $10^{-6}$ bis $10^{-9}$ M, insbesondere von $10^{-7}$ bis $10^{-9}$ M, ermittelt.

In einem weiteren Test kann gezeigt werden, dass die Verbindungen der vorliegenden Erfindung Zellen, die normalerweise von HIV infiziert werden, vor einer solchen Infektion schützen oder zumindest eine solche Infektion verlangsamen. Dabei wird die menschliche T-Zell-Leukämie Zellinie MT-2 (Science 229, 563 (1985)), die empfindlich gegen den zytopathogenen Effekt von HIV ist, mit HIV-1 allein oder mit HIV-1 in Gegenwart einer der erfindungsgemässen Verbindungen inkubiert und nach einigen Tagen die Lebensfähigkeit der so behandelten Zellen beurteilt. Hierzu werden die MT-2-Zellen in RPMI 1640-Medium (Gibco, Schweiz; RPMI 1640 enthält ein Aminosäurengemisch ohne L-Gln), das mit 10% hitzeinaktiviertem fetalem Kälberserum, L-Glutamin, Hepes (2-[4-(2-Hydroxyethyl)-1-piperazino]-ethansulfonsäure) und Standardantibiotika supplementiert ist, bei 37 °C in befeuchteter Luft mit 5% $CO_2$ gehalten. 50 μl der jeweiligen Testverbindung in Kulturmedium und 100 μl HIV-1 in Kulturmedium (800 TCID50/ml) (TCID50 = Tissue Culture Infectious Dose 50 = Dosis, die 50% der MT-2-Zellen infiziert) werden zu $4x10^3$ exponentiell wachsenden MT-2-Zellen in 50 μl Kulturmedium pro Vertiefung auf 96-Loch-Mikrotiterplatten gegeben. Parallele Ansätze auf einer weiteren Mikrotiterplatte mit Zellen und Testverbindung erhalten 100 μl Kulturmedium ohne Virus. Nach 4 Tagen Inkubation wird in 10 μl Zellüberstand die Reverse-Transkriptase (RT)-Aktivität ermittelt. Die RT-Aktivität wird bestimmt in 50 mM Tris (α,α,α-Tris(hydroxymethyl)-methylamin, Ultra pur, Merck, Bundesrepublik Deutschland) pH 7,8; 75 mM KCl, 2 mM Dithiothreitol, 5 mM $MgCl_2$; 0,05% Nonidet P-40 (Detergens; Sigma, Schweiz); 50 μg/ml Polyadenylic Acid (Pharmacia, Schweden); 1,6 μg/ml dT(12-18) (Sigma, Schweiz). Die Mischung wird durch einen 0,45 μ Acrodisc-Filter (Gelman Sciences Inc., Ann Arbor) abfiltriert und bei -20 °C aufbewahrt. Zu Aliquoten dieser Lösung werden 0,1% (v/v) [alpha-$^{32}$P]dTTP zum Erzielen einer radioaktiven Endaktivität von 10 μCi/ml zugegeben. 10 μl des Kulturüberstandes werden auf eine neue 96-Loch-Mikrotiterplatte übertragen und hierzu 30 μl des genannten RT-Cocktails gegeben. Nach Mischen wird die Platte für 1,5 bis 3 h bei 37 °C inkubiert. 5 μl dieser Reaktionsmischung werden auf DE81-Papier (Whatman) überführt. Die getrockneten Filter werden 3-mal für 5 min mit 300 mM NaCl/25 mM Tri-Natriumcitrat und 1-mal mit 95% Ethanol gewaschen und erneut luftgetrocknet. Die Auswertung erfolgt in einem Matrix Packard 96well counter (Packard, Zürich, Schweiz). Die ED90-Werte werden errechnet und sind als die niedrigste Konzentration der jeweiligen Testverbindung definiert, welche die RT-Aktivität um 90% im Vergleich zu nicht mit der Testsubstanz behandelten Zellansätzen senkt. Die RT-Aktivität ist dabei ein Mass für die HIV-1-Vermehrung.

Verbindungen der Formel I zeigen hierbei beispielsweise eine ED90 von $10^{-8}$ bis $10^{-5}$ M, insbesondere von

$10^{-6}$ bis $10^{-7}$ M.

Auch die neuen Verbindungen der Formel I der vorliegenden Erfindung zeigen vorteilhafte pharmakologische Eigenschaften, die erwarten lassen, dass sie in vivo die genannten Hemmwirkungen entfalten. So ist beispielsweise der Blutspiegel bei intravenöser oder intraperitonealer Applikation von 20 mg/kg einer dieser Verbindungen an Mäusen 1 h nach der Applikation vorzugsweise etwa gleich oder höher als die ED90 im Zellassay.

Bei peroraler Gabe von 120 mg/kg einer dieser Verbindungen lassen sich 30 min nach der Applikation beispielsweise Konzentrationen im Mäuseblut finden, die über der ED90 im Zellassay liegen, vorzugsweise etwa das zehnfache der ED90 im Zellassay ausmachen.

Beispielsweise lässt sich mit Boc-Phe[C](p-$CH_3O$)Phe-(L)-Val-(L)-Phe-morpholin-4-ylamid (siehe Beispiele) nach 30 min eine Konzentration von 10,75 $\mu$M erzielen bei einer ED90 von 0,1 $\mu$M.

Die Ermittlung des Blutspiegels wird wie oben für die Verbindungen der Formel I an Mäusen vorgenommen.

Bei den im nachfolgenden genannten Definitionen von Verbindungen der Formel I' können in sinnvoller Weise, beispielsweise zur Ersetzung allgemeinerer durch speziellere Definitionen, Definitionen von Resten aus jeweils weiter oben genannten allgemeinen Definitionen eingesetzt werden oder auch einzelne Definitionen weggelassen werden.

Bevorzugt sind die Verbindungen der Formel I', worin T ein Acylradikal der oben genannten Formel Z bedeutet, worin $R^z$
über ein Ringkohlenstoffatom gebundenes Heterocyclyl ausgewählt aus durch bis zu drei unabhängig voneinander aus Niederalkyl, Phenylniederalkyl, Diphenylniederalkyl, Triphenylniederalkyl, wie Triphenylmethyl, Niederalkanoyl, Hydroxy, Niederalkoxy, Phenylniederalkoxy, wie Benzyloxy, Diphenylmethoxy oder Triphenylmethoxy, Hydroxyniederalkyl, wie Hydroxymethyl, Halogen, wie Fluor, Chlor oder Brom, Cyano, Niederalkoxycarbonyl, wie Methoxy- oder tert-Butoxycarbonyl, Phenylniederalkoxycarbonyl, wie Benzyloxycarbonyl und Haloniederalkyl, wie Chlormethyl oder Trifluormethyl, ausgewählte Reste substituiertem oder vorzugsweise unsubstituiertem Pyrrolyl, 2,5-Dihydropyrrolyl, Indolyl, Indolizinyl, Isoindolyl, Pyrrolidinyl, wie Pyrrolidin-3-yl oder insbesondere Pyrrolidin-2-yl (in der (R,S)- oder vorzugsweise der (R)- oder (S)-Konfiguration), Hydroxypyrrolidinyl, wie 3- oder insbesondere 4-Hydroxypyrrolidinyl, Furyl, wie Furan-3-yl oder insbesondere Furan-2-yl, Tetrahydrofuryl, Thienyl, Cyclohepta[b]pyrrolyl, Imidazolyl, wie Imidazolyl-2-yl, Imidazolyl-3-yl oder insbesondere Imidazolyl-5-yl, N-Triphenylniederalkyl-imidazolyl, wie N-Triphenylmethyl-imidazolyl, Pyrazolyl, insbesondere Pyrazol-3-yl, Oxazolyl, Isoxazolyl, wie Isoxazol-3-yl oder -5-yl, Thiazolyl, Isothiazolyl, wie Isothiazol-3-yl oder -5-yl, Triazolyl, wie 1,2,3-Triazoly-4- oder -5-yl oder 1,2,4-Triazol-5-yl, Tetrazolyl, Pyridyl, wie Pyridin-4-yl oder -3-yl oder insbesondere Pyridin-2-yl, Chinolyl, wie Chinolin-2-yl, Isochinolyl, insbesondere Isochinolin-1-yl oder -3-yl, Piperidyl, insbesondere Piperidin-2-yl, $\gamma$-Pyranyl, 4,5-Dihydropyranyl, 4H-Chromenyl, Chromanyl, $\gamma$-Thiopyranyl, Pyridazinyl, Cinnolyl, Phthalazinyl, Chinazolinyl, Chinoxalinyl, Pyrimidinyl, Pyrazinyl, Phenyzinyl, Phenoxazinyl, Phenothiazinyl, Morpholinyl und Thiazinyl, welche über ein Ringkohlenstoffatom gebunden sind; wobei diejenigen der genannten Reste, die ein Ringheteroatom direkt in Nachbarstellung zum bindenden Ringkohlenstoffatom enthalten, ganz besonders bevorzugt sind, vor allem die Reste Furan-2-yl, (S)- oder (R)-Pyrrolidin-2-yl und Imidazol-4-yl, Pyridin-2-yl, -3-yl oder -4-yl, oder Isochinolin-3-yl; oder ferner oder insbesondere Tetrahydropyranyl bedeutet; oder
durch mindestens einen aus Niederalkoxy, wie Methoxy, Ethoxy oder n-Butoxy; durch einen oder zwei Substituenten, insbesondere Aryl, vor allem Phenyl, Napthyl, Niederalkoxy, wie Ethoxy oder Methoxy, Niederalkylthio, wie Methylthio oder Ethylthio, Niederalkoxyniederalkoxy, wie 2-Methoxyethoxy, Niederalkylthioniederalkoxy, wie 2-Methylthioethoxy, Aryloxy oder Arylthio, insbesondere Phenyloxy oder o-, m- oder p-Chlorphenyloxy, z.B. p-Chlorphenyloxy, Amino, N-Niederalkylamino oder N,N-Diniederalkylamino, wie 2-Amino, 2-(N-Niederalkyl)amino oder 2-(N,N-Diniederalkyl)amino, z.B. 2-Dimethylamino, Heterocyclyl, welches wie zuletzt für über ein Ringkohlenstoffatom gebundenes Heterocyclyl $R^z$ definiert ist, insbesondere 2-, 3- oder 4-Pyridyl, substituiertes Niederalkoxy, wie Methoxy, Ethoxy oder n-Butoxy; Aryloxy, insbesondere Phenyloxy; Niederalkylthio, wie Methylthio, Ethylthio oder n-Butylthio; durch einen oder zwei Substituenten, insbesondere Aryl, vor allem Phenyl oder Napthyl, substituiertes Niederalkylthio, wie Methylthio, Ethylthio oder n-Butylthio; Arylthio, wie Phenylthio; durch Amino oder durch einen oder zwei Reste ausgewählt aus Niederalkyl, wie Methyl, Heterocyclylniederalkyl, worin Heterocyclyl wie für über ein Ringkohlenstoffatom gebundenes Heterocyclyl $R^z$ definiert ist, insbesondere Heterocyclylmethyl, wie über einen Ringkohlenstoff gebundenes Imidazolylmethyl, z.B. 4-Imidazolylmethyl, oder Pyridylmethyl, z.B. 2-, 3- oder 4-Pyridylmethyl, Arylniederalkyl, wie Phenyl- oder Napthylniederalkyl, z.B. Phenyl- oder Naphthylmethyl, Niederalkanoyl, wie Acetyl, Niederalkoxycarbonyl, wie tert-Butoxycarbonyl, Arylniederalkoxycarbonyl, wie Phenylniederalkoxycarbonyl, z.B. Benzyloxycarbonyl, substituiertes Amino, wobei in allererster Linie einer der Aminosubstituenten Niederalkyl, insbesondere Methyl ist, und der andere Wasserstoff oder einen der zuvor als Aminosubstituenten genannten

Reste bedeutet; Heterocyclyl, wie oben für über ein Ringkohlenstoffatom gebundenes Heterocyclyl $R^z$ definiert, insbesondere als Pyridin-2-yl, -3-yl oder -4-yl; und Heterocyclylniederalkyl, worin Niederalkyl vorzugsweise Methyl, 1- oder 2-Ethyl oder 3-Propyl bedeutet und worin Heterocyclyl wie oben für über ein Ringkohlenstoffatom gebundenes Heterocyclyl $R^z$ definiert ist, jedoch auch über ein Ringstickstoffatom gebunden sein kann, insbesondere Imidazol-1-yl, Imidazol-2-yl, Imidazol-5-yl oder vor allem Imidazol-4-yl, N-Triphenylniederalkylimidazolyl, wie N-Triphenylmethyl-imidazol-5-yl oder insbesondere -4-yl, oder Pyrazolyl, wie Pyrazol-1-yl, -3-yl, -4-yl oder -5-yl; und (ferner oder vorzugsweise) durch Niederalkoxycarbonyl, wie Methoxycarbonyl, Niederalkanoyloxyniederalkoxycarbonyl, wie Pivaloyloxymethoxycarbonyl oder Acetyloxymethoxycarbonyl, Carboxy, Phenylniederalkoxycarbonylamino-niederalkoxy, wie 2-(Benzyloxycarbonylamino)-ethoxy, Aminoniederalkoxy, wie 2-Aminoethoxy, Diniederalkylaminoniederalkoxy, wie 2-(Dimethylamino)-ethoxy, N,N-Diniederalkylaminoniederalkoxy, wie 2-(Dimethylamino)-ethoxy; Niederalkoxycarbonyl-niederalkylsulfo, wie Methoxycarbonyl-methylsulfo; Niederalkanoyloxy, wie Acetyloxy; und Tetrahydropyranyloxy, wie 4-Tetrahydropyranyloxy,

ausgewählten Rest substituiertes Niederalkyl (insbesondere Methyl, Ethyl, n-Propyl oder n-Butyl), welches einen weiteren Substituenten Aryl, der wie unten definiert ist, tragen kann, bedeutet; oder

Aryl, insbesondere Chlorphenyl, Chlorniederalkylphenyl, wie o-, m- oder p-Chlormethylphenyl, p-Morpholinomethyl-phenyl, p-Thiomorpholinomethyl-phenyl, oder ferner Phenyl bedeutet;

wobei Aryl in den genannten Definitionen in erster Linie Phenyl, Napthtyl, wie 1- oder 2-Napthtyl, oder Fluorenyl, wie 9-Fluorenyl, bedeutet, welches unsubstituiert oder substituiert durch bis zu drei unabhängig voneinander aus Niederalkyl, Niederalkanoyl, Hydroxy, Niederalkoxy, Phenylniederalkoxy, wie Benzyloxy, Diphenylmethoxy oder Triphenylmethoxy, Hydroxyniederalkyl, wie Hydroxymethyl, Halogen, wie Fluor, Chlor oder Brom, Cyano, Niederalkoxycarbonyl, wie Methoxy- oder tert-Butoxycarbonyl, Phenylniederalkoxycarbonyl, wie Benzyloxycarbonyl, Haloniederalkyl, wie Chlormethyl oder Trifluormethyl, Piperidinomethyl, Piperazin-1-ylmethyl, 4-Niederalkyl-piperazin- 1-yl-methyl, wie 4-Methyl- oder 4-Ethylpiperazin-1-ylmethyl, Morpholinomethyl oder Thiomorpholinomethyl, und Nitro ausgewählte Reste, welche unabhängig voneinander vorliegen können, substituiert ist, insbesondere entsprechend substituiertes Phenyl;

bedeutet;

$R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl, oder ferner oder insbesondere γ-Pyranyl oder Furanyl; und einem ganz oder teilweise gesättigten Derivat dieser Reste, oder worin die Bedeutung Heterocyclyloxycarbonyl für $R_1$ fehlt, oder ferner oder insbesondere Ethoxycarbonyl bedeutet; $B_1$ eine Bindung (bevorzugt) oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, bevorzugt den Rest einer hydrophoben Aminosäure, z. B. Prolin, Phenylalanin, p-Fluorphenylalanin, Phenylglycin, α-Naphthylalanin, Cyclohexylalanin, Cyclohexylglycin oder einer aliphatischen α-Aminosäure ausgewählt aus Glycin, Alanin, Valin, Norvalin, Leucin, Norleucin und Isoleucin, insbesondere Valin, wobei vorzugsweise jede der genannten α-Aminosäuren in der D-, L- oder (D,L)-, vorzugsweise in der L-Form vorliegt, bedeutet, wobei vorzugsweise jede der genannten Aminosäuren mit einem der unter $R_1$ genannten Reste ausgewählt aus Wasserstoff, N-tert-Butoxycarbonyl oder Morpholinocarbonyl substituiert ist, $R_2$ und $R_3$ unabhängig voneinander Phenyl oder Cyclohexyl, wobei diese Reste unsubstituiert oder durch ein bis drei, vorzugsweise ein bis zwei unabhängig voneinander aus Hydroxy, Methoxy, Benzyloxy, Fluor, Sulfo, Niederalkylsulfonyl, Trifluormethyl und Cyano, ferner oder insbesondere aus Isobutyloxy und n-Butyloxy, ausgewählte Reste substituiert sind, wie oben in den allgemeinen Definitionen gezeigt, bedeuten, $A_1$ ein bivalentes Radikal einer hydrophoben α-Aminosäure, wie oben unter den allgemeinen Definitionen gezeigt, bedeutet, welches N-terminal mit der Gruppe -C=O und C-terminal mit $A_2$ verbunden ist, $A_2$ ein bivalentes Radikal einer hydrophoben α-Aminosäure, vorzugsweise wie oben unter den allgemeinen Definitionen definiert, bedeutet, welches N-terminal mit $A_1$ verbunden ist und C-terminal mit dem Rest $NR_4R_5$ verbunden ist, wobei die genannten Aminosäurereste in der (D)- oder (L)-Form, vorzugsweise, ausser Phenylalanin, das in der (L)- oder der (D)-Form vorliegt, in der (L)-Form vorliegen, insbesondere $A_1$ und $A_2$ ein bivalentes Radikal eines Dipeptides der Formel Val-Phe, Ile-Phe, Val-Cha, Ile-Cha, Ile-Gly, Val-Val, Val-Gly, Val-(p-F-Phe), Val-Tyr, Val-(p-$CH_3$O-Phe), Val-(p-BzlOPhe), Val-Ile, Val-Ala, Val-Leu oder Gly-(p-F-Phe), oder (ferner oder insbesondere) Phenylglycyl-(p-$CH_3$O-Phe), Val-(4-isobutyloxy-Phe) oder -Val-(4-n-butyloxy-Phe); bilden, worin die Aminosäuren in der (D)- oder (L)-, insbesondere der (L)-Form vorliegen mit Ausnahme von (L)-Val-Phe, in dem Phe in der (L)- oder (D)-Form vorliegt; oder $A_1$ und $A_2$ zusammen ein bivalentes Radikal eines Dipeptides aus zwei, vorzugsweise der oben unter den allgemeinen De-

finitionen genannten, hydrophoben α-Aminosäuren bilden, dessen zentrale Amidbindung reduziert ist und das N-terminal mit der Gruppe -C=O und C-terminal mit der Gruppe $NR_4R_5$ verbunden ist, wie in den allgemeinen Definitionen aufgeführt, z. B. mit der Formel Val(red)-Phe, und $R_4$ und $R_5$ gemeinsam mit dem bindenden Stickstoffatom Thiomorpholino oder Morpholino, insbesondere Morpholino, oder ferner oder insbesondere 2,6-Dimethylmorpholino bedeuten; und alternativ oder ergänzend hierzu die Verbindungen der Formel I, worin $R_1$ Morpholinosulfonyl oder N-(2-Pyridylmethyl)-N-methyl-aminocarbonyl bedeutet und die übrigen Reste die genannten Bedeutungen haben; und die pharmazeutisch verwendbaren Salze dieser Verbindungen, sofern salzbildende Gruppen vorliegen.

Stärker bevorzugt sind die Verbindungen der Formel I', worin T Pyrrolidin-2-ylcarbonyl oder -3-ylcarbonyl, wie (R)- oder (S)-Pyrrolidin-2-ylcarbonyl ((D)- oder (L)-Prolyl), Furan-3- oder insbesondere Furan-2-ylcarbonyl, Pyridyl-4-, Pyridyl-3- oder insbesondere Pyridyl-2-ylcarbonyl, Isochinolin-1- oder insbesondere Isochinolin-3-ylcarbonyl, Pyrazin-2-ylcarbonyl, Niederalkoxyniederalkylcarbonyl, wie Methoxyacetyl, n-Butoxyacetyl oder 3-Methoxypropionyl, Phenyl- oder Naphthylniederalkoxyniederalkylcarbonyl, wie Benzyloxyacetyl, α-Niederalkoxy-α-phenylniederalkylcarbonyl, wie (R)- oder (S)-α-Methoxy-α-phenylacetyl, Niederalkoxyniederalkoxyniederalkylcarbonyl, wie 2-(Methoxyethoxy)acetyl, Niederalkoxyniederalkoxyniederalkoxyniederalkylcarbonyl, wie 2-(2-(Methoxyethoxy)ethoxy)acetyl, o-, m- oder p-Chlorphenyloxy-niederalkoxyniederalkylcarbonyl, N,N-Diniederalkylaminoniederalkoxyniederalkylcarbonyl, wie 2-(N,N-Dimethylamino)ethyloxyacetyl, 2-, 3- oder 4-Pyridylniederalkyloxyniederalkylcarbonyl, wie Pyridin-2-ylmethoxyacetyl, Phenyloxyniederalkylcarbonyl, wie Phenoxyacetyl, Niederalkylthioniederalkylcarbonyl, wie Methylthioacetyl, Phenylniederalkylthioniederalkylcarbonyl, wie Benzylthioacetyl, N,N-Diniederalkyl-aminoniederalkylcarbonyl, wie N,N-Dimethylaminoacetyl, -3-propionyl oder -4-butyryl, N-Niederalkylaminoniederalkylcarbonyl, wie N-Methylaminoacetyl oder -3-propionyl, N-Imidazol(-2-, -4- oder -5-)ylmethyl-N-niederalkylaminoniederalkylcarbonyl, wie N-(Imidazol-4-ylmethyl)-N-methylaminoacetyl, N-Pyridin(-2-, -3- oder -4-)ylmethyl-N-niederalkylaminoniederalkylcarbonyl, wie N-Pyridin-2-ylmethyl-N-methylaminoacetyl, N-Phenylniederalkoxycarbonyl-N-niederalkylaminoniederalkylcarbonyl, wie N-BenzyloxycarbonylN-methylaminoacetyl, Imidazol(-1-, -2-, -4- oder -5-)ylniederalkylcarbonyl, wie 3-(Imidazol-4-yl)propionyl, N-Triphenylniederalkylimidazol(-4- oder -5-)ylniederalkylcarbonyl, wie 3-(N-Triphenylmethylimidazol-4-yl)propionyl, oder Pyrazol(-1-, -3-, -4- oder -5-)ylniederalkylcarbonyl, wie Pyrazol-1-ylacetyl oder ferner Haloniederalkylbenzoyl, wie p-Chlormethylbenzoyl, p-(Morpholino- oder Thiomorpholinomethyl)-benzoyl oder Benzoyl bedeutet, oder (insbesondere) Niederalkanoyloxy-niederalkylcarbonyl, wie Acetyloxyacetyl, Niederalkoxycarbonylniederalkoxy-niederalkylcarbonyl, wie Methoxycarbonylmethoxyacetyl, Niederalkoxycarbonylniederalkylthio-niederalkylcarbonyl, wie Methoxycarbonylmethylthio-acetyl, Niederalkoxycarbonylniederalkylsulfo-niederalkylcarbonyl, wie Methoxycarbonylmethylsulfo-acetyl, Niederalkanoyloxyniederalkoxycarbonylniederalkoxy-niederalkylcarbonyl, wie Pivaloyloxymethoxycarbonyl-methoxyacetyl oder Acetyloxymethoxycarbonylmethoxy-acetyl, Carboxyniederalkoxyniederalkylcarbonyl, wie Carboxymethoxyacetyl, N-Phenylniederalkoxycarbonylamino-niederalkylcarbonyl, wie 3-Benzyloxycarbonylamino-propionyl, Aminoniederalkylcarbonyl, wie 3-Aminopropionyl, Diniederalkylaminoniederalkoxyniederalkoxy-niederalkylcarbonyl, wie [2-(2-Dimethylaminoethoxy)-ethoxy]-acetyl, N-Phenylniederalkoxycarbonylaminoniederalkoxyniederalkoxy-niederalkylcarbonyl, wie 2-(2-N-Benzyloxycarbonylaminoethoxy)ethoxy-acetyl, Aminoniederalkoxyniederalkoxy--niederalkylcarbonyl, wie 2-(2-Aminoethoxy)ethoxy-acetyl, oder Tetrahydropyranyloxy-niederalkylcarbonyl, wie 2-(4-Tetrahydropyranyloxy)-acetyl oder 2(R)-, 2(S)- oder ferner 2(R,S)-(4-Tetrahydropyranyloxy)-propionyl bedeutet; $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl, oder insbesondere Ethoxycarbonyl, 4-Tetrahydropyranyloxycarbonyl oder Tetrahydrofuran-2(R oder S)-yloxycarbonyl bedeutet; oder alternativ und ergänzend hierzu Morpholinosulfonyl, N-(2-Pyridylmethyl)-N-methyl-aminocarbonyl bedeutet; $B_1$ eine Bindung (bevorzugt) oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, wobei im letzteren Falle $R_1$ bevorzugt Wasserstoff, tert-Butoxycarbonyl oder Morpholinocarbonyl ist, oder alternativ oder ergänzend hierzu Morpholinosulfonyl oder N-(2-Pyridylmethyl)-N-methyl-aminocarbonyl ist, $R_2$ und $R_3$ unabhängig voneinander Phenyl oder Cyclohexyl bedeuten, wobei diese Reste unsubstituiert oder durch ein bis drei, vorzugsweise ein bis zwei unabhängig voneinander aus Hydroxy, Methoxy, Benzyloxy, Fluor, Sulfo, Niederalkylsulfonyl, Cyano und Trifluormethyl, und insbesondere aus Isobutyloxy und n-Butyloxy ausgewählte Reste substituiert sind, $A_1$ ein bivalentes Radikal einer der α-Aminosäuren Glycin, Valin, Isoleucin oder insbesondere Leucin oder Phenylglycin bedeutet, welches N-terminal mit der Gruppe -C=O und C-terminal mit $A_2$ verbunden ist, $A_2$ ein bivalentes Radikal einer der α-Aminosäuren Glycin, Alanin, Valin, Leucin, Isoleucin, Phenylalanin, Tyrosin, Cyclohexylalanin, p-Methoxy-phenylalanin, p-Benzyloxyphenylalanin, p-Fluorphenylalanin oder insbesondere p-(Isobutyloxy)- oder p-(n-Butyloxy)phenylalanin bedeutet, welches N-terminal mit $A_1$ und C-terminal mit der Gruppe $NR_4R_5$ verbunden ist, oder ferner $A_1$ und $A_2$ zusammen ein bivalentes Radikal eines Dipeptides mit reduzierter zentraler Peptidbindung bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Cyclohexylalanin, Tyrosin, p-Methoxyphenylalanin oder p-Fluorphenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino, Morpholino oder vorzugsweise 2,6-Dimethylmorpholino bedeutet, insbesondere Morpholino, und die pharmazeutisch verwendbaren Salze dieser Verbindungen, sofern salzbildende Gruppen vorliegen.

Besonders bevorzugt sind die Verbindungen der Formel I', worin T Pyrrolidin-2-ylcarbonyl oder -3-ylcarbonyl, wie (R)- oder (S)-Pyrrolidin-2-ylcarbonyl ((D)- oder (L)-Prolyl), Furan-3- oder insbesondere Furan-2-ylcarbonyl, Pyridyl-4-, Pyridyl-3- oder insbesondere Pyridyl-2-ylcarbonyl, Isochinolin-1- oder insbesondere Isochinolin-3-ylcarbonyl, Pyrazin-2-ylcarbonyl, Niederalkoxyniederalkylcarbonyl, wie Methoxyacetyl, n-Butoxyacetyl oder 3-Methoxypropionyl, Phenyl- oder Naphthylniederalkoxyniederalkylcarbonyl, wie Benzyloxyacetyl, α-Niederalkoxy-α-phenylniederalkylcarbonyl, wie (R)- oder (S)-α-Methoxy-α-phenylacetyl, Niederalkoxyniederalkoxyniederalkylcarbonyl, wie 2-(Methoxyethoxy)acetyl, Niederalkoxyniederalkoxyniederalkoxyniederalkylcarbonyl, wie 2-(2-(Methoxyethoxy)ethoxy)acetyl, o-, m- oder p-Chlorphenyloxy-niederalkoxy-niederalkylcarbonyl, N,N-Diniederalkylaminoniederalkoxyniederalkylcarbonyl, wie 2-(N,N-Dimethylamino)ethyloxyacetyl, 2-, 3- oder 4-Pyridylniederalkyloxyniederalkylcarbonyl, wie Pyridin-2-ylmethoxyacetyl, Phenyloxyniederalkylcarbonyl, wie Phenoxyacetyl, Niederalkylthioniederalkylcarbonyl, wie Methylthioacetyl, Phenylniederalkylthioniederalkylcarbonyl, wie Benzylthioacetyl, N,N-Diniederalkylaminoniederalkylcarbonyl, wie N,N-Dimethylaminoacetyl, -3-propionyl oder -4-butyryl, N-Niederalkylaminoniederalkylcarbonyl, wie N-Methylaminoacetyl oder -3-propionyl, N-Imidazol(-2-, -4- oder -5-)ylmethyl-N-niederalkylaminoniederalkylcarbonyl, wie N-(Imidazol-4-ylmethyl)-N-methylaminoacetyl, N-Pyridin(-2-, -3- oder -4-)ylmethyl-N-niederalkylaminoniederalkylcarbonyl, wie N-Pyridin-2-ylmethyl-N-methylaminoacetyl, N-Phenylniederalkoxycarbonyl-N-niederalkylaminoniederalkylcarbonyl, wie N-Benzyloxycarbonyl-N-methylaminoacetyl, Imidazol(-1-, -2-, -4- oder -5-)ylniederalkylcarbonyl, wie 3-(Imidazol-4-yl)propionyl, N-Triphenylniederalkylimidazol(-4- oder -5-)ylniederalkylcarbonyl, wie 3-(N-Triphenylmethylimidazol-4-yl)propionyl, oder Pyrazol(-1-, -3-, -4- oder -5-)ylniederalkylcarbonyl, wie Pyrazol-1-ylacetyl oder ferner Haloniederalkylbenzoyl, wie p-Chlormethylbenzoyl, p-(Morpholino- oder Thiomorpholinomethyl)benzoyl oder Benzoyl bedeutet, $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl bedeutet; oder alternativ und ergänzend hierzu Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, wobei im letzteren Falle $R_1$ bevorzugt Wasserstoff, tert-Butoxycarbonyl oder Morpholinocarbonyl ist, oder alternativ oder ergänzend hierzu Morpholinosulfonyl oder N-(2-Pyridylmethyl)-N-methyl-aminocarbonyl ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Benzyloxy, Fluor, Sulfo, Niederalkylsulfonyl, Cyano und Trifluormethyl ausgewählte Reste substituiert sind, $A_1$ ein bivalentes Radikal einer der α-Aminosäuren Glycin, Valin oder Isoleucin bedeutet, welches N-terminal mit der Gruppe -C=O und C-terminal mit $A_2$ verbunden ist, $A_2$ ein bivalentes Radikal einer der α-Aminosäuren Glycin, Alanin, Valin, Leucin, Isoleucin, Phenylalanin, Tyrosin, Cyclohexylalanin, p-Methoxy-phenylalanin, p-Benzyloxyphenylalanin oder p-Fluorphenylalanin bedeutet, welches N-terminal mit $A_1$ und C-terminal mit der Gruppe $NR_4R_5$ verbunden ist, oder ferner $A_1$ und $A_2$ zusammen ein bivalentes Radikal eines Dipeptides mit reduzierter zentraler Peptidbindung bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Cyclohexylalanin, Tyrosin, p-Methoxy-phenylalanin oder p-Fluorphenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino oder Morpholino bedeutet, insbesondere Morpholino, und die pharmazeutisch verwendbaren Salze dieser Verbindungen, sofern salzbildende Gruppen vorliegen.

Ganz besonders bevorzugt ist eine Verbindung der Formel I', worin T Pyrrolidin-2-ylcarbonyl oder -3-ylcarbonyl, wie (R)- oder (S)-Pyrrolidin-2-ylcarbonyl ((D)- oder (L)-Prolyl), Furan-3- oder insbesondere Furan-2-ylcarbonyl, Pyridyl-4-, Pyridyl-3- oder insbesondere Pyridyl-2-ylcarbonyl, Isochinolin-1- oder insbesondere Isochinolin-3-ylcarbonyl, Pyrazin-2-ylcarbonyl, Niederalkoxyniederalkylcarbonyl, wie Methoxyacetyl, n-Butoxyacetyl oder 3-Methoxypropionyl, Phenyl- oder Naphthylniederalkoxyniederalkylcarbonyl, wie Benzyloxyacetyl, α-Niederalkoxy-α-phenylniederalkylcarbonyl, wie (R)- oder (S)-α-Methoxy-α-phenylacetyl, Niederalkoxyniederalkoxyniederalkylcarbonyl, wie 2-(Methoxyethoxy)acetyl, Niederalkoxyniederalkoxyniederalkoxyniederalkylcarbonyl, wie 2-(2-(Methoxyethoxy)ethoxy)acetyl, o-, m- oder p-Chlorphenyloxy-niederalkoxy-niederalkylcarbonyl, N,N-Diniederalkylaminoniederalkoxyniederalkylcarbonyl, wie 2-(N,N-Dimethylamino)ethyloxyacetyl, 2-, 3- oder 4-Pyridylniederalkyloxyniederalkylcarbonyl, wie Pyridin-2-ylmethoxyacetyl, Phenyloxyniederalkylcarbonyl, wie

Phenoxyacetyl, Niederalkylthioniederalkylcarbonyl, wie Methylthioacetyl, Phenylniederalkylthioniederalkylcarbonyl, wie Benzylthioacetyl, N,N-Diniederalkyl-aminoniederalkylcarbonyl, wie N,N-Dimethylaminoacetyl, -3-propionyl oder -4-butyryl, N-Niederalkylaminoniederalkylcarbonyl, wie N-Methylaminoacetyl oder -3-propionyl, N-Imidazol(-2-, -4- oder -5-)ylmethyl-N-niederalkylaminoniederalkylcarbonyl, wie N-(Imidazol-4-ylmethyl)-N-methylaminoacetyl, N-Pyridin(-2-, -3- oder -4-)ylmethyl-N-niederalkylaminoniederalkylcarbonyl, wie N-Pyridin-2-ylmethyl-N-methylaminoacetyl, N-Phenylniederalkoxycarbonyl-N-niederalkylaminoniederalkylcarbonyl, wie N-Benzyloxycarbonyl-N-methylaminoacetyl, Imidazol(-1-, -2-, -4- oder -5-)ylniederalkylcarbonyl, wie 3-(Imidazol-4-yl)propionyl, N-Triphenylniederalkylimidazol(-4- oder -5-)ylniederalkylcarbonyl, wie 3-(N-Triphenylmethylimidazol-4-yl)propionyl, oder Pyrazol(-1-, -3-, -4- oder -5-)ylniederalkylcarbonyl, wie Pyrazol-1-ylacetyl oder ferner Haloniederalkylbenzoyl, wie p-Chlormethylbenzoyl, p-(Morpholino- oder Thiomorpholinomethyl)benzoyl oder Benzoyl bedeutet, $R_1$ tert-Butoxycarbonyl bedeutet, $R_2$ Phenyl, Cyclohexyl, p-Hydroxyphenyl, o-, m- oder p-Methoxyphenyl, p-Benzyloxyphenyl, o-, m- oder p-Fluorphenyl, p-Trifluormethylphenyl oder o-, m- oder p-Cyanophenyl bedeutet, insbesondere Phenyl, p-Hydroxyphenyl, p-Methoxyphenyl, p-Benzyloxyphenyl, p-Fluorphenyl oder p-Trifluormethylphenyl, $R_3$ unabhängig von $R_2$ eine der für $R_2$ genannten Bedeutungen hat, $A_1$ ein bivalentes Radikal einer der $\alpha$-Aminosäuren Glycin, (L)-Valin oder (L)-Isoleucin bedeutet, welches N-terminal mit der Gruppe -C=O und C-terminal mit $A_2$ verbunden ist, $A_2$ ein bivalentes Radikal einer der $\alpha$-Aminosäuren Glycin, Alanin, Valin, Leucin, Isoleucin, Phenylalanin, Tyrosin, Cyclohexylalanin, p-Methoxy-phenylalanin, p-Benzyloxyphenylalanin oder p-Fluorphenylalanin bedeutet, welches N-terminal mit $A_1$ und C-terminal mit der Gruppe $NR_4R_5$ verbunden ist, und der Rest $-NR_5R_5$ Morpholino bedeutet, und Salze davon, sofern salzbildende Gruppen vorliegen, oder (ferner oder insbesondere) die Verbindungen der Formel I', oder deren Salze, worin T Niederalkanoyloxy-niederalkylcarbonyl, wie Acetyloxyacetyl, Niederalkoxycarbonylniederalkoxy-niederalkylcarbonyl, wie Methoxycarbonylmethoxy-acetyl, Niederalkoxycarbonylniederalkylthio-niederalkylcarbonyl, wie Methoxycarbonylmethylthio-acetyl, Niederalkoxycarbonylniederalkylsulfo-niederalkylcarbonyl, wie Methoxycarbonylmethylsulfo-acetyl, Niederalkanoyloxyniederalkoxycarbonylniederalkoxy-niederalkylcarbonyl, wie Pivaloyloxymethoxycarbonyl-methoxyacetyl oder Acetyloxymethoxycarbonylmethoxy-acetyl, Carboxyniederalkoxyniederalkylcarbonyl, wie Carboxymethoxyacetyl, N-Phenylniederalkoxycarbonylamino-niederalkylcarbonyl, wie 3-Benzyloxycarbonylamino-propionyl, Aminoniederalkylcarbonyl, wie 3-Aminopropionyl, Diniederalkylaminoniederalkoxyniederalkoxy-niederalkylcarbony 1, wie [2-(2-Dimethylaminoethoxy)-ethoxy]-acetyl, N-Phenylniederalkoxycarbonyl-aminoniederalkoxyniederalkoxy-niederalkylcarbonyl, wie 2-(2-N-Benzyloxycarbonylaminoethoxy)ethoxy-acetyl, Aminoniederalkoxyniederalkoxy-niederalkylcarbonyl, wie 2-(2-Aminoethoxy)ethoxy-acetyl, oder Tetrahydropyranyloxyniederalkylcarbonyl, wie 2-(4-Tetrahydropyranyloxy)-acetyl oder 2(R)-, 2(S)- oder ferner 2(R,S)-(4-Tetrahydropyranyloxy)-propionyl bedeutet und die übrigen Reste die gerade genannten Bedeutungen haben.

Sehr bevorzugt ist eine Verbindung der Formel I', worin T ein Acylradikal der Formel Z, wie oben gezeigt, bedeutet, worin

$R^z$ Hydrocarbyl, in dem mindestens ein Kohlenstoffatom durch ein Heteroatom ersetzt ist mit der Massgabe, dass ein Heteroatom nicht direkt an das Carbonyl gebunden ist, an das der Rest $R^z$ gebunden ist, Alkyl mit 2 oder mehr Kohlenstoffatomen, Niederalkenyl, Niederalkinyl oder Aryl bedeutet, vorzugsweise wie oben definiert, insbesondere worin $R^z$

über ein Ringkohlenstoffatom gebundenes Heterocyclyl ausgewählt aus durch bis zu drei unabhängig voneinander aus Niederalkyl, Phenylniederalkyl, Diphenylniederalkyl, Triphenylniederalkyl, wie Triphenylmethyl, Niederalkanoyl, Hydroxy, Niederalkoxy, Phenylniederalkoxy, wie Benzyloxy, Diphenylmethoxy oder Triphenylmethoxy, Hydroxyniederalkyl, wie Hydroxymethyl, Halogen, wie Fluor, Chlor oder Brom, Cyano, Niederalkoxycarbonyl, wie Methoxy- oder tert-Butoxycarbonyl, Phenylniederalkoxycarbonyl, wie Benzyloxycarbonyl und Haloniederalkyl, wie Chlormethyl oder Trifluormethyl, ausgewählte Reste substituiertem oder vorzugsweise unsubstituiertem Pyrrolyl, 2,5-Dihydropyrrolyl, Indolyl, Indolizinyl, Isoindolyl, Pyrrolidinyl, wie Pyrrolidin-3-yl oder insbesondere Pyrrolidin-2-yl (in der (R,S)- oder vorzugsweise der (R)- oder (S)-Konfiguration), Hydroxypyrrolidinyl, wie 3- oder insbesondere 4-Hydroxypyrrolidinyl, Furyl, wie Furan-3-yl oder insbesondere Furan-2-yl, Tetrahydrofuryl, Thienyl, Cyclohepta[b]pyrrolyl, Imidazolyl, wie Imidazolyl-2-yl, Imidazolyl-3-yl oder insbesondere Imidazolyl-5-yl, N-Triphenylniederalkyl-imidazolyl, wie N-Triphenylmethyl-imidazolyl, Pyrazolyl, insbesondere Pyrazol-3-yl, Oxazolyl, Isoxazolyl, wie Isoxazol-3-yl oder -5-yl, Thiazolyl, Isothiazolyl, wie Isothiazol-3-yl oder -5-yl, Triazolyl, wie 1,2,3-Triazoly-4- oder -5-yl oder 1,2,4-Triazol-5-yl, Tetrazolyl, Pyridyl, wie Pyridin-4-yl oder -3-yl oder insbesondere Pyridin-2-yl, Chinolyl, wie Chinolin-2-yl, Isochinolyl, insbesondere Isochinolin-1-yl oder -3-yl, Piperidyl, insbesondere Piperidin-2-yl, $\gamma$-Pyranyl, 4,5-Dihydropyranyl, 4H-Chromenyl, Chromanyl, $\gamma$-Thiopyranyl, Pyridazinyl, Cinnolyl, Phthalazinyl, Chinazolinyl, Chinoxalinyl, Pyrimidinyl, Pyrazinyl, Phenyzinyl, Phenoxazinyl, Phenothiazinyl, Morpholinyl und Thiazinyl, welche über ein Ringkohlenstoffatom gebunden sind; wobei diejenigen der genannten Reste, die ein Ringheteroatom direkt in Nachbarstellung zum bindenden Ringkohlenstoffatom enthalten, ganz besonders bevorzugt sind, vor allem die Reste Furan-2-yl, (S)- oder (R)-Pyrrolidin-2-yl und Imidazol-4-yl, Pyridin-2-yl, -3-yl oder -4-yl, oder Isochinolin-3-yl; oder ferner oder insbesondere Tetrahydropyranyl bedeutet; oder

durch mindestens einen aus Niederalkoxy, wie Methoxy, Ethoxy oder n-Butoxy, oder durch einen oder zwei Substituenten, insbesondere Aryl, vor allem Phenyl, Napthyl, Niederalkoxy, wie Ethoxy oder Methoxy, Niederalkylthio, wie Methylthio oder Ethylthio, Niederalkoxyniederalkoxy, wie 2-Methoxyethoxy, Niederalkylthioniederalkoxy, wie 2-Methylthioethoxy, Aryloxy oder Arylthio, insbesondere Phenyloxy oder o-, m- oder p-Chlorphenyloxy, z.B. p-Chlorphenyloxy, Amino, N-Niederalkylamino oder N,N-Diniederalkylamino, wie 2-Amino, 2-(N-Niederalkyl)amino oder 2-(N,N-Diniederalkyl)amino, z.B. 2-Dimethylamino, Heterocyclyl, welches wie zuletzt für über ein Ringkohlenstoffatom gebundenes Heterocyclyl $R^z$ definiert ist, insbesondere 2-, 3- oder 4-Pyridyl, substituiertes Niederalkoxy, wie Methoxy, Ethoxy oder n-Butoxy, Aryloxy, insbesondere Phenyloxy, Niederalkylthio, wie Methylthio, Ethylthio oder n-Butylthio, durch einen oder zwei Substituenten, insbesondere Aryl, vor allem Phenyl oder Napthyl; substituiertes Niederalkylthio, wie Methylthio, Ethylthio oder n-Butylthio, und Arylthio, wie Phenylthio; durch Amino oder durch einen oder zwei Reste ausgewählt aus Niederalkyl, wie Methyl, Heterocyclylniederalkyl, worin Heterocyclyl wie für über ein Ringkohlenstoffatom gebundenes Heterocyclyl $R^z$ definiert ist, insbesondere Heterocyclylmethyl, wie über einen Ringkohlenstoff gebundenes Imidazolylmethyl, z.B. 4-Imidazolylmethyl, oder Pyridylmethyl, z.B. 2-, 3- oder 4-Pyridylmethyl, Arylniederalkyl, wie Phenyl- oder Napthylniederalkyl, z.B. Phenyl- oder Naphthylmethyl, Niederalkanoyl, wie Acetyl, Niederalkoxycarbonyl, wie tert-Butoxycarbonyl, Arylniederalkoxycarbonyl, wie Phenylniederalkoxycarbonyl, z.B. Benzyloxycarbonyl, substituiertes Amino, wobei in allererster Linie einer der Aminosubstituenten Niederalkyl, insbesondere Methyl ist, und der andere Wasserstoff oder einen der zuvor als Aminosubstituenten genannten Reste bedeutet; Heterocyclyl, wie oben für über ein Ringkohlenstoffatom gebundenes Heterocyclyl $R^z$ definiert, insbesondere als Pyridin-2-yl, -3-yl oder -4-yl; und Heterocyclylniederalkyl, worin Niederalkyl vorzugsweise Methyl, 1- oder 2-Ethyl oder 3-Propyl bedeutet und worin Heterocyclyl wie oben für über ein Ringkohlenstoffatom gebundenes Heterocyclyl $R^z$ definiert ist, jedoch auch über ein Ringstickstoffatom gebunden sein kann, insbesondere Imidazol-1-yl, Imidazol-2-yl, Imidazol-5-yl oder vor allem Imidazol-4-yl, N-Triphenylniederalkylimidazolyl, wie N-Triphenylmethylimidazol-5-yl oder insbesondere -4-yl, oder Pyrazolyl, wie Pyrazol-1-yl, -3-yl, -4-yl oder -5-yl; oder (ferner oder vorzugsweise) durch Niederalkoxycarbonyl, wie Methoxycarbonyl, Niederalkanoyloxyniederalkoxycarbonyl, wie Pivaloyloxymethoxycarbonyl oder Acetyloxy- methoxycarbonyl, Carboxy, Phenylniederalkoxycarbonylamino-niederalkoxy, wie 2-(Benzyloxycar- bonylamino)-ethoxy, Aminoniederalkoxy, wie 2-Aminoethoxy, Diniederalkylaminoniederalkoxy, wie 2-(Dimethylamino)-ethoxy, oder N,N-Diniederalkylaminoniederalkoxy, wie 2-(Dimethylamino)-ethoxy; Niederalkoxycarbonyl-niederalkylsulfo, wie Methoxycarbonyl-methylsulfo; Niederalkanoyloxy, wie Acetyloxy; und Tetrahydropyranyloxy, wie 4-Tetrahydropyranyloxy

ausgewählten Rest substituiertes Niederalkyl (insbesondere Methyl, Ethyl, n-Propyl oder n-Butyl), welches einen weiteren Substituenten Aryl, der wie unten definiert ist, tragen kann, bedeutet; oder

Aryl, insbesondere Chlorphenyl, Chlorniederalkylphenyl, wie o-, m- oder p-Chlormethylphenyl, p-Morpholinomethyl-phenyl, p-Thiomorpholinomethyl-phenyl, oder ferner Phenyl bedeutet;

wobei Aryl in den genannten Definitionen in erster Linie Phenyl, Napthtyl, wie 1- oder 2-Napthtyl, oder Fluorenyl, wie 9-Fluorenyl, bedeutet, welches unsubstituiert oder substituiert durch bis zu drei unabhängig voneinander aus Niederalkyl, Niederalkanoyl, Hydroxy, Niederalkoxy, Phenylniederalkoxy, wie Benzyloxy, Diphenylmethoxy oder Triphenylmethoxy, Hydroxyniederalkyl, wie Hydroxymethyl, Halogen, wie Fluor, Chlor oder Brom, Cyano, Niederalkoxycarbonyl, wie Methoxy- oder tert-Butoxycarbonyl, Phenylniederalkoxycarbonyl, wie Benzyloxycarbonyl, Haloniederalkyl, wie Chlormethyl oder Trifluormethyl, Piperidinomethyl, Piperazin-1-ylmethyl, 4-Niederalkyl-piperazin-1-yl-methyl, wie 4-Methyl- oder 4-Ethylpiperazin-1-ylmethyl, Morpholinomethyl oder Thiomorpholinomethyl, und Nitro ausgewählte Reste, welche unabhängig voneinander vorliegen können, substituiert ist, insbesondere entsprechend substituiertes Phenyl;

bedeutet;

wobei T in der bevorzugtesten Form als Pyrrolidin-2-ylcarbonyl oder -3-ylcarbonyl, wie (R)- oder (S)-Pyrrolidin-2-ylcarbonyl ((D)- oder (L)-Prolyl), Furan-3- oder insbesondere Furan-2-ylcarbonyl, Pyridyl-4-, Pyridyl-3- oder insbesondere Pyridyl-2-ylcarbonyl, Isochinolin- 1- oder insbesondere Isochinolin-3-ylcarbonyl, Pyrazin-2-ylcarbonyl, Niederalkoxyniederalkylcarbonyl, wie Methoxyacetyl, n-Butoxyacetyl oder 3-Methoxypropionyl, Phenyl- oder Naphthylniederalkoxyniederalkylcarbonyl, wie Benzyloxyacetyl, $\alpha$-Niederalkoxy-$\alpha$-phenylniederalkylcarbonyl, wie (R)- oder (S)-$\alpha$-Methoxy-$\alpha$-phenylacetyl, Niederalkoxyniederalkoxyniederalkylcarbonyl, wie 2-(Methoxyethoxy)acetyl, Niederalkoxyniederalkoxyniederalkoxyniederalkylcarbonyl, wie 2-(2-(Methoxyethoxy)ethoxy)acetyl, o-, m- oder p-Chlorphenyloxy-niederalkoxy-niederalkylcarbonyl, N,N-Diniederalkylaminoniederalkoxyniederalkylcarbonyl, wie 2-(N,N-Dimethylamino)ethyloxyacetyl, 2-, 3- oder 4-Pyridylniederalkyloxyniederalkylcarbonyl, wie Pyridin-2-ylmethoxyacetyl, Phenyloxyniederalkylcarbonyl, wie Phenoxyacetyl, Niederalkylthioniederalkylcarbonyl, wie Methylthioacetyl, Phenylniederalkylthioniederalkylcarbonyl, wie Benzylthioacetyl, N,N-Diniederalkylaminoniederalkylcarbonyl, wie N,N-Dimethylaminoacetyl, -3-propionyl oder -4-butyryl, N-Niederalkylaminoniederalkylcarbonyl, wie N-Methylaminoacetyl oder -3-propionyl, N-Imidazol(-2-, -4- oder -5-)ylmethyl-N-niederalkylaminoniederalkylcarbonyl, wie N-(Imidazol-4-ylmethyl)-N-methylaminoacetyl, N-Pyridin(-2-, -3- oder -4-)ylmethyl-N-niederalkylaminoniederalkylcarbonyl, wie N-Pyridin-2-ylmethyl-N-methylaminoacetyl, N-Phenylniederalkoxycarbonyl-N-niederalkylaminoniederalkylcarbonyl, wie N-Benzyloxycarbonyl-N-methylaminoacetyl, Imidazol(-1-, -2-, -4- oder -5-)ylniederalkylcarbonyl, wie 3-(Imidazol-4-yl) propionyl, oder N-Triphenylniederalkylimidazol(-4- oder -5-)ylniederalkylcarbonyl, wie 3-(N-Triphenylmethyl-imidazol-4-yl)propionyl, oder Pyrazol(-1-, -3-, -4- oder -5-)ylniederalkylcarbonyl, wie Pyrazol-1-ylacetyl, oder ferner als Haloniederalkylbenzoyl, wie p-Chlormethylbenzoyl, p-(Morpholino- oder Thiomorpholinomethyl)benzoyl oder Benzoyl, insbesondere als Methoxyacetyl oder als 2-Pyridylcarbonyl vorliegt; oder (ferner oder insbesondere) Niederalkanoyloxyniederalkylcarbonyl, wie Acetyloxyacetyl, Niederalkoxycarbo- nylniederalkoxyniederalkylcarbonyl, wie Methoxycarbonylmethoxy-acetyl, Niederalkoxy- carbonylniederalkylthio-niederalkylcarbonyl, wie Methoxycarbonylmethylthio-acetyl, Niederalkoxycarbonyl- niederalkylsulfo-niederalkylcarbonyl, wie Methoxycarbonylmethylsulfo-acetyl, Niederalkanoyloxyniederal- koxycarbonylniederalkoxy-niederalkylcarbonyl, wie Pivaloyloxymethoxycarbonyl-methoxyacetyl oder Acetyloxymethoxycar- bonylmethoxy-acetyl, Carboxyniederalkoxyniederalkylcarbonyl, wie Carboxymethoxy- acetyl, N-Phenylniederalkoxycarbonylaminoniederalkylcarbonyl, wie 3-Benzyloxycarbonylamino-propionyl, Aminoniederalkylcarbonyl, wie 3-Aminopropionyl, Diniederalkylaminoniederalkoxyniederalkoxy-niederalkylcarbonyl, wie [2-(2-Dimethylaminoethoxy)-ethoxy]-acetyl, N-Phenylniederalkoxycarbonyl-aminoniederalkoxyniederalkoxy-niederalkylcarbonyl, wie 2-(2-N-Benzyloxycarbonylaminoethoxy)ethoxy-acetyl, Aminoniederalkoxyniederalkoxy-niederalkylcarbonyl, wie 2-(2-Aminoethoxy)ethoxy-acetyl, oder Tetrahydropyranyl-oxy-niederalkylcarbonyl, wie 2-(4-Tetrahydropyranyloxy)-acetyl oder 2(R)-, 2(S)- oder ferner 2(R,S)-(4-Tetrahydropyranyloxy)-propionyl, bedeutet;
und worin
entweder (besonders bevorzugt) $R_1$ tert-Butoxycarbonyl; $B_1$ eine Bindung; $R_2$ Phenyl; $R_3$ Phenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von Phenylalanin (-(L)-Phe-), welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;
oder $R_1$ tert-Butoxycarbonyl; $B_1$ eine Bindung; $R_2$ Phenyl; $R_3$ p-Methoxyphenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von p-Methoxy-phenylalanin (-(L)-(p-$CH_3O$-Phe)-), welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;
oder $R_1$ tert-Butoxycarbonyl; $B_1$ eine Bindung; $R_2$ Phenyl; $R_3$ p-Fluorphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N-terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;
oder $R_1$ tert-Butoxycarbonyl; $B_1$ eine Bindung; $R_2$ Phenyl; $R_3$ p-Fluorphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von p-Methoxy-phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;
oder (besonders bevorzugt) $R_1$ tert-Butoxycarbonyl; $B_1$ eine Bindung; $R_2$ Phenyl; $R_3$ p-Methoxyphenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von Phenylalanin (-(L)-Phe-), welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;
und pharmazeutisch verwendbare Salze davon, sofern salzbildende Gruppen vorliegen.

Ganz besonders stark bevorzugt ist eine Verbindung der Formel I', worin T 4(S)-(2-Furanylcarboxy)-; 4(S)-[4-(Dimethyl-amino)-butyryloxy]; 4(S)-(N-Z-N-Methyl-aminoacetyloxy); 4(5)-(Methylamino-acetyloxy); 4(S)-[N-(Imidazol-4-methyl)-N-methylaminoacetyloxy]; 4(S)-[3-(1-Triphenylmethyl-imidazol-4-yl)-propionyloxy]; 4(S)-[3-(4-Imidazolyl)-propionyloxy]; 4(S)-(Methoxy-acetyloxy); 4(S)-((2-picolinoyl); 4(S)-(Benzyloxy-acetyloxy); 4(S)-[(S)-α-Methoxy-α-phenyl-acetyloxy]; 4(S)-[(R)-α-Methoxy-α-phenyl-acetyloxy]; 4(S)-(1-Pyrazolylacetyloxy); 4(S)-(Isochinolin-3-carbonyloxy); 4(S)-(Pyrazincarbonyloxy); 4(S)-(4-α-Chlormethyl-benzoyloxy); 4(S)-[4-(4-Morpholino)methyl-benzoyloxy]; 4(S)-(Isonicotinoyloxy); 4(S)-(Nicotinoyloxy); 4(S)-(3-Methoxypropanoyloxy); 4(S)-[(4-Chlorphenoxy)methoxyacetyloxy)]; 4(S)-[2-(2-Methoxyethoxy)acetyloxy)]; 4(S)-(Butyloxyacetyloxy); 4(S)-[2-[2-(2-Methoxyethoxy)ethoxy]acetyloxy)]; 4(S)-(Methoxyacetyloxy); 4(S)-(Phenoxyacetyloxy); 4(S)-[(S)-α-Methoxy-α-phenylacetyloxy); 4(S)-[(R)-α-methoxy-α-phenylacetyloxy)]; (N,N-Dimethyl-aminoacetyloxy); 4(S)-[N-(Pyridin-2-methyl)-N-methyl-aminoacetyloxy]; 4(S)-[3-(Dimethyl-amino)-propionyloxy]; 4(S)-[3-(N-Z-N-Methyl-amino)-propionyloxy]; 4(S)-(3-Methyl-amino-propionyloxy); 4(S)-[(Dimethylaminoethoxy)-acetyloxy]; 4(S)-[(2-Pyridylmethoxy)-acetyloxy]; 4(S)-(Methoxy-acetyloxy); 4(S)-(Pyridin-2-carboxyl); 4(S)-(Methylthioacetyloxy); 4(S)-(Benzylthioacetyloxy); 4(S)-((L)-Prolyloxy); 4(S)-((D)-Prolyloxy); 4(S)-((L)-(N-Z-Prolyl)oxy); 4(S)-((D)-(N-Z-Prolyl)oxy); 3-(N-Z-Amino)-propionyloxy; 3-Amino-propionyloxy; (3-Dimethylamino-propoxy)-acetyloxy; 2-(2-Dimethylamino-ethoxy)-ethoxy-acetyloxy; (4-Dimethylamino-butoxy)-acetyloxy; (2-Benzyloxy)acetyloxy; 2-Acetyloxy)acetyloxy; 2-(4-Tetrahydropyranyloxy)acetyloxy; 2(R)-(4-Tetrahydropyranyloxy)]-propionyloxy; 2-(2-Amino-ethoxy)ethoxy-acetyloxy); 2-(2-Benzyloxycarbonylamino-ethoxy)ethoxy-acetyloxy); (Methoxycarbonyl-methoxy)-acetyloxy; (Methoxycarbonyl-methylthio)-acetyloxy; (Methoxycarbonyl-methylsulfo)-acetyloxy; (Pivaloyloxymethoxycarbonylmethoxy)-acetyloxy; (Carboxymethoxy)-acetyloxy; oder (Acetoxy-methoxycarbonylmethoxy)-acetyloxy bedeutet; und

$R_1$ tert-Butoxycarbonyl; $B_1$ eine Bindung; $R_2$ Phenyl; $R_3$ Phenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von Phenylalanin (-(L)-Phe-), welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder die Verbindung der Formel I', worin T die zuletzt genannten Bedeutungen hat, insbesondere eine der genannten Bedeutungen, und

$R_1$ tert-Butoxycarbonyl; $B_1$ eine Bindung; $R_2$ Phenyl; $R_3$ p-Methoxyphenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von Phenylalanin (-(L)-Phe-), welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

jeweils ein pharmazeutisch verwendbares Salz davon, sofern salzbildende Gruppen vorliegen.

Noch stärker bevorzugt ist eine Verbindung der Formel I', worin T Methoxyacetyl oder Pyridin-2-ylcarbonyl bedeutet und

entweder $R_1$ tert-Butoxycarbonyl; $B_1$ eine Bindung; $R_2$ Phenyl; $R_3$ Phenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N-terminal mit $A_1$ und C-terminal mit der Gruppe $-NR_4R_5$ verbunden ist; und $R_4$ und $R_5$ gemeinsam mit dem bindenden Stickstoffatom Morpholino bedeuten (besonders bevorzugt);

oder $R_1$ tert-Butoxycarbonyl; $B_1$ eine Bindung; $R_2$ Phenyl; $R_3$ p-Methoxyphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von p-Methoxy-phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder $R_1$ tert-Butoxycarbonyl; $B_1$ eine Bindung; $R_2$ Phenyl; $R_3$ p-Fluorphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N-terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder $R_1$ tert-Butoxycarbonyl; $B_1$ eine Bindung; $R_2$ Phenyl; $R_3$ p-Fluorphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von p-Methoxy-phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder $R_1$ tert-Butoxycarbonyl; $B_1$ eine Bindung; $R_2$ Phenyl; $R_3$ p-Methoxyphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N-terminal mit $A_1$ und C-terminal mit der Gruppe $-NR_4R_5$ verbunden ist; und $R_4$ und $R_5$ gemeinsam mit dem bindenden Stickstoffatom Morpholino bedeuten (besonders bevorzugt);

und pharmazeutisch verwendbare Salze davon, sofern salzbildende Gruppen vorliegen.

Am stärksten sind alle oder die einzelnen in den Beispielen genannten Verbindungen der Formel I' und deren pharmazeutisch verwendbare Salze, sofern salzbildende Gruppen vorliegen, bevorzugt.

Die Verbindungen der Formel I' und Salze von solchen Verbindungen mit mindestens einer salzbildenden Gruppe werden nach an sich bekannten Verfahren erhalten, z. B. indem man

a) zur Herstellung von Verbindungen der Formel

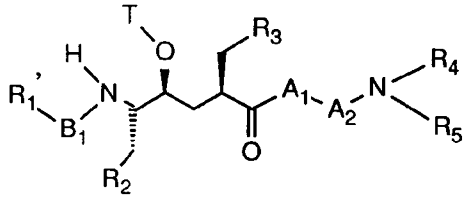

(Ib'),

worin $R_1$' die für Verbindungen der Formel I' genannten Bedeutungen von $R_1$ ausser Wasserstoff hat, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I' genannten Bedeutungen haben, eine Säure der Formel

$R_1$'-OH (II)

oder ein reaktionsfähiges Säurederivat davon, worin $R_1$' ausser Wasserstoff dieselben Bedeutungen hat wie $R_1$ in Verbindungen der Formel I', mit einer Aminoverbindung der Formel

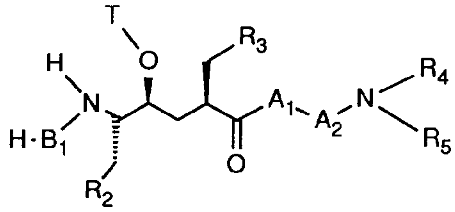

(III')

oder einem reaktionsfähigen Derivat hiervon, worin die Reste die für Verbindungen der Formel I' genannten Bedeutungen haben, kondensiert, wobei in den Ausgangsmaterialien der Formeln II und III' freie funktionelle Gruppen mit Ausnahme der an der Reaktion beteiligten erforderlichenfalls in geschützter Form vorliegen, und gewünschtenfalls vorhandene Schutzgruppen abspaltet, oder

b) zur Herstellung von Verbindungen der Formel

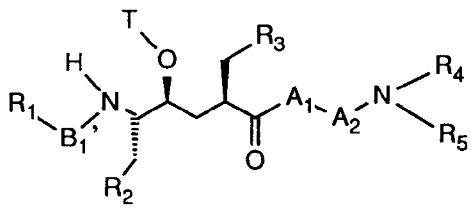

(Ic'),

worin $B_1$' dieselben Reste wie $B_1$ in Verbindungen der Formel I' ausser einer Bindung bedeuten kann, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I' genannten Bedeutungen haben, eine Carbonsäure der Formel

$R_1$-$B_1$'-OH (IV)

oder ein reaktionsfähiges Säurederivat davon, worin $R_1$ die für Verbindungen der Formel I' genannten Bedeutungen hat und $B_1$' die zuletzt genannten Bedeutungen hat, mit einer Aminoverbindung der Formel

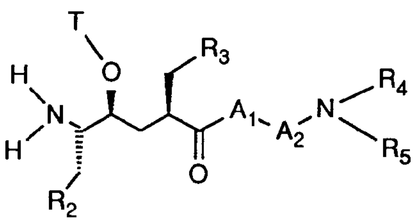

(V'),

oder einem reaktionsfähigen Derivat davon, worin die Reste die für Verbindungen der Formel I' genannten Bedeutungen haben, kondensiert, wobei freie funktionelle Gruppen in den Ausgangsmaterialien der Formeln IV und V' mit Ausnahme der an der Reaktion beteiligten erforderlichenfalls in geschützter Form vorliegen, und gewünschtenfalls vorhandene Schutzgruppen abspaltet, oder

c) eine Carbonsäure der Formel

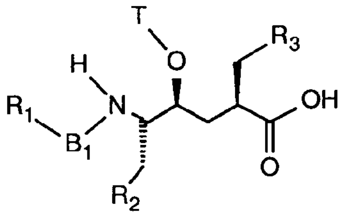

(VI'),

oder ein reaktionsfähiges Derivat davon, worin die Reste die für Verbindungen der Formel I' genannten Bedeutungen haben, mit einer Aminoverbindung der Formel

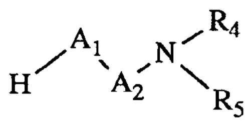

(VII)

oder einem reaktionsfähigen Derivat davon, worin die Reste die für Verbindungen der Formel I' genannten Bedeutungen haben, kondensiert, wobei freie funktionelle Gruppen in den Ausgangsmaterialien der Formeln VI' und VII mit Ausnahme der an der Reaktion beteiligten erforderlichenfalls in geschützter Form vorliegen, und gewünschtenfalls vorhandene Schutzgruppen abspaltet, oder
d) zur Herstellung einer Verbindung der Formel

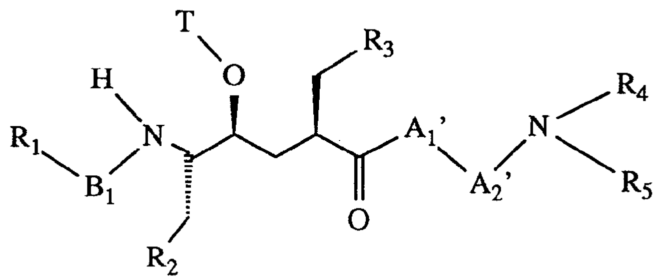

(Id'),

worin $A_1$' und $A_2$' die Bedeutungen von $A_1$ und $A_2$ in Verbindungen der Formel I' haben, wobei $A_1$' jedoch keine Bindung bedeutet und die Peptidbindung zwischen $A_1$' und $A_2$' nicht in reduzierter Form vorliegt, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I' genannten Bedeutungen haben, eine Carbonsäure der Formel

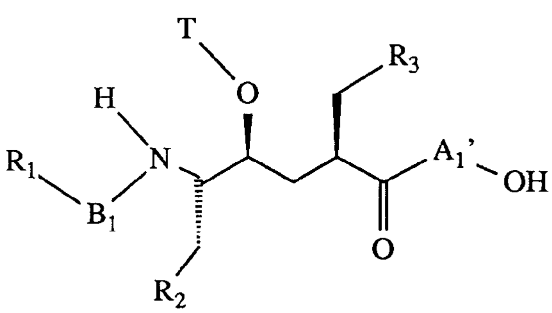

(VIII')

oder ein reaktionsfähiges Derivat davon, worin die Reste die zuletzt genannten Bedeutungen haben, mit einer Aminoverbindung der Formel

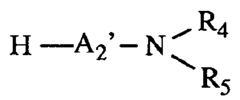

(IX)

oder einem reaktionsfähigen Derivat davon, worin die Reste die zuletzt genannten Bedeutungen haben, kondensiert, wobei freie funktionelle Gruppen in den Ausgangsmaterialien der Formeln VIII' und IX mit Ausnahme der an der Reaktion beteiligten erforderlichenfalls in geschützter Form vorliegen, und gewünschtenfalls vorhandene Schutzgruppen abspaltet, oder

e) eine Carbonsäure der Formel

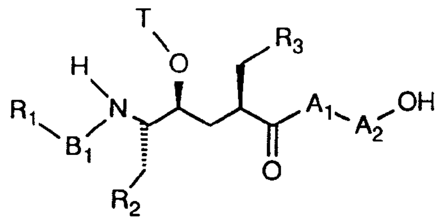

(X')

oder ein reaktionsfähiges Derivat davon, worin die Reste die für Verbindungen der Formel I' genannten Bedeutung haben, mit einer Aminoverbindung der Formel

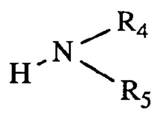

(XI)

oder einem reaktionsfähigen Derivat hiervon, worin die Reste die für Verbindungen der Formel I' genannten Bedeutungen haben, kondensiert, wobei freie funktionelle Gruppen in den Ausgangsmaterialien der Formeln X' und XI mit Ausnahme der an der Reaktion beteiligten erforderlichenfalls in geschützter Form vorliegen, und gewünschtenfalls vorhandene Schutzgruppen abspaltet, oder

f) in einer Verbindung der Formel I', worin die Substituenten die oben genannten Bedeutungen haben mit der Massgabe, dass in der betreffenden Verbindung der Formel I' mindestens eine funktionelle Gruppe durch Schutzgruppen geschützt ist, vorhandene Schutzgruppen abspaltet, oder

g) eine Verbindung der Formel I,

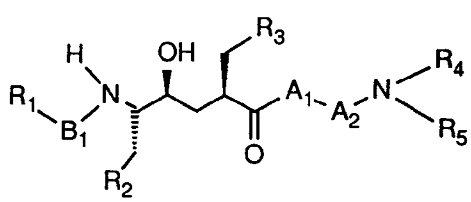

(I)

worin die Reste die für Verbindungen der Formel I' genannten Bedeutungen haben, mit einer Carbonsäure der Formel XXV,

T-OH (XXV)

worin T die bei der Definition von Verbindungen der Formel I' genannten Bedeutungen hat, oder einem reaktionsfähigen Säurederivat davon umsetzt, wobei freie funktionelle Gruppen in den Ausgangsmaterialien der Formeln XXV und I mit Ausnahme der an der Reaktion beteiligten erforderlichenfalls in geschützter Form vorliegen, und gewünschtenfalls vorhandene Schutzgruppen abspaltet, oder

h) zur Herstellung einer Verbindung der Formel I', worin T einen Rest der Formel Z bedeutet, worin $R^z$ ein durch verethertes oder verestertes Hydroxy oder Mercapto, unsubstituiertes oder substituierte Amino oder über Stickstoff gebundenes Heterocyclyl substituiertes Niederalkyl bedeutet und die übrigen Reste die für Verbindungen der Formel I' genannten Bedeutungen haben, eine Verbindung der Formel

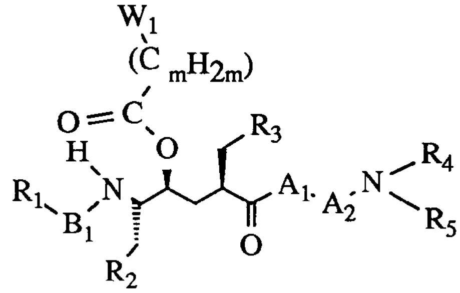

(XXVI)

worin $W_1$ eine Abgangsgruppe bedeutet, $-(C_mH_{2m})-$ einen Niederalkylenrest (m liegt zwischen 1 und 7) bedeutet und die übrigen Reste die für Verbindungen der Formel I' genannten Bedeutungen haben, mit einer Verbindung der Formel

L-H (XXVII),

worin L verethertes oder verestertes Hydroxy oder Mercapto, unsubstituiertes oder substituierte Amino oder über Stickstoff gebundenes Heterocyclyl bedeutet, oder einem reaktionsfähigen Derivat davon umsetzt, wobei freie funktionelle Gruppen in den Ausgangsmaterialien der Formeln XXVI und XXVII mit Ausnahme der an der Reaktion beteiligten erforderlichenfalls in geschützter Form vorliegen, und gewünschtenfalls vorhandene Schutzgruppen abspaltet,
und/oder gewünschtenfalls eine nach einem der vorstehenden Verfahren a) bis h) erhaltene Verbindung der Formel I' mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I' auftrennt und/oder eine erfindungsgemässe Verbindung der Formel I' in eine andere erfindungsgemässe Verbindung der Formel I' umwandelt.
Die oben definierten Verfahren werden nachfolgend näher beschrieben:

Verfahren a) (Herstellung einer Amidbindung)

In Ausgangsmaterialien der Formel II und III' sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Zu den Schutzgruppen für funktionelle Gruppen in Ausgangsmaterialien, deren Umsetzung vermieden werden soll, insbesondere Carboxy-, Amino-, Hydroxy-, Mercapto- und Sulfogruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Schutzgruppen können aber auch in den Endstoffen vorhanden sein. Verbindungen der Formel I' mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkyl-, z. B. Methylgruppen, und eine Amino- oder Carboxygruppe eines zweiten Moleküls der Formel I' substituiert sein. Verbindungen mit solchen Schutzgruppen lassen sich z. B. mit Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl, oder eine in Form eines Lactons, insbesondere eines γ-Lactons, geschützte Carboxygruppe.

Eine geschützte Aminogruppe ist durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl .

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonylprop- 1-en-2-yl, wie 1 -Ethoxycarbonyl-prop- 1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I' substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z. B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl, besonders bevorzugt tert-Butoxycarbonyl oder Benzyloxycarbonyl.

Eine Hydroxygruppe kann beispielsweise durch eine Acylgruppe, z. B. unsubstituiertes oder durch Halogen, wie Chlor, substituiertes Niederalkanoyl, wie Acetyl oder 2,2-Dichloracetyl, oder insbesondere durch einen für geschützte Aminogruppen genannten Acylrest eines Kohlensäurehalbesters geschützt sein. Eine Hydroxygruppe kann auch durch Triniederalkylsilyl, z. B. Trimethylsilyl, Triisopropylsilyl oder tert-Butyldimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl, Benzyl oder Trityl.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines $\gamma$-Lactones, geschützt sein.

Bevorzugt ist eine geschützte Hydroxygruppe durch Triniederalkylsilyl oder als Lacton geschützt, insbesondere durch tert-Butyl-dimethylsilyl oder als $\gamma$-Lacton.

Eine Mercaptogruppe, wie z. B. in Cystein, kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine Sulfogruppe kann beispielsweise durch Niederalkyl, z. B. Methyl oder Ethyl, durch Phenyl oder als Sulfonamid, beispielsweise als Imidazolid, geschützt sein.

Als Schutzgruppe, beispielsweise Carboxyschutzgruppe, im Sinne dieser Anmeldung ist ausdrücklich auch ein in leicht abspaltbarer Weise mit der zu schützenden funktionellen Gruppe, beispielsweise Carboxygruppe, verbundener polymerer Träger zu verstehen, wie er z. B. für die Merrifield-Synthese geeignet ist. Ein solcher geeigneter polymerer Träger ist insbesondere ein durch Copolymerisation mit Divinylbenzol schwach vernetztes Polystyrolharz, das zur reversiblen Bindung geeignete Brückenglieder trägt.

Die Säuren der Formel II sind Carbonsäuren oder Sulfonsäuren.

Die Carbonsäuren der Formel II liegen entweder mit freier Carboxygruppe vor, oder als reaktionsfähiges Derivat hiervon, beispielsweise als von der freien Carboxyverbindung abgeleiteter aktivierter Ester, als reaktionsfähiges Anhydrid, oder ferner als reaktionsfähiges cyclisches Amid. Die reaktionsfähigen Derivate können auch in situ gebildet werden.

Aktivierte Ester von Verbindungen der Formel II mit einer Carboxygruppe sind insbesondere am Verknüpfungskohlenstoffatom des veresternden Restes ungesättigte Ester, z. B. vom Vinylester-Typ, wie Vinylester (erhältlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z. B. durch Behandeln der entsprechenden Säure mit einem geeigneten N,N'-disubstituierten Carbodiimid, z. B. N,N'-Dicyclohexylcarbodiimid; Carbodiimid-Methode), oder N,N-disubstituierte Amidinoester (erhältlich z. B. durch Behandeln der entsprechenden Säure mit einem N,N-disubstituierten Cyananmid; Cyanamid-Methode), geeignete Arylester, insbesondere durch elektronenanziehende Substituenten substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, insbesondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z. B. nach der Anhydrid- oder Carbodiimid-Methode; Methode der aktivierten N-Hydroxyester). Auch innere Ester, z. B. $\gamma$-Lactone, sind einsetzbar.

Anhydride von Säuren können symmetrische oder vorzugsweise gemischte Anhydride dieser Säuren sein, z. B. Anhydride mit anorganischen Säuren, wie Säurehalogenide, insbesondere Säurechloride (erhältlich z. B. durch Behandeln der entsprechenden Säure mit Thionylchlorid, Phosphorpentachlorid oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1 -Niederalkoxycarbonyl-2-niederalkoxy-1,2-dihydrochinolin; Methode der gemischten O-Alkylkohlensäureanhydride), oder Anhydride mit dihalogenierter, insbesondere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-phenylphosphoramidochloridat erhalten kann oder durch Umsetzung von Alkylphosphorsäureamiden in Gegenwart von Sulfonsäureanhydriden und/oder racemisierungssenkenden Additiven, wie N-Hydroxybenztriazol, oder in Gegenwart von Cyanphosphonsäurediethylester) oder mit Phosphorigsäurederivaten, oder Anhydride mit organischen Säuren, wie gemischte Anhydride mit organischen Carbonsäuren (erhältlich z. B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z. B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methan- oder p-Toluolsulfonsäurechlorid; Methode der gemischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride).

Geeignete cyclische Amide sind insbesondere Amide mit fünfgliedrigen Diazacyclen aromatischen Charakters, wie Amide mit Imidazolen, z. B. Imidazol (erhältlich z. B. durch Behandeln der entsprechenden Säure mit N,N'-Carbonyldiimidazol; Imidazol-Methode), oder Pyrazol, z. B. 3,5-Dimethylpyrazol (erhältlich z. B. über das Säurehydrazid durch Behandeln mit Acetylaceton; Pyrazolid-Methode).

Wie erwähnt, können Derivate von Carbonsäuren, die als Acylierungsmittel verwendet werden, auch in situ gebildet werden. So kann man z. B. N,N'-disubstituierte Amidinoester in situ bilden, indem man das Gemisch des Ausgangsmaterials der Formel III' und der als Acylierungsmittel verwendeten Säure der Formel II in Gegenwart eines geeigneten N,N'-disubstituierten Carbodiimids, z. B. N,N'-Cyclohexylcarbodiimid, zur Reaktion bringt, beispielsweise in Gegenwart einer geeigneten Base, wie Triethylamin. Weiter kann man Amino- oder Amidoester der als Acylierungsmittel verwendeten Säuren in Gegenwart des zu acylierenden Ausgangsmaterials der Formel III' bilden, indem man das Gemisch der entsprechenden Säure- und Amino-Ausgangsstoffe in Gegenwart eines N,N'-disubstituierten Carbodiimids, z. B. N,N'-Dicyclohexylcarbodiimid, und eines N-Hydroxyamins oder N-Hydroxy-amids, z. B. N-Hydroxysuccinimid, gegebenenfalls in Anwesenheit einer geeigneten Base, z. B. 4-Dimethylamino-pyridin, umsetzt. Ferner kann man durch Umsetzung mit N,N,N',N'-Tetraalkyluroniumverbindungen, wie O-Benztriazol-1-yl-N,N,N',N'-tetra-methyl-uronium-hexafluorphosphat, Aktivierung in situ erreichen. Schliesslich können Phosphorsäureanhydride der Carbonsäuren der Formel II in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

Die Aminogruppe von Verbindungen der Formel III', die an der Reaktion teilnimmt, trägt vorzugsweise mindestens ein reaktionsfähiges Wasserstoffatom, insbesondere, wenn die damit reagierende Carboxygruppe in reaktionsfähiger Form vorliegt; sie kann aber auch selbst derivatisiert sein, z. B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat einer solchen Verbindung mit einer Aminogruppe ist z. B. auch ein Carbaminsäurehalogenid, wobei die an der Reaktion teilnehmende Aminogruppe durch Halogencarbonyl, z. B. Chlorcarbonyl, substituiert ist.

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure mit dem entsprechenden Amin kann vorzugsweise in Gegenwart eines der üblichen Kondensationsmittel durchgeführt werden. Übliche Kondensationsmittel sind z. B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonylimidazol, 1,2-Oxazoliumverbindungen, z. B. 2-Ethyl-5-phenyl-1,2-oxazolium-3'-sulfonat und 2-tert-Butyl-5-methylisoxazoliumperchlorat, oder eine geeignete Acylaminoverbindung, z. B. 2-Ethoxy-1-ethoxycarbonyl-1,2-dihydrochinolin, N,N,N',N'-Tetraalkyluroniumverbindungen, wie O-Benztriazol-1-yl-N,N,N',N'-tetra-methyluroniumhexafluorphosphat,ferner aktivierte Phosphorsäurederivate, z. B. Diphenylphosphorylazid, Diethylphosphorylcyanid, Phenyl-N-phenylphosphoroamidochloridat, Bis-(2-oxo-3-oxazolidinyl)phosphinsäurechlorid oder 1-Benztriazolyloxy-tris-(dimethylamino)-phosphonium-hexafluorophosphat.

In analoger Weise wie bei den für die Carbonsäuren der Formel II zur Kondensation genannten Reaktionstypen lassen sich auch die Sulfonsäuren der Formel II mit endständiger Sulfonylgruppe bei der Kondensation mit Verbindungen der Formel III' zu den entsprechenden Sulfonamiden der Formel Ib' umsetzen.

So können beispielsweise aktivierte Sulfonsäureester eingesetzt werden, z. B. die entsprechenden, insbesondere durch Nitrogruppen substituierten, Arylester, wie Phenylester, wobei die Aminkomponente der Formel Ib' auch als Alkalimetallamid, z. B. Alkalimetallarylamid, wie Natriumanilinamid, oder als Alkalimetallsalz von stickstoffhaltigen Heterocyclen, z. B. Kalium-pyrrolid, eingesetzt werden kann.

Ferner können reaktionsfähige Anhydride zum Einsatz kommen, wie etwa die entsprechenden symmetrischen (herstellbar z. B. durch Reaktion der alkylsulfonsauren Silbersalze mit Alkylsulfonylchloriden) oder vorzugsweise asymmetrischen Säureanhydride, z. B. Anhydride mit anorganischen Säuren, wie Sulfonylhalogenide, insbesondere Sulfonylchloride (erhältlich z. B. durch Umsetzung der entsprechenden Sulfonsäuren mit anorganischen Säurechloriden, z. B. Thionylchlorid, Sulfurylchlorid oder Phosphorpentachlorid), mit organischen Carbonsäuren (erhältlich z. B. durch Behandeln eines Sulfonsäurehalogenides mit dem Salz einer Carbonsäure, wie einem Alkalimetallsalz, analog der oben genannten Methode zur Herstellung der gemischten Säureanhydride), oder Azide (erhältlich z. B. aus einem entsprechenden Sulfonsäurechlorid und Natriumazid oder über das entsprechende Hydrazid und dessen Behandlung mit salpetriger Säure analog der oben genannten Azidmethode).

Gewünschtenfalls wird eine organische Base zugegeben, z. B. ein Triniederalkylamin mit voluminösen Resten, z. B. Ethyldiisopropylamin, und/oder eine heterocyclische Base, z. B. Pyridin, 4-Dimethylaminopyridin oder bevorzugt N-Methylmorpholin.

Die Kondensation aktivierter Ester, reaktionsfähiger Anhydride oder reaktionsfähiger cyclischer Amide mit den entsprechenden Aminen wird üblicherweise in Gegenwart einer organischen Base, z. B. einfachen Triniederalkylaminen, z. B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z. B. in Gegenwart von anorganischen Carbonaten, z. B. Ammonium- oder Alkalimetallcarbonaten oder -hydrogencarbonaten, wie Natrium- oder Kaliumcarbonat oder -hydrogencarbonat (üblicherweise zusammen mit einem Sulfat), erfolgen, die Reaktion von Sulfonsäurehalogeniden, wie Sulfonsäurechloriden, in Gegenwart von Hydroxiden, z. B. Alkalimetallhydroxiden, wie Natriumhydroxid oder Kaliumhydroxid.

Carbonsäurechloride, beispielsweise die von der Säure der Formel II abgeleiteten Chlorkohlensäurederivate, werden mit den entsprechenden Aminen vorzugsweise in Gegenwart eines organischen Amins, z. B. der vorstehend genannten Triniederalkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z. B. Formamid oder Dimethylformamid, einem halogenierten Kohlenwasserstoff, z. B. Methylenchlorid, Tetrachlorkohlenstoff oder Chlorbenzol, einem Keton, z. B. Aceton, einem cyclischen Ether, z. B. Tetrahydrofuran, einem Ester, z. B. Essigsäureethylester, oder einem Nitril, z. B. Acetonitril, oder in einer Mischung davon, gegebenenfalls bei erniedrigter oder erhöhter Temperatur, z. B. in einem Temperaturbereich von etwa -40 °C bis etwa +100 °C, bevorzugt von etwa -10 °C bis etwa +50 °C, und ohne Inertgas oder unter Inertgas-, z. B. Stickstoff- oder Argonatmosphäre.

Auch wässrige, beispielsweise alkoholische, z. B. Ethanol, oder aromatische Lösungsmittel, z. B. Benzol oder Toluol, sind möglich. Bei Gegenwart von Alkalihydroxiden als Basen kann gegebenenfalls auch Aceton zugesetzt werden.

Die Kondensation kann auch gemäss der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 (1985) beschrieben ist.

Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Verfahren b) (Herstellung einer Amidbindung)

In Ausgangsmaterialien der Formel IV und V' sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III' beschriebenen, wenn man dort anstelle der Carbonsäuren der Formel II diejenigen der Formel IV und anstelle der Aminoverbindungen der Formel III' diejenigen der Formel V' einsetzt.

Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Verfahren c) (Herstellung einer Amidbindung)

In Ausgangsmaterialien der Formel VI' und VII sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und

III' beschriebenen, wenn man dort anstelle der Carbonsäuren der Formel II diejenigen der Formel VI' und anstelle der Aminoverbindungen der Formel III' diejenigen der Formel VII einsetzt.

Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Verfahren d) (Herstellung einer Amidbindung)

In Ausgangsmaterialien der Formel VIII' und IX sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III' beschriebenen, wenn man dort anstelle der Carbonsäuren der Formel II diejenigen der Formel VIII' und anstelle der Aminoverbindungen der Formel III' diejenigen der Formel IX einsetzt.

Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Verfahren e) (Herstellung einer Amidbindung)

In Ausgangsmaterialien der Formel X' und XI sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen, die freien Carbonsäuren und ihre reaktionsfähigen Derivate, die freien Amine und ihre reaktionsfähigen Derivate und die verwendeten Verfahren zur Kondensation sind vollständig analog zu den unter Verfahren a) für die Herstellung einer Amidbindung ausgehend von Verbindungen der Formel II und III' beschriebenen, wenn man dort anstelle der Carbonsäuren der Formel II diejenigen der Formel X' und anstelle der Aminoverbindungen der Formel III' diejenigen der Formel XI einsetzt.

Ein reaktionsfähiges Derivat einer solchen Verbindung der Formel XI mit einer Aminogruppe ist z. B. auch ein Isocyanat, in dem die an der Reaktion teilnehmende Aminogruppe als Isocyanatgruppe abgewandelt ist, wobei im letzteren Falle nur Verbindungen der Formel I' zugänglich sind, die am Stickstoffatom der durch die Reaktion gebildeten Amidgruppe ein Wasserstoffatom tragen.

Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Verfahren f) (Schutzgruppenabspaltung)

Die Abspaltung der Schutzgruppen, die nicht Bestandteil des gewünschten Endproduktes der Formel I' sind, z. B. der Carboxy-, Amino-, Hydroxy-, Mercapto- und/oder Sulfoschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder mittels anderer Reduktionsmittel, sowie Photolyse, gegebenenfalls stufenweise oder gleichzeitig, wobei auch enzymatische Methoden verwendet werden können. Die Abspaltung der Schutzgruppen ist beispielsweise in den weiter vorn im Abschnitt über "Schutzgruppen" genannten Standardwerken beschrieben.

So kann beispielsweise geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff

erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als γ-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, beispielsweise in einem halogenierten Kohlenwasserstoff, wie Methylenchlorid oder Chloroform (insbesondere, wenn gleichzeitig durch Benzyl geschütztes Hydroxy nicht freigesetzt werden soll), gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, z.B. gebunden an ein Trägermaterial, wie Kohle, bevorzugt in polaren Lösungsmitteln, wie Diniederalkylniederalkanoylamiden, z. B. Dimethylformamid, Ethern, wie cyclischen Ethern, z. B. Dioxan, Estern, wie Niederalkansäureniederalkylester, z.B. Essigsäureethylester, oder Alkoholen, wie Methanol, Ethanol oder Propanol, wobei Methanol besonders bevorzugt ist, vorzugsweise etwa bei Raumtemperatur, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig- oder Trifluoressigsäure, gegebenenfalls in Gegenwart von Wasser, Triphenylaminomethyl insbesondere durch Hydrogenolyse mit einem Edelmetall oder Edelmetalloxid als Katalysator, wie Platin, Palladium oder insbesondere Palladiumhydroxid, wobei der Katalysator vorzugsweise an ein Trägermaterial, wie Kohle, Kieselgel oder Aluminiumoxid, gebunden ist, in inerten Lösungsmitteln, wie einem Ether, vorzugsweise einem Niederalkylniederalkanoat, wie Essigsäureethylester, bei Temperaturen von 20 bis 80 °C, insbesondere von 50 bis 70 °C, erforderlichenfalls unter erhöhtem Druck, z.B. zwischen etwa 1 bis 10 bar, gespalten werden, und eine als Silylamino geschützten Aminogruppe kann z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl,

wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl oder tert-Butyldimethylsilyl, mittels Fluoridionen abspalten, vorzugsweise mit einem Fluorid einer organischen quaternären Stickstoffbase, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, oder insbesondere eines Ethers, wie Tetrahydrofuran, bei Temperaturen zwischen 0 und 50 °C, insbesondere etwa bei Raumtemperatur.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thiaaliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Eine durch Benzyloxy geschützte Hydroxygruppe wird beispielsweise mittels Hydrogenolyse freigesetzt, d.h. durch Behandeln mit Wasserstoff in Gegenwart eines geeigneten Hydrierkatalysators, wie eines Palladiumkatalysators, z.B. gebunden an ein Trägermaterial, wie Kohle, bevorzugt in polaren Lösungsmitteln, wie Diniederalkylniederalkanoylamiden, z. B. Dimethylformamid, Ethern, wie cyclischen Ethern, z. B. Dioxan, Estern, wie Niederalkylalkanoaten, z.B. Essigsäureethylester, oder Alkoholen, wie Methanol, Ethanol oder Propanol, wobei Methanol besonders bevorzugt ist, vorzugsweise etwa bei Raumtemperatur. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt. Veresterte Hydroxygruppen, z. B. Niederalkanoyloxy, wie Acetyloxy, können auch durch Esterasen freigesetzt werden, acyliertes Amino beispielsweise durch geeignete Peptidasen.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, beispielsweise Natriumcarbonat, freigesetzt.

Die Temperaturen für die Freisetzung der geschützten funktionellen Gruppen liegen vorzugsweise zwischen -80 und 100 °C, besonders bevorzugt zwischen -20 und 50 °C, beispielsweise zwischen 10 und 35 °C, wie im Bereich der Raumtemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

Verfahren g) (Herstellung eines Carbonsäureesters)

Die Acylierung der Hydroxygruppe erfolgt beispielsweise in an sich bekannter Weise unter Verwendung einer wie oben definierten Säure der Formel XXV, worin T die genannten Bedeutungen ausser unsubstituiertes oder substituiertes Aminocarbonyl hat, oder eines reaktionsfähigen Derivates einer Verbindung der Formel XXV, worin T die für Verbindungen der Formel I genannten Bedeutungen hat. Als reaktionsfähiges Derivat kommt beispielsweise eine Carbonsäure der Formel XXV'

$$T\text{-}Z_1 \qquad (XXV')$$

in Frage, worin T einen der oben bei Verbindungen der Formel I' definierten Reste, vorzugsweise einen der genannten Reste ausser unsubstituiertes oder substituiertes Aminocarbonyl, bedeutet und worin $Z_1$ reaktionsfähig aktiviertes Hydroxy bedeutet (die Verbindung der Formel XXV' enthält somit anstelle einer an die Carbonylgruppe gebundenen Hydroxyfunktion reaktionsfähig aktiviertes Hydroxy, vorzugsweise wie nachfolgend definiert). Die freie Carbonsäure der Formel XXV kann beispielsweise durch starke Säuren, wie Halogenwasserstoff-, Schwefel-, Sulfon-, oder Carbonsäure oder saure Ionenaustauscher, z.B. durch Chlor-, Bromwasserstoff- oder Iodwasserstoffsäure, Schwefelsäure, eine gegebenenfalls, z.B. durch Halogen, substituierte Alkancarbonsäure oder durch eine Säure der Formel XXV, vorzugsweise mit einem Überschuss der Säure der Formel XXV, erforderlichenfalls unter Bindung von entstehendem Reaktionswasser durch wasserbindende Mittel, unter azeotroper Abdestillation des Reaktionswassers oder unter extraktiver Veresterung, durch Säureanhydride, insbesondere anorganische oder vor allem organische Säureanhydride, z.B. Carbonsäureanhydride, wie Niederalkancarbonsäureanhydride (ausser Ameisensäureanhydrid), z.B. Acetanhydrid, oder durch geeignete Aktivierungs- oder Kupplungsreagentien der nachstehend aufgeführten Art, insbesondere auch in situ, aktiviert werden. T-$Z_1$ kann insbesondere auch für ein Carbonsäureazid ($Z_1$ = Azido; erhältlich beispielsweise durch Umsetzung eines entsprechenden Säureesters über das entsprechende Hydrazid und dessen Behandlung mit salpetriger Säure); für ein Carbonsäurehalogenid ($Z_1$ = Halogen, vor allem Chlor oder Brom), insbesondere ein Säurechlorid oder -bromid, welches beispielsweise durch Umsetzung mit organischen Säurehalogeniden, insbesondere mit Oxalyldihalogeniden, wie Oxalyldichlorid, mit anorganischen Säurehalogeniden, z.B. mit Säurehalogeniden des Phosphors oder Schwefels, wie Phosphortrichlorid, Phosphortribromid, Phosphorpentachlorid, Phosphorpentabromid, Phosphoroxidchlorid, Phosphoroxidbromid, Thionylchlorid oder Thionylbromid, oder vor allem unter schonenden Bedingungen mit Tetraniederalkyl-α-halogenoenaminen, z.B. Tetramethyl-α-halogenoenaminen, insbesondere 1-Chlor-N,N,2-trimethyl-1-propenamin (vorzugsweise durch Umsetzung unter Inertgas, wie Stickstoff, in inerten Lösungsmitteln, insbesondere chlorierten Kohlenwasserstoffen, wie Methylenchlorid oder Chloroform, oder Ethern, wie Diethylether, Dioxan oder Tetrahydrofuran, oder Gemischen davon, bei bevorzugten Temperaturen zwischen -78 und 50 °C, insbesondere bei -60 bis 30 °C, z.B. zwischen -10 °C und Raumtemperatur (vgl. Devos, A., et al., J. C. S. Chem. Commun. 1979, 1180-81 und Haveaux, B., et al., Org. Synth. 59, 26 (1980))), wobei das erhaltene Säurehalogenid, z.B. das Säurechlorid der Formel XXV', worin $Z_1$ Chlor bedeutet, auch direkt in situ weiter verwendet werden kann, beispielsweise durch Umsetzung mit der Verbindung der Formel I in Gegenwart von tertiären Stickstoffbasen, wie Pyridin oder 4-Dimethylaminopyridin (DMAP, welches vorzugsweise in katalytischen Mengen zugefügt wird) oder beiden, bei bevorzugten Temperaturen zwischen -20 und 50 °C, insbesondere zwischen 10 und 40 °C) erhalten werden kann; für einen aktivierten Ester, wobei $Z_1$ den Rest eines Alkoholes mit elektronenziehenden Substituenten, insbesondere Cyanmethoxy oder Aryloxy, worin Aryl vorzugsweise Phenyl oder Naphthyl ist, welches durch Halogen, Nitro und/oder Cyano ein- oder mehrfach substituiert ist, z.B. Nitrophenoxy, wie 4-Nitrophenoxy oder 2,4-Dinitrophenyloxy, oder Polyhalogenphenoxy, wie Pentachlorphenoxy, bedeutet; oder für ein symmetrisches oder vorzugsweise asymmetrisches Säureanhydrid stehen, welches beispielsweise durch Einwirkung eines Salzes, z.B. eines Alkalimetallsalzes einer Säure der Formel XXV oder ihres Reaktionspartners, vorzugsweise einer Niederalkancarbonsäure, wie Essigsäure, wie des Natrium- oder Kaliumsalzes, auf ein jeweils komplementäres Säurehalogenid, insbesondere im Falle der Reaktion mit einem Salz einer Carbonsäure der Formel XXV ein Carbonsäurehalogenid, z.B. -Chlorid, wie Acetylchlorid, und im Falle der Reaktion eines Carbonsäurehalogenides der Formel XXV', worin $Z_1$ Halogen, z.B. Chlor oder Brom, bedeutet, mit einem Salz einer Niederalkancarbonsäure, insbesondere Natrium- oder Kaliumacetat, erhalten werden kann. Als Aktivierungs- und Kupplungsreagentien zur Aktivierung von Carbonsäuren der Formel XXV in situ können auch Carbodiimide, z.B. N,N'-Di-$C_1$-$C_4$-alkyl- oder N,N'-Di-$C_5$-$C_7$-cycloalkyl-carbodiimid, wie Diisopropylcarbodiimid oder N,N'-Dicyclohexyl-carbodiimid, vorteilhaft unter Zusatz eines Aktivierungskatalysators, wie N-Hydroxysuccinimid oder gegebenenfalls, z.B. durch Halogen, $C_1$-$C_7$-Alkyl oder $C_1$-$C_7$-Alkoxy, substituiertes N-Hydroxy-benzotriazol oder N-Hydroxy-5-norbornen-2,3-dicarboxamid, $C_1$-$C_4$-Alkylhalogenformiat, z.B. Isobutylchlorformiat, geeignete Carbonylverbindungen, z.B. N,N-Carbonyldiimidazol, geeignete 1,2-Oxazoliumverbindungen, z.B. 2-Ethyl-5-phenyl-1,2-oxazolium-3'-sulfonat oder 2-tert-Butyl-5-methyl-isoxazolium-perchlorat, geeignete Acylaminoverbindungen, z.B. 2-Ethoxy-1-ethoxycarbonyl-1,2-dihydrochinolin, oder geeignete Phosphorylcyanamide

bzw. -azide, z.B. Diethylphosphorylcyanamid oder Diphenylphosphorylazid, ferner Triphenylphosphin-disulfid oder 1-$C_1$-$C_4$-Alkyl-2-halogeno-pyridinium-halogenide, z.B. 1-Methyl-2-chlorpyridinium-iodid, eingesetzt werden.

Falls in der Verbindung der Formel XXV zwei freie Carboxygruppen vorliegen, kann als aktiviertes Säurederivat auch ein inneres Anhydrid vorliegen.

$Z_1$ bedeutet bevorzugt Halogen, wie Chlor oder Brom, sowie Acyloxy, z.B. Niederalkanoyloxy, wie Acetyloxy.

Die Umsetzung mit einem Säurehalogenid, wie -chlorid, der Formel XXV' ($Z_1$=Cl) erfolgt insbesondere in einem Ether, wie Dioxan, Tetrahydrofuran, oder einem Nitril, wie Acetonitril, oder Gemischen davon, in An- oder Abwesenheit von Pyridin und in Ab- oder vorzugsweise Anwesenheit von tertiären Stickstoffbasen, wie 4-Dimethylaminopyridin, Ethyldiisoporopylamin, Triethylamin oder Gemischen von zwei oder mehr dieser Basen, mit oder ohne Schutzgas, wie Argon, bei Temperaturen zwischen 0 °C und 80 °C oder der Rückflusstemperatur, z.B. zwischen Raumtemperatur und 50 °C oder Rückflusstemperatur, falls diese kleiner als 50 °C ist.

Für den Spezialfall, dass $R^z$ in Formel I' unsubstituiertes oder substituiertes Amino bedeutet, eignen sich zur Einführung des entsprechenden Restes T (unsubstituiertes oder substituiertes Aminocarbonyl) bei der Reaktion mit Verbindungen der Formel I insbesondere die Verbindungen der Formel XXV', worin $Z_1$ Halogen, wie Chlor, bedeutet und worin T unsubstituiertes oder substituiertes Aminocarbonyl bedeutet, welche z.B. hergestellt werden können durch Umsetzung der komplementären Amine, beispielsweise unsubstituierter oder substituierter Alkylamine, Arylniederalkylamine oder Arylamine, wie bei der Definition von unsubstituiertem oder substituiertem Amino $R^z$ definiert, mit Phosgen oder ferner Analogen davon, welche statt Chlor andere Halogenatome, insbesondere Brom, enthalten, vorzugsweise in Gegenwart von tertiären Stickstoffbasen, wie Pyridin oder Triethylamin, und in inerten Lösungsmitteln, z.B. chlorierten Kohlenwasserstoffen, wie Methylenchlorid oder Chloroform, Ethern, wie Diethylether, Tetrahydrofuran oder Dioxan, oder Carbonsäureamiden, wie Dimethylformamid. Auch entsprechende N-Carbonylazolide der Formel XXV' ($Z_1$ = N-haltiger Heterocyclus, wie 1-Imidazolido) sind geeignet, welche beispielsweise durch Umsetzung mit den entsprechenden N,N'-Carbonyldiazoliden, wie N,N'-Carbonyldiimidazol, erhalten werden unter Bedingungen, wie gerade für Phosgen und Analoge mit anderen Halogenatomen beschrieben. Die Reaktion der Verbindungen der Formel I mit der entsprechenden Verbindungen der Formel XXV' findet dann ebenfalls unter diesen Bedingungen statt (vgl. Staab, H. A., Angew. Chemie 74, 407 (1962)).

Für den Spezialfall der Einführung von Aminocarbonyl T oder einer N-monosubstituierten Aminocarbonylgruppe T eignet sich als aktiviertes Säurederivat insbesondere das entsprechende Isocyanat der Formel XXV",

$$Q\text{-}N{=}C{=}O \qquad (XXV'')$$

worin Q eine Aminoschutzgruppe, z.B. Trihalogenacetyl, wie Trifluor- oder Trichloracetyl, oder einen der oben bei der Definition von unsubstituiertem oder substituiertem Amino $R^z$, worin die Aminogruppe einen Substituenten trägt, genannten unsubstituierten oder substituierten Niederalkylreste oder Arylreste bedeutet, wobei man, wenn Q eine Aminoschutzgruppe bedeutet, nach der Umsetzung mit der Verbindung der Formel I die entsprechende Verbindung der Formel I', worin $R_5$ freies Aminocarbonyloxy bedeutet, durch Abspaltung der Schutzgruppe Q erhalten kann, wie bei der Freisetzung von durch Acyl geschütztem Amino unter Verfahren f) beschrieben, insbesondere durch saure Hydrolyse, oder, wenn Q einen der genannten substituierten oder unsubstituierten Niederalkylreste oder Arylreste bedeutet, eine entsprechende Verbindung der Formel I mit am Stickstoff monosubstituiertem Aminocarbonyl T. Sowohl Aminocarbonyl als auch N-monosubstituiertes Aminocarbonyl T kann in N-disubstituiertes Aminocarbonyl T überführt werden, indem man mit einem weiteren unsubstituierten oder substituierten Niederalkylrest unter Verwendung geeigneter Ausgangsmaterialien und analogen Bedingungen alkyliert, wie unten bei den zusätzlichen Verfahrensmassnahmen beschrieben.

Die genannten Reaktionen können unter an sich bekannten Reaktionsbedingungen, bei üblichen Temperaturen, in An- oder, insbesondere im Falle der Verwendung von Niederalkanoylanhydriden zur Aktivierung der Carbonsäure der Formel XXV, Abwesenheit von inerten Lösungs- oder Verdünnungsmitteln durchgeführt werden, beispielsweise in Säureamiden, z.B. Carbonsäuramiden, wie Dimethylformamid, Dimethylacetamid oder 1,3-Dimethyl-3,4,5,6-tetrahydro-2(1H)-pyrimidinon (DMPU), oder Amiden anorganischer Säuren, wie Hexamethylphosphorsäuretriamid, Ethern, z.B. cyclischen Ethern, wie Tetrahydrofuran oder Dioxan, oder acyclischen Ethern, wie Diethylether oder Ethylenglykoldimethylether, halogenierten Kohlenwasserstoffen, wie Haloniederalkanen, z.B. Methylenchlorid oder Chloroform, Ketonen, wie Aceton, Nitrilen, wie Acetonitril, Säureanhydriden, wie Acetanhydrid, Estern, wie Essigsäureethylester, Bisalkansulfinen, wie Dimethylsulfoxid, Stickstoffheterocyclen, wie Pyridin, oder Gemischen dieser Lösungsmittel, insbesondere in wasserfreien Lösungsmitteln oder Lösungsmittelgemischen, wobei die jeweils geeigneten Lösungsmittel für die oben genannten Reaktionen ausgewählt werden können, soweit sinnvoll und zweckmässig, unter Verwendung von Salzen der eingesetzten Verbindungen, insbesondere von Metallsalzen eingesetzter Cabonsäuren, wie den Alkali- oder Erdalkalimetallsalzen, z.B. Natrium- oder Kaliumsalzen, in Ab- oder Anwesenheit von Katalysatoren, wie

Dimethylaminopyridin, Kondensationsmitteln oder neutralisierenden Agentien, wie tertiären Stickstoffbasen, z.B. Pyridin, Triethylamin, N-Methylmorpholin, Dimethylaminopyridin oder Ethyldiisopropylamin, je nach Art der Reaktion und/oder der Reaktionsteilnehmer unter atmosphärischem Druck oder in einem geschlossenen Gefäss, unter Normaldruck oder ferner unter erhöhtem Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind Reaktionsbedingungen, die jeweils spezifisch genannt sind oder in erster Linie den in den Beispielen genannten analog sind. Der Reaktionsablauf wird zweckmässig mittels üblicher analytischer Methoden, insbesondere mit Dünnschichtchromatographie, verfolgt. Aus diesen Reaktionsbedingungen können die jeweils geeigneten für alle im vorliegenden Text beschriebenen Reaktionen ausgewählt werden, wobei spezifisch genannte Reaktionsbedingungen besonders bevorzugt sind.

Die erfindungsgemässe Umsetzung wird vorzugsweise unter milden Bedingungen, insbesondere bei Temperaturen zwischen -10 °C und 60 °C, z.B. bei 0 °C bis Raumtemperatur oder leicht erhöhten Temperaturen bis etwa 50 °C, z.B. bei etwa 0 °C bis 40 °C, durchgeführt. Sowohl bei der Umsetzung mit einem Carbonsäurehalogenid der Formel XXV', worin $Z_1$ Halogen, wie Chlor oder Brom bedeutet, als auch bei der Umsetzung mit einem Anhydrid, insbesondere einem symmetrischen Anhydrid ($Z_1$ = O-T), verwendet man insbesondere die gegenüber der Verbindung der Formel I etwa äquimolare Menge oder einen Ueberschuss der entsprechenden Verbindung der Formel XXV' (Halogenid bzw. T-O-T), beispielsweise die 0,95- bis 10-fache molare Menge, vorzugsweise die 1,05- bis 5-fache molare Menge.

In Ausgangsmaterialien der Formel I, XXV, XXV' und XXV" sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen sind vollständig analog zu den unter Verfahren a) für die Herstellung einer Amidbindung ausgehend von Verbindungen der Formel II und III' beschriebenen. Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Verfahren h) (Nukleophile Substitution)

In Ausgangsmaterialien der Formel XXVI und XXII sind funktionelle Gruppen mit Ausnahme der Gruppen, die an der Reaktion teilnehmen sollen oder unter den Reaktionsbedingungen nicht reagieren, unabhängig voneinander durch Schutzgruppen geschützt.

Die Schutzgruppen sind vollständig analog zu den unter Verfahren a) für die Herstellung einer Amidbindung ausgehend von Verbindungen der Formel II und III' beschriebenen.

Eine Abgangsgruppe $W_1$ ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Jodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, p-Bromtoluolsulfonsäure oder p-Toluolsulfonsäure verestertes Hydroxy oder mit Stickstoffwasserstoffsäure verestertes Hydroxy.

Die Reaktion zwischen einer Verbindung der Formel XXVI und einer Verbindung der Formel XXVII, worin L verethertes oder verestertes Hydroxy oder Mercapto bedeutet, findet vorzugsweise in Gegenwart einer Base, z.B. einer hydroxidhaltigen Base, wie einem Metallhydroxid, z.B. einem Alkalimetallhydroxid, wie Natrium- oder Kaliumhydroxid, oder insbesondere eines Metallcarbonates oder -hydrogencarbonates, wie Natrium- oder Kaliumcarbonat oder Natrium- oder Kaliumhydrogencarbonat, wobei in diesen Fällen zusätzlich zu den unten genannten Lösungsmitteln auch vorzugsweise Ketone, wie Niederalkanone, oder auch wässrige und protische Lösungsmittel in Frage kommen, oder in erster Linie unter Verwendung eines Metallalkoholates oder -thiolates als aktivierte Verbindung der Formel XXVII oder dessen Herstellung in situ in Gegenwart einer starken Base, z.B. eines Alkalimetallhydrides, wie Natriumhydrid, oder in Gegenwart eines Alkalimetalles, wie Natrium, in Ab- oder Anwesenheit eines geeigneten Lösungsmittels, insbesondere eines aprotischen Lösungsmittels, z.B. DMPU, eines Ethers, wie Diethylether, Dioxan oder Tetrahydrofuran, oder eines Carbonsäureamides, wie Dimethylformamid, bei Temperaturen zwischen 0 °C und Rückflusstemperatur, insbesondere zwischen 20 °C und Rückflusstemperatur, erforderlichenfalls unter Schutzgas, wie Stickstoff oder Argon, statt.

Bei der Umsetzung von Verbindungen der Formel XXVII, worin L unsubstituiertes oder substituiertes Amino oder über Stickstoff gebundenes Heterocyclyl bedeutet, findet die Reaktion vorzugsweise in Abwesenheit einer Base oder in Gegenwart einer sterisch gehinderten Stickstoffbase, insbesondere einer tertiären

Stickstoffbase, wie 4-Dimethylaminopyridin, Pyridin oder Triethylamin, in einem wässrigen oder nichtwässrigen Lösungsmittel, wie wässrigem oder nichtwässrigem Alkohol, z.B. Ethanol oder Methanol, Ester, wie Diethylester, Ether, wie Diethylether, Dioxan oder Tetrahydrofuran, Carbonsäureamiden, wie Dimethylformamid, oder Acetonitril; bei Temperaturen zwischen 0 °C und Rückflusstemperatur, insbesondere zwischen 20 und 100 °C, statt.

Die Substitution kann je nach den Reaktionsbedingungen als nukleophile Substitution erster oder zweiter Ordnung ablaufen.

Die Freisetzung durch Schutzgruppen geschützter funktioneller Gruppen in den erhaltenen Verbindungen der Formel I' mit geschützten Funktionen erfolgt nach einer oder mehreren der unter Verfahren f) genannten Methoden.

Zusätzliche Verfahrensmassnahmen

Bei den zusätzlichen Verfahrensmassnahmen, die gewünschtenfalls durchgeführt werden, können funktionelle Gruppen der Ausgangsverbindungen, die nicht an der Reaktion teilnehmen sollen, ungeschützt oder in geschützter Form, beispielsweise durch eine oder mehrere der oben unter Verfahren a) genannten Schutzgruppen, vorliegen. Die Schutzgruppen können in den Endprodukten erhalten bleiben oder ganz oder teilweise nach einer der unter Verfahren f) genannten Methoden abgespalten werden.

Salze von Verbindungen der Formel I' mit mindestens einer salzbildenden Gruppe können in an sich bekannter Weise hergestellt werden. So kann man Salze von Verbindungen der Formel I' mit sauren Gruppen z. B. durch Behandeln mit Metallverbindungen, wie Alkalimetallsalzen von geeigneten organischen Carbonsäuren, z. B. dem Natriumsalz der 2-Ethyl-hexansäure, mit organischen Alkali- oder Erdalkalimetallverbindungen, wie den entsprechenden Hydroxiden, Carbonaten oder Hydrogencarbonaten, wie Natrium- und Kaliumhydroxid, -carbonat oder -hydrogencarbonat, mit entsprechenden Calciumverbindungen oder mit Ammoniak oder einem geeigneten organischen Amin bilden, wobei man vorzugsweise stöchiometrische Mengen oder nur einen kleinen Überschuss des salzbildenden Mittels verwendet. Säureadditionssalze von Verbindungen der Formel I' erhält man in üblicher Weise, z. B. durch Behandeln mit einer Säure oder einem geeigneten Anionenaustauscherreagenz. Innere Salze von Verbindungen der Formel I', welche saure und basische salzbildende Gruppen enthalten, z. B. eine freie Carboxygruppe und eine freie Aminogruppe, können z.B. durch Neutralisieren von Salzen, wie Säureadditionssalzen, auf den isoelektrischen Punkt, z. B. mit schwachen Basen, oder durch Behandeln mit Ionenaustauschern gebildet werden.

Salze können in üblicher Weise in die freien Verbindungen überführt werden, Metall- und Ammoniumsalze beispielsweise durch Behandeln mit geeigneten Säuren oder sauren Ionenaustauschern, und Säureadditionssalze beispielsweise durch Behandeln mit einem geeigneten basischen Mittel oder basischen Ionenaustauschern.

Stereoisomerengemische von Verbindungen der Formel I', also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z. B. optisch aktiven Säuren oder Basen, durch Chromatographie an mit optisch aktiven Verbindungen belegten Säulenmaterialien oder durch enzymatische Methoden, z. B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I' selbst erfolgen.

In einer erhältlichen Verbindung der Formel I', worin T einen Rest der Formel Z bedeutet, worin $R^z$ durch Phenyl- oder Naphthylmethoxycarbonyl substituiertes Amino oder Imino, z.B. in durch diese Reste N-substituiertem Aminoniederalkyl oder durch Phenyl- oder Naphthylmethoxycarbonyl und Niederalkyl N,N-disubstituiertem Aminoniederalkyl, in N-Phenyl- oder Naphthylmethoxycarbonyl-pyrrolidin-2-yl oder -aminoniederalkoxyniederalkoxyniederalkyl, bedeutet, kann die Amino- oder Iminogruppe im betreffenden Rest T durch Abspaltung von Phenyl- oder Napthylniederalkoxycarbonyl in die entsprechende Amino- oder N-Niederalkylaminoniederalkylcarbonylgruppe überführt werden, beispielsweise durch Hydrogenolyse, d.h. durch Behandeln mit Wasserstoff in Gegenwart eines geeigneten Hydrierkatalysators, wie eines Palladiumkatalysators, z.B. gebunden an ein Trägermaterial, wie Kohle, bevorzugt in polaren Lösungsmitteln, wie Diniederalkylniederalkanoylamiden, z. B. Dimethylformamid, Ethern, wie cyclischen Ethern, z. B. Dioxan, Estern, wie Niederalkansäureniederalkylester, z.B. Essigsäureethylester, oder Alkoholen, wie Methanol oder Ethanol, insbesondere bei Raumtemperatur.

Durch reduktive Aminierung kann durch Heterocyclylniederalkyl, wie über einen Ringkohlenstoff gebundenes Imidazolylmethyl oder Pyridylmethyl, Arylniederalkyl, wie Phenyl- oder Naphthylniederalkyl, oder insbesondere durch Niederalkyl einfach N-substituiertes Aminoniederalkyl $R^z$ als Bestandteil von T in einer Verbindung der Formel I' in durch einen zusätzlichen Rest ausgewählt aus Heterocyclylniederalkyl, wie über einen Ringkohlenstoff gebundenes Imidazolylmethyl oder Pyridylmethyl, oder Arylniederalkyl, wie Phenyl- oder Naphthylniederalkyl, N,N-disubstituiertes Niederalkyl $R^z$ überführt werden. Die Reaktion findet zwischen einer entsprechenden einfach N-substituierten Verbindung der Formel I' und einem entsprechenden Heterocyclyl- oder Arylniederalkylketon oder -niederalkanaldehyd statt unter katalytischer Hydrierung, beispielsweise in Gegenwart eines Edelmetallkatalysators, wie Platin oder insbesondere Palladium, welcher an einen Träger, vorzugsweise Kohle, gebunden ist, oder eines Schwermetallkatalysators, wie Raney-Nickel, bei Normaldrucken oder Drucken von 1 bis 100 bar, vorzugsweise in Gegenwart eines Edelmetallkatalysators etwa unter Normaldruck, in organischen Lösungsmitteln, z.B. Alkoholen, wie Ethanol oder insbesondere Methanol, in Ab- oder vorzugsweise Anwesenheit einer Carbonsäure, wie Niederalkansäure, z.B. Essigsäure, bei bevorzugten Temperaturen von 0 °C bis 50 °C, z.B. bei Raumtemperatur, oder unter Reduktion mittels eines komplexen Borhydrids, wie Natriumcyanoborhydrid, statt.

In einer Verbindung der Formel I', worin T N-Triphenylmethyl-imidazolylniederalkylcarbonyl bedeutet, kann der Triphenylmethylrest abgespalten werden, wie unter Verfahren f) beschrieben. Man erhält die entsprechende Verbindung der Formel I', worin T Imidazolyl(-2-, -4- oder -5-)yl bedeutet.

In einer Verbindung der Formel I', worin einer der Reste $R_2$, $R_3$ oder $A_2$, oder mehrere dieser Reste, durch Benzyloxy substituiert sind, kann der Benzyloxyrest abgespalten werden, wie unter Verfahren f) beschrieben. Man erhält die entsprechenden Verbindungen der Formel I', worin anstelle von Benzyloxy Hydroxy vorliegt.

In einer erhältlichen Verbindung der Formel I' kann man eine Amino- oder Carboxamidgruppe substituieren, eine in freier oder in reaktionsfähiger Form vorliegende Carboxygruppe verestern oder amidieren oder eine veresterte oder amidierte Carboxygruppe in eine freie Carboxygruppe überführen.

Die Substitution einer Carboxamidgruppe oder einer anderen primären oder sekundären Aminogruppe, beispielsweise zur Herstellung der oben als Substituent von aus $R_4$ und $R_5$ zusammen mit dem bindenden Stickstoffatom gebildetem Thiomorpholino oder Morpholino genannten Carbamoylderivate Mono- oder Diniederalkylcarbamoyl, oder Mono- oder Di-hydroxyniederalkylcarbamoyl, oder unter Bildung der oben genannten Derivate des Substituenten Amino an aus $R_4$ und $R_5$ gemeinsam mit dem bindenden Stickstoffatom gebildetem Thiomorpholino oder Morpholino, oder zur Herstellung von N,N-disubstituiertem Amino $R^z$ in Verbindungen der Formel I', in denen der Stickstoff der umzusetzenden Aminogruppen an Wasserstoff gebunden ist, erfolgt z. B. durch Alkylierung.

Geeignete Mittel zur Alkylierung einer Carboxamidgruppe in einer Verbindung der Formel I' sind beispielsweise Diazoverbindungen, z. B. Diazomethan. Man kann Diazomethan in einem inerten Lösungsmittel zersetzen, wobei das gebildete freie Methylen mit der Carboxamidgruppe in der Verbindung der Formel I' reagiert. Die Zersetzung von Diazomethan erfolgt vorzugsweise katalytisch, z. B. in Gegenwart eines Edelmetalls in fein verteilter Form, z. B. Kupfer, oder eines Edelmetallsalzes, z. B. Kupfer(I)-chlorid oder Kupfer(II)-sulfat.

Alkylierungsmittel sind auch in der Deutschen Offenlegungsschrift 2 331 133 genannt, z. B. Alkylhalogenide, Sulfonsäureester, Meerweinsalze oder 1-substituierte 3-Aryltriazene, welche man unter den dort genannten Reaktionsbedingungen mit einer Verbindung der Formel I' mit einer Carboxamidgruppe umsetzen kann.

Weitere Alkylierungsmittel sind ausgewählt aus entsprechenden Alkylverbindungen, die einen Substituenten X tragen, worin X eine Abgangsgruppe ist. Eine Abgangsgruppe ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Iodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure, verestertes Hydroxy oder mit Stickstoffwasserstoffsäure verestertes Hydroxy.

Die Reaktion kann unter den Bedingungen einer nukleophilen Substitution erster oder zweiter Ordnung ablaufen.

Beispielsweise kann man eine der Verbindungen mit einem Substituenten X, worin X für eine Abgangsgruppe mit hoher Polarisierbarkeit der Elektronenhülle, z. B. für Brom oder Iod, steht, in einem polaren aprotischen Lösungsmittel, z. B. Aceton, Acetonitril, Nitromethan, Dimethylsulfoxid oder Dimethylformamid, umsetzen. Die Substitutionsreaktion wird gegebenenfalls bei erniedrigter oder erhöhter Temperatur, z. B. in einem Temperaturbereich von etwa -40 ° bis etwa 100 °C, bevorzugt von etwa -10 ° bis etwa 50 °C, und gegebenenfalls unter Inertgas, z. B. Stickstoff- oder Argonatmosphäre, durchgeführt.

Zur Veresterung oder Amidierung einer Carboxygruppe in einer Verbindung der Formel I', beispielsweise

zur Amidierung einer freien Carboxygruppe einer Aminosäure, wie Glu oder Asp, mit Ammoniak, oder einer freien Carboxygruppe an durch $R_4$ und $R_5$ gemeinsam mit dem bindenden Stickstoffatom gebildetem Thiomorpholino oder Morpholino, kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z. B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z. B. mit Diazomethan, Alkylhalogeniden, Sulfonsäureestern, Meerweinsalzen oder 1-substituierten 3-Aryltriazenen, etc.

Zur Überführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung unter üblichen, wie den im Organikum, 17. Auflage, VEB Deutscher Verlag der Wissenschaften, Berlin 1988, genannten Reaktionsbedingungen angewendet werden.

In einer Verbindung der Formel I' kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den üblichen, wie den im Organikum, 15. Auflage, VEB Deutscher Verlag der Wissenschaften, Berlin (Ost) 1976, für derartige Umsetzungen genannten Reaktionsbedingungen erfolgen.

In einer Verbindung der Formel I' kann man eine vorhandene freie Aminogruppe acylieren, beispielsweise zur Einführung eines der für $R_1$ ausser Wasserstoff genannten Reste. Die Acylierung erfolgt nach der oben unter Verfahren a) oder einer der für Schutzgruppen genannten Methoden oder beispielsweise nach einem der im Organikum, 17. Auflage, VEB Deutscher Verlag der Wissenschaften, Berlin (Ost) 1988, genannten Verfahren.

In einer erhältlichen Verbindung der Formel I', worin die Substituenten die genannten Bedeutungen haben und mindestens eine freie Hydroxygruppe vorhanden ist und die übrigen funktionellen Gruppen in geschützter Form vorliegen, kann man die freie Hydroxygruppe acylieren oder verethern, beispielsweise die Hydroxygruppe an Thiomorpholino oder Morpholino, das aus $R_4$ und $R_5$ gemeinsam mit dem bindenden Stickstoffatom gebildet wird.

Die Acylierung kann erfolgen mit acylierenden Reagentien nach einer der unter Verfahren a) bis e) oder g) oder nach einer der für Schutzgruppen genannten Methoden oder nach einem der im Organikum, 17. Auflage, VEB Deutscher Verlag der Wissenschaften, Berlin (Ost) 1988, genannten Verfahren.

Die Veretherung kann mit den oben genannten Alkylierungsmitteln und unter den gleichen Reaktionsbedingungen erfolgen, z. B. mit Diazomethan, Alkylhalogeniden, Sulfonsäureestern, Meerweinsalzen, 1-substituierten 3-Aryltriazenen, etc. Bevorzugt ist die Umsetzung mit entsprechenden Alkylhalogeniden, wie Niederalkyliodiden oder -bromiden, in Gegenwart von Caesiumcarbonat in geegneten Lösungsmitteln oder Lösungsmittelgemischen, z.B. in N,N-Diniederalkyl-niederalkanoylamiden, wie Dimethylformamid oder -acetamid, oder Ethern, wie Dioxan, oder Gemischen davon, bei Temperaturen uwischen 0 °C bis zur Rückflusstemperatur, vorzugsweise bei 30 bis 60 °C, z.B. bei etwa 50 °C.

In einer Verbindung der Formel I' kann man vorhandene Gruppen, die Schutzgruppen entesprechen, oder ferner geeignete Reste $R_1$ ausser Wasserstoff nach einem der unter Verfahren f) genannten Verfahren abspalten, insbesondere durch Hydrolyse, beispielsweise in Gegenwart von Basen, wie Alkali- oder Erdalkalihydroxiden, z. B. Natriumhydroxid, oder Säuren, wie organischen Säuren oder Mineralsäuren, z. B. Halogenwasserstoff, wie Chlorwasserstoff. Die Hydrolyse erfolgt unter den üblichen Bedingungen, beispielsweise in wässriger Lösung oder in wasserfreien Lösungsmitteln, insbesondere in Ethern, wie Dioxan, bei Temperaturen zwischen -50 °C und der Rückflusstemperatur der entsprechenden Reaktionsgemische, z. B. zwischen 0 °C und 50 °C, vorzugsweise in Gegenwart eines Schutzgases, wie Argon oder Stickstoff, oder durch Hydrogenolyse (z.B. im Falle von Benzyloxycarbonylresten), vorzugsweise in polaren Lösungsmitteln, wie Alkoholen, z.B. Methanol oder Ethanol, oder von Estern, wie Niederalkylniederalkanoaten, z.B. Essigsäureethylester, bei den zuletzt genannten Temperaturen und in Gegenwart von geeigneten Hydrierkatalysatoren, wie eines Palladiumkatalysators, der vorzugsweise an einen Träger, wie Kohle, gebunden ist.

In einer Verbindung der Formel I', in der wenigstens einer der Reste $R_2$ oder $R_3$ eine Phenylgruppe bedeutet und/oder einer oder mehrere der Reste $B_1$, $A_1$ oder $A_2$ Phenylalanin bedeuten, wobei die Phenylreste auch jeweils, wie oben beschrieben, substituiert sein können, kann der oder können die entsprechenden Phenylreste selektiv zu entsprechenden Cyclohexylresten reduziert, beispielsweise hydriert, werden. Die Hydrierung erfolgt vorzugsweise in Gegenwart eines Katalysators, der die selektive Hydrierung von Doppelbindungen in Gegenwart von Peptidbindungen erlaubt, insbesondere eines Katalysators aus Schwermetalloxiden, wie eines Rh(III)/Pt(VI)-Oxidkatalysators nach Nishimura (S. Nishimura, Bull. Chem. Soc. Japan 33, 566 (1960), in geeigneten Lösungsmitteln, insbesondere Wasser, Alkoholen, wie Methanol oder Ethanol, Estern, wie Essigsäureethylester, oder Ethern, wie Dioxan, z. B.in Methanol, bei Temperaturen von 0 bis 150 °C, vorzugsweise 10 bis 50 °C, z. B. bei Raumtemperatur, und bei Wasserstoffdrucken von 1 bis 50 bar, z. B. bei Normaldruck.

Pharmazeutische Präparate:

Die Erfindung betrifft auch pharmazeutische Präparate enthaltend Verbindungen der Formel I', sowie neue Verbindungen der Formel I.

Die pharmakologisch verwendbaren Verbindungen der vorliegenden Erfindung können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, bukkalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die Erfindung betrifft auch pharmazeutische Präparate und eine Methode zur Behandlung von durch Retroviren verursachten Krankheiten, z. B. von AIDS oder dessen Vorstufen, insbesondere, wenn HIV-2 oder vor allem HIV-1 die Erkrankung verursacht, vorzugsweise dadurch gekennzeichnet, dass eine therapeutisch gegen retrovirale Erkrankungen, wie AIDS oder dessen Vorstufen, wirksame Menge einer erfindungsgemässen Verbindung der Formel I' in einem pharmazeutischen Präparat umfasst ist, welches zur Verabreichung an einen Warmblüter, insbesondere Menschen, zur Behandlung von einer retroviralen Erkrankung, wie AIDS, geeignet ist bzw. dass eine therapeutisch wirksame Menge einer erfindungsgemässen Verbindung der Formel I' bei der Behandlungsmethode an einen Warmblüter, z. B. Menschen, der wegen einer der genannten Erkrankungen, insbesondere AIDS, einer derartigen Behandlung bedarf, in therapeutisch gegen retrovirale Erkrankungen, wie AIDS oder dessen Vorstufen, wirksamer Menge verabreicht wird. Die an Warmblüter, z. B. Menschen von etwa 70 kg Körpergewicht, zu verabreichenden Dosismengen liegen zwischen etwa 3 mg und etwa 10 g, vorzugsweise zwischen etwa 40 mg und etwa 4 g, z. B. bei ungefähr 300 mg bis 1,5 g pro Person und Tag, verteilt auf vorzugsweise 1 bis 3 Einzeldosen, die z. B. gleich gross sein können. Ueblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z. B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen oder Dispersionen, und zwar insbesondere isotonische wässrige Lösungen, Dispersionen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z. B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Miglyol 812" (Triglycerid gesättigter Fettsäuren der Kettenlänge $C_8$ bis $C_{12}$ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und, wenn erwünscht oder notwendig, nach Zugabe von geeigneten Hilfsstoffen, zu Tabletten, Dragée-Kernen oder Kapseln verarbeitet, oder durch Herstellung von Dispersionen, vorzugsweise mit Phospholipiden, die in Gläschen abgefüllt werden. Dabei kann man die Wirkstoffe auch in Kunststoffträger einbauen, die sie dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl, Fettsäureester von Niederalkylglykolen, wie 1,2-Propylenglykol-monolaurat (Gemisch beider Konstitutionsisomeren; Gattefossé S.A., Saint Priest, Frankreich), ®Gelucire (Glyceride und Teilpolyglyceride von Fettsäure; Gattefossé S.A., Saint Priest, Frankreich) oder flüssigen Polyethylenglykolen, wie PEG 300, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, beigefügt werden.

Als pharmazeutische Präparate besonders bevorzugt sind durch Phospholipide stabilisierte Dispersionen des Wirkstoffes, vorzugsweise zur oralen Applikation, enthaltend

a) ein Phospholipid oder mehrere Phospholipide der Formel

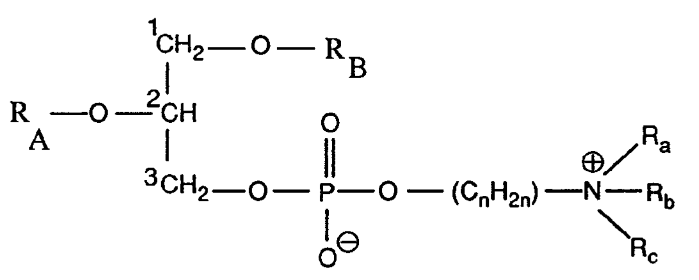

(A),

worin $R_A$ $C_{10-20}$-Acyl, $R_B$ Wasserstoff oder $C_{10-20}$-Acyl, $R_a$, $R_b$ und $R_c$ Wasserstoff oder $C_{1-4}$-Alkyl und n eine ganze Zahl von zwei bis vier darstellen, gewünschtenfalls b) ein weiteres Phospholipid oder mehrere weitere Phospholipide

c) den Wirkstoff und

d) eine pharmazeutisch annehmbare Trägerflüssigkeit und gewünschtenfalls weitere Hilfsstoffe und/oder Konservierungsmittel.

Das Herstellungsverfahren für diese Dispersionen ist dadurch gekennzeichnet, dass man eine Lösung oder Suspension der Komponenten a) und c) oder a), b) und c), vorzugsweise von a) und b) in einem Gewichtsverhältnis von 20 : 1 bis 1 : 5, insbesondere von 5 : 1 bis 1 : 1, durch Verdünnung mit Wasser in eine Dispersion umwandelt, anschliessend das organische Lösungsmittel entfernt, beispielsweise durch Zentrifugation, Gelfiltration, Ultrafiltration oder insbesondere durch Dialyse, z. B. tangentiale Dialyse, vorzugsweise gegen

Wasser, die erhaltene Dispersion, vorzugsweise nach Zugabe von Hilfsstoffen oder Konservierungsmitteln, erforderlichenfalls unter Einstellung eines annehmbaren pH-Wertes durch Zugabe von pharmazeutisch annehmbaren Puffern, wie Phosphatsalzen oder organischen Säuren (rein oder gelöst in Wasser), wie Essigssäure oder Zitronensäure, vorzugsweise zwischen pH 3 und 6, z. B. pH 4 - 5, falls sie nicht bereits die richtige Wirkstoffkonzentration hat, konzentriert, vorzugsweise auf eine Wirkstoffkonzentration von 2 bis 30 mg/ml, insbesondere von 10 bis 20 mg/ml, wobei die Konzentrierung vorzugsweise nach den zuletzt genannten Methoden zur Entfernung eines organischen Lösungsmittels erfolgt, insbesondere durch Ultrafiltration, z. B. unter Verwendung einer Apparatur zur Durchführung tangentialer Dialyse und Ultrafiltration.

Die nach diesem Verfahren herstellbare, durch Phospholipide stabilisierte Dispersion ist bei Zimmertemperatur mindestens mehrere Stunden stabil, reproduzierbar bezüglich des Mengenanteils der Komponenten und toxikologisch unbedenklich und daher insbesondere für die orale Applikation am Menschen geeignet.

Die Grössenordnung der erhaltenen Partikel in der Dispersion ist variabel und liegt vorzugsweise zwischen ca. 1,0 x $10^{-8}$ bis ca. 1,0 x $10^{-5}$ m, insbesondere zwischen etwa $10^{-7}$ und etwa 2 x $10^{-6}$ m.

Die Nomenklatur der Phospholipide der Formel I und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

In einem Phospholipid der Formel A sind RA und RB mit den Bedeutungen $C_{10-20}$-Acyl vorzugsweise geradkettiges $C_{10-20}$-Alkanoyl mit einer geraden Anzahl an C-Atomen und geradkettiges $C_{10-20}$-Alkenoyl mit einer Doppelbindung und einer geraden Anzahl an C-Atomen.

Geradkettiges $C_{10-20}$-Alkanoyl $R_A$ und $R_B$ mit einer geraden Anzahl an C-Atomen sind beispielsweise n-Dodecanoyl, n-Tetradecanoyl, n-Hexadecanoyl oder n-Octadecanoyl.

Geradkettiges $C_{10-20}$-Alkenoyl $R_A$ und $R_B$ mit einer Doppelbindung und einer geraden Anzahl an C-Atomen sind beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (Oleoyl).

In einem Phospholipid der Formel A ist n eine ganze Zahl von zwei bis vier, vorzugsweise zwei. Die Gruppe der Formel -($C_nH_{2n}$)- stellt unverzweigtes oder verzweigtes Alkylen dar, z.B. 1,1-Ethylen, 1,1-, 1,2- oder 1,3-Propylen oder 1,2-, 1,3- oder 1,4-Butylen. Bevorzugt ist 1,2-Ethylen (n=2).

Phospholipide der Formel A sind beispielsweise natürlich vorkommende Kephaline, worin $R_a$, $R_b$ und $R_c$ Wasserstoff bedeuten oder natürlich vorkommende Lecithine, worin $R_a$, $R_b$ und $R_c$ Methyl bedeuten, z.B. Kephalin oder Lecithin aus Sojabohnen, Rinderhirn, Rinderleber oder Hühnerei mit verschiedenen oder identischen Acylgruppen $R_A$ und $R_B$ oder Mischungen davon.

Bevorzugt sind synthetische, im wesentlichen reine Phospholipide der Formel A mit verschiedenen oder identischen Acylgruppen $R_A$ und $R_B$.

Der Begriff "synthetisches" Phospholipid der Formel A definiert Phospholipide, welche bezüglich $R_A$ und $R_B$ eine einheitliche Zusammensetzung haben. Solche synthetischen Phospholipide sind vorzugsweise die unten definierten Lecithine und Kephaline, deren Acylgruppen $R_A$ und $R_B$ eine definierte Struktur haben und von einer definierten Fettsäure mit einem Reinheitsgrad höher als ca. 95 % abgeleitet sind. $R_A$ und $R_B$ können gleich oder verschieden und ungesättigt oder gesättigt sein. Bevorzugt ist $R_A$ gesättigt, z.B. n-Hexadecanoyl, und $R_B$ ungesättigt, z.B. 9-cis-Octadecenoyl (= Oleoyl).

Der Begriff "natürlich vorkommende" Phospholipide der Formel A definiert Phospholipide, welche bezüglich $R_A$ und $R_B$ keine einheitliche Zusammensetzung haben. Solche natürlichen Phospholipide sind ebenfalls Lecithine und Kephaline, deren Acylgruppen RA und $R_B$ strukturell undefinierbar und von natürlich vorkommenden Fettsäuregemischen abgeleitet sind.

Der Begriff "im wesentlichen reines" Phospholipid definiert einen Reinheitsgrad von mehr als 70 % (Gew.) des Phospholipids der Formel A, welcher anhand geeigneter Bestimmungsmethoden, z.B. papierchromatographisch, nachweisbar ist.

Besonders bevorzugt sind synthetische, im wesentlichen reine Phospholipide der Formel A, worin RA die Bedeutung geradkettiges $C_{10-20}$-Alkanoyl mit einer geraden Anzahl an C-Atomen und $R_B$ die Bedeutung geradkettiges $C_{10-20}$-Alkenoyl mit einer Doppelbindung und einer geraden Anzahl an C-Atomen haben. $R_a$, $R_b$ und $R_c$ sind Methyl und n ist zwei.

In einem besonders bevorzugten Phospholipid der Formel A bedeuten RA n-Dodecanoyl, n-Tetradecanoyl, n-Hexadecanoyl oder n-Octadecanoyl und $R_B$ 9-cis-Dodecenoyl, 9-cis-Tetradecenoyl, 9-cis-Hexadecenoyl, 9-cis-Octadecenoyl oder 9-cis-Icosenoyl. $R_a$, $R_b$ und $R_c$ sind Methyl und n ist zwei.

Ein ganz besonders bevorzugtes Phospholipid der Formel A ist synthetisches 1-n-Hexadecanoyl-2-(9-cis-octadecenoyl)-3-sn-phosphatidylcholin mit einer Reinheit von mehr als 95 %.

Bevorzugte natürliche, im wesentlichen reine Phospholipide der Formel A sind insbesondere Lecithin (L-α-Phosphatidylcholin) aus Sojabohnen oder Hühnerei.

Für die Acylreste in den Phospholipiden der Formeln A sind auch die in Klammern angegebenen Bezeichnungen gebräuchlich: 9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Weitere Phospholipide sind vorzugsweise Ester von Phosphatidsäure (3-sn-Phosphatidsäure) mit den genannten Aclyresten, wie Phosphatidylserin und Phosphatidylethanolamin.

Schwerlösliche Wirkstoffe können auch als wasserlösliche, pharmazeutisch annehmbare Salze vorliegen, wie oben definiert.

In der Trägerflüssigkeit d) sind die Komponenten a), b) und c) oder a) und c) als Liposomen so enthalten, dass sich mehrere Tage bis Wochen keine Feststoffe oder feste Aggregate wie Mizellen zurückbilden und die Flüssigkeit mit den genannten Komponenten, gegebenenfalls nach Filtration, vorzugsweise oral, applizierbar ist.

In der Trägerflüssigkeit d) können pharmazeutisch annehmbare, nicht toxische Hilfsstoffe enthalten sein, z.B. wasserlösliche Hilfsstoffe welche zur Herstellung von isotonischen Bedingungen geeignet sind, z.B. ionische Zusätze wie Kochsalz oder nichtionische Zusätze (Gerüstbildner) wie Sorbit, Mannit oder Glucose oder wasserlösliche Stabilisatoren für die Liposomendispersion wie Lactose, Fructose oder Sucrose.

Zusätzlich zu den wasserlöslichen Hilfsstoffen können in der Trägerflüssigkeit für flüssige pharmazeutische Formulierungen verwendbare Emulgatoren, Netzmittel oder Tenside vorhanden sein, insbesondere Emulgatoren wie Ölsäure, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Bevorzugte Konservierungsmittel sind z. B. Antioxidantien, wie Ascorbinsäure, oder Microbizide, wie Sorbinsäure oder Benzoesäure.

Ausgangsmaterialien:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Alle Ausgangsmaterialien können vorzugsweise analog den in den Beispielen und Referenzbeispielen genannten Verfahren hergestellt werden.

Bei der Herstellung aller Ausgangsmaterialien können freie funktionelle Gruppen, die nicht an der jeweiligen Reaktion teilnehmen sollen, ungeschützt oder in geschützter Form vorliegen, beispielsweise geschützt durch die oben unter Verfahren a) genannten Schutzgruppen. Wenn erforderlich, liegen die funktionellen Gruppen, die nicht an der Reaktion teilnehmen sollen, immer in geschützter Form vor (vgl. z.B. den Schutz von Verbindungen der Formel XXI bei der Umsetzung z.B. zu Verbindungen der Formel XXII', s. unten, an der nicht umzusetzenden Carboxygruppe, um eine Lactonisierung zu vermeiden). Diese Schutzgruppen können zu geeigneten Zeitpunkten durch die unter Verfahren f) beschriebenen Reaktionen freigesetzt werden. Die Verbindungen mit salzbildenden Gruppen können jeweils auch als Salze Verwendung finden und Salze auf jeder Stufe hergestellt oder wieder in die freien Verbindungen überführt werden.

In den Formeln wird, sofern nicht die Stereochemie asymmetrischer Kohlenstoffatome direkt durch die Wahl entsprechender Bindungssymbole definiert wird, die Konfiguration asymmetrischer Kohlenstoffatome durch die jeweils angegebene Konfigurationsbezeichnung ausgewählt aus (S), (R) und (S,R) bezeichnet.

Die Carbon- oder Sulfonsäuren der Formel II, oder reaktionsfähige Derivate hiervon, sind bekannt und sind kommerziell erhältlich oder können nach an sich bekannten Verfahren hergestellt werden.

Die Verbindungen der Formel III' sind bekannt oder können nach an sich bekannten Verfahren hergestellt werden. Beispielsweise sind sie zugänglich aus Verbindungen der Formel

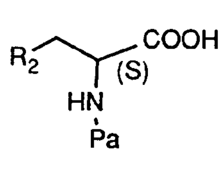

(XII),

worin $R_2$ die für Verbindungen der Formel I' genannten Bedeutungen hat und Pa eine Aminoschutzgruppe, insbesondere Niederalkoxycarbonyl, wie tert-Butoxycarbonyl, oder Phenylniederalkoxycarbonyl, wie Benzyloxycarbonyl, bedeutet (oder Analogen davon, worin anstelle von Pa Wasserstoff steht, die dann anschliessend geschützt werden können) durch Reduktion in eine Verbindung der Formel

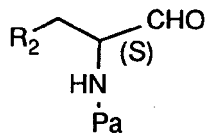

(XIII)

überführt (oder das Analoge mit Wasserstoff statt Pa), worin die Reste die zuletzt genannten Bedeutungen haben.

Die Reduktion von Aminosäurederivaten der Formel XII zu den entsprechenden Aldehyden XIII erfolgt beispielsweise durch Reduktion zu den entsprechenden Alkoholen und anschliessende Oxidation zu den Aldehyden der Formel XIII.

Die Reduktion zu den Alkoholen erfolgt insbesondere durch Hydrogenierung der entsprechenden Säurehalogenide oder anderweitiger, unter Verfahren a) genannter aktivierter Carbonsäurederivate, oder durch Umsetzung aktivierter Carbonsäurederivate der Verbindungen der Formel XII, insbesondere von Anhydriden mit organischen Carbonsäuren, vorzugsweise solcher von Halogenameisensäureestern, wie Chlorameisensäureisobutylester (die vorzugsweise durch Umsetzung der Verbindungen der Formel XII in Gegenwart von basischen Aminen, z. B. Triniederalkylaminen, wie Triethylamin, in organischen Lösungsmitteln, wie cyclischen Ethern, z. B. Dioxan, bei Temperaturen zwischen -50 und 80 °C, vorzugsweise zwischen 0 und 50 °C, erhalten werden) mit komplexen Hydriden, wie Alkaliborhydriden, z. B. Natriumborhydrid, in wässriger Lösung in An- oder Abwesenheit der zuletzt verwendeten organischen Lösungsmittel bei Temperaturen zwischen -50 und 80 °C, vorzugsweise zwischen 0 und 50 °C. Die anschliessende Oxidation der erhaltenen Alkohole erfolgt vorzugsweise mit solchen Oxidationsmitteln, welche selektiv die Hydroxy- in eine Aldehydgruppe umwandeln, beispielsweise Chromsäure oder ihre Derivate, wie Pyridiniumchromat oder tert-Butylchromat, Dichromat/Schwefelsäure, Schwefeltrioxid in Gegenwart von heterocyclischen Basen, wie Pyridin/$SO_3$ (vorzugsweise gelöst in Diniederalkylsulfoxiden, wie Dimethylsulfoxid, aromatischen Lösungsmitteln, wie Toluol, oder Gemischen dieser Lösungsmittel), ferner Salpetersäure, Braunstein oder Selendioxid, in Wasser, wässrigen oder organischen Lösungsmitteln, wie halogenierten Lösungsmitteln, z. B. Methylenchlorid, Carbonsäureamiden, wie Dimethylformamid, und/oder cyclischen Ethern, wie Tetrahydrofuran, in Gegenwart oder Abwesenheit von basischen Aminen, z. B. Triniederalkylaminen, wie Triethylamin, bei Temperaturen zwischen -70 bis 100 °C, vorzugsweise zwischen -70 bis -50 °C oder bei -10 bis 50 °C, beispielsweise wie in der Europäischen Patentanmeldung EP-A-0 236 734 beschrieben.

Auch die direkte Reduktion der Verbindungen der Formel XII zu den Aldehyden ist möglich, beispielsweise durch Hydrierung in Gegenwart eines partiell vergifteten Palladiumkatalysators oder durch Reduktion der entsprechenden Aminosäureester, z. B. der Niederalkylester, wie Ethylester, mit komplexen Hydriden, z. B. Borhydriden, wie Natriumborhydrid, oder vorzugsweise Aluminiumhydriden, z. B. Lithiumaluminiumhydrid, Lithiumtri-(tert-butoxy)aluminiumhydrid oder insbesondere Diisobutylaluminiumhydrid, in apolaren Lösungsmitteln, z. B. in Kohlenwasserstoffen oder aromatischen Lösungsmitteln, wie Toluol, bei -100 bis 0 °C, bevorzugt -70 bis -30 °C, und anschliessende Umsetzung zu den entsprechenden Semicarbazonen, z. B. mit den entsprechenden Säuresalzen von Semicarbazonen, wie Semicarbazidhydrochlorid, in wässrigen Lösungsmittelsystemen, wie Alkohol/Wasser, z. B. Ethanol/Wasser, bei Temperaturen zwischen -20 und 60 °C, bevorzugt 10 bis 30 °C, und Umsetzung des erhaltenen Semicarbazones mit einem reaktiven Aldehyd, z. B. Formaldehyd, in einem inerten Lösungsmittel, beispielsweise einem polaren organischen Lösungsmittel, z. B. einem Carbonsäureamid, wie Dimethylformamid, bei Temperaturen zwischen -30 und 60 °C, bevorzugt 0 bis 30 °C, und dann einer Säure, beispielsweise einer starken Mineralsäure, wie Halogenwasserstoff, in wässriger Lösung, gegebenenfalls in Gegenwart des vorher verwendeten Lösungsmittels, bei Temperaturen zwischen -40 und 50 °C, bevorzugt zwischen -10 und 30 °C, umsetzt. Die entsprechenden Ester werden durch Umsetzung der Aminosäuren mit den entsprechenden Alkoholen, beispielsweise Ethanol, analog den bei der Kondensation unter Verfahren b) verwendeten Bedingungen gewonnen, beispielsweise durch Umsetzung mit anorganischen Säurehalogeniden, wie Thionylchlorid, in organischen Lösungsmittelgemischen, wie Mischungen aus aromatischen und alkoholischen Lösungsmitteln, z. B. Toluol und Ethanol, bei Temperaturen zwischen -50 und 50 °C, bevorzugt zwischen -10 und 20 °C.

Die Herstellung der Verbindungen der Formel XIII erfolgt in besonders bevorzugter Weise unter Bedingungen analog den in J. Org. Chem. 47, 3016 (1982), J. Org. Chem. 43, 3624 (1978) oder J. Org. Chem. 51,

3921 (1986) genannten Reaktionsbedingungen.

Zur Synthese der Verbindungen der Formel III' werden sodann die Verbindungen der Formel XIII mit einem reaktiven Tetraalkylsilan, vorzugsweise einem Halogenmethyltriniederalkylsilan, wie Chlormethyltrimethylsilan, in einem inerten Lösungsmittel, beispielsweise einem Ether, wie Diethylether, einem cyclischen Ether, wie Dioxan, oder einem Ester, wie Essigsäureethylester, bei Temperaturen zwischen -100 und 50 °C, bevorzugt zwischen -65 und 40 °C, umgesetzt, wobei Verbindungen der Formel

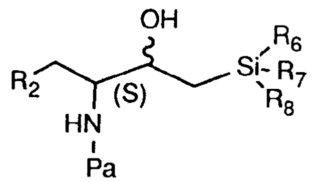

(XIV)

erhalten werden, worin $R_6$, $R_7$ und $R_8$ Niederalkyl, z. B. Methyl, bedeuten und die übrigen Reste die zuletzt genannten Bedeutungen haben, die erhaltenen Verbindungen in Gegenwart einer Lewissäure, wie Bortrifluoridethyletherat, in einem inerten Lösungsmittel, insbesondere einem halogenierten Kohlenwasserstoff, wie Methylenchlorid oder Chloroform, mit anschliessender Nachbehandlung mit wässriger Base, z. B. Natronlauge, bei Temperaturen zwischen -30 und 80 °C, insbesondere zwischen 0 und 50 °C, unter Elimination und Schutzgruppenabspaltung in olefinische Verbindungen der Formel

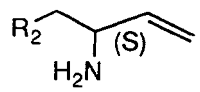

(XV)

überführt, worin $R_2$ die für Verbindungen der Formel I' genannten Bedeutungen hat, in das entsprechende Olefin erneut eine Aminoschutzgruppe Pa einführt, wie unter Verfahren a) für die Einführung von Aminoschutzgruppen beschrieben, insbesondere mit Hilfe eines Säureanhydrides in einem chlorierten Kohlenwasserstoff, wie Methylenchlorid oder Chloroform, bei Temperaturen zwischen -50 und 80 °C, insbesondere zwischen 0 und 35 °C, wobei ein geschütztes Amino-olefin der Formel

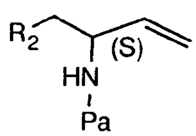

(XVI)

erhalten wird, in dem die Reste die zuletzt genannten Bedeutungen haben, die Doppelbindung in ein Oxiran umwandelt, vorzugsweise stereoselektiv unter Verwendung von Peroxiden, insbesondere Peroxycarbonsäuren, z. B. Halogeno-perbenzoesäure, wie m-Chlorperbenzoesäure, in einem inerten organischen Lösungsmittel, vorzugsweise einem halogenierten Kohlenwasserstoff, wie Methylenchlorid oder Chloroform, bei Temperaturen zwischen -50 und 60 °C, insbesondere zwischen -10 und 25 °C, und, falls erforderlich, eine Diastereomerentrennung vornimmt, wobei Epoxide der Formel

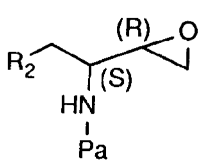

(XVII),

in denen die Reste die zuletzt genannten Bedeutungen haben, erhalten werden, an die betreffenden Olefine einen geeigneten Malonsäurediester, z. B. Malonsäuredimethylester oder Malonsäurediethylester, addiert, beispielsweise durch Aktivierung der Methylengruppe des Malonsäurediesters mittels eines Alkalimetalls, z. B. Natrium, in einem polaren wasserfreien Lösungsmittel, wie einem Alkohol, z. B. Methanol oder Ethanol, bei Temperaturen zwischen -50 und 80 °C, insbesondere zwischen 0 und 35 °C, und die Lösung mit einer Säure, wie einer Carbonsäure, z. B. Zitronensäure, behandelt, wobei ein Lacton der Formel

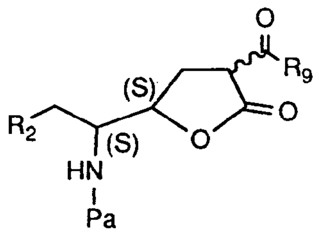

(XVIII),

worin $R_9$ Niederalkoxy, z. B. Methoxy oder Ethoxy, bedeutet und die übrigen Reste die zuletzt genannten Bedeutungen haben, erhalten wird, gewünschtenfalls in solchen Verbindungen, in denen $R_2$ Phenyl, das unsubstituiert oder wie für Verbindungen der Formel I' beschrieben substituiert ist, bedeutet, diesen Rest zu Cyclohexyl reduziert, insbesondere durch Hydrierung, vorzugsweise in Gegenwart von Katalysatoren, wie Edelmetalloxiden, z. B. Gemischen von Rh(III)/Pt(VI)-Oxiden (nach Nishimura), bevorzugt in polaren Lösungsmitteln, wie Alkoholen, z. B. Methanol, bei Normaldruck oder bis zu 5 bar, bevorzugt bei Normaldruck, bei Temperaturen von -20 bis 50 °C, bevorzugt 10 bis 35 °C, die direkt oder nach der Hydrierung erhaltenen Verbindungen der Formel XVIII mit einem den Rest $R_3$-$CH_2$- einführenden Reagens, beispielsweise der Formel $R_3$-$CH_2$-W, worin $R_3$ die für Verbindungen der Formel I' genannten Bedeutungen hat und W eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Iodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure verestertes Hydroxy oder mit Stickstoffwasserstoffsäure verestertes Hydroxy, insbesondere Bromid, bedeutet, in einem wasserfreien polaren Lösungsmittel, z. B. einem Alkohol, wie Ethanol, in Gegenwart eines Alkalimetalles, z. B. Natrium, bei Temperaturen zwischen -50 und 80 °C, bevorzugt zwischen 0 und 35 °C, umsetzt unter Erhalt von Verbindungen der Formel

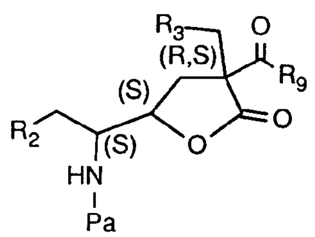

(XIX),

worin die Reste die zuletzt genannten Bedeutungen haben, die Verbindungen der Formel XIX hydrolysiert und decarboxyliert, beispielsweise durch Hydrolyse mittels einer Base, wie einem Alkalimetallhydroxid, z. B. Lithiumhydroxid oder NaOH, bei Temperaturen zwischen -50 und 80 °C, vorzugsweise zwischen etwa 0 und 35 °C, in einem organischen Lösungsmittel, z. B. einem Ether, wie Dimethoxyethan, oder einem Alkohol, wie Ethanol, und anschliessende Decarboxylierung durch Erhitzen in einem inerten Lösungsmittel, vorzugsweise einem Kohlenwasserstoff, z. B. einem aromatischen Kohlenwasserstoff, wie Toluol, auf Temperaturen zwischen 40 und 120 °C, vorzugsweise zwischen 70 und 120 °C, wobei eine Verbindung der Formel

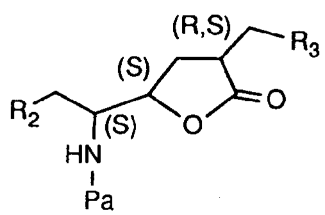

(XX),

worin die Reste die zuletzt genannten Bedeutungen haben, erhalten wird, durch Säulenchromatographie die erhaltenen (R,S,S)- und (S,S,S)-Isomeren auftrennt, das (R,S,S)-Isomere weiterverwendet und zur Öffnung des Lactonringes mit einer Base, wie einem Alkalimetallhydroxid, z. B. Lithiumhydroxid oder Natriumhydroxid, in einem inerten Lösungsmittel, wie einem Ether, z. B. Dimethoxyethan, oder einem Alkohol, wie Ethanol, umsetzt unter Erhalt einer Verbindung der Formel

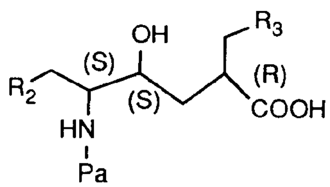

(XXI),

worin die Reste die zuletzt genannten Bedeutungen haben, in die erhaltene Verbindung eine Hydroxyschutzgruppe Py, beispielsweise eine der unter Verfahren a) genannten Hydroxyschutzgruppen unter den dort genannten Bedingungen, insbesondere eine Triniederalkylsilylgruppe mit Hilfe des entsprechenden Halogen-triniederalkylsilanes, z. B. tert-Butyldimethylchlorsilanes, in einem polaren Lösungsmittel, wie einem Diniederalkyl-niederalkanoylamid, wie Dimethylformamid, in Gegenwart einer sterisch gehinderten Aminoverbindung, wie eines cyclischen Amins, z. B. Imidazol, bei Temperaturen von -50 bis 80 °C, bevorzugt von 0 bis 35 °C, einführt unter Erhalt einer Verbindung der Formel

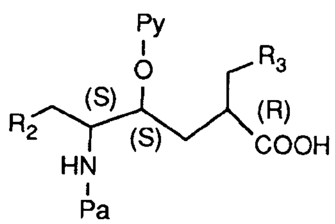

(XXII),

worin die Reste die zuletzt genannten Bedeutungen haben, oder direkt mit einer Verbindung der Formel XXV oder einem reaktionsfähigen Derivat hiervon, wie oben definiert, unter Einführung des Restes T acyliert, wie oben unter Verfahren g) beschrieben, wobei man die entsprechende Verbindung der Formel

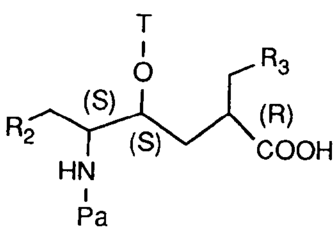

(XXII')

erhält, worin anstelle von Py der Rest T steht und die übrigen Reste die genannten Bedeutungen haben; und die Verbindungen der Formel III' mit den unter Verfahren a) angegebenen Resten aus einer der Verbindungen der Formel XXII oder XXII' beispielsweise herstellt durch Kondensation mit einer Verbindung der Formel VII, worin die Reste die unter Verfahren c) angegebenen Bedeutungen haben, unter den für Verfahren a) angegebenen Bedingungen, insbesondere durch in-situ-Reaktion in Gegenwart eines Kondensationsmittels, wie Benzotriazol-1-yl-oxy-tris-(di-methylamino)-phosphonium-hexafluorphosphat oder O-Benztriazol-1-yl-N,N,N',N'-tetra-methyl-uronium-hexafluorphosphat, eines sterisch gehinderten Amins, wie N-Methylmorpholin, und einer racemisierungshindernden Verbindung, wie 1-Hydroxy-benz-triazol, in einem polaren Lösungsmittel, vorzugsweise einem Säureamid, z. B. einem Diniederalkylamino-niederalkanoylamid, wie Dimethylformamid, bei Temperaturen zwischen -50 und 80 °C, insbesondere zwischen 0 und 35 °C, und durch anschliessende Schutzgruppenabspaltung von Pa, wie unter Verfahren f) beschrieben, sofern Pa keinen dem Radikal $H-B_1$- mit den oben für Verbindungen der Formel I' dargestellten Bedeutungen, ausser einer Bindung, entsprechenden Rest bedeutet, Kondensation mit einer Verbindung der Formel $H-B_1'-OH$, worin $B_1'$ die unter Verfahren b) genannten Bedeutungen hat, unter den zuletzt genannten Kondensationsbedingungen und schliesslich die Abspaltung von Py (nur im Falle der Verbindung der Formel XXII) und/oder weiteren Schutzgruppen, wie unter Verfahren f) beschrieben, und erforderlichenfalls (im Falle der aus Verbindungen der Formel XXII hergestellten Verbindungen mit freier Hydroxygruppe) Einführung von T, wie unter Verfahren g) beschrieben. Zur Herstellung der Verbindungen der Formel III' ist auch die sukzessive Kondensation der Verbindungen der Formel XXII oder XXII' mit Verbindungen möglich, welche die Radikale $-B_1-$, $-A_1-$, $-A_2-$, $-A_1-A_2-$, $A_2-NR_4R_5$ und/oder $-NR_4R_5$ der Verbindung der Formel VII einführen, unter Bedingungen analog den für Verfahren a) genannten, wobei im Falle der Verwendung von Verbindungen der Formel XXII die Schutzgruppe Py nach einer der unter Verfahren f)

beschriebenen Methoden abgespalten wird und dann mit einer Verbindung der Formel XXV oder einem reaktionsfähigen Derivat davon unter den für Verfahren g) genannten Reaktionsbedingungen der Rest T eingeführt werden muss.

Der Weg von einer oben genannten Verbindung der Formel XVIII zu einer Verbindung der Formel XX kann auch folgendermassen geführt werden:

Verseifung einer racemischen Verbindung der Formel XVIII (herstellbar aus dem Racemat einer Verbindung der Formel XVI über das entsprechende Racemat einer Verbindung der Formel XVII) und Decarboxylierung unter Bedingungen analog denen für die Verseifung und Decarboxylierung von Verbindungen der Formel XIX führt zu einer Verbindung analog der Verbindung der Formel XIX, worin jedoch die Reste $R_3$-$CH_2$- und $R_9$-(C=O)- fehlen und die als Racemat vorliegt; diese wird anschliessend mit einer Verbindung der Formel $R_3$-$CH_2$-W, wie unterhalb von Formel XVIII definiert, worin W eine der dort genannten nukleofugen Abgangsgruppen, insbesondere Halo, wie Brom oder Chlor, bedeutet, umgesetzt indem sie zunächst in Gegenwart einer starken Base, wie einem Alkalimetallbis(triniederalkylsilyl)amid, z.B. Lithium-bis(trimethylsilyl)-amid, deprotoniert wird und dann mit der Verbindung der Formel $R_3$-$CH_2$-W alkyliert (vorzugsweise unter Erhalt der 1'(S),3(R)-($R_3$-$CH_2$-),5(S)- und der 1'(R),3(S)-($R_3$-$CH_2$-),5(R)-Verbindung der Formel XX, d.h. eines Racemates).

Die vorgenannten Verbindungen der Formel XV können auch am den Rest -$NH_2$ tragenden Kohlenstoffatom in der (R,S)-Konfiguration anstelle der gezeigten (S)-Konfiguration vorliegen, die Verbindungen der Formel XII, XIII, XIV und insbesondere die der Formel XVI, XVII, XVIII, XIX, XX, XXI und/oder XXII können auch am den Rest Pa-NH- tragenden Kohlenstoffatom in der (R,S)-Konfiguration anstelle der (S)-Konfiguration vorliegen. Auch die vorgenannten Verbindungen der Formel XVI, XVII und XVIII können als Racemate vorliegen, d. h. die optischen Antipoden der gezeigten Formeln sind ebenfalls möglich. Aus diesen Racematen können z.B. entsprechende Verbindungen der Formel VI' erhalten werden (beispielsweise Racemate, falls $R_1$ und $T_1$ keine Asymmetriezentren enthalten), so dass auf diese Weise Verbindungen der Formel I' zugänglich sind, in denen entweder das $R_2$-$CH_2$- tragende Kohlenstoffatom in der (S)-Konfiguration, das T-O- tragende Kohlenstoffatom in der (S)-Konfiguration und das $R_3$-$CH_2$- tragende Kohlenstoffatom in der (R)-Konfigaration vorliegt (2R,4S,5S), oder die genannten Kohlenstoffatome die entgegengesetzte Konfiguration haben (2S,4R,5R); oder auch Gemische von Verbindungen der Formel VI' oder I' mit diesen beiden Konfigurationen vorliegen. Entsprechende racemische Gemische oder Diastereomerengemische können auf allen Stufen (vorzugsweise) in die einzelnen Isomeren aufgetrennt werden.

Verbindungen der Formel XX, in denen die Reste die genannten Bedeutungen haben, werden auch aus Verbindungen der Formel XIII hergestellt, in denen die Reste die genannten Bedeutungen haben, indem man die Aldehyde der Formel XIII mit 2-Halogenpropionsäureestern, insbesondere 2-Iod-propionsäureestern, wie 2-Jod-propionsäureethylester, umsetzt, wobei man die Verbindungen der Formel

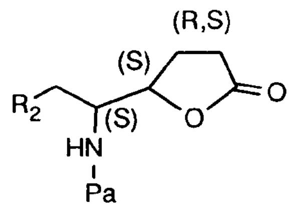

(XXIII)

erhält, worin die Reste die genannten Bedeutungen haben und worin das den Rest Pa-NH-tragende Kohlenstoffatom auch alternativ in der (R,S)-Konfiguration vorliegen kann.

Die Umsetzung erfolgt zunächst unter Bildung des Homoenolates des 2-Halogenpropionsäureesters in Gegenwart einer Mischung von Zn/Cu in einem Diniederalkyl-niederalkanoylamid, wie Dimethylacetamid, bei Temperaturen zwischen 0 und 100 °C, insbesondere zwischen 20 und 80 °C. In einem weiteren Ansatz wird, vorzugsweise unter Schutzgas, wie Stickstoff oder Argon, ein Tetraniederalkyl-orthotitanat, wie Tetraisopropyl-orthotitanat, in einem aromatischen Lösungsmittel, wie Toluol oder Xylol, in Gegenwart eines Halogenkohlenwasserstoffes, wie Methylenchlorid, mit einem Titantetrahalogenid, wie Titantetrachlorid, versetzt und bei 0 bis 50 ° C, insbesondere bei 20 bis 30 °C, gerührt, wobei das entsprechende Di-Halogen-titan-diniederalkylat oder vorzugsweise das Trihalogen-titan-niederalkylat, insbesondere Trichlortitan-diisopropylat, gebildet wird. Die Zn-Homoenolat-Lösung wird zu diesem bei Temperaturen zwischen -50 und 0°C, insbesondere von -40 bis -25 °C, zugetropft und anschliessend der Aldehyd der Formel XIII in einem Halogenkohlenwasserstoff, z. B. Methylenchlorid, zugetropft, wobei die Umsetzung bei -50 bis 30 °C, vorzugsweise bei etwa -20 bis 5 °C, stattfindet unter Bildung des Esters, insbesondere Ethylesters, der Verbindung der Formel XXIII. Dieser Ester wird dann hydrolysiert unter Bildung der Verbindung der Formel XIII, wie oben definiert, vorzugsweise in einem

organischen Lösungsmittel, wie einem Aromaten, z. B. in Toluol oder Xylol, in Gegenwart einer Säure, wie einer Carbonsäure, z. B. Essigsäure, bei Temperaturen zwischen 20 °C und dem Siedepunkt des Reaktionsgemisches, insbesondere zwischen 70 und 100 °C. Falls erforderlich, wird eine Diastereomerentrennung, beispielsweise durch Chromatographie, z. B. an Kieselgel mit einem organischen Lösungsmittelgemisch, wie einem Gemisch aus Alkan und Ester, wie Niederalkan und Niederalkylniederalkanoylester, wie Hexan/Essigsäureethylester, durchgeführt.

Aus der Verbindung der Formel XXIII wird dann durch Deprotonierung mit einer starken Base unter Erhalt des am $\alpha$-Kohlenstoff neben der Oxogruppe des Lactons gebildeten Carbanions und anschliessende nukleophile Substitution des Restes W einer Verbindung der Formel $R_3$-$CH_2$-W, worin $R_3$ und W wie oben bei der Herstellung von Verbindungen der Formel XIX definiert sind (W ist insbesondere Bromo), die entsprechende Verbindung der Formel XX erhalten, wobei die Reaktion vorzugsweise stereoselektiv zur (R)-Konfiguration am den Rest $R_3$-$CH_2$- tragenden Kohlenstoffatom in der Verbindung der Formel XX führt. Die Umsetzung mit der starken Base, insbesondere mit einer Alkalimetall-Organosiliciumamid-Verbindung, beispielsweise einem Alkalimetall-bis(triniederalkylsilyl)amid, wie Lithium-bis(trimethylsilyl)amid, oder ferner einem Alkalimetall-diniederalkylamid, wie Lithiumdiisopropylamid, erfolgt vorzugsweise in einem inerten organischen Lösungsmittel, insbesondere einem Ether, z. B. einem cyclischen Ether, wie Tetrahydrofuran, oder 1,3-Dimethyl-3,4,5,6-tetrahydro-2(1H)-pyrimidinon (DMPU), oder Gemischen dieser Lösungsmittel, bei Temperaturen zwischen - 100 und 0 °C, vorzugsweise zwischen -78 und -50 °C, die nukleophile Substitution in situ durch Zugabe der Verbindung der Formel $R_3$-$CH_2$-W, im selben Lösungsmittel bei Temperaturen zwischen - 100 und 0 °C, vorzugsweise zwischen -60 und -40 °C.

Eine Verbindung der Formel XV, worin die Reste die genannten Bedeutungen haben, und worin vorzugsweise das die Gruppe -$NH_2$ tragende Kohlenstoffatom in der (R,S)-Konfiguration vorliegt, kann auch erhalten werden, indem man einen Ameisensäureester, z. B. Ameisensäureniederalkylester, wie Ameisensäureethylester, durch Reaktion mit Allylamin bei Temperaturen zwischen 20 und 70 °C, insbesondere zwischen 50 und 60 °C, umsetzt zu Ameisensäureallylamid. Dieses Amid wird dann unter Schutzgas, wie Stickstoff oder Argon, dehydratisiert, vorzugsweise mit einem Säurehalogenid, wie Phosphoroxidchlorid, Phosgen oder insbesondere einem organischen Sulfonsäurehalogenid, z. B. einem Arylsulfonsäurechlorid, wie Toluolsulfonsäurechlorid, in Gegenwart einer Base, z. B. einem Triniederalkylamin, wie Triethylamin, oder insbesondere einem mono- oder bicyclischen Amin, wie Pyridin oder Chinolin, bei Temperaturen zwischen 50 und 100 °C, insbesondere zwischen etwa 80 und etwa 100 °C. Dabei entsteht Allylisocyanid, das durch Umsetzung mit einem Organolithiumsalz, z. B. Niederalkyllithium, wie n-Butyllithium, in das entsprechende Lithiumsalz überführt wird, wobei die Reaktion vorzugsweise in einem inerten organischen Lösungsmittel, insbesondere einem Ether, wie Dioxan oder Diethylether, oder einem Alkan, z. B. Hexan, oder einem Gemisch dieser Lösungsmittel, bei Temperaturen von -120 bis -50, insbesondere von etwa -100 bis -90 °C, durchgeführt wird. Das gebildete Lithiumsalz wird dann in situ mit einer Verbindung der Formel $R_2$-$CH_2$-W, worin $R_2$ die für Verbindungen der Formel I genannten Bedeutungen hat und W die oben für Verbindungen der Formel $R_3$-$CH_2$-W genannten Bedeutungen hat, insbesondere Brom bedeutet, umgesetzt, vorzugsweise durch Zutropfen von $R_2$-$CH_2$-W in einem organischen Lösungsnmittel, z. B. einem Ether, wie Tetrahydrofuran, bei den zuletzt genannten Temperaturen und anschliessendes Erwärmen auf 0 bis 50 °C, vorzugsweise auf 20 bis 30 °C. Dabei entsteht ein Isocyanid der Formel

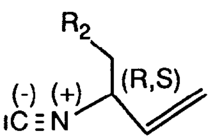

(XXIV),

worin die Reste die genannten Bedeutungen haben. Die Verbindung der Formel XXIV wird anschliessend hydrolysiert, vorzugsweise in wässriger Lösung, der eine Säure zugesetzt ist, z. B. in wässriger Halogenwasserstoffsäure, wie Salzsäure, insbesondere in konzentrierter Salzsäure, bei Temperaturen zwischen -20 und 30 °C, insbesondere zwischen etwa 0 und 10 °C, und man erhält die Verbindung der Formel XV, worin die Reste wie zuletzt definiert sind und worin vorzugsweise das die Gruppe -$NH_2$ tragende Kohlenstoffatom in der (R,S)-Konfiguration vorliegt.

Verbindungen der Formel IV sind bekannt oder nach an sich bekannten Verfahren herstellbar, beispielsweise durch Kondensation von Carbon- oder Sulfonsäuren der Formel II, oder reaktionsfähigen Derivaten hiervon, mit Aminoverbindungen der Formel H-$B_1$'-OH, worin $B_1$' die für Verbindungen der Formel IV genannten Bedeutungen hat, wobei die Kondensation wie zuletzt beschrieben erfolgt, oder im Falle von Verbindungen der Formel II, worin $R_1$' N-(Heterocyclylniederalkyl)-N-niederalkyl-aminocarbonyl, wie N-(2-Pyridylmethyl)-N-methyl-aminocarbonyl, bedeutet, analog EP 0 402 646 vom 19.12. 1990, Beispiel 218.

Verbindungen der Formel V' werden beispielsweise aus Verbindungen der Formel XXII oder XXII' durch Kondensation mit einer Verbindung der Formel VII oder sukzessive Kondensation mit Verbindungen (z. B. H-$A_1$'-OH, H-$A_2$'-OH, H-$A_1$-$A_2$-OH, oder die Verbindung der Formel XI, worin jeweils die Reste die oben genannten Bedeutungen haben) hergestellt, welche Fragmenten der Verbindung der Formel VII entsprechen. Die Kondensationsbedingungen sind analog zu den für die Herstellung der Verbindungen der Formel III' beschriebenen, wobei im Falle der Verwendung einer Verbindung der Formel XXII anschliessend noch T durch Umsetzung mit einer Verbindung der Formel XXV oder einem reaktionsfähigen Derivat davon, wie unter Verfahren g) definiert, unter den für Verfahren g) genannten Bedingungen eingeführt wird.

Verbindungen der Formel VI' werden beispielsweise aus den Aminoverbindungen der Formel XXII oder XXII', z. B. durch Einführung einer Carboxyschutzgruppe, wie unter Verfahren a) beschrieben, und Abspaltung der Schutzgruppe Pa, wie unter Verfahren f) beschrieben, hergestellt durch Kondensation mit einer Carbonsäure der Formel $R_1$-$B_1$-OH, worin die Reste die für Verbindungen der Formel I' genannten Bedeutungen haben. Im Falle der Verwendung einer Verbindung der Formel XXII wird anschliessend noch T durch Umsetzung mit einer Verbindung der Formel XXV oder einem reaktionsfähigen Derivat davon, wie unter Verfahren g) definiert, unter den für Verfahren g) genannten Bedingungen eingeführt.

Verbindungen der Formel VII werden beispielsweise aus den entsprechenden Aminosäuren H-$A_1$'-OH oder H-$A_2$'-OH oder den Peptiden H-$A_1$-$A_2$-OH und den Aminkomponenten der Formel XI, worin die Reste jeweils die oben genannten Bedeutungen haben, durch Kondensation analog dem unter Verfahren a) beschriebenen Verfahren hergestellt. Zur Herstellung von Verbindungen mit reduzierter Peptidbindung zwischen $A_1$ und $A_2$ kann die Peptidbindung zwischen $A_1$ und $A_2$, vorzugsweise auf der Stufe des Dipeptides, reduziert werden, beispielsweise mit Wasserstoff in Gegenwart von Schwer- oder Edelmetallkatalysatoren, wie Platin oder Palladium, gegebenenfalls auf Trägern, wie Aktivkohle, oder durch komplexe Hydride, vorzugsweise komplexe Hydride, z. B. Lithiumaluminiumhydrid oder Disiamylboran in polaren Lösungsmitteln, wie Alkoholen, z. B. Ethanol, oder Ethern, wie cyclischen Ethern, z. B. Tetrahydrofuran, bei Temperaturen zwischen 0 und 150 °C, bevorzugt zwischen 20 °C und dem Siedepunkt des betreffenden Reaktionsgemisches. Das Amin der Formel XI ist bekannt oder wird nach an sich bekannten Methoden hergestellt.

Verbindungen der Formel VIII' lassen sich beispielsweise aus Verbindungen der Formel VI' durch Kondensation mit einer den Rest $A_1$' einführenden Aminosäure herstellen. Die Umsetzung erfolgt analog den unter Verfahren a) beschriebenen Bedingungen.

Verbindungen der Formel IX werden beispielsweise aus einer Aminosäure H-$A_2$'-OH, worin $A_2$' die unter Verfahren d) genannten Bedeutungen hat, und einem Amin der Formel XI, worin die Reste die für Verbindungen der Formel I genannten Bedeutungen haben, durch Kondensation hergestellt.

Verbindungen der Formel X' werden beispielsweise aus Verbindungen der Formel VI' und aus den entsprechenden Aminosäuren H-$A_1$'-OH oder H-$A_2$'-OH oder den Peptiden H-$A_1$-$A_2$-OH, worin die Reste jeweils die oben genannten Bedeutungen haben, durch Kondensation analog dem unter Verfahren a) beschriebenen Verfahren hergestellt. Zur Herstellung von Verbindungen mit reduzierter Peptidbindung zwischen $A_1$ und $A_2$ wird die Peptidbindung zwischen $A_1$ und $A_2$, vorzugsweise auf der Stufe des Dipeptides, reduziert, beispielsweise mit Wasserstoff in Gegenwart von Schwermetall- oder Edelmetallkatalysatoren, wie Platin oder Palladium, gegebenenfalls auf Trägern, wie Aktivkohle, oder' durch komplexe Hydride, vorzugsweise durch komplexe Hydride, z. B. Lithiumaluminiumhydrid oder Disiamylboran in polaren Lösungsmitteln, wie Alkoholen, z. B. Ethanol, oder Ethern, wie cyclischen Ethern, z. B. Tetrahydrofuran, bei Temperaturen zwischen 0 und 150 °C, bevorzugt zwischen 20 °C und dem Siedepunkt des Reaktionsgemisches.

Das Amin der Formel XI ist bekannt, kommerziell erhältlich oder wird nach an sich bekannten Methoden hergestellt.

Verbindungen der Formel XXV sind bekannt oder nach an sich bekannten Verfahren herstellbar, oder sie sind kommerziell erhältlich.

Als Beispiel erwähnt sei die Herstellung einer Verbindung der Formel XXV, worin T durch Heterocyclylniederalkyl, worin Heterocyclyl über ein Ringstickstoffatom gebunden ist, substituiertes Arylcarbonyl bedeutet, welche vorzugsweise durch Umsetzung einer durch Halogenniederalkyl, wie Chlor- oder Brommethyl, substituierten Arylcarbonsäure, wie Chlormethylbenzoesäure oder Brommethylbenzoesäure, mit einer entsprechenden heterocyclischen Stickstoffbase, wie Piperidin, Piperazin, 1-Niederalkylpiperazin oder insbesondere Morpholin oder Thiomorpholin, unter nukleophiler Substitution des Halogenatomes erfolgt.

Verbindungen der Formel I können nach den in der Europäischen Patentanmeldung mit der Veröffentlichungsnummer EP 0 532 466 (veröffentlicht am 17. März 1993; ein englischssprachiges Äquivalent ist beispielsweise in Südafrika angemeldet) angegebenen Verfahren hergestellt werden. Diese europäische Patentanmeldung wird daher durch Bezugnahme in den vorliegenden Text aufgenommen.

Verbindungen der Formel I lassen sich vorzugsweise analog den Verbindungen der Formel I' nach einem

der Verfahren a) bis und mit f) herstellen, wenn man in Verfahren a) anstelle einer Verbindung der Formel III' eine Verbindung der Formel III,

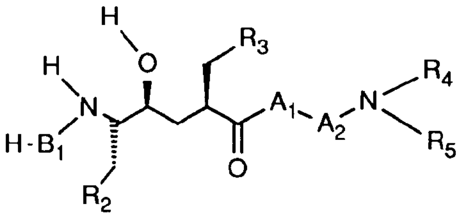

(III)

worin die Reste die für Verbindungen der Formel III' genannten Bedeutungen haben, unter Erhalt der Verbindung der Formel Ib, worin anstelle von T in einer Verbindung der Formel Ib' ein Wasserstoffatom steht, worin die übrigen Reste wie für Verbindungen der Formel Ib' definiert sind (herstellbar analog einer Verbindung der Formel III' aus einer Verbindung der Formel XXII unter Weglassen der Acylierung mit einer Verbindung der Formel XXV), oder in Verfahren b) anstelle einer Verbindung der Formel V' eine Verbindung der Formel V,

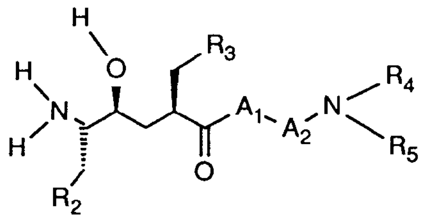

(V)

worin die Reste die für Verbindungen der Formel V' genannten Bedeutungen haben, unter Erhalt einer Verbindung der Formel Ic, worin anstelle von T in einer Verbindung der Formel Ic' ein Wasserstoffatom steht und die übrigen Reste wie für Verbindungen der Formel Ic' definiert sind (herstellbar analog einer Verbindung der Formel V' aus einer Verbindung der Formel XXII unter Weglassen der Acylierung mit einer Verbindung der Formel XXV), oder in Verfahren c) anstelle einer Verbindung der Formel VI' eine Verbindung der Formel VI,

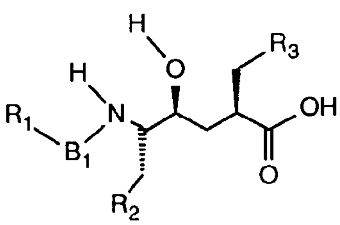

(VI)

worin die Reste die für Verbindungen der Formel VI' genannten Bedeutungen haben (herstellbar analog einer Verbindung der Formel VI' aus einer Verbindung der Formel XXII unter Weglassen der Acylierung mit einer Verbindung der Formel XXV), oder in Verfahren d) zur Herstellung einer Verbindung der Formel Id, worin anstelle von T in einer Verbindung der Formel Id' ein Wasserstoffatom steht und die übrigen Reste die für Verbindungen der Formel Id' genannten Bedeutungen haben, anstelle einer Verbindung der Formel VIII' eine Verbindung der Formel VIII,

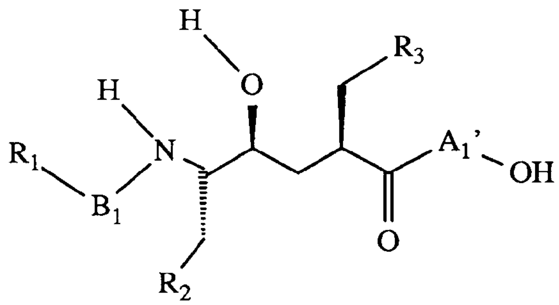

(VIII)

worin die Reste die für Verbindungen der Formel VIII' genannten Bedeutungen haben (herstellbar analog einer Verbindung der Formel VIII' aus einer Verbindung der Formel VI), oder in Verfahren e) anstelle einer Verbindung der Formel X' eine Verbindung der Formel X,

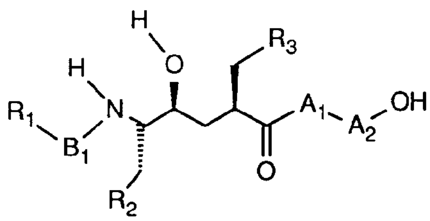

(X)

worin die Reste die für Verbindungen der Formel X' genannten Bedeutungen haben (herstellbar analog einer Verbindung der Formel X' aus einer Verbindung der Formel VI), oder in Verfahren f) anstelle einer Verbindung der Formel I', worin mindestens eine funktionelle Gruppe in geschützter Form vorliegt, eine Verbindung der Formel I mit mindestens einer geschützten funktionellen Gruppe unter Schutzgruppenabspaltung umsetzt.

Die Zwischenverbindungen der Formel I und ihre Salze, sofern salzbildende Gruppen vorliegen, sind, soweit sie neu sind, ebenfalls Gegenstand der vorliegenden Erfindung. Sie sind ebenfalls pharmazeutisch wirksam, wie oben für die aus den Verbindungen der Formel I' freisetzbaren Verbindungen der Formel I beschrieben, und können in pharmazeutischen Präparaten anstelle von Verbindungen der Formel I' vorliegen; die entsprechenden pharmazeutischen Präparate erhält man, indem man anstelle von Verbindungen der Formel I' oben bei der Beschreibung der pharmazeutischen Präparate solche der Formel I einsetzt. Somit sind die Verbindungen auch nützlich zur Behandlung von Krankheiten und können wie die Verbindungen der Formel I' in pharmazeutische Präparate eingearbeitet und zur Behandlung von retroviralen Erkrankungen, wie AIDS, insbesondere durch Hemmung der HIV-1- oder HIV-2-Protease, verwendet werden.

Vorzugsweise handelt es sich dabei um Verbindungen der Formel I,

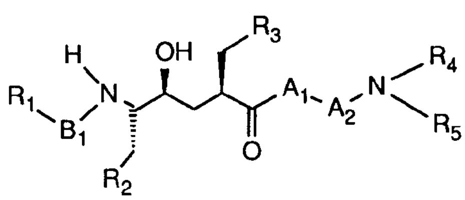

(I)

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, wie für Verbindungen der Formel I' definiert, insbesondere tert-Butoxycarbonyl; $R_2$ Phenyl, Cyclohexyl, Niederalkoxyphenyl, insbesondere o-, m- oder p-Methoxyphenyl, Benzyloxyphenyl, insbesondere p-Benzyloxyphenyl, p-Fluor-phenyl, p-Trifluormethylphenyl oder p-Hydroxyphenyl bedeutet, $R_3$ Phenyl, Niederalkoxyphenyl, insbesondere o-, m- oder p-Methoxyphenyl, p-Trifluormethylphenyl, o-, m- oder p-Cyanophenyl, Benzyloxyphenyl, insbesondere p-Benzyloxyphenyl, o-, m- oder p-Flu-

orphenyl oder Hydroxyphenyl bedeutet, $A_1$ das bivalente Radikal der Aminosäure (L)-Valin, (L)-Isoleucin oder Glyin bedeutet, welches N-terminal mit der Gruppe -C=O und C-terminal mit $A_2$ verbunden ist, $A_2$ das bivalente Radikal der Aminosäure Glycin, Alanin, Valin, Leucin, Isoleucin, Phenylalanin, Tyrosin, Cyclohexylalanin, p-Niederalkoxyphenylalanin, wie p-Methoxy-phenylalanin, p-Benzyloxyphenylalanin oder p-Fluorphenylalanin, welches N-terminal mit A1 und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino, insbesondere Morpholino, bedeuten,

mit der Massgabe, dass mindestens entweder einer der Reste $R_2$ und $R_3$ Benzyloxyphenyl bedeutet oder $A_2$ das bivalente Radikal von p-Benzyloxyphenylalanin bedeutet, während die übrigen Reste die genannten Bedeutungen haben, wenn entweder $R_3$ eine andere Bedeutung als o- oder m-Fluorphenyl, o- oder m-Cyanophenyl oder o- oder m-Methoxyphenyl hat, oder wenn $A_2$ eine andere Bedeutung als Alanin oder Leucin hat; oder Salze dieser Verbindungen, sofern salzbildende Gruppen vorliegen.

Bevorzugt hiervon sind die Verbindungen der Formel I, worin $R_1$ tert-Butoxycabonyl bedeutet, $R_2$ Phenyl, p-Benzyloxyphenyl oder o-, m- oder p-Methoxyphenyl bedeutet, $R_3$ Phenyl, p-Benzyloxyphenyl, o- m- oder p-Methoxyphenyl, o-, m- oder p-Fluorphenyl oder Hydroxyphenyl bedeutet, A1 das bivalente Radikal der Aminosäure (L)-Valin, (L)-Isoleucin oder Glyin, insbesondere von (L)-Valin, bedeutet, welches N-terminal mit der Gruppe -C=O und C-terminal mit $A_2$ verbunden ist, $A_2$ das bivalente Radikal der Aminosäure Glycin, Alanin, Valin, Leucin, Isoleucin, Phenylalanin, Tyrosin, Cyclohexylalanin, p-Niederalkoxyphenylalanin, wie p-Methoxy-phenylalanin, p-Benzyloxyphenylalanin oder p-Fluorphenylalanin, insbesondere von Phenylalanin, Tyrosin, p-Methoxy-phenylalanin, p-Benzyloxyphenylalanin oder p-Fluorphenylalanin, welches N-terminal mit $A_1$ und C-terminal mit der Gruppe $NR_4R_5$ verbunden ist, und $R_4$ und $R_5$ gemeinsam mit dem bindenden Stickstoffatom Morpholino bedeuten.

mit der Massgabe, dass mindestens entweder einer der Reste $R_2$ und $R_3$ Benzyloxyphenyl bedeutet oder $A_2$ das bivalente Radikal von p-Benzyloxyphenylalanin bedeutet, während die übrigen Reste die genannten Bedeutungen haben.

Besonders bevorzugt sind auch die Verbindungen der Formel I, worin $R_1$ tert-Butoxycarbonyl bedeutet, $R_2$ Phenyl bedeutet, $R_3$ o- oder m-Fluorphenyl, o- oder m-Cyanophenyl oder o- oder m-Niederalkoxy*phenyl*, wie o- oder m-Methoxy*phenyl*, bedeutet, $A_1$ das bivalente Radikal der Aminosäure (L)-Valin, (L)-Isoleucin oder Glyin, insbesondere von (L)-Valin, bedeutet, welches N-terminal mit der Gruppe -C=O und C-terminal mit $A_2$ verbunden ist, $A_2$ das bivalente Radikal der Aminosäure Glycin, Alanin, Valin, Leucin, Isoleucin, Phenylalanin, Tyrosin, Cyclohexylalanin, p-Niederalkoxyphenylalanin, wie p-Methoxy-phenylalanin, p-Benzyloxyphenylalanin oder p-Fluorphenylalanin, insbesondere von Phenylalanin, Tyrosin, p-Niederalkoxyphenylalanin, wie p-Methoxy-phenylalanin, p-Benzyloxyphenylalanin oder p-Fluorphenylalanin, welches N-terminal mit $A_1$ und C-terminal mit der Gruppe $NR_4R_5$ verbunden ist, und $R_4$ und $R_5$ gemeinsam mit dem bindenden Stickstoffatom Morpholino bedeuten.

Besonders bevorzugt ist eine Verbindung der Formel I mit der Bezeichnung
Boc-Phe[C](o-CN)Phe-(L)-Val-(L)-Phe-morpholin-4-ylamid,
Boc-Phe[C](m-CN)Phe-(L)-Val-(L)-Phe-morpholin-4-ylamid,
Boc-Phe[C](p-BzlO)Phe-(L)-Val-(L)-Phe-morpholin-4-ylamid,
Boc-Phe[C](p-BzlO)Phe-(L)-Val-(L)-(p-BzlOPhe)-morpholin-4-ylamid,
Boc-(p-BzlO)Phe[C]Phe-(L)-Val-(L)-Phe-morpholin-4-ylamid,
Boc-(p-BzlO)Phe[C]Phe-(L)-Val-(L)-(p-BzlOPhe)-morpholin-4-ylamid,
Boc-(p-BzlO)Phe[C](p-BzlO)Phe-(L)-Val-(L)-Phe-morpholin-4-ylamid,
Boc-(p-BzlO)Phe[C](p-BzlO)Phe-(L)-Val-(L)-(p-BzlOPhe)-morpholin-4-ylamid,
Boc-Phe[C](o-F)Phe-(L)-Val-(L)-Phe-morpholin-4-ylamid,
Boc-Phe[C](o-F)Phe-(L)-Val-(L)-(p-$CH_3$O-Phe)-morpholin-4-ylamid,
Boc-Phe[C](m-F)Phe-(L)-Val-(L)-Phe-morpholin-4-ylamid,
Boc-Phe[C](m-F)Phe-(L)-Val-(L)-(p-$CH_3$O-Phe)-morpholin-4-ylamid,
Boc-Phe[C]Phe-(L)-Val-(L)-Leu-morpholin-4-ylamid,
Boc-Phe[C]Phe-(L)-Val-(L)-Ala-morpholin-4-ylamid,
Boc-(p-$CH_3$O)Phe[C](p-BzlO)Phe-(L)-Val-(L)-Phe-morpholin-4-ylamid,
Boc-(p-$CH_3$O)Phe[C](p-BzlO)Phe-(L)-Val-(L)-(p-$CH_3$O-Phe)-morpholin-4-ylamid,
Boc-(p-$CH_3$O)Phe[C](p-BzlO)Phe-(L)-Val-(L)-Tyr-morpholin-4-ylamid,
Boc-(p-$CH_3$O)Phe[C](3-$CH_3$O)Phe-(L)-Val-(L)-Phe-morpholin-4-ylamid,
Boc-(p-$CH_3$O)Phe[C](2-$CH_3$O)Phe-(L)-Val-(L)-Phe-morpholin-4-ylamid,
Boc-Phe[C](3-$CH_3$O)Phe-(L)-Val-(L)-Phe-morpholin-4-ylamid,
Boc-Phe[C](2-$CH_3$O)Phe-(L)-Val-(L)-Phe-morpholin-4-ylamid,
Boc-Phe[C](3-$CH_3$O)Phe-(L)-Val-(L)-(p-$CH_3$O-Phe)-morpholin-4-ylamid,

Boc-Phe[C](2-$CH_3O$)Phe-(L)-Val-(L)-(p-$CH_3O$-Phe)-morpholin-4-ylamid oder
Boc-Phe[C](p-BzlO)Phe-(L)-Val-(L)-(p-$CH_3O$-Phe)-morpholin-4-ylamid
oder auch eine Verbindung mit der Bezeichnung
Boc-(p-$CH_3O$)Phe[C]Phe-(L)-Val-(L)-Phe-morpholin-4-ylamid (ganz besonders bevorzugt),
Boc-(p-$CH_3O$)Phe[C](p-$CH_3O$)Phe-(L)-Val-(L)-Phe-morpholin-4-ylamid (ganz besonders bevorzugt),
Boc-(p-$CH_3O$)Phe[C]Tyr-(L)-Val-(L)-Phe-morpholin-4-ylamid,
Boc-(p-$CH_3O$)Phe[C]Phe-(L)-Val-(L)-(p-$CH_3O$-Phe)-morpholin-4-ylamid,
Boc-(p-$CH_3O$)Phe[C]Phe-(L)-Val-(L)-Tyr-morpholin-4-ylamid,
Boc-(p-$CH_3O$)Phe[C](p-$CH_3O$)Phe-(L)-Val-(L)-(p-$CH_3O$-Phe)-morpholin-4-ylamid,
Boc-(p-$CH_3O$)Phe[C](p-$CH_3O$)Phe-(L)-Val-(L)-Tyr-morpholin-4-ylamid,
Boc-(p-$CH_3O$)Phe[C]Tyr-(L)-Val-(L)-(p-$CH_3O$-Phe)-morpholin-4-ylamid oder
Boc-(p-$CH_3O$)Phe[C]Tyr-(L)-Val-(L)-Tyr-morpholin-4-ylamid.

In ganz besonderer Weise bevorzugt ist schliesslich die Verbindung der Formel I mit der Bezeichnung Boc-Phe[c](p-$CH_3O$)Phe-(L)-Val-(L)-Phe-morpholin-4-ylamid (= 5(S)-(tert-Butoxycarbonylamino)-4(S)-hydroxy-2(R)-(p-methoxy-phenylmethyl)-6-phenyl-hexanoyl-(L)-valyl-(L)-phenyl-alanyl-morpholin-4-ylamid.

Eine Verbindung der Formel XXVI kann beispielsweise aus einer Verbindung der Formel I durch Umsetzung mit einer Carbonsäure der Formel

$$W_1\text{-}(C_mH_{2m})COOH \qquad (XXVII)$$

worin die Reste die für Verbindungen der Formel XXVI genannten Bedeutungen haben, oder einem reaktionsfahigen Säurederivat davon, welches anstelle der Carboxygruppe einen Rest -(C=O)-$Z_1$ trägt, worin $Z_1$ die für Verbindungen der Formel XXV' genannten Bedeutungen hat, unter Bedingungen analog den unter Verfahren g) genannten hergestellt werden.

Die Isocyanate der Formel XXV" lassen sich beispielsweise aus den entsprechenden Aminvorstufen durch Umwandlung der Aminogruppe in die Isocyanatogruppe herstellen, z.B. durch Umsetzung mit Phosgen in der Hitze, z.B. unter Rückflussbedingungen, oder durch Zutropfen des flüssigen oder in einem Lösungsmittel gelösten primären, sekundären oder tertiären Amins zu einem Überschuss von Phosgen in einem geeigneten Lösungsmittel (Toluol, Xylol, Ligroin, Chlorbenzen, α-Chlornaphthalen usw.) unter Kühlung (beispielsweise auf - 50 bis 0 °C), wobei sich intermediär eine Mischung aus Carbamoylchlorid und Aminhydrochlorid bildet, die dann bei erhöhter Temperatur (besipielsweise bei 50 ° C bis Rückflusstemperatur) weiter bis zur vollständigen Lösung phosgeniert wird, unter HCl-Eliminierung.

Die übrigen Ausgangsverbindungen sind bekannt, werden nach an sich bekannten Verfahren hergestellt und/oder sind käuflich.

Für alle vor- und nachstehend genannten Verfahren gilt allgemein folgendes:

Infolge der engen Beziehung zwischen den Verbindungen der Formel I' und ihren Salzen und Ausgangsmaterialien (Ausgangsstoffe und Zwischenprodukte) in freier Form und in Form ihrer Salze sind vor- und nachstehend unter den freien Verbindungen bzw. ihren Salzen sinn- und zweckmässig gegebenenfalls auch die entsprechenden Salze bzw. freien Verbindungen zu verstehen.

Alle vorstehend angeführten Verfahrensschritte können unter an sich bekannten, vorzugsweise den spezifisch genannten, Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, z. B. Ionenaustauschern, wie Kationenaustauschern, z. B. in der $H^+$-Form, je nach Art der Reaktion und/oder der Reaktionsteilnehmer bei erniedrigter, normaler oder erhöhter Temperatur, z.B. im Temperaturbereich von etwa -100°C bis etwa 190°C, vorzugsweise von etwa -80°C bis etwa 150°C, z.B. bei - 80 bis -60 °C, bei Raumtemperatur, bei - 20 bis 40 °C oder bei Rückflusstemperatur, unter atmosphärischem Druck oder in einem geschlossenen Gefäss, gegebenenfalls unter Druck, und/oder in einer inerten Atmosphäre, z.B. unter einer Argon- oder Stickstoffatmosphäre.

Auf allen Reaktionsstufen können auftretende Isomerengemische in die einzelnen Isomeren, z. B. Diastereomeren oder Enantiomeren, oder in beliebige Gemische von Isomeren, z. B. Racemate oder Diastereomerengemische, aufgetrennt werden, beispielsweise analog zu den Methoden, die unter den "Zusätzlichen Verfahrensmassnahmen" beschrieben sind.

In bestimmten Fällen, beispielsweise bei Hydrierungen, ist es möglich, stereoselektive Reaktionen zu erzielen, so dass z. B. eine erleichterte Gewinnung von einzelnen Isomeren möglich ist.

Zu den Lösungsmitteln, aus denen die für die jeweilige Reaktion geeigneten ausgewählt werden können, zählen beispielsweise Wasser, Ester, wie Niederalkylniederalkanoate, z. B. Essigsäurediethylester, Ether, wie aliphatische Ether, z. B. Diethylether, oder cyclische Ether, z. B. Tetrahydrofuran, flüssige aromatische Kohlenwasserstoffe, wie Benzol oder Toluol, Alkohole, wie Methanol, Ethanol oder 1- oder 2-Propanol, Nitrile, wie Acetonitril, Halogenkohlenwasserstoffe, wie Methylenchlorid, Säureamide, wie Dimethylformamid, Basen, wie

heterocyclische Stickstoffbasen, z. B. Pyridin, Carbonsäureanhydride, wie Niederalkansäureanhydride, z. B. Acetanhydrid, cyclische, lineare oder verzweigte Kohlenwasserstoffe, wie Cyclohexan, Hexan oder Isopentan, oder Gemische dieser Lösungsmittel, z. B. wässrige Lösungen, soweit bei der Beschreibung der Verfahren nichts anderes angegeben ist. Derartige Lösungsmittelgemische können auch bei der Aufarbeitung, beispielsweise durch Chromatographie oder Verteilung, Verwendung finden.

Die Verbindungen, einschliesslich ihrer Salze, können auch in Form von Hydraten erhalten werden, oder ihre Kristalle können z.B. das zur Kristallisation verwendete Lösungsmittel einschliessen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. in geschützter Form oder als Salz, verwendet wird, oder eine nach dem erfindungsgemässen Verfahren erhältliche Verbindung unter den Verfahrensbedingungen erzeugt und in situ weiterverarbeitet. Im Verfahren der vorliegenden Erfindung werden vorzugsweise solche Ausgangsstoffe eingesetzt, die zu den eingangs als besonders wertvoll beschriebenen Verbindungen führen. In erster Linie sind Reaktionsbedingungen bevorzugt, die den in den Beipielen genannten analog sind.

Soweit erforderlich, können auf allen Verfahrensstufen geschützte Ausgangsverbindungen eingesetzt und die Schutzgruppen auf geeigneten Reaktionstufen entfernt werden.

Schutzgruppen, ihre Einführung und ihre Freisetzung sind wie unter Verfahren a) und f) beschrieben.

Die folgenden Beispiele dienen zur Illustration der Erfindung, schränken deren Umfang jedoch in keiner Weise ein. Die anfangs aufgeführten Referenzbeispiele sind Ausgangsverbindungen (der Formel I) für die weiter unten aufgeführten Beispiele (der Formel I').

Bezugnahme auf "Referenz-Beispiele" bezieht sich entweder auf die unten spezifisch beschriebenen "Referenz-Beispiele" oder auf die in der europäischen Patentanmeldung EP 0 532 466 (publiziert am 17. März 1993, durch Bezugnahme in den vorliegenden Text aufgenommen) genannten Beispiele (die Nummer dieser Beispiele ist dann identisch mit der unten genannten "Referenz-Beispiel"-Nummer).

Temperaturen werden in Celsiusgraden (°C) angegeben. Sofern keine Temperatur angegeben ist, erfolgt die Reaktion bei Raumtemperatur. Die $R_f$-Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt:

DC-Laufmittelsysteme:

| | | |
|---|---|---|
| A | Hexan/Essigsäureethylester | 1:1 |
| B | Essigsäureethylester | - |
| C | Hexan/Essigsäureethylester | 4:1 |
| D | Hexan/Essigsäureethylester | 2:1 |
| E | Hexan/Essigsäureethylester | 3:1 |
| F | Methylenchlorid/Methanol | 9:1 |
| G | Chloroform/Methanol/Wasser/Eisessig | 85:13:1,5:0,5 |
| H | Essigsäureethylester/Methanol | 9:1 |

| | | |
|---|---|---|
| I | Hexan/Essigsäureethylester | 1:2 |
| J | Cloroform/Methanol/Essigsäure/Wasser | 75:27:5:0,5 |
| K | Essigsäureethylester/Essigsäure | 19:1 |
| L | Methylenchlorid/Methanol | 7:3 |
| M | Methylenchlorid/Ether | 49:1 |
| N | Methylenchlorid/Ether | 3:1 |
| O | Essigsäureethylester/THF | 9:1 |
| P | Hexan/Essigsäureethylester | 8:1 |
| Q | Methylenchlorid/Hexan/Ether | 10:10:1 |
| R | Methanol | - |
| S | Essigsäureethylester/Hexan | 3:1 |
| T | Essigsäureethylester/Ethanol | 97:3 |
| U | THF/Essigsäureethylester | 3:1 |
| V | Methylenchlorid/THF | 2:1 |
| W | Methylenchlorid/$^{i}$Propanol/ Methanol/Triethylamin | 8:3:3:1 |
| X | Acetonitril/Essigsäureethylester | 1:1 |
| Y | Essigsäureethylester/Ethanol | 95:5 |
| Z | Methylenchlorid/Methanol | 12:1 |
| A' | Methylenchlorid/Diethylether | 1:1 |
| B' | Methylenchlorid/Tetrahydrofuran | 4:1 |
| C' | Methylenchlorid/Tetrahydrofuran | 1:1 |

Zum Teil werden die oben genannten Buchstabencodes für DC-Mittel auch für die Angabe der Elutionsmittel bei Säulenchromatographie verwendet.

Die Abkürzung "$R_f(A)$" bedeutet beispielsweise, dass der $R_f$-Wert im Lösungsmittelsystem A ermittelt wurde. Das Mengenverhältnis von Lösungsmitteln zueinander ist stets in Volumenanteilen (v/v) angegeben. Auch bei der Definition der Fliessmittel-Systeme für die Säulenchromatographie werden die Mengenverhältnisse der verwendeten Lösungsmittel in Volumenanteilen (v/v) angegeben.

Die weiteren verwendeten Kurzbezeichnungen und Abkürzungen haben die folgenden Bedeutungen:

| | |
|---|---|
| abs. | absolut |
| atm | physikalische Atmosphären |
| Anal. | Elementaranalyse |
| ber. | berechnet |
| | (Druckeinheit) - 1 atm entspricht 1,013 bar |
| Boc | tert-Butoxycarbonyl |
| BOP | Benzotriazol-1-yl-oxy-tris-(di-methylamino)-phosphonium-hexafluor-phosphat |
| DC | Dünnschichtchromatographie |
| DCC | Dicyclohexylcarbodiimid |
| DEPC | Pyrocarbonsäurediethylester |
| DIPE | Diisopropylether |
| DMAP | 4-Dimethylaminopyridin |
| DMF | Dimethylformamid |
| DMPU | 1,3-Dimethyl-3,4,5,6-tetrahydro-2(1H)-pyrimidinon |
| DMSO | Dimethylsulfoxid |
| Essigester | Essigsäureethylester |
| Ether | Diethylether |

| | |
|---|---|
| FAB-MS | Fast-Atom-Bombardment-Massenspektroskopie |
| gef. | gefunden |
| ges. | gesättigt |
| h | Stunde(n) |
| HBTU | O-Benztriazol-1-yl-N,N,N',N'-tetramethyl-uronium-hexafluorphosphat |
| HOBt | 1-Hydroxy-benztriazol |
| HV | Hochvakuum |
| IR | Infrarotspektroskopie |
| min | Minute(n) |
| NMM | N-Methylmorpholin |
| org. | organisch |
| Pd/C | Palladium auf Aktivkohle (Katalysator) |
| $^{i}$Propanol | Isopropanol |
| RT | Raumtemperatur |
| RV | Rotationsverdampfer |
| Smp. | Schmelzpunkt |
| Sole | gesättigte Kochsalzlösung |
| TBAF | Tetrabutylammoniumfluorid (Trihydrat) |
| TFA | Trifluoressigsäure |
| THF | Tetrahydrofuran |
| Z | Benzyloxycarbonyl |

Massenspektroskopische Messwerte werden nach der "Fast-Atom-Bombardment" (FAB-MS)-Methode erhalten. Die Massenangaben beziehen sich auf das protonierte Molekülion $(M+H)^+$.

Die Werte für IR-Spektren werden in $cm^{-1}$ angegeben, in runden Klammern findet sich das jeweilige Lösungsmittel.

Zur Bezeichnung von bivalenten Radikalen von natürlichen α-Aminosäuren werden die in der Peptidchemie üblichen Abkürzungen verwendet. Die Konfiguration am α-Kohlenstoffatom wird durch Voranstellen von (L)- oder (D)- angegeben. -Cha- steht für Cyclohexylalanyl, -(p-F-Phe)- für in p-Stellung des Phenylringes mit Fluor, -(p-$CH_3$O-Phe)- für in p-Stellung des Phenylringes mit einer Methoxygruppe, -(p-BzlOPhe)- für in p-Stellung mit einer Benzyloxygruppe, -(p-CN-Phe)- für in p-Stellung des Phenylringes mit einer Cyanogruppe, -(4-n-butyloxy-Phe)- für in p-Stellung des Phenylringes durch n-Butoxy und -(4-isobutyloxy-Phe)- für in p-Stellung des Phenylringes durch Isobutoxy substituiertes Phenylalanyl.

HPLC-Gradienten:

| | |
|---|---|
| I | 20 % → 100 % a) in b) während 20 min. |
| II | 20 % → 100 % a) in b) während 35 min. |
| III | 20 % → 100 % a) in b) während 30 min. |
| IV | 20 % → 100 % a) in b) während 20 min und anschliessend 100 % a) während 8 min. |

Laufmittel a): Acetonitril + 0,05 % TFA; Laufmittel b): Wasser + 0,05 % TFA. Säule (250 x 4,6 mm) gefüllt mit "Reversed-Phase"-Material $C_{18}$-Nucleosil® (5 μm mittlere Korngrösse, mit Octadecylsilanen kovalent derivatisiertes Silicagel, Macherey & Nagel, Düren, BRD). Detektion durch UV-Absorption bei 215 nm. Die Retentionszeiten ($t_{Ret}$) werden in Minuten angegeben. Fliessgeschwindigkeit 1 ml/min.

Die folgenden Kurzbezeichnungen für Reste werden durch die entsprechenden Formelbilder und Bezeichnungen definiert:

Der Rest mit der Kurzbezeichnung -Phe[C]Phe- bedeutet das bivalente Radikal von 5(S)-Amino-2(R)-benzyl-4(S)-hydroxy-6-phenyl-hexansäure und hat die Formel

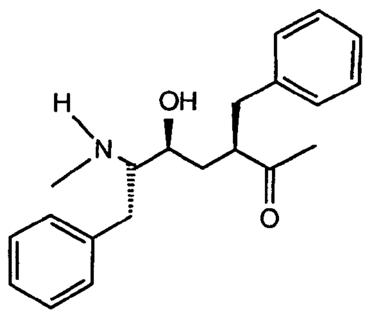

.

Der Rest mit der Kurzbezeichnung -Cha[C](p-CN)Phe- bedeutet das bivalente Radikal von 5(S)-Amino-2(R)-(p-cyanophenylmethyl)-6-cyclohexyl-4(S)-hydroxy-hexansäure und hat die Formel

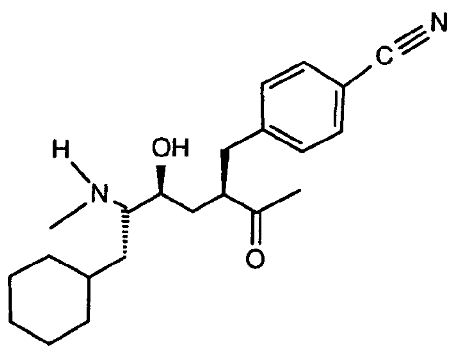

.

Der Rest mit der Kurzbezeichnung -Cha[C]Cha- bedeutet das bivalente Radikal von 5(S)-Amino-6-cyclohexyl-2(R)-cyclohexylmethyl-4(S)-hydroxy-hexansäure und hat die Formel

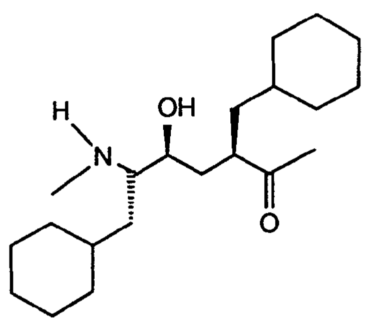

.

Der Rest mit der Kurzbezeichnung -Cha[C](p-F)Phe- bedeutet das bivalente Radikal von 5(S)-Amino-6-cyclohexyl-2(R)-(p-fluorphenylmethyl)4(S)-hydroxy-hexansäure und hat die Formel

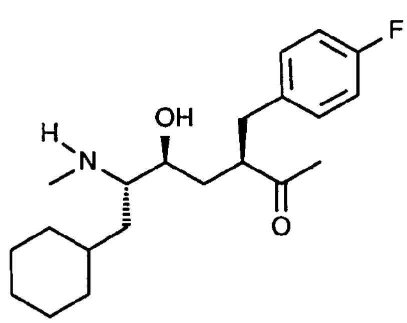

.

Der Rest mit der Kurzbezeichnung -(p-F)Phe[C]Phe- bedeutet das bivalente Radikal von 5(S)-Amino-2(R)-benzyl-6-(p-fluorphenyl)-4(S)-hydroxy-hexansäure und hat die Formel

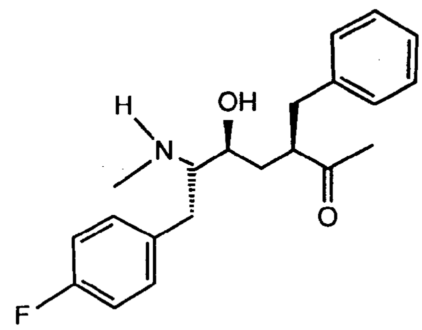

Die nachfolgend gezeigten weiteren Formelbilder der Zentralbausteine entsprechen den folgenden Kurzbezeichnungen, die in den nachfolgenden Beispielen verwendet werden:

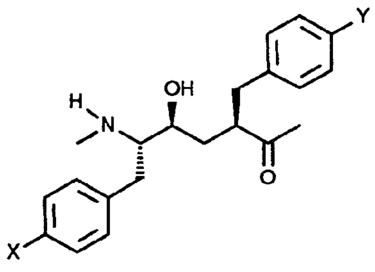

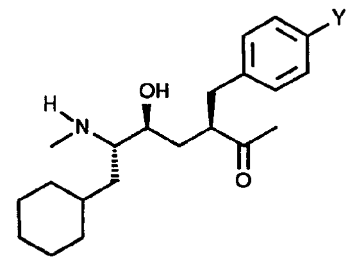

| | X | Y |
|---|---|---|
| -Phe[C](p-F)Phe- | H | F |
| -Phe[C](p-CN)Phe- | H | CN |
| -Phe[C](p-$CH_3O$)Phe- | H | $CH_3O$ |
| -Phe[C](p-$CF_3$)Phe- | H | $CF_3$ |
| -(p-F)Phe[C](p-F)Phe- | F | F |
| -(p-F)Phe[C](p-CN)Phe- | F | CN |
| -Tyr[C]Tyr- | OH | OH |
| -Tyr[C]Phe- | OH | H |
| -Phe[C]Tyr- | H | OH |
| -(p-BzlO)Phe[C](p-BzlO)Phe- | $C_6H_5CH_2O$ | $C_6H_5CH_2O$ |
| -(p-BzlO)Phe[C]Phe- | $C_6H_5CH_2O$ | H |
| -Phe[C](BzlO)Phe- | H | $C_6H_5CH_2O$ |
| -(p-$CH_3O$)Phe[C](p-$CH_3O$)Phe- | $CH_3$-O | $CH_3$-O |
| -(p-$CH_3O$)Phe[C]Phe- | $CH_3$-O | H |
| -(p-$CH_3O$)Phe[C](p-BzlO)Phe- | $CH_3$-O | $C_6H_5CH_2O$ |
| -(p-$CH_3O$)Phe[C]Tyr- | $CH_3$-O | OH |

| | Y |
|---|---|
| -Cha[C](p-$CH_3O$)Phe- | $CH_3O$ |
| -Cha[C](p-$CF_3$)Phe- | $CF_3$ |
| -Cha[C](p-Bzlo)Phe- | $C_6H_5$-$CH_2$-O |
| -Cha[C]Tyr- | OH |

Demnach entspricht -Phe[C](p-F)Phe- dem bivalenten Radikal von 5(S)-Amino-2(R)-(p-fluorphenylmethyl)-4(S)-hydroxy-6-phenyl-hexansäure; -Phe[C](p-CN)Phe- dem bivalenten Radikal von 5(S)-Amino-2(R)-(p-cyanophenylmethyl)-4(S)-hydroxy-6-phenyl-hexansäure; -Phe[C](p-$CH_3O$)Phe- dem bivalenten Radikal von 5(S)-Amino-4(S)-hydroxy-2(R)-(p-methoxyphenylmethyl)-6-phenyl-hexansäure; -Phe[C](p-$CF_3$)Phe- dem bivalenten Radikal von 5(S)-Amino-4(S)-hydroxy-6-phenyl-2(R)-(p-trifluormethylphenylmethyl)-hexansäure; -(p-F)Phe[C](p-F)Phe- dem bivalenten Radikal von 5(S)-Amino-6-(p-fluorphenyl)-2(R)-(p-fluorphenylmethyl)-4(S)-hydroxy-hexansäure; -(p-F)Phe[C](p-CN)Phe- dem bivalenten Radikal von 5(S)-Amino-2(R)-(p-cyanophenylmethyl)-6-(p-fluorphenyl)-4(S)-hydroxy-hexansäure; -Cha[C](p-$CH_3O$)Phe- dem bivalenten Radikal von 5(S)-Amino-2(R)-(p-methoxyphenylmethyl)-6-cyclohexyl-4(S)-hydroxy-hexansäure; -Cha[C](p-$CF_3$)Phe- dem bivalenten Radikal von 5(S)-Amino-6-cyclohexyl-4(S)-hydroxy-2(R)-(p-trifluormethylphenyl-

methyl)-hexansäure; -Tyr[C]Tyr- dem bivalenten Radikal von 5(S)-Amino-4(S)-hydroxy-6-(p-hydroxyphenyl)-2(R)-(p-hydroxyphenylmethyl)-hexansäure; -Phe[C]Tyr- dem bivalenten Radikal von 5(S)-Amino-4(S)-hydroxy-2(R)-(p-hydroxyphenylmethyl)-6-phenylhexansäure-; -Tyr[C]Phe- dem bivalenten Radikal von 5(S)-Amino-4(S)-hydroxy-6-(p-hydroxyphenyl)-2(R)-benzyl-hexansäure; -(p-BzlO)Phe[C](p-BzlO)Phe- dem bivalenten Radikal von 5(S)-Amino-6-(p-benzyloxyphenyl)-2(R)-(p-benzyloxyphenylmethyl)-4(S)-hydroxy-hexansäure; -(p-BzlO)Phe[C]Phe- dem bivalenten Radikal von 5(S)-Amino-6-(p-benzyloxyphenyl)-4(S)-hydroxy-2(R)-benzyl-hexansäure; -Phe[C](p-BzlO)Phe- dem bivalenten Radikal von 5(S)-Amino-2(R)-(p-benzyloxyphenylmethyl)-4(S)-hydroxy-6-phenyl-hexansäure; -(p-$CH_3O$)Phe[C](p-$CH_3O$)Phe- dem bivalenten Radikal von 5(S)-Amino-4(S)-hydroxy-6-(p-methoxyphenyl)-2(R)-(p-methoxyphenylmethyl)-hexansäure; -(p-$CH_3O$)Phe[C]Phe- dem bivalenten Radikal von 5(S)-Amino-4(S)-hydroxy-6-(p-methoxyphenyl)-2(R)-phenylmethyl-hexansäure; -(p-$CH_3O$-)Phe[C](p-BzlO)Phe- dem bivalenten Radikal von 5(S)-Amino-2(R)-(p-benzyloxyphenylmethyl)-4(S)-hydroxy-6-(p-methoxyphenyl)-hexansäure; und -(p-$CH_3O$)Phe[C]Tyr- dem bivalenten Radikal von 5(S)Amino-4(S)-hydroxy-2(R)-(p-hydroxyphenylmethyl)-6-(p-methoxyphenyl)-hexansäure.

-Cha[C](p-BzlO)Phe- bedeutet das bivalente Radikal von 5(S)-Amino-2(R)-(p-benzyloxybenzyl)-6-cyclohexyl-2(R)-4(S)-hydroxy-hexansäure; -Cha[C]Tyr- bedeutet entsprechend das bivalente Radikal von 5(S)-Amino-6-cyclohexyl-4(S)-hydroxy-2(R)-(4-hydroxybenzyl)-hexansäure.

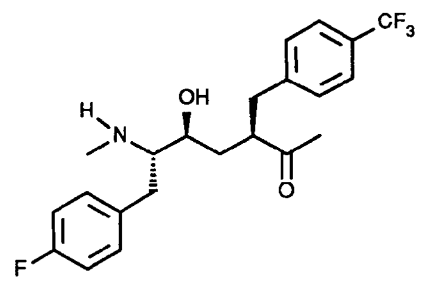

**-(p-F)Phe[C](p-$CF_3$)Phe-**

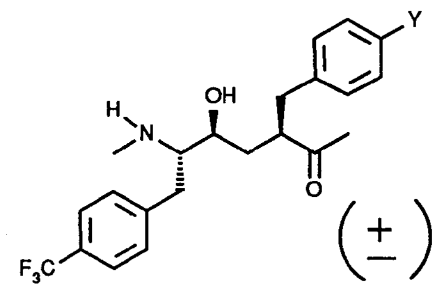

| | **Y** |
|---|---|
| **-($CF_3$)Phe[C]Phe-** | **H** |
| **-($CF_3$)Phe[C](p-F)Phe-** | **F** |
| **-($CF_3$)Phe[C](p-$CF_3$)Phe-** | **$CF_3$** |

Der Rest -(p-F)Phe[C](p-$CF_3$)Phe- entspricht demnach dem bivalenten Radikal von 5(S)-Amino-4(S)-hydroxy-6-(p-fluorphenyl)-2(R)-(p-trifluormethylphenylmethyl)-hexansäure.

Das Symbol (±) soll zum Ausdruck bringen, dass die Reste -($CF_3$)Phe[C]Phe-, -($CF_3$)Phe[C](p-F)Phe- und -($CF_3$)Phe[C](p-$CF_3$)Phe-, welche den bivalenten Radikalen von 5-Amino-2-phenyl-4-hydroxy-6-(p-trifluormethylphenylmethyl)-hexansäure, 5-Amino-2-(p-fluorphenylmethyl)-4-hydroxy-6-(p-trifluormethylphenyl)-hexansäure und 5-Amino-2-(p-trifluormethylphenylmethyl)4-hydroxy-6-(p-trifluormethylphenyl)-hexansäure entsprechen, in den entsprechenden Beispielen als Gemisch des 2(R),4(S),5(S)-Isomeren mit dem 2(S),4(R),5(R)-Isomeren vorliegen können.

Weitere Bedeutungen von entsprechenden bivalenten Resten sind: -Phe[C](m-CN)Phe- bedeutet das bivalente Radikal von
5(S)-Amino-2(R)-(m-cyanophenylmethyl)-4(S)-hydroxy-6-phenyl-hexansäure; -Phe[C](o-CN)Phe- bedeutet das bivalente Radikal von
5(S)-Amino-2(R)-(m-cyanophenylmethyl)-4(S)-hydroxy-6-phenyl-hexansäure; -Phe[C](o-F)Phe- bedeutet das bivalente Radikal von
5(S)-Amino-2(R)-(o-fluorphenylmethyl)-4(S)-hydroxy-6-phenyl-hexansäure; -Phe[C](m-F)Phe- bedeutet das bivalente Radikal von
5(S)-Amino-2(R)-(m-fluorphenylmethyl)-4(S)-hydroxy-6-phenyl-hexansäure; -(p-$CH_3O$)Phe[C](3-$CH_3O$)Phe- bedeutet das bivalente Radikal von
5(S)-Amino-2(R)-(m-methoxyphenylmethyl)-4(S)-hydroxy-6-(p-methoxyphenyl)-hexansäure; -(p-$CH_3O$)Phe[C]

(2-$CH_3$O)Phe- bedeutet das bivalente Radikal von 5(S)-Amino-2(R)-(o-methoxyphenylmethyl)-4(S)-hydroxy-6-(p-methoxyphenyl)-hexansäure; -Phe[C](3-$CH_3$O) Phe- bedeutet das bivalente Radikal von 5(S)-Amino-2(R)-(m-methoxyphenylmethyl)-4(S)-hydroxy-6-phenyl-hexansäure; und -Phe[C](2-$CH_3$O)Phe- bedeutet das bivalente Radikal von 5(S)-Amino-2(R)-(o-methoxyphenylmethyl)-4(S)-hydroxy-6-phenyl-hexansäure.

Schliesslich entspricht -Phe[C](p-isobutyloxy)Phe- dem bivalenten Radikal von 5(S)-Amino-4(S)-hydroxy-2(R)-(p-isobutyloxybenzyl)-6-phenyl-hexansäure; -Phe[C](3,4-dimethoxy)Phe- dem bivalenten Radikal von 5(S)-Amino-2(R)-(3,4-dimethoxybenzyl)-4(S)-hydroxy-6-phenyl-hexansäure; -Phe[C](3,4,5-trimethoxy)Phe- dem bivalenten Radikal von 5(S)-Amino-4(S)-hydroxy-6-phenyl-2(R)-(3,4,5-trimethoxybenzyl)-hexansäure und -Phe[C](2,3,4-trimethoxy)Phe- dem bivalenten Radikal von 5(S)-Amino-4(S)-hydroxy-6-phenyl-2(R)-(2,3,4-trimethoxybenzyl)-hexansäure.

**Referenzbeispiele** (diese werden nachstehend nur dann mit Herstellungsbeschreibung genannt, wenn eine zusätzliche Synthese beschrieben wird):

Die Referenzbeispiele 1 bis 41 und die Beschreibung ihrer Herstellung (wobei gegebenenfalls eine neue Synthese angegeben wird) finden sich in der oben genannten EP 0 532 466. Es sind dies:

**Referenz-Beispiel 1: Boc-Cha[C](p-F)Phe-(L)-Val-(L)-Phe-morpholin-4-ylamid,**

**Referenz-Beispiel 2: Boc-Phe[C]Phe-(L)-Val-(L)-Phe-morpholin-4-ylamid** (Anmerkung: Das in Referenzbeispiel 2 b) als erste Verbindung genannte **5(S)-[1(S)-(Boc-amino)-2-phenylethyl]-3(R)-phenylmethyl-dihydrofuran2-(3H)on** wird vorzugsweise folgendermassen hergestellt (Herstellungsmethode, welche auch für grössere Mengen geeignet ist):

i) 5(S)-[1(S)-(Boc-amino)-2-phenylethyl]-dihydrofuran-2-(3H)-on (A.E. DeCamp, A.T. Kawaguchi, R.P. Volante, and I. Shinkai, Tetrahedron Lett. 32, 1867 (1991)). Unter $N_2$-Atmosphäre gibt man zu einer Lösung von 375 g (1,65 Mol) 3-Jodpropionsäureethylester [Referenz-Beispiel 21 D)1)a)] in 1700 ml Toluol 173 g Zn/Cu (Herstellung: R.D. Smith, H.E. Simmons, W.E. Parham, M.D. Bhavsar, Org. Synth., Coll. Vol 5, 855 (1973)) und 280 ml Dimethylacetamid und rührt kräftig während 1 h bei RT und 4 h bei 80°C nach (→ Zn-Homoenolatlösung). In einer zweiten Apparatur ($N_2$-Atmosphäre) wird eine Lösung von 122 ml (0,40 Mol) Tetraisopropyl-orthotitanat in 350 ml Toluol und 1900 ml Methylenchlorid unter leichtem Kühlen bei einer Innentemperatur von 15 bis 25°C mit 127 ml (1,14 Mol) Titan-tetrachlorid versetzt, 15 min bei RT gerührt (→ gelbe Lösung) und auf -40°C abgekühlt (→ teilweise Kristallisation des Trichlor-titan-isopropylats). Die auf RT abgekühlte Zn-Homoenolatlösung filtriert man unter Argonatmosphäre durch eine G3-Glasfritte und tropft sie zum Trichlor-titan-isopropylat, wobei die Temperatur bei -30°C bis -25°C gehalten wird (→ tiefrote Lösung), rührt während 5 min bei -25°C und kühlt auf -40°C ab. Anschliessend tropft man eine Lösung von 233 g (0,85 Mol) N-Boc-phenylalaninal (Herstellung: D.J. Kempf, J. Org. Chem. 51, 3921 (1986), dann Kristallisation aus Hexan (0°C, ca. 18 h), Waschen mit kaltem Hexan, Trocknen) in 1500 ml Methylenchlorid zu und rührt 15 h bei -22 bis -18°C und schliesslich 1 h bei 0°C nach. Das Reaktionsgemisch wird in 10 l Eiswasser und 12 l tert-Butylmethylether aufgenommen und 7-10 min kräftig gerührt. Man trennt die wässrige Phase ab; extrahiert sie 2x mit 10 l Ether; wäscht die organischen Phasen mit 8 l Wasser, 8 l ges. Natriumhydrogencarbonat-Lösung, 8 l Wasser und 5 l Sole; trocknet sie mit Natriumsulfat und dampft sie ein (→ kristalliner 5(S)-(Boc-amino)-4(S)-hydroxy-6-phenyl-hexansäure-ethylester). Das obige Zwischenprodukt wird in 6500 ml Toluol und 230 ml Essigsäure unter Argonatmosphäre während 2,5 h auf 100°C erhitzt. Man giesst das erkaltete Reaktionsgemisch unter Rühren auf 6 l Eiswasser, trennt die wässrige Phase ab, extrahiert sie 2x mit 2000 ml Toluol, wäscht die org. Phasen mit 5 l ges. Natriumhydrogencarbonat Lösung, 5 140 %-iger Natriumhydrogensulfit Lösung, 41 Wasser und 4 l Sole und trocknet sie mit Natriumsulfat. Eindampfen der org. Phasen bis zu einem Rückstand von ca. 300 g und Versetzen mit 800 ml Hexan (mehrere Stunden ausrühren) liefert kristallines Lacton, das laut HPLC ca. 10 % des (5R)-Epimeren (DC $R_f$(E)=0,08; $t_{Ret}$(II)=18,8 min) enthält. Dieses Material wird in der nächsten Stufe eingesetzt. Die reine Titelverbindung kann nach Säulenchromatographie ($SiO_2$, Hexan/Essigsäureethylester 2:1) erhalten werden: DC $R_f$(E)=0,14; $t_{Ret}$(II)=19,2 min; $[\alpha]^D$=17,7° (c=1; Ethanol).

*ii)* **5(S)-[1(S)-(Boc-amino)-2-phenylethyl]-3(R)-phenylmethyl-dihydrofuran-2-(3H)-on** (A.K. Ghosh, S.P. McKee, and W.J. Thompson, J. Org. Chem. 56, 6500 (1991)). Unter $N_2$-Atmosphäre wird eine Lösung von 1943 g (6,32 Mol) 5(S)-[1(S)-(Boc-amino)-2-phenylethyl]-dihydrofuran-2-(3H)-on in 12,01 THF und 1,9 l 1,3-Dimethyl-3,4,5,6-tetrahydro-2(1H)-pyrimidinon auf -75°C abgekühlt, bei einer Innentemperatur von unter -70°C mit 14000 ml Lithiumbis(trimethylsilyl)amid (1 M) in THF (Aldrich) versetzt und 20 min bei -75°C nachgerührt. Dazu tropft man während 1 h 835 ml (7,00 Mol) Benzylbromid, wobei die Innentemperatur -70°C

nicht übersteigen darf, und rührt während 30 min bei -75°C aus. Zur klaren Lösung gibt man anschliessend bei -75 bis -70°C 2320 ml Propionsäure (90 min) und dann 2320 ml Wasser (1 h), wobei man die Temperatur auf -10°C ansteigen lässt. Das Reaktionsgemisch wird auf 30 l Essigsäureethylester und 35 1 10 %-ige Zitronensäure-Lösung gegossen, die wässrige Phase abgetrennt und mit 2x 101 Essigsäureethylester nachextrahiert. Die organischen Phasen wäscht man mit 3x 12 l ges. Natriumbicarbonat Lösung, 201 Sole und 2x 20 l Wasser, engt sie ein, nimmt den öligen Rückstand in 101 Toluol auf und dampft bis zu einem Restvolumen von ca. 5 l ein. Filtrieren des Eindampfrückstandes durch 4 kg Kieselgel Merck (0,063-0,200 mm), Nachwaschen mit Toluol und Kristallisieren des Rohprodukts aus Hexan (4 1 Hexan/kg Rohprodukt) liefert die Titelverbindung: DC $R_f$(D)=0,54; FAB-MS $(M+H)^+$=414.]),

**Referenz-Beispiel 3: Boc-Cha[C](p-CN)Phe-(L)-Val-(L)-Phe-morpholin-4-ylamid,**
**Referenz-Beispiel 4: H-Phe[C]Phe-(L)-Val-(L)-Phe-morpholin-4-ylamid,**
**Referenz-Beispiel 5: 3-Benzofuranoyl-Phe[C]Phe-(L)-Val-(L)-Phe-morpholin-4-ylamid,**
**Referenz-Beispiel 6: Nicotinoyl-Phe[C]Phe-(L)-Val-(L)-Phe-morpholin-4-ylamid,**
**Referenz-Beispiel 7: Morpholinocarbonyl-Val-Phe[C]Phe-(L)-Val-(L)-Phe-morpholin-4- ylamid,**
**Referenz-Beispiel 8: Boc-Cha[C]Cha-(L)-Val-(L)-Cha-morpholin-4-ylamid,**
**Referenz-Beispiel 9: Boc-Cha[C](p-F)Phe-(L)-Val-(L)-(p-F-Phe)-morpholin-4-ylamid,**
**Referenz-Beispiel 10: Boc-Cha[C](p-F)Phe-(L)-Val-(L)-(p-$CH_3O$-Phe)-morpholin-4-ylami d,**
**Referenz-Beispiel 11: Boc-Cha[C](p-F)Phe-(L)-Val-(L)-Cha-morpholin-4-ylamid,**
**Referenz-Beispiel 12: 1,2,3,4-Tetrahydroisochinolin-3(S)-carbonyl-Val-Phe[C]Phe-(L) - Val-(L)-Phe-morpholin-4-ylamid,**
**Referenz-Beispiel 13: Boc-Phe[C]Phe-(L)-Val-(L)-(p-F-Phe)-morpholin-4-ylamid,**
**Referenz-Beispiel 14: Boc-Phe[C]Phe-(L)-Val-(L)-(p-$CH_3O$-Phe)-morpholin-4-ylamid,**
**Referenz-Beispiel 15: Boc-Phe[C]Phe-(L)-Val-(L)-Cha-morpholin-4-ylamid,**
**Referenz-Beispiel 16: Boc-Phe[C]Phe-(L)-Ile-(L)-Phe-morpholin-4-ylamid,**
**Referenz-Beispiel 17: Boc-Phe[C]Phe-(L)-Val-Gly-morpholin-4-ylamid,**
**Referenz-Beispiel 18: Boc-Phe[C]Phe-(L)-Ile-Gly-morpholin-4-ylamid,**
**Referenz-Beispiel 19: Boc-Phe[C]Phe-(L)-Val-(L)-Val-morpholin-4-ylamid,**
**Referenz-Beispiel 20: Boc-Phe[C]Phe-(L)-Val-(L)-Phe-thiomorpholin-4-ylamid,**
**Referenz-Beispiel 21:**

**A)** **1) eine der nachstehend unter B) bis J) genannten Verbindungen, worin anstelle von -morpholin-4-ylamid der Rest -thiomorpholin-4-ylamid steht;**

**B)**

**1) Boc-(p-F)Phe[C](p-F)Phe-(L)-Val-(L)-Phe-morpholin-4-ylamid,**
**2) Boc-(p-F)Phe[C](p-F)Phe-(L)-Val-(L)-(p-F-Phe)-morpholin-4-ylamid,**
**3) Boc-(p-F)Phe[C](p-F)Phe-(L)-Val-(L)-(p-$CH_3O$-Phe)-morpholin-4-ylamid,**
**4) Boc-(p-F)Phe[C](p-F)Phe-(L)-Val-(L)-Cha-morpholin-4-ylamid,**
**5) Boc-(p-F)Phe[C](p-F)Phe-(L)-Ile-(L)-Phe-morpholin-4-ylamid,**

**C)**

**1) Boc-(p-F)Phe[C](p-CN)Phe-(L)-Val-(L)-Phe-morpholin-4-ylamid,**
**2) Boc-(p-F)Phe[C](p-CN)Phe-(L)-Val-(L)-(p-F-Phe)-morpholin-4-ylamid,**
**3) Boc-(p-F)Phe[C](p-CN)Phe-(L)-Val-(L)-(p-$CH_3O$-Phe)-morpholin-4-ylamid,**
**4) Boc-(p-F)Phe[C](p-CN)Phe-(L)-Val-(L)-Cha-morpholin-4-ylamid,**
**5) Boc-(p-F)Phe[C](p-CN)Phe-(L)-Ile-(L)-Phe-morpholin-4-ylamid,**

**und**

**D)**

**1) Boc-Phe[C](p-F)Phe-(L)-Val-(L)-Phe-morpholin-4-ylamid.**

**2) Boc-Phe[C](p-F)Phe-(L)-Val-(L)-(p-F-Phe)-morpholin-4 -ylamid**

Analog Ref.-Bsp. 1) werden 350 mg (0,395 mMol) 5(S)-(Boc-amino)-4(S)-(tert-butyldimethylsilyloxy)-6-phenyl-2(R)-(p-fluor-phenylmethyl)-hexanoyl-(L)-Val-(L)-(p-F-Phe)-morpholin-4-ylamid mit 374 mg (1,19 mMol) TBAF in 3 ml DMF zur Titelverbindung entschützt: $t_{Ret}$(II)=23,3 min; FAB-MS $(M+H)^+$=765.

Das Ausgangsmaterial wird wie folgt hergestellt:

**2) a) 5(S)-(Boc-amino)-4(S)-(tert-butyldimethylsilyloxy)-6-phenyl-2(R)-(p-fluor-phenylmethyl)-hexanoyl-(L)-Val-(L)-(p-F-Phe)-morpholin-4-ylamid**

Analog Ref.-Bsp. 9f) werden 265 mg (0,485 mMol) 5(S)-(Boc-amino)-4(S)-(tert-butyldimethylsilyloxy)-6-phenyl-2(R)-(p-fluorphenylmethyl)-hexansäure [Ref.-Bsp. 21 D) 1)e)] und 188 mg (0,53 mMol) H-(L)-Val-(L)-(p-F-Phe)-morpholin-4-ylamid (Ref.-Bsp. 9e) in 4,6 ml NMM/$CH_3CN$ 0,25 M mit 202,6 mg (0,53 mMol) HBTU umgesetzt: DC $R_f$(D)=0,28; $t_{Ret}$(II)=33,4 min.

**3) Boc-Phe[C](p-F)Phe-(L)-Val-(L)-(p-$CH_3O$-Phe)-morpholin-4-ylamid**

Analog Ref.-Bsp. 1) werden 280 mg (0,312 mMol) 5(S)-(Boc-amino)-4(S)-(tert-butyldimethylsilyloxy)-6-phenyl-2(R)-(p-fluor-phenylmethyl)-hexanoyl-(L)-Val-(L)-(p-$CH_3O$-Phe)-morpholin-4-ylamid mit 295 mg (0,94 mMol) TBAF in 3 ml DMF zur Titelverbindung entschützt.
Säulenchromatographie ($SiO_2$, Essigsäureethylester/Hexan 4:1 → Essigsäureethylester) liefert die reine Titelverbindung: DC $R_f$(B)=0,56; $t_{Ret}$(II)=23,1 min; FAB-MS $(M+H)^+$=777.
Das Ausgangsmaterial wird wie folgt hergestellt:

**3) a) 5(S)-(Boc-amino)-4(S)-(tert-butyldimethylsilyloxy)-6-phenyl-2(R)-(p-fluor-phenylmethyl)-hexanoyl-(L)-Val-(L)-(p-$CH_3O$-Phe)-morpholin-4-ylamid**

Analog Ref.-Bsp. 9f) werden 200 mg (0,366 mMol) 5(S)-(Boc-amino)-4(S)-(tert-butyldimethylsilyloxy)-6-phenyl-2(R)-(p-fluorphenylmethyl)-hexansäure [Ref.-Bsp. 21 D) 1)e)] und 146 mg (0,402 mMol) H-(L)-Val-(L)-(p-$CH_3O$-Phe)-morpholin-4-ylamid (Ref.-Bsp. 10e) in 3,6 ml NMM/CH3CN 0,25 M mit 153 mg (0,402 mMol) HBTU umgesetzt: DC $R_f$(D)=0,22; $t_{Ret}$(II)=33,1 min.

**4) Boc-Phe[C](p-F)Phe-(L)-Val-(L)-Cha-morpholin-4-ylamid**

(beschrieben in EP 0 532 466)

**5) Boc-Phe[C](p-F)Phe-(L)-Ile-(L)-Phe-morpholin-4-ylamid**

Analog Ref.-Bsp. 1) werden 220 mg (0,251 mMol) 5(S)-(Boc-amino)-4(S)-(tert-butyldimethylsilyloxy)-6-phenyl-2(R)-(p-fluor-phenylmethyl)-hexanoyl-(L)-Ile-(L)-Phe-morpholin-4-ylamid mit 240 mg (0,75 mMol) TBAF in 3 ml DMF zur Titelverbindung entschützt. Säulenchromatographie ($SiO_2$, Essigsäureethylester/THF 9:1) liefert die reine Titelverbindung: DC $R_f$(O)=0,3; $t_{Ret}$(II)=23,9 min; FAB-MS $(M+H)^+$=761.
Das Ausgangsmaterial wird wie folgt hergestellt:

**5) a) 5(S)-(Boc-amino)-4(S)-(tert-butyldimethylsilyloxy)-6-phenyl-2(R)-(p-fluor-phenylmethyl)-hexanoyl-(L)-Ile-(L)-Phe-morpholin-4-ylamid**

Analog Ref.-Bsp. 9f) werden 200 mg (0,366 mMol) 5(S)-(Boc-amino)-4(S)-(tert-butyldimethylsilyloxy)-6-phenyl-2(R)-(p-fluorphenylmethyl)-hexansäure [Ref.-Bsp. 21 D) 1)e)] und 140 mg (0,403 mMol) H-(L)-Ile-(L)-Phe-morpholin-4-ylamid (Ref.-Bsp. 16b) in 3,5 ml NMM/$CH_3CN$ 0,25 M mit 153 mg (0,40 mMol) HBTU umgesetzt: DC $R_f$(D)=0,16; $t_{Ret}$(II) =34,4 min.

E)

**1) Boc-Phe[C](p-CN)Phe-(L)-Val-(L)-Phe-morpholin-4-ylamid**

(beschrieben in EP 0 532 466)

**2)** Boc-Phe[C](p-CN)Phe-(L)-Val-(L)-(p-F-Phe)-morpholin-4-ylamid
(beschrieben in EP 0 532 466)

**3)** Boc-Phe[C](p-CN)Phe-(L)-Val-(L)-(p-$CH_3O$-Phe)-morpholin-4-ylamid

**4)** Boc-Phe[C](p-CN)Phe-(L)-Val-(L)-Cha-morpholin-4-ylamid

**5) Boc-Phe[C](p-CN)Phe-(L)-Ile-(L)-Phe-morpholin-4-ylamid**

(beschrieben in EP 0 532 466)

F)

**1) Boc-Phe[C](p-$CH_3O$)Phe-(L)-Val-(L)-(Phe-morpholin-4-ylamid**

Analog Ref.-Bsp. 1) werden 417 mg (0,48 mMol) 5(S)-(Boc-amino)-4(S)-(tert-butyldimethylsilyloxy)-6-phenyl-2(R)-(p-methoxy-phenylmethyl)-hexanoyl-(L)-Val-(L)-Phe-morpholin-4-ylamid mit 301 mg (0,95 mMol) TBAF in 5 ml DMF zur Titelverbindung entschützt: DC $R_f$(F)=0,4; $t_{Ret}$(I)=15,8 min; FAB-MS $(M+H)^+$=759.
Das Ausgangsmaterial wird wie folgt hergestellt:

**1) a) p-Methoxy-benzyljodid**

Eine Lösung von 1,7 ml (12,8 mMol) 4-Methoxy-benzylchlorid (Fluka; Buchs/Schweiz) in 25 ml Aceton wird mit 9,4 g (62,6 mMol) Natriumjodid bei RT gerührt. Ein Gaschromatogramm des Reaktionsgemisches nach 90 min zeigt vollständigen Umsatz an, deshalb giesst man das Reaktionsgemisch auf Ether und wäscht es mit 10-%iger Natriumthiosulfat Lösung und Sole. Trocknen der organischen Phase mit $Na_2SO_4$ und Eindampfen liefert die Titelverbindung: $^1$H-NMR (200 MHz, $CD_3OD$: 3,78 (s, 3 H), 4,54 (s, 2 H), 6,8-6,95 und 7,2-7,4 (2m, je 2 H).

**1) b) 5(S)-[1(S)-(Boc-amino)-2-phenyl-ethyl]-3(R)-(p-methoxy-phenylmethyl)-dihydrofuran-2-(3H)-on**

Analog Ref.-Bsp. 21 D) 1)c) werden 2,98 g (9,74 mMol) 5(S)-[1(S)-(Boc-amino)-2-phenylethyl]-dihydrofuran-2-(3H)-on[Ref.-Bsp. 21 D) 1)b)] gelöst in 40 ml THF bei -75°C mit 19,5 ml Lithium-bis-(trimethylsilyl)amid 1 M in THF deprotoniert und mit 2,9 g (11,7 mMol) p-Methoxy-benzyljodid in 20 ml THF alkyliert (45 min). Säulenchromatographie ($SiO_2$, Hexan/Essigsäureethylester 2:1) und Digerieren aus DIPE liefert die reine Titelverbindung: DC $R_f$(D)=0,32; $t_{Ret}$(I)=16,7 min.

**1) c) 5(S)-(Boc-amino)-4(S)-hydroxy-6-phenyl-2(R)-(p-methoxy-phenylmethyl)-hexansäure**

Analog Ref.-Bsp. 1i) werden 1,7 g (3,99 mMol) 5(S)-[1(S)-(Boc-amino)-2-phenylethyl]-3(R)-(p-methoxy-phenylmethyl)-dihydrofuran-2(3H)-on in 43 ml Dimethoxyethan und 11 ml Wasser mit 16 ml 1 M Lithiumhydroxid Lösung hydrolysiert. Verrühren in Ether liefert die reine Titelverbindung: DC $R_f$(F)=0,53; $t_{Ret}$(I)=14,2 min; FAB-MS $(M+Na)^+$=466.

**1) d) 5(S)-(Boc-amino)-4(S)-(tert-butyldimethylsilyloxy)-6-phenyl-2(R)-(p-methoxy-phenylmethyl)-hexansäure**

Analog Ref.-Bsp. 1j) werden 0,93 g (2,10 mMol) 5(S)-(Boc-amino)-4(S)-hydroxy-6-phenyl-2(R)-(p-methoxy-phenylmethyl)-hexansäure in 20 ml DMF mit 1,4 g (9,64 mMol) tert-Butyldimethylchlorsilan und 1,17 g (17,2 mMol) Imidazol silyliert. Hydrolyse der Silylesterfunktion mit 1,7 g Kaliumcarbonat in Methanol (23 ml)/THF (7 ml)/Wasser (7 ml) und Verrühren des Rohproduktes in Hexan liefert die Titelverbindung: $t_{Ret}$(I)=20,6 min; FAB-MS $(M+H)^+$=558.

**1) e) 5(S)-(Boc-amino)-4(S)-(tert-butyldimethylsilyloxy)-6-phenyl-2(R)-(p-methoxy-phenylmethyl)-hexanoyl-(L)-Val-(L)-Phe-morpholin-4-ylamid**

Analog Ref.-Bsp. 9f) werden 300 mg (0,537 mMol) 5(S)-(Boc-amino)-4(S)-(tert-butyldimethylsilyloxy)-6-phenyl-2(R)-(p-methoxy-phenylmethyl)-hexansäure und 197 mg (0,59 mMol) H-(L)-Val-(L)-Phe-morpholin-4-ylamid (Ref.-Bsp. lo) in 5,2 ml NMM/$CH_3CN$ 0,25 M mit 224 mg (0,59 mMol) HBTU umgesetzt: $t_{Ret}$(I)=22,1 min; FAB-MS $(M+H)^+$=873.

2) Boc-Phe[C](p-$CH_3O$)Phe-(L)-Val-(L)-(p-F-Phe)-morpholin-4-ylamid

**3) Boc-Phe[C](p-$CH_3O$)Phe-(L)-Val-(L)-(p-$CH_3O$-Phe)-morpholin-4-ylamid**

Analog Ref.-Bsp. 1) werden 200 mg (0,22 mMol) 5(S)-(Boc-amino)-4(S)-(tert-butyldimethylsilyloxy)-6-phenyl-2(R)-(p-methoxy-phenylmethyl)-hexanoyl-(L)-Val-(L)-(p-$CH_3O$-Phe)-morpholin-4-ylamid mit 210 mg (0,66 mMol) TBAF in 3 ml DMF zur Titelverbindung entschützt. Säulenchromatographie ($SiO_2$, Methylenchlorid/Methanol 19:1) liefert die reine Titelverbindung: DC $R_f$(F)=0,66; $t_{Ret}$(II)=22,5 min; FAB-MS $(M+H)^+$=789.

Das Ausgangsmaterial wird wie folgt hergestellt:

**3) a) 5(S)-(Boc-amino)-4(S)-(tert-butyldimethylsilyloxy)-6-phenyl-2(R)-(p-methoxy-phenylmethyl)-hexanoyl-(L)-Val-(L)-(p-$CH_3O$-Phe)morpholin-4-ylamid**

Analog Ref.-Bsp. 9f) werden 200 mg (0,358 mMol) 5(S)-(Boc-amino)-4(S)-(tert-butyldimethylsilyloxy)-6-phenyl-2(R)-(p-methoxy-phenylmethyl)-hexansäure und 143 mg (0,39 mMol) H-(L)-Val-(L)-(p-$CH_3O$-Phe)-morpholin-4-ylamid (Ref.-Bsp. 10e) in 3,6 ml NMM/$CH_3CN$ 0,25 M mit 149 mg (0,39 mMol) HBTU umgesetzt: $t_{Ret}$(II)=33,2 min.

**4)** Boc-Phe[C](p-$CH_3O$)Phe-(L)-Val-(L)-Cha-morpholin-4-ylamid

**5)** Boc-Phe[C](p-$CH_3O$)Phe-(L)-Ile-(L)-Phe-morpholin-4-ylamid

**G)**

**1) Boc-Phe[C](p-$CF_3$)Phe-(L)-Val-(L)-Phe-morpholin-4-ylamid**

Analog Ref.-Bsp. 1) werden 120 mg (0,13 mMol) 5(S)-(Boc-amino)-4(S)-(tert-butyldimethylsilyloxy)-6-phenyl-2(R)-(p-trifluormethyl-phenylmethyl)-hexanoyl-(L)-Val-(L)-Phe-morpholin-4-ylamid mit 124 mg (0,39 mMol) TBAF in 3 ml DMF zur Titelverbindung entschützt. Fällen mit DIPE aus einer konzentrierten Lösung in DMF liefert die reine Titelverbindung: $t_{Ret}$(II)=24,7 min; FAB-MS $(M+H)^+$=797.

Das Ausgangsmaterial wird wie folgt hergestellt:

**1) a) 5(S)-[1(S)-(Boc-amino)-2-phenyl-ethyl]-3(R)-(p-trifluormethylphenylmethyl)-dihydrofuran-2-(3H)-on**

Analog Ref.-Bsp. 21 D) 1)c) werden 1,0 g (3,26 mMol) 5(S)-[1(S)-(Boc-amino)-2-phenylethyl]-dihydrofuran-2-(3H)-on [Ref.-Bsp. 21 D) 1)b)] gelöst in 20 ml THF bei -75°C mit 6,5 ml Lithium-bis-(trimethylsilyl)amid 1 M in THF deprotoniert und mit 0,93 g (3,91 mMol) p-Trifluormethyl-benzylbromid (Fluka; Buchs/Schweiz) bei anfangs -75°C alkyliert (Erwärmung während 45 min auf -60°C). Eine Säulenchromatographie ($SiO_2$, Hexan/Essigsäureethylester 2:1) liefert die reine Titelverbindung: DC $R_f$(D)=0,4; $t_{Ret}$(II)=27,0 min; FAB-MS $(M+H\text{-Buten})^+$=408.

**1) b) 5(S)-(Boc-amino)-4(S)-hydroxy-6-phenyl-2(R)-(p-trifluormethyl-phenylmethyl)-hexansäure**

Analog Ref.-Bsp. 1i) werden 4,3 g (9,3 mMol) 5(S)-[1(S)-(Boc-amino)-2-phenylethyl]-3(R)-(p-trifluormethyl-phenylmethyl)-dihydrofuran-2-(3H)-on in 100 ml Dimethoxyethan und 25 ml Wasser mit 37 ml 1 M Lithiumhydroxid-Lösung zur Titelverbindung hydrolysiert: DC $R_f$(H)=0,68; $t_{Ret}$(II)=22,5 min.

**1) c) 5(S)-(Boc-amino)-4(S)-(tert-butyldimethylsilyloxy)-6-phenyl-2(R)-(p-trifluormethyl-phenylmethyl)-hexansäure**

Analog Ref.-Bsp. lj) werden 3,2 g (6,65 mMol) 5(S)-(Boc-amino)-4(S)-hydroxy-6-phenyl-2(R)-(p-trifluormethyl-phenylmethyl)-hexansäure in 25 ml DMF mit 4,6 g (30,6 mMol) tert-Butyldimethylchlorsilan und 3,7 g (54,5 mMol) Imidazol silyliert. Hydrolyse der Silylesterfunktion mit 5,5 g Kaliumcarbonat in Methanol (75 ml)/THF (22 ml)/Wasser (12 ml) liefert die Titelverbindung: $t_{Ret}$(II)=32,7 min.

**1) d) 5(S)-(Boc-amino)-4(S)-(tert-butyldimethylsilyloxy)-6-phenyl-2(R)-(p-trifluormethyl-phenylmethyl)-hexanoyl-(L)-Val-(L)-Phe-morpholin-4-ylamid**

Analog Ref.-Bsp. 9f) werden 200 mg (0,335 mMol) 5(S)-(Boc-amino)-4(S)-(tert-butyldimethylsilyloxy)-6-phenyl-2(R)-(p-trifluormethyl-phenylmethyl)-hexansäure und 123 mg (0,369 mMol) H-(L)-Val-(L)-Phe-morpholin-4-ylamid (Ref.-Bsp. lo) in 3,2 ml NMM/$CH_3CN$ 0,25 M mit 140 mg (0,37 mMol) HBTU umgesetzt: $t_{Ret}$(II)=34,8 min.

**2)** Boc-Phe[C](p-$CF_3$)Phe-(L)-Val-(L)-(p-F-Phe)-morpholin-4-ylamid
**3)** Boc-Phe[C](p-$CF_3$)Phe-(L)-Val-(L)-(p-$CH_3$O-Phe)-morpholin-4-ylamid
**4)** Boc-Phe[C](p-$CF_3$)Phe-(L)-Val-(L)-Cha-morpholin-4-ylamid
**5)** Boc-Phe[C](p-$CF_3$)Phe-(L)-Ile-(L)-Phe-morpholin-4-ylamid

H)

**1)** Boc-Cha[C](p-CN)Phe-(L)-Val-(L)-(p-F-Phe)-morpholin-4-ylamid
**2)** Boc-Cha[C](p-CN)Phe-(L)-Val-(L)-(p-$CH_3$O-Phe)-morpholin-4-ylamid
**3)** Boc-Cha[C](p-CN)Phe-(L)-Val(L)-Cha-morpholin-4-ylamid
**4)** Boc-Cha[C](p-CN)Phe-(L)-Ile-(L)-Phe-morpholin-4-ylamid

I)

**1)** Boc-Cha[C](p-$CH_3$O)Phe-(L)-Val-(L)-Phe-morpholin-4-ylamid
**2)** Boc-Cha[C](p-$CH_3$O)Phe-(L)-Val-(L)-(p-F-Phe)-morpholin-4-ylamid
**3)** Boc-Cha[C](p-$CH_3$O)Phe-(L)-Val-(L)-(p-$CH_3$O-Phe)-morpholin-4-ylamid
**4)** Boc-Cha[C](p-$CH_3$O)Phe-(L)-Val-(L)-Cha-morpholin-4-ylamid
**5)** Boc-Cha[C](p-$CH_3$O)Phe-(L)-Ile-(L)-Phe-morpholin-4-ylamid

J)

**1)** Boc-Cha[C](p-$CF_3$)Phe-(L)-Val-(L)-Phe-morpholin-4-ylamid
**2)** Boc-Cha[C](p-$CF_3$)Phe-(L)-Val-(L)-(p-F-Phe)-morpholin-4-ylamid
**3)** Boc-Cha[C](p-$CF_3$)Phe-(L)-Val-(L)-(p-$CH_3$O-Phe)-morpholin- 4-ylamid
**4)** Boc-Cha[C](p-$CF_3$)Phe-(L)-Val-(L)-Cha-morpholin-4-ylamid
**5)** Boc-Cha[C](p-$CF_3$)Phe-(L)-Ile-(L)-Phe-morpholin-4-ylamid

**Referenz-Beispiel 22:** Analog einem der vorgenannten Referenz-Beispiele, oder auf die jeweils im Detail angegebene Weise, werden durch Wahl entsprechender Ausgangsverbindungen hergestellt:

**A)** Boc-(L)-Val-Phe[C]Phe-(L)-Val-(L)-Phe-morpholin-4-ylamid;
**B)** H-(L)-Val-Phe[C]Phe-(L)-Val-(L)-Phe-morpholin-4-ylamid;
**C)** **Boc-Cha[C](p-F)Phe-(L)-Ile-(L)-Phe-morpholin-4-ylamid**
(beschrieben in EP 0 532 466)
**D)** Boc-Cha[C](p-F)Phe-(L)-Val-(L)-Cha-morpholin-4-ylamid;
**E)** Boc-Phe[C]Phe-(L)-Val-(D)-Phe-morpholin-4-ylamid;
**F)** Boc-Phe[C]Phe-(L)-Val(red)-(L)-Phe-morpholin-4-ylamid;
**G)** Isobutyloxycarbonyl-Phe[C]Phe-(L)-Val-(L)-Phe-morpholin-4-ylamid;
**H)** Boc-Cha[C](p-CN)Phe-(L)-Val-(L)-Phe-thiomorpholin-4-ylamid;
**I)** **Boc-Cha[C](p-F)Phe-(L)-Val-(L)-Phe-thiomorpholin-4-ylamid**
(beschrieben in EP 0 532 466)
**J)** Die Verbindungen aus Referenz-Beispiel 22 A) bis 22 G), worin anstelle von -morpholin-4-ylamid der Rest -thiomorpholin-4-ylamid steht.

Anmerkung: die Referenzbeispiele 23 bis 32 betreffen keine Verbindungen der Formel I.

**Referenz-Beispiel 33: Boc-(p-F)Phe[C](p-$CF_3$)Phe-(L)-Val-(L)-Phe-morpholin-4-ylamid**
(beschrieben in EP 0 532 466)

**Referenz-Beispiel 34: Boc-(p-F)Phe[C](p-$CF_3$)Phe-(L)-Val-(L)-(p-F-Phe)-morpholin-4-ylamid**
(beschrieben in EP 0 532 466)

**Referenz-Beispiel 35: Boc-(p-F)Phe[C](p-$CF_3$)Phe-(L)-Val-(L)-(p-$CH_3$O-Phe)-morpholin-4-ylamid**
(beschrieben in EP 0 532 466)

**Referenz-Beispiel 36: Morpholinosulfonyl-(L)-Val-Phe[C]Phe-(L)-Val-(L)-Phe-morpholin-4-ylamid**
(beschrieben in EP 0 532 466)

**Referenz-Beispiel 37: Morpholinosulfonyl-Phe[C]Phe-(L)-Val-(L)-Phe-morpholin-4-ylamid**
(beschrieben in EP 0 532 466)

**Referenz-Beispiel 38: N-(N-(2-Pyridylmethyl)-N-methyl-aminocarbonyl)-(L)-Val-Phe[C]Phe-(L)-Val-(L)-Phe-morpholin-4-ylamid**
(beschrieben in EP 0 532 466)

**Referenz-Beispiel 39: 5(S)-(Boc-amino)-4(S)-(acetoxy)-6-phenyl-2(R)-phenylmethylhexanoyl-(L)-Val-(L)-Phe-morpholin-4-ylamid**

(beschrieben in EP 0 532 466)

**Referenz-Beispiel 40:**

Auf analoge Weise, wie in einem der vorstehenden Referenz-Beispiele beschrieben, werden die folgenden Verbindungen erhalten:

A) Boc-(p-$CF_3$)Phe[C]Phe-(L)-Val-(L)-Phe-morpholin-4-ylamid

B) Boc-(p-$CF_3$)Phe[C]Phe-(L)-Val-(L)-(p-F)Phe-morpholin-4-ylamid

C) Boc-(p-$CF_3$)Phe[C]Phe-(L)-Val-(L)-(p-$CH_3$O)Phe-morpholin-4-ylamid

D) Boc-(p-$CF_3$)Phe[C]Phe-(L)-Val-(L)-Cha-morpholin-4-ylamid

E) Boc-(p-$CF_3$)Phe[C]Phe-(L)-Ile-(L)-Phe-morpholin-4-ylamid

F) **Boc-(p-$CF_3$)Phe[C](p-F)Phe-(L)-Val-(L)-Phe-morpholin-4-ylamid**

Analog Referenz-Beispiel 1) werden 136 mg (0,146 mMol) eines Gemisches von 5(S)-(Boc-amino)-4(S)-(tert-butyldimethylsilyloxy)-6-(p-trifluormethyl-phenyl)-2(R)-(p-fluor-phenylmethyl)-hexanoyl-(L)-Val-(L)-Phe-morpholin-4-ylamid und 5(R)-(Boc-amino)-4(R)-(tert-butyldimethylsilyloxy)-6-(p-trifluormethyl-phenyl)-2(S)-(p-fluor-phenylmethyl)-hexanoyl-(L)-Val-(L)-Phe-morpholin-4-ylamid mit 92 mg (0,293 mMol) TBAF in 1,5 ml DMF zur Titelverbindung entschützt: $t_{Ret}$(II)=26,0 min; FAB-MS $(M+H)^+$=815.

Das Ausgangsmaterial wird wie folgt hergestellt:

a) **rac. N-Boc-1-(p-trifluormethyl-phenyl)-3-buten-2-amin**

Analog Referenz-Beispiel 1*d*) werden 10,5 g (48,8 mMol) rac. 1-(p-Trifluormethyl-phenyl)-3-buten-2-amin (Referenz-Beispiel 40 *P*) c)) und 13,8 g (63,4 mMol) Boc-Anhydrid in 100 ml Methylenchlorid umgesetzt. Das Reaktionsgemisch wird mit 0,1 N HCl und 2 Portionen Sole gewaschen und die wässrigen Phasen werden mit 2 Portionen Methylenchlorid extrahiert. Trocknen der organischen Phasen mit $Na_2SO_4$, Eindampfen und Fällen mit Hexan aus einer konzentrierten Lösung in Methylenchlorid liefert die Titelverbindung: DC $R_f$(P)=0,27; $t_{Ret}$(II)=25,5 min; Anal: ber. C 60,94 %, H 6,39 %, N 4,44 %, F 18,07 %; gef. C 61,15 %, H 6,43 %, N 4,27 %, F 18,09 %.

**b: 2(R)-[1(S)-(Boc-amino)-2-(p-trifluormethyl-phenyl)-ethyl]-oxiran und 2(S)-[1(R)-(Boc-amino)-2-(p-trifluormethyl-phenyl)-ethyl]-oxiran**

Analog Referenz-Beispiel 1d) werden 13,5 g (42,8 mMol) rac. N-Boc-1-(p-trifluormethyl-phenyl)-3-buten-2-amin und 36,8 g (214 mMol) m-Chlorperbenzoesäure in 284 ml Chloroform umgesetzt. Verteilen des Reaktionsgemisches zwischen 3 Portionen Methylenchlorid, 10 %-iger $Na_2SO_3$-Lösung, ges. $Na_2CO_3$-Lösung, Wasser und Sole und Säulenchromatographie ($SiO_2$, Hexan/Essigsäureethylester 4:1) des Rohproduktes liefert das Racemat der Titelverbindungen: DC $R_f$(C)=0,15; $t_{Ret}$(II)=22,9 min; Anal: ber. C 58,00 %, H 6,08 %, N 4,23 %, F 17,20 %; gef. C 58,03 %, H 6,33 %, N 4,45 %, F 17,02 %.

**c: 5(S)-[1(S)-(Boc-amino)-2-(p-trifluormethylphenyl)-ethyl]-3(R,S)carbethoxy-dihydrofuran-2-(3H)-on und 5(R)-[1(R)-(Boc-amino)-2-(p-trifluormethylphenyl)-ethyl]-3(S,R)-carbethoxy-dihydrofuran-2-(3H)-on**

Analog Referenz-Beispiel 1e) werden 9,6 g (29,0 mMol) eines Gemisches von 2(R)-[1(S)-(Boc-amino)-2-(p-trifluormethyl-phenyl)-ethyl]-oxiran und 2(S)-[1(R)-(Boc-amino)-2-(p-trifluormethyl-phenyl)-ethyl]-oxiran in 48 ml Ethanol und 5 ml THF mit Natrium-diethylmalonat (Hergestellt aus 153 ml Ethanol, 2 g (87 mMol) Natrium und 15,4 ml (101 mMol) Malonsäurediethylester) umgesetzt. Kristallisieren durch Zugabe von Hexan zu einer konzentrierten Lösung in Essigsäureethylester liefert ein Gemisch der Titelverbindungen: DC $R_f$(D)=0,40; $t_{Ret}$(II)=24,1 min und 24,6 min; FAB-MS $(M+Na)^+$=468.

**d: 5(S)-[1(S)-(Boc-amino)-2-(p-trifluormethylphenyl)-ethyl]-dihydrofuran-2-(3H)-on und 5(R)-[1(R)-(Boc-amino)-2-(p-trifluormethylphenyl)-ethyl]-dihydrofuran-2-(3H)-on**

Analog Referenz-Beispiel 1h) werden 9,0 g (20,2 mMol) eines Gemisches von 5(S)-[1(S)-(Boc-amino)-2-(p-trifluormethylphenyl)-ethyl]-3(R,S)-carbethoxy-dihydrofuran-2-(3H)-on und 5(R)-[1(R)-(Boc-amino)-2-(p-trifluormethylphenyl)-ethyl]-3(S,R)-carbethoxy-dihydrofuran-2-(3H)-on in 166 ml Dimethoxyethan mit 86,9 ml 1 N wässriger LiOH-Lösung verseift. Decarboxylierung der erhaltenen Carbonsäuren in 350 ml Toluol (9 h 120°C) und Kristallisieren des Rohproduktes durch Zugabe von Hexan zu einer konzentrierten Lösung in Essigsäureethylester liefert die Titelverbindung als Racemat: $t_{Ret}$(II)=23,2 min; Anal: ber. C 57,90 %, H 5,94 %, N 3,75 %, F 15,26 %; gef. C 57,70 %, H 5,78 %, N 3,82 %, F 15,42 %.

**e: 5(S)-[1(S)-(Boc-amino)-2-(p-trifluormethylphenyl)-ethyl]-3(R)-(p-fluor-phenylmethyl)-dihydrofuran-2-(3H)-on und 5(R)-[1(R)-(Boc-amino)-2-(p-trifluormethylphenyl)-ethyl]-3(S)-(p-fluorphenylmethyl)-dihydrofuran-2-(3H)-on**

Analog Referenz-Beispiel 21 D) 1)c) werden 700 mg (1,88 mMol) eines Gemisches von 5(S)-[1(S)-(Boc-amino)-2-(p-trifluormethylphenyl)-ethyl]-dihydrofuran-2-(3H)-on und 5(R)-[1(R)-(Boc-amino)-2-(p-trifluormethylphenyl)-ethyl]-dihydrofuran-2-(3H)-on gelöst in 3,4 ml THF und 0,38 ml 1,3-Dimethyl-3,4,5,6-tetrahydro-2(1H)-pyrimidinon bei -75°C mit 3,67 ml Lithiumbis(trimethylsilyl)amid 1 M in THF deprotoniert und mit 0,242 ml (1,88 mMol) 4-Fluor-benzylbromid (Fluka; Buchs/Schweiz) bei -75°C alkyliert (40 min). Eine Säulenchromatographie ($SiO_2$, Hexan/Essigsäureethylester 2:1) liefert die Titelverbindung: DC $R_f$(D)=0,59; $t_{Ret}$(II)=26,6 min; FAB-MS $(M+H)^+$=482.

f) **5(S)-(Boc-amino)-4(S)-hydroxy-6-(p-trifluormethyl-phenyl)-2(R)-(p-fluor-phenylmethyl)-hexansäure und 5(R)-(Boc-amino)-4(R)-hydroxy-6-(p-trifluormethyl-phenyl)-2(S)-(p-fluor-phenylmethyl)-hexansäure**

Analog Referenz-Beispiel 1i) werden 1,1 g (2,28 mMol) eines Gemisches von 5(S)-[1(S)-(Boc-amino)-2-(p-trifluormethylphenyl)-ethyl]-3(R)-(p-fluor-phenylmethyl)-dihydrofuran-2-(3H)-on und 5(R)-[1(R)-(Boc-amino)-2-(p-trifluormethylphenyl)-ethyl]-3(S)-(p-fluor-phenylmethyl)-dihydrofuran-2-(3H)-on in 37 ml Dimethoxyethan und 19 ml Wasser mit 9,1 ml 1 M Lithiumhydroxid Lösung hydrolysiert. Das teilweise eingedampfte Reaktionsgemisch giesst man auf ein Gemisch von Eis, 112 ml ges. $NH_4Cl$-Lösung, 9,4 ml 10 %-iger Zitronensäure-Lösung und 46 ml Methylenchlorid und setzt Methanol zu, bis sich der ausgefallene Festkörper klar in den 2 Phasen löst. Die wässrige Phase wird mit 2 Portionen Methylenchlorid/Methanol ca. 9:1 extrahiert, die organischen Phasen mit Sole gewaschen, mit $Na_2SO_4$ getrocknet und eingedampft: DC $R_f$(D)=0,15; $t_{Ret}$(II)=22,7 min.

g) **5(S)-(Boc-amino)-4(S)-(tert-butyldimethylsilyloxy)-6-(p-trifluormethyl-phenyl)-2(R)-(p-fluorphenyl-methyl)-hexansäure und 5(R)-(Boc-amino)-4(R)-(tert-butyldimethylsilyloxy)-6-(p-trifluormethyl-phenyl)-2(S)-(p-fluorphenyl-methyl)-hexansäure**

Analog Referenz-Beispiel 1j) werden 1,1 g (2,20 mMol) eines Gemisches von 5(S)-(Boc-amino)-4(S)-hydroxy-6-(p-trifluormethyl-phenyl)-2(R)-(p-fluor-phenylmethyl)-hexansäure und 5(R)-(Boc-amino)-4(R)-hydroxy-6-(p-trifluormethyl-phenyl)-2(S)-(p-fluor-phenylmethyl)-hexansäure in 2,4 ml DMF mit 1,52 g (10,1 mMol) tert-Butyldimethylchlorsilan und 1,2 g (18,0 mMol) Imidazol silyliert. Hydrolyse der Silylesterfunktion mit 1,8 g Kaliumcarbonat in 50 ml Methanol/THF/Wasser 3:1:1 liefert nach Extraktion und Säulenchromatographie ($SiO_2$, Hexan/Essigsäureethylester 2:1) die Titelverbindung: DC $R_f$(D)=0,16; $t_{Ret}$(II)=32,7 min; FAB-MS $(M+H)^+$=614.

h) **5(S)-(Boc-amino)-4(S)-(tert-butyldimethylsilyloxy)-6-(p-trifluormethyl-phenyl)-2(R)-(p-fluorphenyl-methyl)-hexanoyl-(L)-Val-(L)-Phe-morpholin-4-ylamid und5(R)-(Boc-amino)-4(R)-(tert-butyldimethylsilyloxy)-6-(p-trifluormethyl-phenyl)-2(S)-(p-fluorphenyl-methyl)hexanoyl-(L)-Val-(L)-Phe-morpholin-4-ylamid**

Analog Referenz-Beispiel 9f) werden 200 mg (0,326 mMol) eines Gemisches von 5(S)-(Boc-amino)-4(S)-(tert-butyldimethylsilyloxy)-6-(p-trifluormethyl-phenyl)-2(R)-(p-fluorphenyl-methyl)-hexansäure und 5(R)-(Boc-amino)-4(R)-(tert-butyldimethylsilyloxy)-6-(p-trifluormethyl-phenyl)-2(S)-(p-fluorphenyl-methyl)-hexansäure und 119 mg (0,358 mMol) H-(L)-Val-(L)-Phe-morpholin-4-ylamid (Referenz-Beispiel lo) in 3,1 ml NMM/CH3CN 0,25 M mit 136 mg (0,36 mMol) HBTU zur Titelverbindung umgesetzt: $t_{Ret}$(II)=34,5 min; FAB-MS $(M+H)^+$=929.

G) Boc-(p-$CF_3$)Phe[C](p-F)Phe-(L)-Val-(L)-(p-F)Phe-morpholin-4- ylamid

H) Boc-(p-$CF_3$)Phe[C](p-F)Phe-(L)-Val-(L)-Cha-morpholin-4-ylamid

I) Boc-(p-$CF_3$)Phe[C](p-F)Phe-(L)-Val-(L)-(p-$CH_3O$)Phe-morpholin-4-ylamid

J) Boc-(p-$CF_3$)Phe[C](p-F)Phe-(L)-Ile-(L)-Phe-morpholin-4-ylamid

K) **Boc-(p-$CF_3$)Phe[C](p-$CF_3$)Phe-(L)-Val-(L)-Phe-morpholin-4-ylamid**

Analog Referenz-Beispiel 1) werden 110 mg (0,112 mMol) eines Gemisches von 5(S)-(Boc-amino)-4(S)-(tert-butyldimethylsilyloxy)-6-(p-trifluormethyl-phenyl)-2(R)-(p-trifluormethyl-phenyl-methyl)-hexanoyl-(L)-Val-(L)-Phe-morpholin-4-ylamid und 5(R)-(Boc-amino)-4(R)-(tert-butyldimethylsilyloxy)-6-(p-trifluormethyl-phenyl)-2(S)-(p-trifluormethyl-phenyl-methyl)-hexanoyl-(L)-Val-(L)-Phe-morpholin-4-ylamid mit 71 mg (0,225 mMol) TBAF in 1,1 ml DMF zur Titelverbindung entschützt: $t_{Ret}$(I)=18,0 und 18,6 min (12%); FAB-MS $(M+H)^+$=865.

Das Ausgangsmaterial wird wie folgt hergestellt:

a: **5(S)-[1(S)-(Boc-amino)-2-(p-trifluormethylphenyl)-ethyl]-3(R)-(p-trifluormethylphenylmethyl)-dihydrofuran-2-(3H)-on und5(R)-[1(R)-(Boc-amino)-2-(p-trifluormethylphenyl)-ethyl]-3(S)-(p-trifluormethyl-phenylmethyl)-dihydrofuran-2-(3-H)-on**

Analog Referenz-Beispiel 21 D) 1)c) werden 1,5 g (4,02 mMol) eines Gemisches von 5(S)-[1(S)-(Boc-amino)-2-(p-trifluormethylphenyl)-ethyl]-dihydrofuran-2-(3H)-on und 5(R)-[1(R)-(Boc-amino)-2-(p-trifluormethylphenyl)-ethyl]-dihydrofuran-2-(3H)-on gelöst in 7,3 ml THF und 0,81 ml 1,3-Dimethyl-3,4,5,6-tetrahydro-2(1H)-pyrimidinon bei -75°C mit 7,86 ml Lithiumbis(trimethylsilyl)amid 1 M in THF deprotoniert und mit 1,01 g (4,02 mMol) 4-Trifluormethyl-benzylbromid (Fluka; Buchs/Schweiz) bei -75°C alkyliert (40 min). Eine Säulenchromatographie ($SiO_2$, Methylenchlorid/Hexan/Ether 10:10:1iefert die Titelverbindung: DC $R_f$(Q)=0,23; $t_{Ret}$(II)=27,7 min; FAB-MS $(M+H-Boc)^+$=432.

**b) 5(S)-(Boc-amino)-4(S)-hydroxy-6-(p-trifluormethyl-phenyl)-2(R)-(p-trifluormethyl-phenylmethyl)-hexansäure und 5(R)-(Boc-amino)-4(R)-hydroxy-6-(p-trifluormethyl-phenyl)-2(S)-(p-trifluormethyl-phenylmethyl)-hexansäure**

Analog Referenz-Beispiel 1i) werden 1,43 g (2,69 mMol) eines Gemisches von 5(S)-[1(S)-(Boc-amino)-2-(p-trifluormethylphenyl)-ethyl]-3(R)-(p-trifluormethyl-phenylmethyl)-dihydrofuran-2-(3H)-on und 5(R)-[1(R)-(Boc-amino)-2-(p-trifluormethylphenyl)-ethyl]-3(S)-(p-trifluormethyl-phenylmethyl)-dihydrofuran-2-(3H)-on in 43 ml Dimethoxyethan und 22 ml Wasser mit 10,7 ml 1 M Lithiumhydroxid-Lösung hydrolysiert. Das teilweise eingedampfte Reaktionsgemisch giesst man auf ein Gemisch von Eis, 132 ml ges. $NH_4Cl$-Lösung, 11 ml 10 %-iger Zitronensäure-Lösung und 54 ml Methylenchlorid und setzt Methanol zu, bis sich der ausgefallene Festkörper löst. Die wässrige Phase wird mit 2 Portionen Methylenchlorid/Methanol ca. 4:1 extrahiert, die organischen Phasen mit Sole gewaschen, mit $Na_2SO_4$ getrocknet und eingedampft: $t_{Ret}$(II)=24,2 min; FAB-MS $(M+H)^+$=550.

**c) 5(S)-(Boc-amino)-4(S)-(tert-butyldimethylsilyloxy)-6-(p-trifluormethyl-phenyl)-2(R)-(p-trifluormethylphenyl-methyl)-hexansäure und 5(R)-(Boc-amino)-4(R)-(tert-butyldimethylsilyloxy)-6-(p-trifluormethyl-phenyl)-2(S)-(p-trifluormethylphenyl-methyl)-hexansäure**

Analog Referenz-Beispiel 1j) werden 1,38 g (2,51 mMol) eines Gemisches von 5(S)-(Boc-amino)-4(S)-hydroxy-6-(p-trifluormethyl-phenyl)-2(R)-(p-trifluormethyl-phenylmethyl)-hexansäure und 5(R)-(Boc-amino)-4(R)-hydroxy-6-(p-trifluormethyl-phenyl)-2(S)-(p-trifluormethyl-phenylmethyl)-hexansäure in 5,7 ml DMF mit 1,74 g (11,6 mMol) tert-Butyldimethylchlorsilan und 1,4 g (20,6 mMol) Imidazol silyliert. Hydrolyse der Silylesterfunktion mit 2,1 g Kaliumcarbonat in 55 ml Methanol/THF/Wasser 3:1:1 liefert die Titelverbindung: DC $R_f$(A)=0,25; $t_{Ret}$(I)=21,8 min.

**d) 5(S)-(Boc-amino)-4(S)-(tert-butyldimethylsilyloxy)-6-(p-trifluormethyl-phenyl)-2(R)-(p-trifluormethyl-phenyl-methyl)-hexanoyl-(L)-Val-(L)-Phe-morpholin-4-ylamid und 5(R)-(Boc-amino)-4(R)-(tert-butyldimethylsilyloxy)-6-(p-trifluormethyl-phenyl)-2(S)-(p-trifluormethyl-phenyl-methyl)hexanoyl-(L)-Val-(L)-Phe-morpholin-4-ylamid**

Analog Referenz-Beispiel 9f) werden 200 mg (0,301 mMol) eines Gemisches von 5(S)-(Boc-amino)-4(S)-(tert-butyldimethylsilyloxy)-6-(p-trifluormethyl-phenyl)-2(R)-(p-trifluormethyl-phenyl-methyl)-hexansäure und 5(R)-(Boc-amino)-4(R)-(tert-butyldimethylsilyloxy)-6-(p-trifluormethyl-phenyl)-2(S)-(p-trifluormethyl-phenyl-methyl)-hexansäure und 110 mg (0,331 mMol) H-(L)-Val-(L)-Phe-morpholin-4-ylamid (Referenz-Beispiel 1o) in 2,8 ml NMM/$CH_3CN$ 0,25 M mit 126 mg (0,33 mMol) HBTU umgesetzt. Dabei fällt das eine Diastereomere aus dem Reaktionsgemisch aus und kann abfiltriert werden (→ 5(R),4(R),2(S)). Durch Extraktion (analog Referenz-Beispiel 9f) der Mutterlauge und Säulenchromatographie ($SiO_2$, Hexan/Essigsäureethylester 1:1) kann das andere (→ 5(S),4(S),2(R)) in ca. 90 %-iger diastereomerer Reinheit (gemäss $^1$H-NMR-Spektroskopie) isoliert werden: DC $R_f$(A)=0,15; $t_{Ret}$(I)=23,0 min (Diastereomere nicht getrennt); $^1$H-NMR (300 MHz, $CD_3OD$, aus Kristallisation (→ 5(R),4(R),2(S))): u.a. 0,60 (dd, $(H_3C)_2CH$), $^1$H-NMR (300 MHz, $CD_3OD$, nach Chromatographie (→ 5(S),4(S),2(R))): u.a. 0,91 (dd, $(H_3C)_2CH$).

L) Boc-(p-$CF_3$)Phe[C](p-$CF_3$)Phe-(L)-Val-(L)-(p-F)Phe-morpholin- 4- ylamid

M) Boc-(p-$CF_3$)Phe[C](p-$CF_3$)Phe-(L)-Ile-(L)-Phe-morpholin-4-ylamid

N) Boc-(p-$CF_3$)Phe[C](p-$CF_3$)Phe-(L)-Val-(L)-(p-$CH_3$O)Phe-morpholin-4-ylamid

O) Boc-(p-$CF_3$)Phe[C](p-$CF_3$)Phe-(L)-Val-(L)-Cha-morpholin-4-ylamid

P) Die Verbindungen gemäss den vorstehenden Referenz-Beispielen A) bis O), worin der Rest -morpholin-4-ylamid durch den Rest thiomorpholin-4-ylamid ersetzt ist.

Das Ausgangsmaterial wird wie folgt hergestellt:

**a) N-Allylformamid**

Eine Lösung von 300 ml Allylamin in 1288 ml Ameisensäureethylester wird während 8 h auf 60°C erwärmt. Das Reaktionsgemisch wird am Rotationsverdampfer eingeengt und der Rückstand über eine Vigreux Kolonne destilliert (77°C; 1 mbar): $^1$H-NMR (200 MHz, $CDCl_3$): 8,2-7,95 (m, 1 H), 6,5-5,8 (sb, 1 H), 5,9-5,7 (m, 1 H), 5,3-5,05 (m, 2 H), 3,95-3,75 (m, 2 H).

**b) Allylisocyanid**

(U. Schöllkopf, R. Jentsch, K. Madawinata und R. Harms, Liebigs Ann. Chem., (1976) 2105). Unter $N_2$-Atmosphäre werden 517 g Chinolin und 286 g p-Toluolsulfonsäurechlorid bei 90°C vorgelegt. Man legt ein Vakuum von 2-4 mbar an und tropft 85 g N-Allylformamid zu, wobei das entstehende Isocyanid bei einer Innentemperatur von 85-95°C kontinuierlich über eine Vigreux Kolonne in die Kühlfalle (Aceton/Trockeneis) abdestilliert. Nach beendeter Umsetzung wird das Destillat unverzüglich nochmals über eine Vigreux Kolonne destilliert ($N_2$-Atmosphäre Normaldruck; 100°C): $^1$H-NMR (200 MHz, $CDCl_3$): 5,9-5,7 (m, 1 H), 5,45 (d, 16 Hz, 1 H), 5,32 (d, 10 Hz, 1 H), 4,05 (m, 2 H); IR (CH2$Cl_2$): 2150, 1650.

**c) rac. 1-(p-Trifluormethyl-phenyl)-3-buten-2-amin**

Unter $N_2$-Atmosphäre werden 4,5 g Allylisocyanid in 100 ml THF/Ether/Pentan abs 4:1:1 gelöst und auf -100°C abgekühlt. Man tropft bei -100 bis -90°C 42 ml n-Butyllithium (1,6 M in Hexan) zu, wobei erst eine Gelbfärbung auftritt und sich kurz vor Ende der Zugabe ein Festkörper ausscheidet. Langsam lässt man das Reaktionsgemisch auf -70°C aufwärmen und kühlt dann wieder auf - 100°C ab. Bei - 100 bis -85°C wird eine Lösung von 16 g p-Trifluormethyl-benzylbromid (Fluka; Buchs/Schweiz) in 10 ml THF zugetropft und langsam auf RT aufgewärmt. Das Reaktionsgemisch wird am Rotationsverdampfer (80 mbar; 30 °C) eingedampft, der Rückstand auf 150 ml Eiswasser gegossen und 3 mal mit Ether extrahiert. Die Ether-Phasen dampft man ein, versetzt den braunen Rückstand bei 0°C mit 85 ml Methanol und 17 ml konz. Salzsäure und lässt über Nacht im Kühlschrank stehen. Das Gemisch wird am Rotationsverdampfer eingedampft und der Rückstand verteilt zwischen 2 x 150 ml 2 M Salzsäure und 2 x 200 ml Ether. Die vereinigten wässrigen Phasen stellt man unter Kühlen mit festem Natriumhydroxid alkalisch und extrahiert sie mit 3 Portionen Essigsäureethylester. Waschen der organischen Phasen mit Sole, Trocknen mit Natriumsulfat, Eindampfen und Destillation am Kugelrohr (0,1 mbar; 170 °C) liefert die reine Titelverbindung: $^1$H-NMR (200 MHz, $CDCl_3$): 7,56 und 7,32 (2d, 8 Hz, je 2 H), 5,96-5,78 (m, 1 H), 5,19-5,02 (m, 2 H), 3,68-3,55 (m, 1 H), 2,87 und 2,71 (AB x d, $J_{ab}$= 13 Hz, $J_1$=6 Hz, $J_2$= 8 Hz, 2 H), 1,4 (sb, 2 H).

Die weitere Umsetzung analog Referenz-Beispiel 1 d) bis 1 k) und 1), 9 f) und 9, 10 f) und 10, 15 a) und 15 oder 16 c) und 16 führt zu den oben unter a) bis o) genannten Verbindungen.

**Referenz-Beispiel 41:**

Analog einem der vorstehenden Referenz-Beispiele werden hergestellt:

A) **Boc-Phe[C]Phe-(L)-Val-(L)-Tyr-morpholin-4-ylamid**

Analog Referenz-Beispiel 1) werden 360 mg (0,418 mMol) 5(S)-(Boc-amino)-4(S)-(tert-butyldimethylsilyloxy)-6-phenyl-2(R)-phenylmethyl-hexanoyl-(L)-Val-(L)-Tyr-morpholin-4-ylamid mit 263 mg (0,84 mMol) TBAF in 7 ml DMF zur Titelverbindung entschützt: DC $R_f$(B)=0,28; $t_{Ret}$(II)=20,1 min; FAB-MS $(M+H)^+$=745.

Das Ausgangsmaterial wird wie folgt hergestellt:

**a: Z-(L)-Tyr-morpholin-4-ylamid**

Zu einer eisgekühlten Suspension von 14,04 g (40 mMol) Z-(L)-Tyr-OH in 750 ml Methylenchlorid gibt man 9,08 g (44 mMol) DCC und rührt 20 min. Danach werden 10,81 g (80 mMol) HOBT und eine Lösung von 5,23 g (60 mMol) Morpholin in 50 ml Methylenchlorid zugesetzt. Nach 18 h Rühren bei RT wird filtriert. Das Filtrat wäscht man mit ges. $NaHCO_3$-Lösung, Wasser und Sole und extrahiert die wässrigen Phasen mit 2 Portionen Methylenchlorid. Eine Säulenchromatographie ($SiO_2$, Essigsäureethylester) der mit $Na_2SO_4$ getrockneten und eingedampften organischen Phasen liefert die Titelverbindung: DC $R_f$(B)=0,39.

**b: H-(L)-Tyr-morpholin-4-ylamid**

Eine Lösung von 2,05 g (5,3 mMol) Z-(L)-Tyr-morpholin-4-ylamid in 91 ml Methanol wird in Gegenwart von 0,5 g 10 %-igem Pd/C während 1,5 h bei RT hydriert. Filtration durch ®Celite (Filterhilfsmittel aus Kieselgur, John-Manville Corp.) und Eindampfen des Filtrats liefert die Titelverbindung: DC $R_f$(R)=0,34.

**c: Z-(L)-Val-(L)-Tyr-morpholin-4-ylamid**

Bei 0°C gibt man zu einer Lösung von 6,3 g (25 mMol) Z-(L)-Val-OH in 400 ml Methylenchlorid 5,18 g (25 mMol) DCC und 3,73 g (27,5 mMol) HOBT und rührt 20 min nach. Anschliessend wird eine Lösung von 6,27 g (25 mMol) H-(L)-Tyr-morpholin-4-ylamid in 600 ml Methylenchlorid zugesetzt. Nach 18 h Rühren bei RT arbeitet man wie in Referenz-Beispiel 41 A) a) beschrieben auf: DC $R_f$(B)=0,50.

**d: H-(L)-Val-(L)-Tyr-morpholin-4-ylamid**

Hydrieren einer Lösung von 4,83 g (10 mMol) Z-(L)-Val-(L)-Tyr-morpholin-4-ylamid in 182 ml Methanol in Gegenwart von 1 g 10 %-igem Pd/C während 1,5 h bei RT liefert nach Filtration durch ®Celite, Eindampfen des Filtrats und Säulenchromatographie ($SiO_2$, Methylenchlorid/Methanol 9:1) die Titelverbindung: DC $R_f$(F)=0,30; FAB-MS $(M+H)^+$=350.

**e: 5(S)-(Boc-amino)-4(S)-(tert-butyldimethylsilyloxy)-6-phenyl-2(R)-phenylmethylhexanoyl-(L)-Val-(L)-Tyr-morpholin-4-ylamid**

Analog Referenz-Beispiel 1k) werden 300 mg (0,569 mMol) 5(S)-(Boc-amino)-4(S)-(tert-butyldimethylsilyloxy)-6-phenyl-2(R)-phenylmethyl-hexansäure (Referenz-Beispiel 2d) in 5 ml DMF mit 277 mg (0,626 mMol) BOP, 84,5 mg (0,625 mMol) HOBT und 157 µl (1,42 mMol) NMM aktiviert und mit 198,6 mg (0,568 mMol) H-(L)-Val-(L)-Tyr-morpholin-4-ylamid in 2 ml DMF umgesetzt (2 h RT). Eine Säulenchromatographie ($SiO_2$, Essigsäureethylester/Hexan 2:1) liefert die Titelverbindung: DC $R_f$(I)=0,26; $t_{Ret}$(II)=32,2 min; FAB-MS $(M+H)^+$=859.

B) **Boc-Tyr[C]Phe-(L)-Val-(L)-Phe-morpholin-4-ylamid**
Hydrieren von 165 mg (0,197 mMol) Boc-(p-BzlO)Phe[C]Phe-(L)-Val-(L)-Phe-morpholin-4-ylamid (Referenz-Beispiel 46) gelöst in 12 ml Methanol in Gegenwart von 82 mg 10 %-igem Pd/C bei RT liefert nach Filtration durch ®Celite und Eindampfen des Filtrats die Titelverbindung: $t_{Ret}$(I)=13,9 min; FAB-MS $(M+H)^+$=745.

C) **Boc-Tyr[C]Phe-(L)-Val-(L)-Tyr-morpholin-4-ylamid**
Hydrieren von 235 mg (0,25 mMol) Boc-(p-BzlO)Phe[C]Phe-(L)-Val-(L)-(p-BzlOPhe)-morpholin-4-ylamid (Referenz-Beispiel 47) gelöst in 30 ml Methanol in Gegenwart von 120 mg 10 %-igem Pd/C bei RT liefert nach Filtration durch ®Celite, Eindampfen des Filtrats und Kristallisation aus Essigsäureethylester/Hexan die Titelverbindung: DC $R_f$(B)=0,43; $t_{Ret}$(I)=12,1 min; FAB-MS $(M+H)^+$=761.

D) **Boc-Phe[C]Tyr-(L)-Val-(L)-Phe-morpholin-4-ylamid**
Hydrieren von 140 mg (0,168 mMol) Boc-Phe[C](p-BzlO)Phe-(L)-Val-(L)-Phe-morpholin-4-ylamid (Referenz-Beispiel 44) gelöst in 10 ml Methanol in Gegenwart von 70 mg 10 %-igem Pd/C bei RT liefert nach Filtration durch ®Celite und Eindampfen des Filtrats die Titelverbindung: $t_{Ret}$(II)=19,9 min; FAB-MS $(M+H)^+$=745.

E) **Boc-Phe[C]Tyr-(L)-Val-(L)-Tyr-morpholin-4-ylamid**
Hydrieren von 140 mg (0,159 mMol) Boc-Phe[C](p-BzlO)Phe-(L)-Val-(L)-(p-BzlOPhe)-morpholin-4-ylamid (Referenz-Beispiel 45) gelöst in 10 ml Methanol in Gegenwart von 75 mg 10 %-igem Pd/C bei RT liefert nach Filtration durch ®Celite und Eindampfen des Filtrats die Titelverbindung: $t_{Ret}$(II)=17,2 min; FAB-MS $(M+H)^+$=761.

F) **Boc-Tyr[C]Tyr-(L)-Val-(L)-Phe-morpholin-4-ylamid**
Hydrieren von 188 mg (0,178 mMol) Boc-(p-BzlO)Phe[C](BzlO)Phe-(L)-Val-(L)-Phe-morpholin-4-ylamid (Referenz-Beispiel 48) gelöst in 20 ml Methanol in Gegenwart von 85 mg 10 %-igem Pd/C bei RT liefert nach Filtration durch ®Celite und Eindampfen des Filtrats die Titelverbindung: DC $R_f$(B)=0,50; $t_{Ret}$(I)=12,1 min; FAB-MS $(M+H)^+$=761.

G) **Boc-Tyr[C]Tyr-(L)-Val-(L)-Tyr-morpholin-4-ylamid**;
Hydrieren von 209 mg (0,20 mMol)
Boc-(p-BzlO)Phe[C](p-BzlO)Phe-(L)-Val-(L)-(p-BzlOPhe)-morpholin-4-ylamid (Referenz-Beispiel 49) gelöst in 20 ml Methanol in Gegenwart von 85 mg 10%-igem Pd/C bei RT liefert nach Filtration und Eindampfen des Filtrats die Titelverbindung: DC $R_f$(B)=0,15; $t_{Ret}$(I)=10,6 min; FAB-MS $(M+H)^+$=777.

H) die Verbindungen gemäss den vorstehend genannten Referenz-Beispielen A) bis G), worin der Rest -morpholin-4-ylamid durch den Rest thiomorpholin-4-ylamid ersetzt ist.

Die nachfolgend genannten Referenzbeispiele sind nicht explizit in EP 0 532 466 offenbart.

**Referenz-Beispiel 42: Boc-Phe[C](o-CN)Phe-(L)-Val-(L)-Phe-morpholin-4-ylamid**

Analog Referenz-Beispiel 1) werden 145 mg (0,167 mMol) 5(S)-(Boc-amino)-4(S)-(tert-butyldimethylsilyloxy)-6-phenyl-2(R)-(o-cyano-phenylmethyl)-hexanoyl-(L)-Val-(L)-Phe-morpholin-4-ylamid mit 105 mg (0,334 mMol) TBAF in 4 ml DMF entschützt. Fällen mit DIPE aus einer konzentrierten Lösung in DMF liefert die reine Titelverbindung: $t_{Ret}$(I)=15,7 min; FAB-MS $(M+H)^+$=754.

Das Ausgangsmaterial wird wie folgt hergestellt:

**a) 5(S)-[1(S)-(Boc-amino)-2-phenylethyl]-3(R)-(o-cyano-phenylmethyl)-dihydrofuran-2-(3H)-on**
Analog Referenz-Beispiel 21 D) 1)c) werden 2,0 g (6,55 mMol) 5(S)-[1(S)-(Boc-amino)- 2-phenylethyl]-dihydrofuran-2-(3H)-on [Referenz-Beispiel 21 D) 1)b)] gelöst in 40 ml THF mit 13,1 ml Lithiumbis(trimethylsilyl)amid 1 M in THF deprotoniert und mit 1,4 g (7,2 mMol) 2-Brommethylbenzonitril (Fluka; Buchs/Schweiz) alkyliert 75 min). Säulenchromatographie ($SiO_2$, Hexan/Essigsäureethylester 2:1) und Verrühren in Hexan liefert die reine Titelverbindung: DC $R_f$(D)=0,45; $t_{Ret}$(I)=16,2 min.

**b) 5(S)-(Boc-amino)-4(S)-hydroxy-6-phenyl-2(R)-(o-cyano-phenylmethyl)hexansäure**
Analog Referenz-Beispiel 1i) werden 1,67 g (3,97 mMol) 5(S)-[1(S)-(Boc-amino)-2-phenylethyl]-3(R)-(o-cyano-phenylmethyl)-dihydrofuran-2-(3H)-on in 37 ml Dimethoxyethan und 20 ml Wasser mit 16 ml 1 M Lithiumhydroxid Lösung zur Titelverbindung hydrolysiert: $t_{Ret}$(I)=13,8 min.

**c) 5(S)-(Boc-amino)-4(S)-(tert-butyldimethylsilyloxy)-6-phenyl-2(R)-(o-cyano-phenylmethyl)-hexansäure**
Analog Referenz-Beispiel 1j) werden 0,85 g (1,93 mMol) 5(S)-(Boc-amino)-4(S)-hydroxy-6-phenyl-2(R)-(o-cyano-phenylmethyl)-hexansäure in 10 ml DMF mit 1,34 g (8,9 mMol) tert-Butyldimethylchlorsilan und 1,08 g (15,9 mMol) Imidazol silyliert. Hydrolyse der Silylesterfunktion mit 1,6 g Kaliumcarbonat in 40 ml Methanol/THF/Wasser 5:1:2 liefert nach Säulenchromatographie ($SiO_2$, Essigsäureethylester/Hexan 1:1) die Titelverbindung: DC $R_f$(A)=0,4; $t_{Ret}$(I)=20,0 min.

**d) 5(S)-(Boc-amino)-4(S)-(tert-butyldimethylsilyloxy)-6-phenyl-2(R)-(o-cyano-phenylmethyl)-hexanoyl-(L)-Val-(L)-Phe-morpholin-4-ylamid**

Bei 5°C werden 100 mg (0,18 mMol) 5(S)-(Boc-amino)-4(S)-(tert-butyldimethylsilyloxy)-6-phenyl-2(R)-(o-cyano-phenylmethyl)-hexansäure in 3 ml THF mit 41 mg (0,20 mMol) DCC versetzt. Nach 10 min gibt man 66 mg (0,20 mMol) H-(L)-Val-(L)-Phe-morpholin-4-ylamid (Referenz-Beispiel 1o) und 27 mg (0,20 mMol) HOBT zu und rührt 17 h bei RT. Das Reaktionsgemisch wird filtriert und das Filtrat verteilt zwischen 3 Portionen Essigsäureethylester, 10 %-iger Zitronensäure Lösung, Wasser, ges. $NaHCO_3$-Lösung und Sole. Trocknen der organischen Phase mit $Na_2SO_4$, Eindampfen und Digerieren in DIPE liefert die Titelverbindung: $t_{Ret}$(I)=21,8 min; FAB-MS $(M+H)^+$=868.

**Referenz-Beispiel 43: Boc-Phe[C](m-CN)Phe-(L)-Val-(L)-Phe-morpholin-4-ylamid**

Analog Referenz-Beispiel 1) werden 131 mg (0,151 mMol) 5(S)-(Boc-amino)-4(S)-(tert-butyldimethylsilyloxy)-6-phenyl-2(R)-(m-cyano-phenylmethyl)-hexanoyl-(L)-Val-(L)-Phe-morpholin-4-ylamid mit 95 mg (0,301 mMol) TBAF in 4 ml DMF entschützt. Fällen mit DIPE aus einer konzentrierten Lösung in DMF liefert die reine Titelverbindung: $t_{Ret}$(I)=15,7 min; FAB-MS $(M+H)^+$=754.

Das Ausgangsmaterial wird wie folgt hergestellt:

**a) 5(S)-[1(S)-(Boc-amino)-2-phenylethyl]-3(R)-(m-cyano-phenylmethyl)-dihydrofuran-2-(3H)-on**

Analog Referenz-Beispiel 21 D) 1)c) werden 2,0 g (6,55 mMol) 5(S)-[1(S)-(Boc-amino)-2-phenylethyl]-dihydrofuran-2-(3H)-on [Referenz-Beispiel 21 D) 1)b)] gelöst in 40 ml THF bei -75°C mit 13,1 ml Lithiumbis(trimethylsilyl)amid 1 M in THF deprotoniert und mit 1,4 g (7,2 mMol) 3-Brommethylbenzonitril (Fluka; Buchs/Schweiz) alkyliert (60 min -60°C). Säulenchromatographie ($SiO_2$, Hexan/Essigsäureethylester 2:1) und Verrühren in Hexan liefert die reine Titelverbindung: DC $R_f$(D)=0,41; $t_{Ret}$(I)=16,1 min.

**b) 5(S)-(Boc-amino)-4(S)-hydroxy-6-phenyl-2(R)-(m-cyano-phenylmethyl)-hexansäure**

Analog Referenz-Beispiel 1i) werden 1,6 g (3,8 mMol) 5(S)-[1(S)-(Boc-amino)-2-phenylethyl]-3(R)-(m-cyano-phenylmethyl)-dihydrofuran-2-(3H)-on in 37 ml Dimethoxyethan und 20 ml Wasser mit 15,2 ml 1 M Lithiumhydroxid Lösung zur Titelverbindung hydrolysiert: $t_{Ret}$(I)=13,8 min.

**c) 5(S)-(Boc-amino)-4(S)-(tert-butyldimethylsilyloxy)-6-phenyl-2(R)-(m-cyano-phenylmethyl)-hexansäure**

Analog Referenz-Beispiel 1j) werden 1,4 g (3,2 mMol) 5(S)-(Boc-amino)-4(S)-hydroxy-6-phenyl-2(R)-(m-cyano-phenylmethyl)-hexansäure in 20 ml DMF mit 2,2 g (14,6 mMol) tert-Butyldimethylchlorsilan und 1,8 g (26 mMol) Imidazol silyliert. Hydrolyse der Silylesterfunktion mit 2,6 g Kaliumcarbonat in 55 ml Methanol/THF/Wasser 8:1:2 liefert nach Säulenchromatographie ($SiO_2$, Essigsäureethylester/Hexan 1:1) die Titelverbindung: DC $R_f$(A)=0,39; $t_{Ret}$(I)=19,8 min.

**d) 5(S)-(Boc-amino)-4(S)-(tert-butyldimethylsilyloxy)-6-phenyl-2(R)-(m-cyano-phenylmethyl)-hexanoyl-(L)-Val-(L)-Phe-morpholin-4-ylamid**

Analog Referenz-Beispiel 42 d) werden 100 mg (0,18 mMol) 5(S)-(Boc-amino)-4(S)-(tert-butyldimethylsilyloxy)-6-phenyl-2(R)-(m-cyano-phenyl methyl)-hexansäure in 3 ml THF mit 41 mg (0,20 mMol) DCC aktiviert und mit 66 mg (0,20 mMol) H-(L)-Val-(L)-Phe-morpholin-4-ylamid (Referenz-Beispiel lo) und 27 mg (0,20 mMol) HOBT zur Titelverbindung umgesetzt: $t_{Ret}$(I)=21,5 min; FAB-MS $(M+H)^+$=868.

**Referenz-Beispiel 44: Boc-Phe[C](p-BzlO)Phe-(L)-Val-(L)-Phe-morpholin-4-ylamid**

Analog Referenz-Beispiel 1) werden 560 mg (0,59 mMol) 5(S)-(Boc-amino)-4(S)-(tert-butyldimethylsilyloxy)-6-phenyl-2(R)(p-benzyloxyphenyl-methyl)-hexanoyl-(L)-Val-(L)-Phe-morpholin-4-ylamid mit 372 mg (1,18 mMol) TBAF in 8,4 ml DMF zur Titelverbindung entschützt: $t_{Ret}$(II)=26,1 min; FAB-MS $(M+H)^+$=835.

Das Ausgangsmaterial wird wie folgt hergestellt:

**a) p-Benzyloxy-benzyljodid**

Eine Lösung von 1,0 g (4,3 mMol) 4-Benzyloxy-benzylchlorid (Fluka; Buchs/Schweiz) in 8 ml Aceton wird mit 3,13 g (20,9 mMol) Natriumjodid bei RT gerührt. Laut Gaschromatogramm ist die Umsetzung nach 90 min beendet. Aufarbeitung wie in Referenz-Beispiel 21 F) 1) a) beschrieben liefert die Titelverbindung: $^1$H-NMR (200 MHz, $CDCl_3$: 4,48 (s, 2 H), 5,06 (s, 2 H), 6,85-6,95 (m, 2 H), 7,25-7,48 (m, 7 H).

**b) 5(S)-[1(S)-(Boc-amino)-2-phenyl-ethyl]-3(R)-(p-benzyloxy-phenylmethyl)-dihydrofuran-2-(3H)-on**

Analog Referenz-Beispiel 21 D) 1)c) werden 1,13 g (3,70 mMol) 5(S)-[1(S)-(Boc-amino)-2-phenylethyl]-dihydrofuran-2-(3H)-on [Referenz-Beispiel 21 D) 1)b)] gelöst in 4,8 ml THF und 0,75 ml 1,3-Dimethyl-3,4,5,6-tetrahydro-2(1H)-pyrimidinon bei -75°C mit 7,25 ml Lithiumbis(trimethylsilyl)amid 1 M in THF deprotoniert und mit 1,2 g (3,7 mMol) p-Benzyloxy-benzyljodid in 2 ml THF alkyliert (15 min). Säulenchro-

matographie ($SiO_2$, Hexan/Essigsäureethylester 2:1) liefert die reine Titelverbindung: DC $R_f$(D)=0,30; $t_{Ret}$(II)=28,2 min; FAB-MS $(M+H)^+$=502.

**c) 5(S)-(Boc-amino)-4(S)-hydroxy-6-phenyl-2(R)-(p-benzyloxy-phenylmethyl)-hexansäure**

Analog Referenz-Beispiel 1i) werden 1,4 g (2,79 mMol) 5(S)-[1(S)-(Boc-amino)-2-phenylethyl]-3(R)-(p-benzyloxy-phenylmethyl)-dihydrofuran-2-(3H)-on in 45 ml Dimethoxyethan und 23 ml Wasser mit 11 ml 1 M Lithiumhydroxid Lösung hydrolysiert. Das teilweise eingedampfte Reaktionsgemisch giesst man auf ein Gemisch von Eis, 137 ml ges. $NH_4Cl$-Lösung, 11 ml 10 %-iger Zitronensäure Lösung und 56 ml Methylenchlorid und setzt Methanol zu, bis sich der ausgefallene Festkörper löst. Die wässrige Phase wird mit 2 Portionen Methylenchlorid/Methanol ca. 10:1 extrahiert, die organischen Phasen mit Sole gewaschen, mit $Na_2SO_4$ getrocknet und eingedampft: $t_{Ret}$(II)=24,0 min; FAB-MS $(M+H)^+$=520.

**d) 5(S)-(Boc-amino)-4(S)-(tert-butyldimethylsilyloxy)-6-phenyl-2(R)-(p-benzyloxy-phenyl-methyl)-hexansäure**

Analog Referenz-Beispiel 1j) werden 1,4 g (2,69 mMol) 5(S)-(Boc-amino)-4(S)- hydroxy-6-phenyl-2(R)-(p-benzyloxy-phenylmethyl)-hexansäure in 2,9 ml DMF mit 1,87 g (12,4 mMol) tert-Butyldimethylchlorsilan und 1,5 g (22 mMol) Imidazol silyliert. Hydrolyse der Silylesterfunktion mit 2,2 g Kaliumcarbonat in 63 ml Methanol/THF/Wasser 3:1:1 und Säulenchromatographie ($SiO_2$, Hexan/Essigsäureethylester 2:1) des Rohproduktes liefert die Titelverbindung: DC $R_f$(D)=0,17; $t_{Ret}$(II)=33,7 min; FAB-MS $(M+H)^+$=634.

**e) 5(S)-(Boc-amino)-4(S)-(tert-butyldimethylsilyloxy)-6-phenyl-2(R)-(p-benzyloxy-phenyl-methyl)-hexanoyl-(L)-Val-(L)-Phe-morpholin-4-ylamid**

Analog Referenz-Beispiel 9f) werden 400 mg (0,631 mMol) 5(S)-(Boc-amino)-4(S)-(tert-butyldimethylsilyloxy)-6-phenyl-2(R)-(p-benzyloxy-phenylmethyl)-hexansäure und 231 mg (0,69 mMol) H-(L)-Val-(L)-Phe-morpholin-4-ylamid (Referenz-Beispiel 1o) in 5,7 ml NMM/$CH_3CN$ 0,25 M mit 263 mg (0,69 mMol) HBTU umgesetzt: DC $R_f$(A)=0,3; $t_{Ret}$(II)=35,8 min; FAB-MS $(M+H)^+$=949.

**Referenz-Beispiel 45: Boc-Phe[C](p-BzlO)Phe-(L)-Val-(L)-(P-BzlOPhe)-morpholin-4-ylamid**

Analog Referenz-Beispiel 1) werden 665 mg (0,63 mMol) 5(S)-(Boc-amino)-4(S)-(tert-butyldimethylsilyloxy)-6-phenyl-2(R)-(p-benzyloxyphenyl-methyl)-hexanoyl-(L)-Val-(L)-(p-BzlOPhe)-morpholin-4-ylamid mit 398 mg (1,26 mMol) TBAF in 9 ml DMF entschützt. Fällen mit DIPE aus einer konzentrierten Lösung in Methylenchlorid liefert die reine Titelverbindung: $t_{Ret}$(II)=28,7 min; FAB-MS $(M+H)^+$=941.

Das Ausgangsmaterial wird wie folgt hergestellt:

**a: Boc-(L)-(p-BzlOPhe)-morpholin-4-ylamid**

Zu einer eisgekühlten Suspension von 14,84 g (40 mMol) Boc-(L)-Tyr(Bzl)-OH (Bachem; Bubendorf/Schweiz) in 650 ml Methylenchlorid gibt man 9,08 g (44 mMol) DCC und rührt 20 min. Danach werden 10,81 g (80 mMol) HOBT und eine Lösung von 5,23 g (60 mMol) Morpholin in 50 ml Methylenchlorid zugesetzt. Nach 18 h Rühren bei RT wird filtriert. Das Filtrat wäscht man mit ges. $NaHCO_3$-Lösung, Wasser und Sole und extrahiert die wässrigen Phasen mit 2 Portionen Methylenchlorid. Erneutes Lösen der eingedampften organischen Phasen in wenig Methylenchlorid, Abfiltrieren des restlichen ungelösten Dicyclohexylharnstoffs und eindampfen des Filtrats liefert die Titelverbindung: DC $R_f$(B)=0,69.

**b: H-(L)-(p-BzlOPhe)-morpholin-4-ylamid**

Bei 0°C versetzt man eine Lösung von 1,0 g (2,69 mMol) Boc-(L)-(p-BzlOPhe)-morpholin-4-ylamid in 30 ml Methylenchlorid mit 30 ml TFA. Nach 45 min wird das Lösungsmittel abgedampft, der Rückstand in Essigsäureethylester gelöst und mit ges. $NaHCO_3$ Lösung, 2x Wasser und Sole gewaschen. Die wässrigen Phasen extrahiert man mit 2 Portionen Essigsäureethylester. Trocknen der organischen Phasen mit $Na_2SO_4$ und Eindampfen liefert die Titelverbindung: DC $R_f$(F)=0,42.

**c: Boc-(L)-Val-(L)-(p-BzlOPhe)-morpholin-4-ylamid**

Bei 0°C gibt man zu einer Lösung von 2,8 g (13 mMol) Boc-(L)-Val-OH in 350 ml Methylenchlorid 2,68 g (13 mMol) DCC und 1,93 g (14,3 mMol) HOBT und rührt 20 min nach. Anschliessend wird eine Lösung von 4,41 g (13 mMol) H-(L)-(p-BzlOPhe)-morpholin-4-ylamid und 1,8 ml (13 mMol) Triethylamin in 250 ml Methylenchlorid zugesetzt. Nach 18 h Rühren bei RT arbeitet man wie in Referenz-Beispiel 41 A) a) beschrieben auf. Dies liefert nach Säulenchromatographie ($SiO_2$, Essigsäureethylester/Hexan 3:1) die reine Titelverbindung: DC $R_f$(S)=0,50.

**d: H-(L)-Val-(L)-(p-BzlOPhe)-morpholin-4-ylamid**

Analog Referenz-Beispiel 45 b) werden 5,7 g (10,6 mMol) Boc-(L)-Val-(L)-(p-BzlOPhe)-morpholin-4-ylamid in 120 ml Methylenchlorid mit 120 ml TFA zur Titelverbindung gespalten: DC $R_f$(F)=0,42.

**e) 5(S)-(Boc-amino)-4(S)-(tert-butyldimethylsilyloxy)-6-phenyl-2(R)-(p-benzyloxy-phenyl-methyl)-hexanoyl-(L)-Val-(L)-(p-BzlOPhe)-morpholin-4-yl-amid**

Analog Referenz-Beispiel 9f) werden 400 mg (0,631 mMol) 5(S)-(Boc-amino)-4(S)-(tert-butyldimethylsi-

lyloxy)-6-phenyl-2(R)-(p-benzyloxy-phenylmethyl)-hexansäure (Referenz-Beispiel 44 d) und 305 mg (0,69 mMol) H-(L)-Val-(L)-(p-BzlOPhe)-morpholin-4-ylamid in 5,7 ml NMM/$CH_3CN$ 0,25 M mit 263 mg (0,69 mMol) HBTU umgesetzt: DC $R_f(A)=0,31$; $t_{Ret}(II)=37,1$ min; FAB-MS $(M+H)^+=1055$.

**Referenz-Beispiel 46: Boc-(p-BzlO)Phe[C]Phe-(L)-Val-(L)-Phe-morpholin-4-ylamid**

Analog Referenz-Beispiel 1) werden 664 mg (0,70 mMol) 5(S)-(Boc-amino)-4(S)-(tert-butyldimethylsilyloxy)-6-(p-benzyloxyphenyl)-2(R)-(phenyl-methyl)-hexanoyl-(L)-Val-(L)-Phe-morpholin-4-ylamid mit 440 mg (1,40 mMol) TBAF in 7 ml DMF entschützt. Kristallisieren aus $^i$Propanol/Hexan liefert die reine Titelverbindung: DC $R_f(D)=0,32$; $t_{Ret}(I)=17,8$ min; FAB-MS $(M+H)^+=835$.
Das Ausgangsmaterial wird wie folgt hergestellt:

**a: N-Boc-(p-benzyloxy-phenylalaninol)**

Analog Referenz-Beispiel 21 B) 1)b) werden 37,1 g (100 mMol) Boc-(L)-(p-BzlOPhe)-OH (Bachem; Bubendorf/Schweiz) in 116 ml THF bei -5°C bis -10°C mit 15,33 ml (110 mMol) Triethylamin und 14,36 ml (110 mMol) Chlorameisensäureisobutylester in 70 ml THF aktiviert. Eintropfen des filtrierten Reaktionsgemisches in 7,57 g (200 mMol) Natriumborhydrid, Behandlung mit Wasser und Digerieren in Hexan führt zur Titelverbindung: DC $R_f(A)=0,50$; FAB-MS $(M+H)^+=358$.

**b: N-Boc-(p-benzyloxy-phenylalaninal)**

Analog Referenz-Beispiel 21 B) 1)c) werden 4,76 g (37,5 mMol) Oxalylchlorid in 33,6 ml Methylenchlorid bei -60°C mit einer Lösung von 3,5 ml (49 mMol) DMSO in 60 ml Methylenchlorid versetzt. Zugabe von 8,94 g (25 mMol) N-Boc-(p-benzyloxy-phenylalaninol) in 150 ml Methylenchlorid, 14 ml (100 mMol) Triethylamin in 30 ml Methylenchlorid und wässrige Aufarbeitung (Extraktion der wässrigen Phasen mit Essigsäureethylester) liefert die kristalline Titelverbindung: DC $R_f(A)=0,71$; $^1H$-NMR (200 MHz, $CDCl_3$): 1,44 (s, 9 H), 3,06 (d, J=6 Hz, 2 H), 4,39 (m, 1 H), 6,86-6,98 und 7,03-7,15 (2m, je 2 H), 7,30-7,48 (m, 5 H), 9,62 (s, 1 H).

**c: 5(S)-[1(S)-(Boc-amino)-2-(p-benzyloxy-phenyl)ethyl]-dihydrofuran-2-(3H)-on**

Analog Referenz-Beispiel 21 D) 1)b) wird aus 7,7 ml (57,1 mMol) 2-Jodpropionsäureethylester in 100 ml Toluol, 6,0 g (91,8mMol) Zn/Cu und 9,69 ml Dimethylacetamid das Zn-Homoenolat gebildet. Dieses überführt man mittels Kanüle zum auf -40°C bis -25°C abgekühlten Trichlor-titan-isopropylat (hergestellt aus 4,17 ml (14,2 mMol) Tetraisopropyl-orthotitanat und 4,41 ml (40,2 mMol) Titan-tetrachlorid in 12 ml Toluol und 69 ml Methylenchlorid). Man erwärmt während 5 min auf -25°C und kühlt wiederum auf -40°C ab. Anschliessend tropft man eine Lösung von 9,7 g (27 mMol) N-Boc-(p-benzyloxyphenylalaninal) in 24,5 ml Methylenchlorid zu und rührt 15 h bei ca. -20°C und schliesslich 1 h bei 0°C nach. Das Reaktionsgemisch wird auf 0,4 kg Eiswasser und 0,5 l Ether gegossen und 10 min kräftig gerührt. Man trennt die wässrige Phase ab, extrahiert sie mit 2 Portionen Ether; wäscht die organischen Phasen mit Wasser, ges. Natriumhydrogencarbonat Lösung, Wasser und Sole, trocknet sie mit Natriumsulfat und dampft sie ein (→ kristallinen 5(S)-(Boc-amino)-4(S)-hydroxy-6-(p-benzyloxyphenyl)-hexansäure-ethylester).
Das obige Zwischenprodukt wird in 220 ml Toluol und 6,73 ml Essigsäure während 2,5 h auf 100°C erhitzt. Man versetzt das erkaltete Reaktionsgemisch mit 0,5 l Wasser, trennt die wässrige Phase ab, extrahiert sie mit 2 Portionen Ether, wäscht die org. Phasen mit ges. Natriumhydrogencarbonat Lösung, Wasser und Sole, trocknet sie mit Natriumsulfat und dampft ein. Kristallisation des Rückstandes aus Ether/Hexan liefert die reine Titelverbindung: DC $R_f(E)=0,28$; $t_{Ret}(II)=23,5$ min; $^1H$-NMR (200 MHz, $CDCl_3$): 1,40 (s, 9 H), 2,03-2,2 und 2,44-2,64 und 2,73-2,98 (3m, je 2 H), 3,95 und 4,48 (2m, je 1 H), 4,62 (d, J=9 Hz, 1 H), 6,87-6,97 und 7,09-7,21 (2m, je 2 H), 7,27-7,48 (m, 5 H).

**d: 5(S)-[1(S)-(Boc-amino)-2-(p-benzyloxyphenyl)-ethyl]-3(R)-(phenylmethyl)-dihydrofuran-2-(3H)-on**

Analog Referenz-Beispiel 21 D) 1)c) werden 2,47 g (6,0 mMol) 5(S)-[1(S)-(Boc-amino)-2-(p-benzyloxyphenyl)ethyl]-dihydrofuran-2-(3H)-on gelöst in 12 ml THF und 1,2 ml 1,3-Dimethyl-3,4,5,6-tetrahydro-2(1H)-pyrimidinon bei -70°C mit 11,73 ml Lithiumbis(trimethylsilyl)amid 1 M in THF deprotoniert und mit 0,713 ml (6,0 mMol) Benzylbromid in 3 ml THF alkyliert (75 min). Säulenchromatographie ($SiO_2$, Hexan/Essigsäureethylester 4:1 → 2:1) und Kristallisation aus Ether/Hexan liefert die reine Titelverbindung: DC $R_f(D)=0,36$.

**e) 5(S)-(Boc-amino)-4(S)-hydroxy-6-(p-benzyloxyphenyl)-2(R)-(phenylmethyl)-hexansäure**

Analog Referenz-Beispiel 1i) werden 0,502 g (1,00 mMol) 5(S)-[1(S)-(Boc-amino)-2-(p-benzyloxyphenyl)-ethyl]-3(R)-(phenylmethyl)-dihydrofuran-2-(3H)-on in 16 ml Dimethoxyethan und 8,6 ml Wasser mit 4 ml 1 M Lithiumhydroxid Lösung hydrolysiert. Das teilweise eingedampfte Reaktionsgemisch giesst man auf ein Gemisch von Eis, 49 ml ges. $NH_4Cl$-Lösung, 4,1 ml 10 %-iger Zitronensäure Lösung und Methylenchlorid und setzt Ethanol zu, bis sich der ausgefallene Festkörper löst. Die wässrige Phase wird mit 2 Portionen Methylenchlorid/Ethanol ca. 9:1 extrahiert, die organischen Phasen mit Sole gewaschen, mit $Na_2SO_4$ getrocknet und eingedampft: DC $R_f$(A)=0,22.

f) **5(S)-(Boc-amino)-4(S)-(tert-butyldimethylsilyloxy)-6-(p-benzyloxy-phenyl)-2(R)-(phenyl-methyl)-hexansäure**

Analog Referenz-Beispiel 1j) werden 1,04 g (2,00 mMol) 5(S)-(Boc-amino)-4(S)-hydroxy-6-(p-benzyloxy-phenyl)-2(R)-(phenylmethyl)-hexansäure in 7 ml DMF mit 1,39 g (9,0 mMol) tert-Butyldimethylchlorsilan und 1,12 g (16,4 mMol) Imidazol silyliert. Hydrolyse der Silylesterfunktion mit 1,6 g Kaliumcarbonat in 46 ml Methanol/THF/Wasser 3:1:1 und Säulenchromatographie ($SiO_2$, Hexan/Essigsäureethylester 4:1 → 2:1 → 1:2) des Rohproduktes liefert die Titelverbindung: DC $R_f$(A)=0,69.

g) **5(S)-(Boc-amino)-4(S)-(tert-butyldimethylsilyloxy)-6-(p-benzyloxyphenyl)-2(R)-(phenyl-methyl)-hexanoyl-(L)-Val-(L)-Phe-morpholin-4-ylamid**

Analog Referenz-Beispiel 9f) werden 126,7 mg (0,200 mMol) 5(S)-(Boc-amino)-4(S)-(tert-butyldimethylsilyloxy)-6-(p-benzyloxy-phenyl)-2(R)-(phenyl-methyl)-hexansäure und 73,3 mg (0,22 mMol) H-(L)-Val-(L)-Phe-morpholin-4-ylamid (Referenz-Beispiel 1o) in 1,88 ml NMM/CH3CN 0,25 M mit 83,4 mg (0,22 mMol) HBTU umgesetzt: DC $R_f$(A)=0,33; FAB-MS $(M+H)^+$=949.

**Referenz-Beispiel 47: Boc-(p-BzlO)Phe[C]Phe-(L)-Val-(L)-(P-BzlOPhe)-morpholin-4-ylamid**

Analog Referenz-Beispiel 1) werden 788 mg (0,85 mMol) 5(S)-(Boc-amino)-4(S)-(tert-butyldimethylsilyloxy)-6-(p-benzyloxyphenyl)-2(R)-(phenyl-methyl)-hexanoyl-(L)-Val-(L)-(p-BzlOPhe)-morpholin-4-ylamid mit 534 mg (1,70 mMol) TBAF in 8,5 ml DMF entschützt. Kristallisieren aus $^i$Propanol/Hexan liefert die reine Titelverbindung: DC $R_f$(B)=0,51; $t_{Ret}$(I)=19,0 min; FAB-MS $(M+H)^+$=941.

Das Ausgangsmaterial wird wie folgt hergestellt:

a) **5(S)-(Boc-amino)-4(S)-(tert-butyldimethylsilyloxy)-6-(p-benzyloxyphenyl)-2(R)-(phenyl-methyl)-hexanoyl-(L)-Val-(L)-(p-BzlOPhe)-morpholin-4-ylamid**

Analog Referenz-Beispiel 9f) werden 560 mg (0,88 mMol) 5(S)-(Boc-amino)-4(S)-(tert-butyldimethylsilyloxy)-6-(benzyloxy-phenyl)-2(R)-(phenyl-methyl)-hexansäure und 425 mg (0,968 mMol) H-(L)-Val-(L)-(p-BzlOPhe)-morpholin-4-ylamid (Referenz-Beispiel 45 d) in 8,27 ml NMM/$CH_3CN$ 0,25 M mit 366,9 mg (0,968 mMol) HBTU umgesetzt: DC $R_f$(A)=0,33; FAB-MS $(M+H)^+$=1055.

**Referenz-Beispiel 48: Boc-(p-BzlO)Phe[C](p-BzlO)Phe-(L)-Val-(L)-Phe-morpholin-4-ylamid**

Analog Referenz-Beispiel 1) werden 1,10 g (1,0 mMol) 5(S)-(Boc-amino)-4(S)-(tert-butyldimethylsilyloxy)-6-(p-benzyloxyphenyl)-2(R)-(p-benzyloxyphenyl-methyl)-hexanoyl-(L)-Val-(L)-Phe-morpholin-4-ylamid mit 628 mg (2,0 mMol) TBAF in 10 ml DMF entschützt. Kristallisieren aus heissem $^i$Propanol liefert die reine Titelverbindung: DC $R_f$(A)=0,61; $t_{Ret}$(I)=19,1 min; FAB-MS $(M+H)^+$=941.

Das Ausgangsmaterial wird wie folgt hergestellt:

a: **5(S)-[1(S)-(Boc-amino)-2-(p-benzyloxyphenyl)-ethyl]-3(R)-(p-benzyloxyphenylmethyl)-dihydrofuran-2-(3H)-on**

Analog Referenz-Beispiel 21 D) 1)c) werden 2,47 g (6,0 mMol) 5(S)-[1(S)-(Boc-amino)-2-(p-benzyloxyphenyl)ethyl]-dihydrofuran-2-(3H)-on (Referenz-Beispiel 46 c) gelöst in 12 ml THF und 1,2 ml 1,3-Dimethyl-3,4,5,6-tetrahydro-2(1H)-pyrimidinon bei -70°C mit 11,73 ml Lithiumbis(trimethylsilyl)amid 1 M in THF deprotoniert und mit 1,946 g (6,0 mMol) p-Benzyloxybenzyljodid (Referenz-Beispiel 44a) in 3 ml THF alkyliert (60 min). Säulenchromatographie ($SiO_2$, Hexan/Essigsäureethylester 4:1) und Kristallisation aus Essigsäureethylester/Hexan liefert die reine Titelverbindung: DC $R_f$(D)=0,45; $t_{Ret}$(I)=19,9 min; FAB-MS $(M+H)^+$=608.

b) **5(S)-(Boc-amino)-4(S)-hydroxy-6-(p-benzyloxyphenyl)-2(R)-(p-benzyloxyphenylmethyl)-hexansäure**

Analog Referenz-Beispiel 1i) werden 2,7 g (4,43 mMol) 5(S)-[1(S)-(Boc-amino)-2-(p-benzyloxyphenyl)-ethyl]-3(R)-(p-benzyloxyphenylmethyl)-dihydrofuran-2-(3H)-on in 59 ml Dimethoxyethan und 31,8 ml Wasser mit 14,8 ml 1 M Lithiumhydroxid Lösung hydrolysiert. Das teilweise eingedampfte Reaktionsgemisch giesst man auf ein Gemisch von Eis, 181 ml ges. $NH_4Cl$-Lösung, 16,2 ml 10 %-iger Zitronensäure Lösung und 400 ml Essigsäureethylester und setzt THF zu, bis sich der ausgefallene Festkörper löst. Die wässrige Phase wird mit 2 Portionen Essigsäureethylester extrahiert, die organischen Phasen mit Sole gewaschen, mit $Na_2SO_4$ getrocknet, eingedampft und in Hexan digeriert: DC $R_f$(A)=0,07.

c) **5(S)-(Boc-amino)-4(S)-(tert-butyldimethylsilyloxy)-6-(p-benzyloxy-phenyl)-2(R)-(p-benzyloxy-phenyl-methyl)-hexansäure**

Analog Referenz-Beispiel 1j) werden 2,44 g (3,90 mMol) 5(S)-(Boc-amino)-4(S)-hydroxy-6-(p-benzyloxy-

phenyl)-2(R)-(p-benzyloxyphenylmethyl)-hexansäure in 14 ml DMF mit 2,70 g (17,6 mMol) tert-Butyldimethylchlorsilan und 2,18 g (32 mMol) Imidazol silyliert. Hydrolyse der Silylesterfunktion mit 3,2 g Kaliumcarbonat in 90 ml Methanol/THF/Wasser 3:1:1 und Säulenchromatographie ($SiO_2$, Hexan/Essigsäureethylester 2:1 → 1:1) des Rohproduktes liefert die Titelverbindung: DC $R_f(A)=0,53$; FAB-MS $(M+H)^+=740$.

**d) 5(S)-(Boc-amino)-4(S)-(tert-butyldimethylsilyloxy)-6-(p-benzyloxyphenyl)-2(R)-(p-benzyloxyphenyl-methyl)-hexanoyl-(L)-Val-(L)-Phe-morpholin-4-ylamid**

Analog Referenz-Beispiel 9f) werden 740 mg (1,00 mMol) 5(S)-(Boc-amino)-4(S)-(tert-butyldimethylsilyloxy)-6-(p-benzyloxy-phenyl)-2(R)-(p-benzyloxyphenyl-methyl)-hexansäure und 367 mg (1,10 mMol) H-(L)-Val-(L)-Phe-morpholin-4-ylamid (Referenz-Beispiel 1o) in 9,39 ml NMM/$CH_3CN$ 0,25 M mit 417 mg (1,10 mMol) HBTU umgesetzt: DC $R_f(A)=0,27$; FAB-MS $(M+H)^+=1055$.

**Referenz-Beispiel 49: Boc-(p-BzlO)Phe[C](p-BzlO)Phe-(L)-Val-(L)-(p-BzlOPhe)-morpholin-4-ylamid**

Analog Referenz-Beispiel 1) werden 0,85 g (0,73 mMol) 5(S)-(Boc-amino)-4(S)-(tert-butyldimethylsilyloxy)-6-(p-benzyloxyphenyl)-2(R)-(p-benzyloxyphenyl-methyl)-hexanoyl-(L)-Val-(L)-(p-BzlOPhe)-morpholin-4-ylamid mit 460 mg (1,46 mMol) TBAF in 7,3 ml DMF entschützt. Digerieren in Essigsäureethylester/Hexan liefert die reine Titelverbindung: DC $R_f(B)=0,66$; $t_{Ret}(I)=20,2$ min; FAB-MS $(M+H)^+=1047$.

Das Ausgangsmaterial wird wie folgt hergestellt:

**a: 5(S)-(Boc-amino)-4(S)-(tert-butyldimethylsilyloxy)-6-(p-benzyloxyphenyl)-2(R)-(p-benzyloxyphenyl-methyl)-hexanoyl-(L)-Val-(L)-(p-BzlOPhe)-morpholin-4-ylamid**

Analog Referenz-Beispiel 9f) werden 740 mg (1,00 mMol) 5(S)-(Boc-amino)-4(S)-(tert-butyldimethylsilyloxy)-6-(p-benzyloxy-phenyl)-2(R)-(p-benzyloxyphenyl-methyl)-hexansäure (Referenz-Beispiel 48) und 483 mg (1,10 mMol) H-(L)-Val-(L)-(p-BzlOPhe)-morpholin-4-ylamid (Referenz-Beispiel 45) in 9,39 ml NMM/$CH_3CN$ 0,25 M mit 417 mg (1,10 mMol) HBTU umgesetzt: DC $R_f(A)=0,19$.

**Referenz-Beispiel 50: Boc-Phe[C](o-F)Phe-(L)-Val-(L)-Phe-morpholin-4-ylamid**

Analog Referenz-Beispiel 1) werden 190 mg (0,219 mMol) 5(S)-(Boc-amino)-4(S)-(tert-butyldimethylsilyloxy)-6-phenyl-2(R)-(o-fluor-phenylmethyl)-hexanoyl-(L)-Val-(L)-Phe-morpholin-4-ylamid mit 138 mg (0,438 mMol) TBAF in 3 ml DMF zur Titelverbindung entschützt: DC $R_f(A)=0,23$; $t_{Ret}(I)=16,0$ min; FAB-MS $(M+H)^+=$ 747

Das Ausgangsmaterial wird wie folgt hergestellt:

**a) 5(S)-[1(S)-(Boc-amino)-2-phenyl-ethyl]-3(R)-(o-fluorphenylmethyl)-dihydrofuran-2-(3H)-on** Analog Referenz-Beispiel 21 D) 1)c) werden 5,0 g (16,37 mMol) 5(S)-[1(S)-(Boc-amino)-2-phenylethyl]-dihydrofuran-2-(3H)-on [Referenz-Beispiel 21 D) 1)b)] gelöst in 75 ml THF bei -75°C mit 32,7 ml Lithiumbis(trimethylsilyl)amid 1 M in THF deprotoniert und mit 2,1 ml (18,0 mMol) o-Fluorbenzylbromid (Fluka; Buchs/Schweiz) bei anfangs -75°C alkyliert (Aufwärmung während 60 min auf max. -60°C). Säulenchromatographie ($SiO_2$, Hexan/Essigsäureethylester 3:1) liefert die Titelverbindung: DC $R_f(D)=0,61$.

**b) 5(S)-(Boc-amino)-4(S)-hydroxy-6-phenyl-2(R)-(o-fluorphenylmethyl)-hexansäure**

Analog Referenz-Beispiel 1i) werden 4,5 g (10,8 mMol) 5(S)-[1(S)-(Boc-amino)-2-phenylethyl]-3(R)-(o-fluorphenylmethyl)-dihydrofuran-2-(3H)-on in 170 ml Dimethoxyethan mit 43,5 ml 1 M Lithiumhydroxid Lösung hydrolysiert. Den Eindampfrückstand des Reaktionsgemisches giesst man auf ein Gemisch von Eis, 120 ml ges. Ammoniumchlorid Lösung und 240 ml 10 %-iger Zitronensäure Lösung und extrahiert mit 3 Portionen Methylenchlorid. Die organischen Phasen werden mit Wasser und Sole gewaschen, über $Na_2SO_4$ getrocknet und eingedampft: $t_{Ret}(I)=14,5$ min.

**c) 5(S)-(Boc-amino)-4(S)-(tert-butyldimethylsilyloxy)-6-phenyl-2(R)-(o-fluorphenyl-methyl)-hexansäure**

Analog Referenz-Beispiel 1j) werden 1,5 g (3,47 mMol) 5(S)-(Boc-amino)-4(S)-hydroxy-6-phenyl-2(R)-(o-fluorphenylmethyl)-hexansäure in 15 ml DMF mit 2,4 g (16 mMol) tert-Butyldimethylchlorsilan und 1,95 g (28,5 mMol) Imidazol silyliert. Hydrolyse der Silylesterfunktion mit 2,8 g Kaliumcarbonat in 50 ml Methanol/THF/Wasser 4:1:1 liefert nach Säulenchromatographie ($SiO_2$, Hexan/Essigsäureethylester 2:1) die Titelverbindung: DC $R_f(D)=0,33$; $t_{Ret}(I)=20,7$ min.

**d) 5(S)-(Boc-amino)-4(S)-(tert-butyldimethylsilyloxy)-6-phenyl-2(R)-(o-fluorphenyl-methyl)-hexanoyl-(L)-Val-(L)-Phe-morpholin-4-ylamid**

Analog Referenz-Beispiel 9f) werden 150 mg (0,27 mMol) 5(S)-(Boc-amino)-4(S)-(tert-butyldimethylsilyloxy)-6-phenyl-2(R)-(o-fluorphenyl-methyl)-hexansäure und 101 mg (0,30 mMol) H-(L)-Val-(L)-Phe-morpholin-4-ylamid (Referenz-Beispiel 1o) in 2,6 ml NMM/$CH_3CN$ 0,25 M mit 115 mg (0,30 mMol) HBTU umgesetzt: $t_{Ret}$(I)=22,3 min.

**Referenz-Beispiel 51: Boc-Phe[C](o-F)Phe-(L)-Val-(L)-(p-$CH_3$O-Phe)-morpholin-4-ylamid**

Analog Referenz-Beispiel 1) werden 332 mg (0,37 mMol) 5(S)-(Boc-amino)-4(S)-(tert-butyldimethylsilyloxy)-6-phenyl-2(R)-(o-fluor-phenylmethyl)-hexanoyl-(L)-Val-(L)-(p-$CH_3$O-Phe)-morpholin-4-ylamid mit 350 mg (1,11 mMol) TBAF in 3 ml DMF entschützt. Digerieren aus DIPE und Ether liefert die Titelverbindung: DC $R_f$(B)=0,50; $t_{Ret}$(I)=16,0 min; FAB-MS $(M+H)^+$=777.

Das Ausgangsmaterial wird wie folgt hergestellt:

**a) 5(S)-(Boc-amino)-4(S)-(tert-butyldimethylsilyloxy)-6-phenyl-2(R)-(o-fluorphenyl-methyl)-hexanoyl-(L)-(p-$CH_3$O-Phe)-morpholin-4-ylamid**

Analog Referenz-Beispiel 9f) werden 205 mg (0,375 mMol) 5(S)-(Boc-amino)-4(S)-(tert-butyldimethylsilyloxy)-6-phenyl-2(R)-(o-fluorphenyl-methyl)-hexansäure (Referenz-Beispiel 50c) und 150 mg (0,412 mMol) H-(L)-Val-(L)-(p-$CH_3$O-Phe)-morpholin-4-ylamid (Referenz-Beispiel 10e) in 3,6 ml NMM/$CH_3$CN 0,25 M mit 156 mg (0,412 mMol) HBTU umgesetzt. Verrühren des Rohproduktes (Schaum) liefert die Titelverbindung: DC $R_f$(A)=0,16; $t_{Ret}$(I)=22,6 min.

**Referenz-Beispiel 52: Boc-Phe[C](m-F)Phe-(L)-Val-(L)-Phe-morpholin-4-ylamid**

Analog Referenz-Beispiel 1) werden 181 mg (0,24 mMol) 5(S)-(Boc-amino)-4(S)-(tertbutyldimethylsilyloxy)-6-phenyl-2(R)-(m-fluor-phenylmethyl)-hexanoyl-(L)-Val-(L)-Phe-morpholin-4-ylamid mit 151 mg (0,478 mMol) TBAF in 3 ml DMF entschützt. Digerieren in Hexan liefert die Titelverbindung: DC $R_f$(A)=0,54; $t_{Ret}$(I)=16,2 min; FAB-MS $(M+H)^+$=747.

Das Ausgangsmaterial wird wie folgt hergestellt:

**a) 5(S)-[1(S)-(Boc-amino)-2-phenyl-ethyl]-3(R)-(m-fluorphenylmethyl)-dihydrofuran-2-(3H)-on**

Analog Referenz-Beispiel 21 D) 1)c) werden 5,0 g (16,37 mMol) 5(S)-[1(S)-(Boc-amino)-2-phenylethyl]-dihydrofuran-2-(3H)-on [Referenz-Beispiel 21 D) 1)b)] gelöst in 75 ml THF bei -75°C mit 32,7 ml Lithiumbis(trimethylsilyl)amid 1 M in THF deprotoniert und mit 3,4 g (18,0 mMol) 3-Fluorbenzylbromid (Fluka; Buchs/Schweiz) bei anfamgs -75°C alkyliert (Aufwärmung während 60 min auf max. -50°C). Säulenchromatographie ($SiO_2$, Hexan/Essigsäureethylester 3:1) liefert die Titelverbindung: DC $R_f$(D)=0,6; $t_{Ret}$(I)=17,2 min.

**b) 5(S)-(Boc-amino)-4(S)-hydroxy-6-phenyl-2(R)-(m-fluorphenylmethyl)-hexansäure**

Analog Referenz-Beispiel 1i) werden 3,7 g (8,95 mMol) 5(S)-[1(S)-(Boc-amino)-2-phenylethyl]-3(R)-(m-fluorphenylmethyl)-dihydrofuran-2-(3H)-on in 140 ml Dimethoxyethan mit 35,8 ml 1 M Lithiumhydroxid Lösung hydrolysiert. Extraktion des Eindampfrückstands des Reaktionsgemisches aus einem Gemisch von Eis, 120 ml ges. Ammoniumchlorid Lösung und 240 ml 10 %-iger Zitronensäure Lösung mit viel Methylenchlorid (Löslichkeit!) liefert die Titelverbindung: $t_{Ret}$(I)=14,6 min.

**c) 5(S)-(Boc-amino)-4(S)-(tert-butyldimethylsilyloxy)-6-phenyl-2(R)-(m-fluorphenyl-methyl)-hexansäure**

Analog Referenz-Beispiel 1j) werden 2,7 g (6,25 mMol) 5(S)-(Boc-amino)-4(S)-hydroxy-6-phenyl-2(R)-(m-fluorphenylmethyl)-hexansäure in 30 ml DMF mit 4,33 g (28,8 mMol) tert-Butyldimethylchlorsilan und 3,51 g (51,3 mMol) Imidazol silyliert. Hydrolyse der Silylesterfunktion mit 5,1 g Kaliumcarbonat in 100 ml Methanol/THF/Wasser 4:1:1 liefert nach Säulenchromatographie ($SiO_2$, Hexan/Essigsäureethylester 2:1) die Titelverbindung: $t_{Ret}$(I)=20,8 min.

**d) 5(S)-(Boc-amino)-4(S)-(tert-butyldimethylsilyloxy)-6-phenyl-2(R)-(m-fluorphenyl-methyl)-hexanoyl-(L)-Val-(L)-Phe-morpholin-4-ylamid**

Analog Referenz-Beispiel 9f) werden 150 mg (0,27 mMol) 5(S)-(Boc-amino)-4(S)-(tert-butyldimethylsilyloxy)-6-phenyl-2(R)-(m-fluorphenyl-methyl)-hexansäure und 101 mg (0,30 mMol) H-(L)-Val-(L)-Phe-morpholin-4-ylamid (Referenz-Beispiel 1o) in 2,6 ml NMM/$CH_3$CN 0,25 M mit 115 mg (0,30 mMol) HBTU umgesetzt. Säulenchromatographie ($SiO_2$, Hexan/Essigsäureethylester 1:1) liefert die Titelverbindung: DC $R_f$(A)=0,28; $t_{Ret}$(I)=23,0 min.

**Referenz-Beispiel 53: Boc-Phe[C](m-F)Phe-(L)-Val-(L)-(p-$CH_3$O-Phe)-morpholin-4-ylamid**

Analog Referenz-Beispiel 1) werden 336 mg (0,37 mMol) 5(S)-(Boc-amino)-4(S)-(tert-butyldimethylsilyloxy)-6-phenyl-2(R)-(m-fluor-phenylmethyl)-hexanoyl-(L)-Val-(L)-(p-$CH_3$O-Phe)-morpholin-4-ylamid mit 350 mg (1,12 mMol) TBAF in 3 ml DMF entschützt. Digerieren aus DIPE und Ether liefert die Titelverbindung: DC $R_f$(B)=0,48; $t_{Ret}$(I)=16,1 min; FAB-MS $(M+H)^+$=777.

Das Ausgangsmaterial wird wie folgt hergestellt:

**a) 5(S)-(Boc-amino)-4(S)-(tert-butyldimethylsilyloxy)-6-phenyl-2(R)-(m-fluorphenyl-methyl)-hexanoyl-(L)-Val-(L)-(p-$CH_3$O-Phe)-morpholin-4-ylamid**

Analog Referenz-Beispiel 9f) werden 205 mg (0,375 mMol) 5(S)-(Boc-amino)-4(S)-(tert-butyldimethylsilyloxy)-6-phenyl-2(R)-(m-fluorphenyl-methyl)-hexansäure (Referenz-Beispiel 52c) und 150 mg (0,412 mMol) H-(L)-Val-(L)-(p-$CH_3$O-Phe)-morpholin-4-ylamid (Referenz-Beispiel 10e) in 3,6 ml NMM/$CH_3$CN (0,25 M) mit 156 mg (0,412 mMol) HBTU umgesetzt. Verrühren des Rohproduktes im Ultraschallbad in Hexan liefert die Titelverbindung: DC $R_f$(A)=0,16; $t_{Ret}$(I)=22,5 min.

**Referenz-Beispiel 54:Boc-Phe[C]Phe-(L)-Val-(L)-Leu-morpholin-4-ylamid**

Analog Referenz-Beispiel 1 werden 900 mg (1,11 mMol) 5(S)-Boc-amino-4(S)-(tert-butyldimethylsilyloxy)-6-phenyl-2(R)-phenylmethyl-hexanoyl-(L)-Val-(L)-Leu-morpholin-4-ylamid mit 608 mg (1,93 mMol) TBAF in 12 ml DMF zur Titelverbindung entschützt: $t_{Ret}$ (I) = 16 min; FAB-MS ($M+H^+$)= 695.

Das Ausgangsmaterial wird wie folgt hergestellt:

a) **Z-(S)-Val-(L)-Leu-morpholin-4-ylamid**

Analog Referenz-Beispiel 41 A) a) werden 3,98 g (10,9 mMol) Z-(L)-Val-(L)-Leu-OH (Bachem, Schweiz) mit 0,87 ml (10 mMol) Morpholin in die Titelverbindung überführt. DC $R_f$ (Methylenchlorid/Methanol 9:1) = 0,6.

b) **H-(S)-Val-(L)-Leu-morpholin-1-ylamid**

Analog Referenz-Beispiel 41 A) b) werden 4,7 g (10,9 mMol) Z-(L)-Val-(L)-Leu-morpholin-4-ylamid durch Hydrierung in Gegenwart von 1 g 10 % Pd/C in die Titelverbindung überführt. DC $R_f$ (Methylenchlorid/Methanol 9:1) = 0,3: FAB-MS ($M+H^+$)= 300.

c) **5(S)-(Boc-amino)-4(S)-(tert-butyldimethylsilyloxy)-6-phenyl-2(R)-phenylmethylhexanoyl-(L)-Val-(L)-Leu-morpholin-4-ylamid**

Analog Referenz-Beispiel 1 k) werden 750 mg (1,42 mMol) 5(S)-(Boc-amino-4(S)-(tert-butyldimethyl-silyloxy)-6-phenyl-2(R)-phenylmethylhexansäure und 508 mg (1,696 mMol) H-(L)-Val-(L)-Leu-morpholin-4-ylamid in DMF umgesetzt: $t_{Ret}$(I) = 22,4 min; FAB-MS ($M+H^+$)= 809.

**Referenz-Beispiel 55: Boc-Phe[C]Phe-(L)-Val-(L)-Ala-morpholin-4-ylamid**

Analog Referenz-Beispiel 1 werden 623 mg (0,81 mMol) 5(S)-(Boc-amino)-4(S)-(tert-butyldimethylsilyloxy)-6-phenyl-2(R)-phenylmethyl-hexanoyl-(L)-Val-(L)-Ala-morpholin-4-ylamid mit 456,5 mg (1,447 mMol) TBAF in 11 ml DMF zur Titelverbindung entschützt: $t_{Ret}$(II)= 19,6 min; FAB-MS ($M+H^+$)= 653.

Das Ausgangsmaterial wird wie folgt hergestellt:

a) **Z-(L)-Val-(L)-Ala-morpholin-1-ylamid.**

Analog Referenz-Beispiel 41 A) a) werden 2 g (6,21 mMol) Z-(L)-Val-(L)-Ala-OH (Bachem, Schweiz) mit 0,54 ml (6,21 mMol) Morpholin in die Titelverbindung überführt. DC $R_f$(Methylenchlorid/Methanol: 9/1)= 0,61.

b) **H-(L)-Val-(L)-Ala-morpholin-1-ylamid.**

Analog Referenz-Beispiel 41 A) b) werden 2,4 g (6,2 mMol) Z-(L)-Val-(L)-Ala-morpholin-1-ylamid durch Hydrierung in Gegenwart von 0,4 g 10% Pd/C in die Titelverbindung überführt. DC $R_f$(Methylenchlorid/Methanol: 9/1)=0,53; FAB-MS ($M+H^+$)= 258.

c) **5(S)-(Boc-amino)-4(S)-(tert-butyldimethylsilyloxy)-6-phenyl-2(R)-phenylmethyl-hexanoyl-(L)-Val-(L)-Ala-morpholin-1-ylamid**

Analog Referenz-Beispiel 1k werden 500 mg (0,947 mMol) 5(S)-(Boc-amino)-4(S)--(tert-butyldimethyl-silyloxy)-6-phenyl-2(R)-phenylmethyl-hexansäure und 292,4 mg (1,136 mMol) H-(L)-Val-(L)-Ala-morpholin-4-ylamid in DMF umgesetzt: $t_{Ret}$(II)= 32,4 min; FAB-MS ($M+H^+$)= 767.

Referenz-Beispiel 56: Analog einem der vorgenannten Referenzbeispiel werden folgende Verbindungen hergestellt:

A) Boc-(p-$CH_3$O)Phe[C]Phe-(L)-Val-(L)-Phe-morpholin-4-ylamid

B) Boc-(p-$CH_3$O)Phe[C](p-$CH_3$O)Phe-(L)-Val-(L)-Phe-morpholin-4-ylamid

C)Boc-(p-$CH_3$O)Phe[C](p-BzlO)Phe-(L)-Val-(L)-Phe-morpholin-4-ylamid

D) Boc-(p-$CH_3$O)Phe[C]Tyr-(L)-Val-(L)-Phe-morpholin-4-ylamid

E) Boc-(p-$CH_3$O)Phe[C]Phe-(L)-Val-(L)-(p-$CH_3$O-Phe)-morpholin-4-ylamid

F) Boc-(p-$CH_3$O)Phe[C]Phe-(L)-Val-(L)-Tyr-morpholin-4-ylamid

G)Boc-(p-$CH_3$O)Phe[C](p-$CH_3$O)Phe-(L)-Val-(L)-(p-$CH_3$O-Phe)-morpholin-4-ylamid

H)Boc-(p-($CH_3O$)Phe[C](p-$CH_3O$)Phe-(L)-Val-(L)-Tyr-morpholin-4-ylamid

I)Boc-(p-$CH_3O$)Phe[C](p-BzlO)Phe-(L)-Val-(L)-(p-$CH_3O$-Phe)-morpholin-4-ylamid

J) Boc-(p-$CH_3O$)Phe[C](p-BzlO)Phe-(L)-Val-(L)-Tyr-morpholin-4-ylamid

K) Boc-(p-$CH_3O$)Phe[C]Tyr-(L)-Val-(L)-(p-$CH_3O$-Phe)-morpholin-4-ylamid

L)Boc-(p-$CH_3$ O)Phe[C]Tyr-(L)-Val-(L)-Tyr-morpholin-4-ylamid

M) Boc-(p-$CH_3O$)Phe[C](3-$CH_3O$)Phe-(L)-Val-(L)-Phe-morpholin-4-ylamid

N) Boc-(p-$CH_3O$)Phe[C](2-$CH_3O$)Phe-(L)-Val-(L)-Phe-morpholin-4-ylamid

**O) Boc-Phe[C](2-$CH_3O$)Phe-(L)-Val-(L)-Phe-morpholin-4-ylamid**

Eine Lösung von 140,3 mg 5(S)-(Boc-amino)-4(S)-(tert-butyldimethylsilyloxy)-6-phenyl-2(R)-(2-methoxyphenylmethyl)-hexanoyl-(L)-Val-(L)-Phe-morpholin-4-ylamid in 3 ml DMF wird mit 101,6 mg TBAF versetzt und das Reaktionsgemisch 21 h bei RT gerührt. Die leicht gelbliche Lösung wird mit ca. 30 ml Essigsäureethylester verdünnt und nacheinander je einmal mit Wasser und ges. Natriumbicarbonat-Lösung und zweimal mit Sole neutral gewaschen und über Natriumsulfat getrocknet. Nach Entfernen des Lösungsmittels wird der Rückstand an Kieselgel gereinigt (Laufmittel B) und die Titelverbindung erhalten. DC $R_f$(B)=0,26. FAB-MS $(M+H)^+$=759.

Das Ausgangsmaterial wird wie folgt hergestellt:

***56 O) a) 2-Methoxybenzylchorid***

Zu 10 ml 2-Methoxybenzylalkohol (Fluka, Buchs, Schweiz) und 53,76 g Diisopropylaminomethyl-polystyrol (Polyhünigbase; Fluka, Buchs, Schweiz; Copolymer aus 98 % Styrol und 2 % Divinylbenzol, diisopropylaminomethyliert) in 200 ml abs. Ether tropft man während ca. 30 min 16,8 ml Thionylchlorid. Nach weiteren 1,5 h Rühren bei 0°C wird abgenutscht und das Filtrat am Rotationsverdampfer und am Hochvakuum eingeengt. Die Reinigung des Rückstandes erfolgt durch Chromatographie an Kieselgel (Laufmittel: Hexan/Essigsäureethylester 6:1). DC $R_f$(C)=0,5. $^1$H-NMR (200 MHz, $CDCl_3$): 7,42-7,24 (m, 2H); 7,0-6,84 (m, 2H); 4,68 (s, 2H); 3,9 (s, 3H).

**56 O) b) 2-Methoxybenzyljodid**

2 g 2-Methoxybenzylchlorid in 22 ml abs. Aceton werden mit 9,3 g Natriumjodid versetzt und bei RT über Nacht gerührt. Das Reaktionsgemisch wird mit 250 ml Ether verdünnt und mit 10%-iger Natriumthiosulfat-Lösung und Sole gewaschen. Nach Trocknen über Natriumsulfat und Entfernen des Lösungsmittels erhält man die Titelverbindung, die ohne Reinigen weiterverarbeitet wird. DC $R_f$(C)=0,46. $^1$H-NMR (200 MHz, $CDCl_3$): 7,36-7,2 (m, 2H); 6,92-6,8 (m, 2H); 4,48 (s, 2H); 3,91 (s, 3H).

**56 O) c) 5(S)-[1(S)-(Boc-amino)-2-phenylethyl]-3(R)-(2-methoxyphenylmethyl)-dihydro-furan-2-(3H)-on**

Unter $N_2$-Atmosphäre wird eine Lösung von 1 g 5(S)-[1(S)-(Boc-amino)-2-phenyl-ethyl]dihydrofuran-2-(3H)-on [Herstellung s. unter Referenz-Beispiel 2 i)] in 4 ml THF abs. und 0,66 ml DMPU auf -75°C abgekühlt, bei einer Innentemperatur von unter -70°C mit 6,42 ml Lithium-bis(trimethylsilyl)amid (1M) in THF (Aldrich, Steinheim, BRD) versetzt und 20 min bei -75°C nachgerührt. Zur Reaktionslösung tropft man innerhalb von 10 min mit einer Spritze 812 mg 2-Methoxybenzyljodid in 2 ml abs. THF, wobei die Innentemperatur -70°C nicht übersteigen darf, und rührt während 1 h bei -75°C aus. Zur klaren Lösung gibt man anschliessend mit einer Spritze bei -75°C bis -70°C 1,22 ml Propionsäure, gefolgt von 1,22 ml Wasser. Dabei steigt die Temperatur auf -30°C. Danach wird das Reaktionsgemisch mit 50 ml Essigsäureethylester verdünnt und mit 20 ml 10%-iger Zitronensäure-Lösung während 5 min kalt verrührt (Eis/Wasser-Kühlung). Die wässerige Phase wird abgetrennt, die organische Phase nacheinander mit Sole, ges. Natriumbicarbonat-Lösung und wieder mit Sole gewaschen. Die vereinigten wässerigen Phasen werden 2 mal mit Essigsäureethylester nachextrahiert. Die vereinigten organischen Phasen werden über Natriumsulfat getrocknet und eingeengt. Man erhält die Titelverbindung als bräunliches Öl. Die Reinigung erfolgt durch Chromatographie an Kieselgel. Chromatographie an Kieselgel (Laufmittel E) gibt die reine Titelverbindung. DC Rf(Hexan/Essigsäureethylester 2,5:1)=0,54. MS $M^+$=425.

**56 O) d) 5(S)-(Boc-amino)-4(S)-hydroxy-6-phenyl-2(R)-(2-methoxyphenylmethyl)-hexansäure**

Eine Lösung von 474 mg 5(S)-[1(S)-(Boc-amino)-2-phenylethyl]-3(R)-(2-methoxyphenylmethyl)-dihydrofuran-2-(3H)-on in 18 ml Dimethoxyethan und 9,07 ml Wasser wird tropfenweise mit 4,45 ml einer 1M Lithiumhydroxyd-Lösung bei RT versetzt. Danach wird das Reaktionsgemisch 3 h bei RT gerührt, mit Essigsäureethylester und THF verdünnt und im Scheidetrichter mit einem Gemisch aus 54,78 ml ges. Ammoniumchlorid-Lösung und 4,58 ml 10%-iger Zitronensäure-Lösung gefolgt von Sole und Wasser neutral gewaschen. Nach Trocknung über Natriumsulfat und Entfernung des Lösungsmittels erhält man die Titelverbindung, die ohne weitere Reinigung weiterverarbeitet wird. DC $R_f$(Hexan/Essigsäureethylester 2,5:1)=0,15.

**56 O) e) 5(S)-(Boc-amino)-4(S)-(tert-butyldimethylsilyloxy)-6-phenyl-2(R)-(2-methoxyphenyl-**

**methyl)-hexansäure**

Eine Lösung von 500 mg 5(S)-(Boc-amino)-4(S)-hydroxy-6-phenyl-2(R)-(2-methoxyphenylmethyl)-hexansäure in 5 ml DMF wird unter Rühren mit 614 mg Imidazol und 796 mg tert-Butyldimethylchlorsilan versetzt. Nach 20 h Rühren bei RT giesst man die Reaktionslösung auf Eiswasser und extrahiert mit Essigsäureethylester. Die organische Phase wird mit 10%-iger Zitronensäure-Lösung und Sole gewaschen. Nach Trocknen über Natriumsulfat wird das Lösungsmittel eingedampft und der Rückstand an Kieselgel chromatographiert (Laufmittel E und A) und die Titelverbindung erhalten. DC $R_f$(Hexan/Essigsäureethylester 2,5:1)=0,12. FAB-MS $(M+H)^+$=558.

**56 O) f) 5(S)-(Boc-amino)-4(S)-(tert-butyldimethylsilyloxy)-6-phenyl-2(R)-(2-methoxyphenylmethyl)-hexanoyl-(L)-Val-(L)-Phe-morpholin-4-ylamid**

Ein Gemisch von 100 mg 5(S)-(Boc-amino)-4(S)-(tert-butyldimethylsilyloxy)-6-phenyl-2 (R)-(2-methoxyphenylmethyl)-hexansäure, 74,71 mg HBTU und 65,68 mg H-(L)-Val-(L)-Phe-morpholin-4-ylamid [Herstellung s. unter Referenzbeispiel 1 o)] in 1,68 ml einer 0.25 M Lösung von NMM in Acetonitril wird 15 h bei RT unter Argon gerührt. Die Lösung wird zur Trockne eingeengt, der Rückstand in Essigsäureethylester aufgenommen und nacheinander mit 10%-iger Zitronensäure, Wasser, ges. Natriumbicarbonat-Lösung und Sole gewaschen. Nach Trocknen über Natriumsulfat und Entfernung des Lösungsmittels erhält man die Titelverbindung, die ohne Reinigen weiterverarbeitet wird. DC $R_f$(A)=0,20. FAB-MS $(M+H)^+$=873.

P) **Boc-Phe[C](3-$CH_3$O)Phe-(L)-Val-(L)-Phe-morpholin-4-ylamid**

Analog Ref.-Bsp. 56 O) werden 356 mg 5(S)-(Boc-amino)-4(S)-(tert-butyldimethylsilyloxy)-6-phenyl-2(R)-(3-methoxyphenylmethyl)-hexanoyl-(L)-Val-(L)-Phe-morpholin-4-ylamid in 6,09 ml DMF mit 206 mg TBAF zur Titelverbindung desilyliert. Die Reinigung erfolgt durch Chromatographie an Kieselgel (Laufmittel B). DC $R_f$(B)=0,37. FAB-MS $(M+H)^+$=759.

Das Ausgangsmaterial wird wie folgt hergestellt:

**56 P) a) 3-Methoxybenzyljodid**

Analog Referenz-Beispiel 56 O) b) erhält man aus 2 ml 3-Methoxybenzylchlorid (Fluka, Buchs, Schweiz) und 9,72 g Natriumjodid in 23 ml abs. Aceton die Titelverbindung. DC $R_f$(Hexan/Essigsäureethylester 2,5:1)=0,71. $^1$H-NMR (200 MHz, $CDCl_3$): 7,20 (m, 1H); 7,0-6,87 (m, 2H); 6,78 (dxd, 1H); 4,42 (s, 2H); 3,8 (s, 3H).

**56 P) b) 5(S)-[1(S)-(Boc-amino)-2-phenylethyl]-3(R)-(3-methoxyphenylmethyl)-dihydrofuran-2-(3H)-on**

Analog zu Referenz-Beispiel 56O/c) werden 1,5 g 5(S)-[1(S)-(Boc-amino)-2-phenylethyl]-dihydrofuran-2-(3H)-on [hergestellt nach Ref.-Bsp. 2i)] in 3 ml abs. THF mit 9,62 ml Lithium-bis-(trimethylsilyl-)amid (1M in THF) und unter Zugabe von 0,998 ml DMPU deprotoniert (-75°C) und mit 1,22 g 3-Methoxybenzyljodid alkyliert. Chromatographie an Kieselgel (Laufmittel E) liefert die reine Titelverbindung. DC $R_f$ (Hexan/Essigsäureethylester 2,5:1)=0,32. FAB-MS $(M+H)^+$=426.

**56 P) c) 5(S)-(Boc-amino)-4(S)-hydroxy-6-phenyl-2(R)-(3-methoxyphenylmethyl)-hexansäure**

Analog Referenz-Beispiel 56 O) d) werden 1,315 g 5(S)-[1(S)-(Boc-amino)-2-phenylethyl]-3(R)-(3-methoxyphenylmethyl)-d ihydrofuran-2-(3H)-on in 49,9 ml Dimethoxyethan und 25,16 ml Wasser mit 12,36 ml Lithiumhydroxyd-Lösung 1M zur Titelverbindung hydrolysiert, die direkt weiterverarbeitet wird. DC $R_f$(A)=0,09. FAB-MS (M+H)+ =444.

**56 P) d) 5(S)-(Boc-amino)-4(S)-(tert-butyldimethylsilyloxy)-6-phenyl-2(R)-(3-methoxyphenylmethyl)-hexansäure**

Analog Referenz-Beispiel 56 O) e) werden 1,3 g 5(S)-(Boc-amino)-4(S)-hydroxy-6-phenyl-2(R)-(3-methoxyphenylmethyl)-hexansäure in 13 ml DMF mit 1,987 g tert-Butyldimethylchlorsilan und 1,646 g Imidazol silyliert. Chromatographie an Kieselgel (Laufmittel: E, D und A) ergibt die reine Titelverbindung. DC $R_f$(D)=0,06. FAB-MS $(M+H)^+$=558.

**56 P) e) 5(S)-(Boc-amino)-4(S)-(tert-butyldimethylsilyloxy)-6-phenyl-2(R)-(3-methoxy-phenylmethyl)-hexanoyl-(L)-Val-(L)-Phe-morpholin-4-ylamid**

Analog Ref.-Bsp. 56 O) f) werden 192,2 mg 5(S)-(Boc-amino)-4(S)-(tert-butyldimethylsilyloxy)-6-phenyl-2( R)-(3-methoxyphenylmethyl)-hexansäure und 126,4 mg H-(L)-Val-(L)-Phe-morpholin-4-ylamid [hergestellt nach Ref.-Bsp. 1o)] in 3,23 ml NMM/$CH_3CN$ 0,25 M mit 143,7 mg HBTU zur Titelverbindung umgesetzt. DC $R_f$(A)=0,25. FAB-MS $(M+H)^+$=873.

Q)Boc-Phe[C](3-$CH_3$O)Phe-(L)-Val-(L)-(p-$CH_3$O-Phe)-morpholin-4-ylamid

R)Boc-Phe[C](2-$CH_3$O)Phe-(L)-Val-(L)-(p-$CH_3$O-Phe)-morpholin-4-ylamid

S) **Boc-Phe[C](p-BzlO)Phe-(L)-Val-(L)-(p-$CH_3$O-Phe)-morpholin-4-ylamid**

Analog Referenz-Beispiel 1) werden 345 mg (0,352 mMol) 5(S)-(Boc-amino)-4(S)-(tert-butyldimethylsilyloxy)-6-phenyl-2(R)-[(p-benzyloxy-phenyl)methyl] -hexanoyl-(L)-Val-(L)-(p-$CH_3$O-Phe)-morpholin-4-yla-mid mit 222 mg (0,704 mMol) TBAF in 5 ml DMF entschützt. Digerieren aus DIPE im Ultraschallbad liefert die Titelverbindung: DC $R_f(B)=0,28$; $t_{Ret}(I)=17,6$ min; FAB-MS $(M+H)^+=865$.

Das Ausgangsmaterial wird wie folgt hergestellt:

a) **5(S)-(Boc-amino)-4(S)-(tert-butyldimethylsilyloxy)-6-phenyl-2(R)-[(p-benzyloxyphenyl)methyl-]hexanoyl-(L)-Val-(L)-(p-$CH_3$O-Phe)-morpholin-4-ylamid**

Analog Referenz-Beispiel 9f) werden 263 mg (0,415 mMol) 5(S)-(Boc-amino)-4(S)-(tert-butyldimethylsilyloxy)-6-phenyl-2(R)-[(p-benzyloxy-phenyl)methyl]-hexansäure (Referenz-Beispiel 44d) und 137 mg (0,377 mMol) H-(L)-Val-(L)-(p-$CH_3$O-Phe)-morpholin-4-ylamid (Referenz-Beispiel 10e) in 3,6 ml NMM/$CH_3$CN 0,25 M mit 157 mg (0,415 mMol) HBTU zur Titelverbindung umgesetzt: $t_{Ret}(I)=23,5$ min.

**Referenz-Beispiel 57: Boc-Phe[C]Tyr-(L)-Val-(L)-(p-$CH_3$O-Phe)-morpholin-4-ylamid**

Hydrieren von 70 mg (0,081 mMol) Boc-Phe[C](p-BzlO)Phe-(L)-Val-(L)-(p-$CH_3$O-Phe)-morpholin-4-ylamid (Referenz-Beispiel 56S)) in 8 ml Methanol in Gegenwart von 35 mg 10% Pd/C liefert nach Filtration durch Celite und Eindampfen die Titelverbindung: DC $R_f(B)=0,17$; $t_{Ret}(I)=14,1$ min; FAB-MS $(M+H)^+=775$.

**Referenz-Beispiel 58:Boc-Phe[C](p-CN)Phe-(L)-Val-(L)-(p-$CH_3$O-Phe)-morpholin-4-ylamid**

Analog Referenz-Beispiel 1) werden 322 mg (0,36 mMol) 5(S)-(Boc-amino)-4(S)-(tert-butyldimethylsilyloxy)-6-phenyl-2(R)-(p-cyano-phenylmethyl)-hexanoyl-(L)-Val-(L)-(p-$CH_3$O-Phe)-morpholin-4-ylamid mit 340 mg (1,08 mMol) TBAF in 3 ml DMF während 19 h entschützt. Digerieren des Rohproduktes aus DIPE im Ultraschallbad liefert die Titelverbindung: DC $R_f(Y)=0,41$; $t_{Ret}(I)=15,4$ min; FAB-MS $(M+H)^+=784$.

Das Ausgangsmaterial wird wie folgt hergestellt:

a) **5(S)-(Boc-amino)-4(S)-(tert-butyldimethylsilyloxy)-6-phenyl-2(R)-(p-cyanophenylmethyl)-hexanoyl-(L)-Val-(L)-(p-$CH_3$O-Phe)-morpholin-4-ylamid**

Unter $N_2$-Atmosphäre werden 200 mg (0,362 mMol) 5(S)-(Boc-amino)-4(S)-(tert-butyldimethylsilyloxy)-6-phenyl-2(R)-(p-cyano-phenylmethyl)-hexansäure [Referenz-Beispiel 21 E)1)c)] in 6 ml abs. THF gelöst und bei 5°C mit 82 mg (0,398 mMol) DCC versetzt. Nach 10 min gibt man 145 mg (0,398 mMol) H-(L)-Val-(L)-(p-$CH_3$O-Phe)-morpholin-4-ylamid (Referenz-Beispiel 10e) und 54 mg (0,398 mMol) HOBT zu und rührt während 17 h bei RT. Das Reaktionsgemisch wird filtriert, das Filtrat in Essigsäureethylester aufgenommen und mit 10 %-iger Zitronensäure Lösung, Wasser ges. $NaHCO_3$ Lösung und Sole gewaschen. Zweimaliges Extrahieren der wässrigen Phasen mit Essigsäureethylester, Trocknen der organischen Phasen mit $Na_2SO_4$, Eindampfen und Digerieren in DIPE liefert die Titelverbindung: $t_{Ret}(I)=21,8$ min; FAB-MS $(M+H)^+=898$.

**Referenz-Beispiel 59: 5(S)-(Boc-amino)-4(S)-hydroxy-6-phenyl-2(R)-[(2,4-difluorphenyl)-methyl]-hexanoyl-(L)-Val-(L)-Phe-morpholin-4-ylamid**

Analog Referenz-Beispiel 1) werden 264 mg (0,30 mMol) 5(S)-(Boc-amino)-4(S)-(tert-butyldimethylsilyloxy)-6-phenyl-2(R)-[(2,4-difluorphenyl)-methyl]-hexanoyl-(L)-Val-(L)-Phe-morpholin-4-ylamid mit 190 mg (0,60 mMol) TBAF in 5 ml DMF während 17 h entschützt. Digerieren des Rohproduktes aus wenig Methylenchlorid und DIPE/Hexan 3:1 im Ultraschallbad liefert die Titelverbindung: DC $R_f(B)=0,71$; $t_{Ret}(I)=16,1$ min; FAB-MS $(M+H)^+=765$.

Das Ausgangsmaterial wird wie folgt hergestellt:

a) **5(S)-[1(S)-(Boc-amino)-2-phenyl-ethyl]-3(R)-[(2,4-difluorphenyl)methyl]-dihydrofuran-2-(3H)-on**

Analog Referenz-Beispiel 21 D) 1)c) werden 5,0 g (16,37 mMol) 5(S)-[1(S)-(Boc-amino)-2-phenylethyl]-dihydrofuran-2-(3H)-on [Referenz-Beispiel 21 D) 1)b)] gelöst in 100 ml THF bei -75°C mit 32,7 ml Lithiumbis(trimethylsilyl)amid 1 M in THF deprotoniert und mit 2,51 ml (19,6 mMol) 2,4-Difluorbenzylbromid (Aldrich; Milwaukee/USA) bei anfangs -75°C alkyliert (Aufwärmung während 2 h auf max. -60°C). Säulenchromatographie ($SiO_2$, Hexan/Essigsäureethylester 2:1) liefert die Titelverbindung: DC $R_f(D)=0,5$; $t_{Ret}(I)=17,2$ min.

b) **5(S)-(Boc-amino)-4(S)-hydroxy-6-phenyl-2(R)-[(2,4-difluorphenyl)methyl]hexansäure**

Analog Referenz-Beispiel 1i) werden 3,1 g (7,18 mMol) 5(S)-[1(S)-(Boc-amino)-2-phenyl-ethyl]-3(R)-[(2,4-difluorphenyl)methyl]-dihydrofuran-2-(3H)-on in 77 ml Dimethoxyethan und 19 ml Wasser mit 28,7 ml 1 M Lithiumhydroxid Lösung hydrolysiert (19 h RT): $t_{Ret}(I)=14,7$ min.

c) **5(S)-(Boc-amino)-4(S)-(tert-butyldimethylsilyloxy)-6-phenyl-2(R)-[(2,4-difluorphenyl)methyl]-hexansäure**

Analog Referenz-Beispiel 1j) werden 3,2 g (7,12 mMol) 5(S)-(Boc-amino)-4(S)-hydroxy-6-phenyl-2(R)-[(2,4-difluorphenyl)methyl]-hexansäure in 67 ml DMF mit 4,93 g (32,7 mMol) tert-Butyldimethylchlorsilan

und 3,97 g (58,4 mMol) Imidazol silyliert. Hydrolyse der Silylesterfunktion mit 5,9 g Kaliumcarbonat in 77 ml Methanol, 20 ml THF und 20 ml Wasser liefert nach Säulenchromatographie ($SiO_2$, Hexan/Essigsäureethylester 2:1) die Titelverbindung: DC $R_f$(D)=0,22; $t_{Ret}$(I)=20,8 min.

d) **5(S)-(Boc-amino)-4(S)-(tert-butyldimethylsilyloxy)-6-phenyl-2(R)[(2,4-difluorphenyl)-methyl]-hexanoyl-(L)-Val-(L)-Phe-morpholin-4-ylamid**

Analog Referenz-Beispiel 9f) werden 200 mg (0,35 mMol) 5(S)-(Boc-amino)-4(S)-(tert-butyldimethylsilyloxy)-6-phenyl-2(R)-[(2,4-difluorphenyl)methyl]-hexansäure und 130 mg (0,39 mMol) H-(L)-Val-(L)-Phe-morpholin-4-ylamid (Referenz-Beispiel 1o) in 3,4 ml NMM/$CH_3CN$ 0,25 M mit 148 mg (0,39 mMol) HBTU zur Titelverbindung umgesetzt: $t_{Ret}$(I)=22,4 min.

**Referenz-Beispiel 60: Boc-Phe[C]Phe-(L)-Val-(L)-Phe-trans-(2,6)-dimethyl-morpholin-4-ylamid:**

Analog Referenzbeispiel 1 werden 770 mg (0,89 mMol) 5(S)-(Boc-amino)-4(S)-(tert-butyldimethylsilyloxy)-6-phenyl-2(R)-(phenylmethyl)-hexanoyl-(L)-Val-(L)-Phe-trans-(2,6)-dimethyl-morpholin-4-ylamid mit 280 mg (0,89 mMol) TBAF in 10 ml DMF entschützt. Digerieren aus DIPE und Ether liefert die Titelverbindung: DC $R_f$(D')=0,26; $t_{Ret}$(I)=17,1 min.; FAB-MS$(M+H)^+$=757.

Die Ausgangsverbindung wird folgendermassen hergestellt:

a) **5(S)-(Boc-amino)-4(S)-(tert-butyldimethylsilyloxy)-6-phenyl-2(R)-(phenylmethyl)hexanoyl-(L)-Val-(L)-Phe-trans-(2,6)-dimethyl-morpholin-4-ylamid:**

Analog Referenzbeispiel 9f) werden 420 mg (0,97 mMol) 5(S)-(Boc-amino)-4(S)-(tert-butyldimethylsilyloxy)-6-phenyl- 2(R)-(phenylmethyl)-hexansäure (Referenzbeispiel 50c) und 512 mg (0,97 mMol) H-(L)-Val-(L)-Phe-trans-(2,6)-dimethyl-morpholin-4-ylamid in 10 ml NMM/$CH_3CN$ 0,25M mit 405 mg HBTU umgesetzt. Chromatographie auf Kieselgel mit Essigsäureethylester/Hexan (1:1) liefert die Titelverbindung: $t_{Ret}$(I)=22,9 min.

b) **H-(L)-Val-(L)-Phe-trans-(2,6)-dimethyl-morpholin-4-ylamid:**

Eine Lösung von 400 mg (1 mMol) Z-(L)-Phe-(L)-Val-OH und 120 mg (1 mMol) trans-(2,6)-Dimethylmorpholin in 10 ml NMM/$CH_3CN$ 0,25 M wird mit 402 mg (1,1 mMol) HBTU versetzt und während 96 h bei RT gerührt. Das Reaktionsgemisch wird mit Ether verdünnt und nacheinander mit Wasser, 10 % Citronensäure, ges. Natriumbicarbonatlösung, Wasser und ges. Kochsalzlösung gewaschen. Die organischen Phasen werden über Natriumsulfat getrocknet und eingedampft. Der Rückstand wird wie in Referenzbeispiel 1 m) beschrieben mit Pd/C in Methanol hydriert und ergibt die Titelverbindung als amorphen Festkörper.

**Referenz-Beispiel 61: Boc-Phe[C](p-isobutyloxy)Phe-(L)-Val-(L)-Phe-morpholin-4-ylamid:**

Eine Lösung von 155 mg (0,2 mMol) Boc-Phe[C]Tyr-(L)-Val-(L)-Phe-morpholin-4-ylamid aus Referenzbeispiel 41D in 18 ml Dioxan wird mit 233 mg (0,7 mMol) Caesiumcarbonat versetzt und während 12 h gerührt. Zur milchigen Suspension gibt man 1,1 ml (9,4 mMol) Isobutyliodid, rührt das Reaktiongemisch während 2 h bei RT und erhitzt anschliessend während 6 h auf 80 °C. Nach dem Abkühlen wird mit Methylenchlorid verdünnt, vom Festkörper abfiltriert und das Filtrat eingedampft. Man erhält die Titelverbindung nach chromatographischer Reinigung auf Kieselgel mit Essigsäureethylester/Hexan (95:5): DC $R_f$(C')=0,56; $t_{Ret}$(I)=18,1 min.; FAB-MS $(M+H)^+$=801.

**Referenz-Beispiel 62: Boc-Phe[C](p-isobutyloxy)Phe-(L)-Val-(L)-(p-isobutyloxy-Phe)-morpholin-4-ylamid:**

Analog Referenzbeispiel 61 erhält man ausgehend von 114 mg (0,14 mMol) 5(S)-(Bocamino)-4(S)-hydroxy-6-phenyl-2(R)-(4-hydroxyphenylmethyl)-hexanoyl-(L)-Val-(L)-(p-isobutyloxy-Phe)-morpholin-4-ylamid, 163 mg (0,5 mMol) Caesiumcarbonat und 0,4 ml (3,4 mMol) Isobutyliodid die Titelverbindung nach chromatographischer Reinigung auf Kieselgel mit Essigsäureethylester: DC $R_f$(C')=0,55 $t_{Ret}$(I)=20,1.;FAB-MS $(M+H)^+$=873.

Das Ausgangsmaterial wird folgendermassen hergestellt:

a) **5(S)-(Boc-amino)-4(S)-hydroxy-6-phenyl-2(R)-(4-hydroxyphenylmethyl)-hexanoyl-(L)-Val-(L)-(p-isobutyloxy-Phe)-morpholin-4-ylamid:**

Eine Lösung von 408 mg (0,4 mMol) 5(S)-(Boc-amino)-4(S)-(tert-butyldimethylsilyloxy)-6-phenyl-2(R)-(4-benzyloxy-phenylmethyl)-hexanoyl-(L)-Val-(L)-(p-isobutyloxy-Phe)-morpholin-4-ylamid in 6 ml Methanol wird mit 76 mg Pd/C 5 % in Gegenwart von 1 atm Wasserstoffdruck während 5 h hydriert. Man filtriert vom Katalysator ab, dampft das Filtrat ein und chromatographiert den Rückstand auf Kieselgel mit Essigsäureethylester/Methanol (19:1). Auf diese Weise erhält man nebst 5(S)-(Boc-amino)-4(S)-(tert-butyldimethylsilyloxy)-6-phenyl-2(R)-(4-hydroxy-phenylmethyl)-hexanoyl-(L)-Val-(L)-(p-isobutyloxy-Phe)-mor pholin-4-ylamid als Nebenprodukt die Titelverbindung: DC $R_f$(C')=0,37.

b) 5(S)-(Boc-amino)-4(S)-(tert-butyldimethylsilyloxy)-6-phenyl-2-(R)-(4-benzyloxyphenylmethyl)-hexanoyl-(L)-Val-(L)-(p-isobutyloxy-Phe)-morpholin-4-ylamid:

Analog Referenzbeispiel 9f werden 291 mg (0,45 mMol) 5(S)-(Boc-amino)-4(S)-(tert-butyldimethylsilyloxy)-6-phenyl-2(R)-(4-benzyloxy-phenylmethyl)-hexansäure (Referenzbeispiel 44d) und 203 mg (0,5 mMol) H-(L)-Val-(L)-(p-isobutyloxy-Phe)-morpholin-4-ylamid (Referenzbeispiel 70b) in 4,6 ml NMM/$CH_3CN$ 0,25 M mit 193 mg HBTU umgesetzt: DC $R_f$(E')=0,39; FAB-MS $(M+H)^+$=1021.

**Referenz-Beispiel 63: 5(S)-[4-(Tetrahydropyranyl)oxycarbonyl-amino]-4(S)-hydroxy-6-phenyl-2(R)-benzy1-hexanoyl-(L)-Val-(L)-Phe-morpholin-4-ylamid**

Es werden 500 mg (0,795 mMol) 5(S)-(Amino)-4(S)-hydroxy-6-phenyl-2(R)-benzylhexanoyl-(L)-Val-(L)-Phe-morpholin-4-ylamid mit 0,331 ml (2,38 mMol) Triethylamin versetzt und unter Abkühlen auf 5°C mit 262 mg (1,59 mMol) Chlorameisensäure-4-tetrahydropyranylester (Chemical Abstracts-Registry No. 89641-80-5) umgesetzt. Nach einer weiteren Stunde Rühren bei RT wird das Reaktionsgemisch auf Wasser gegossen und 3 mal mit Essigsäureethylester extrahiert. Die vereinigten organischen Phasen werden mit Wasser, gesättigter Natriumbicarbonat-Lösung und Sole gewaschen und nach Trocknen über Natriumsulfat unter vermindertem Druck eingeengt. Die Titelverbindung wird durch Säulenchromatographie gereinigt ($SiO_2$, Methylenchlorid/Methanol); DC $R_f$ (B)= 0,3; $t_{Ret}$(I) = 14,28 min; FAB-MS $(M+H^+)$= 757.

**Referenz-Beispiel 64: 5-(S)-[3(S)-(Tetrahydrofuranyl)oxycarbonyl-amino]-4(S)-hydroxy-6-phenyl-2(R)-benzyl-hexanoyl-(L)-Val-(L)-Phe-morpholin-4-ylamid**

Analog Referenz-Beispiel 63 werden 500 mg (0,795 mMol) 5(S)-(Amino)-4(S)-hydroxy-6-phenyl-2(R)-benzyl-hexanoyl-(L)-Val-(L)-Phe-morpholin-4-ylamid mit 363 mg (2,41 mMol) Chlorameisensäure-3(S)-tetrahydrofuranylester umgesetzt. Die Titelverbindung wird durch Fällung mit Ether gereinigt; DC $R_f$ (B)= 0,25; $t_{Ret}$(I)= 13,86 min; FAB-MS $(M+H^+)$= 743.

Das Ausgangsmaterial wird folgendermassen hergestellt:

a) Chlorameisensäure-3(S)-tetrahydrofuranylester

14,1 mL (27,24 mMol) einer 20 %igen Lösung von Phosgen in Toluol wird tropfenweise zu 14 mL Toluol gegeben. Nach Kühlung im Eisbad wird mit einer Lösung von 2 g (22,7 mMol) (S)-(+)-3-Hydroxy-tetrahydrofuran (JPS CHIMIE, Bevaix, Schweiz) in wenig Toluol versetzt, und nach 1 h Rühren bei RT wird das überschüssige Phosgen mit Argon ausgetrieben. Nach Einengen am Rotationsverdampfer unter vermindertem Druck erfolgt zwecks reinigung eine Destillation. Kochpunkt bei 12 torr: 130 °C.

**Referenz-Beispiel 65: 5(S)-[3(R)-(Tetrahydrofuranyl)oxycarbonyl-amino]-4(S)-hydroxy-6-phenyl-2(R)-benzyl-hexanoyl-(L)-Val-(L)-Phe-morpholin-4-ylamid**

Analog Referenz-Beispiel 63 werden 500 mg (0,795 mMol) 5(S)-(Amino)-4(S)-hydroxy-6-phenyl2(R)-benzyl-hexanoyl-(L)-Val-(L)-Phe-morpholin-4-ylamid mit 363 mg (2,41 mMol) Chlorameisensäure-3(R)-tetrahydrofuranylester umgesetzt. Die Titelverbindung wird durch Säulenchromatographie gereinigt ($SiO_2$, Essigsäureethylester bis Essigsäureethylester/Aceton:9/1 ); DC $R_f$(Essigsäureethylester/Aceton: 4/1)= 0,62; $t_{Ret}$(IV) = 14,06 min; FAB-MS $(M+H^+)$= 743.

Das Ausgangsmaterial wird folgendermassen hergestellt:

a) Chlorameisensäure-3(R)-tetrahydrofuranylester

12,5 mL (24,15 mMol) einer 20 %igen Lösung von Phosgen in Toluol werden nach Kühlung im Eisbad tropfenweise mit einer Lösung von 1,06 g (12,03 mMol) (R)-(+)-3-Hydroxytetrahydrofuran (JPS CHIMIE, Bevaix, Schweiz) in wenig Toluol versetzt, und nach 2 h Rühren bei RT wird das überschüssige Phosgen mit Argon ausgetrieben. Nach Einengen am Rotationsverdampfer unter vermindertem Druck wird die rohe Titelverbindung ohne weitere Reinigung weiterverarbeitet.

**Referenz-Beispiel 66: 5(S)-Ethoxycarbonyl-amino]-4(S)-hydroxy-6-phenyl-2(R)-benzyl-hexanoyl-(L)-Val-(L)-Phe-morpholin-4-ylamid**

Analog Referenz-Beispiel 63 werden 500 mg (0,795 mMol) 5(S)-(Amino)-4(S)-hydroxy-6-phenyl-2(R)-benzyl-hexanoyl-(L)-Val- (L)-Phe-morpholin-4-ylamid mit 0,151 ml (1,585 mMol) Chlorameisensäure-ethylester

Fluka, Buchs, Schweiz) umgesetzt. Die Titelverbindung wird durch Kristallisation (Essigsäureethylester/Ether) gereinigt; DC $R_f$(B)= 0,4; $t_{Ret}$(IV)= 14,51 min; FAB-MS (M+H$^+$)= 701.

**Referenz-Beispiel 67: 5(S)-(Boc-amino)-4(S)-hydroxy-6-cyclohexyl-2(R)-(4-benzyl-oxyphenyl-methyl)-hexanoyl-(L)-Val-(L)-Phe-morpholin-4-ylamid**

Analog Referenz-Beispiel 1 werden 1,45 g (1,517 mMol) 5(S)-(Boc-amino)-4(S)-(tertbutyldimethylsilyloxy)-6-cyclohexyl-2(R)-(4-benzyloxybenzyl)-hexanoyl-(L)-Val-(L)-Phe-morpholin-4-ylamid in 12 ml DMF mit 0,958 g (3,03 mMol) TBAF-Trihydrat zur Titelverbindung umgesetzt. Die Titelverbindung wird durch Kristallisation (Hexan) gereinigt. DC $R_f$ (B)= 0,5; $t_{Ret}$(IV)= 18,99 min; FAB-MS (M+H$^+$)= 841.
Die Ausgangsverbindungen werden folgendermassen hergestellt:

**a) 5(S)-[1(S)-(Boc-amino)-2-cyclohexylethyl]-dihydrofuran-2-(3H)-on**

Eine Lösung von 5 g (16,37 mMol) 5(S)-[1(S)-(Boc-amino)-2-phenylethyl]-dihydrofuran-2-(3H)-on in 50 ml Methanol wird in Gegenwart von 0,5 g Nishimura-Katalysator unter Normaldruck 2 h bei RT hydriert. Nach Abfiltrieren des Katalysators wird am Rotationsverdampfer eingeengt und am Hochvakuumgetrocknet. DC $R_f$ (D)= 0,5; FAB-MS (M+H$^+$)= 312.

**b)5(S)-[1(S)-(Boc-amino)-2-cyclohexylethyl]-3(R)-(4-benzyloxybenzyl)-dihydro**furan-2-(3H)-on

Analog Referenz-Beispiel 21 D) 1) c) werden 30,9 g (99,26 mMol) 5(S)-[1(S)-(Boc-amino)-2-cyclohexylethyl]-dihydrofuran-2-(3H)-on mit 200 ml (200 mMol) Lithium-bis(trimethylsilyl)amid 1M in THF und 34 g (104,8 mMol) 4-Benzyloxybenzyljodid zur Titelverbindung umgesetzt. Die Titelverbindung wird durch Säulenchromatographie ($SiO_2$, Hexan/Essigsäureethylester: 4/1 bis 1/1) und Kristallisation (Hexan/Essigsäureethylester) gereinigt; DC $R_f$ (C)= 0,33; $t_{Ret}$(IV)= 20,41 min; FAB-MS (M+H$^+$)= 508.

**c) 5(S)-(Boc-amino)-4(S)-hydroxy-6-cyclohexyl-2(R)-(4-benzyloxy-benzyl)-hexansäure**

Analog Referenz-Beispiel 1 i) werden 2,4 g (4,728 mMol) 5(S)-[1(S)-(Boc-amino)-2-cyclohexylethyl]-3(R)-(4-benzyloxy-benzyl)-dihydrofuran-2-(3H)-on in 10 ml 1,2-Dimethoxyethan mit 9,45 ml 1M LiOH-Lösung zur Titelverbindung umgesetzt. Diese wird durch Kristallisation aus Hexan gereinigt. DC $R_f$ (E) = 0,33; $t_{Ret}$(IV)= 18 min; FAB-MS (M+H$^+$)= 526.

**d) 5(S)-(Boc-amino)-4(S)-(tert-butyldimethyl)silyloxy-6-cyclohexyl-2(R)-(4-benzyloxy-benzyl)-hexansäure.**

Analog Referenz-Beispiel 1 j) werden 28,8 g (54,8 mMol) 5(S)-(Boc-amino)-4(S)-hydroxy-6-cyclohexyl-2(R)-(4-benzyloxy-benzyl)-hexansäure in 288 ml DMF mit 35,8 g (237,6 mMol) tert-Butyldimethylchlorsilan und 30 g (237,6 mMol) Imidazol in die Titelverbindung überführt. Die Titelverbindung wird durch Säulenchromatographie gereinigt ($SiO_2$, Hexan/Essigsäureethylester: 4/1 bis 1/1); DC $R_f$ (E)= 0,33; $t_{Ret}$(IV)= 23,72 min;

**e) 5(S)-(Boc-amino)-4(S)-(tert-butyldimethyl)silyloxy-6-cyclohexyl-2(R)-(4-hydroxyphenyl-methyl)-hexanoyl-(L)-Val-(L)-Phe-morpholin-4-ylamid**

Es werden 3 g (4,69 mMol) 5(S)-(Boc-amino)-4(S)-(tert-butyldimethylsilyloxy)-6-cyclohexyl-2(R)-(4-benzyloxy-benzyl)-hexansäure in 40 ml DMF mit 1,91 g (5,16 mMol) H-(L)-Val-(L)-Phe-morpholin-4-ylamid im Eisbad auf 5 °C gekühlt und mit 0,783 ml (5,16 mMol) DEPC und 2,3 ml (16,41 mMol) Triethylamin versetzt. Nach 1,5 h Rühren bei RT wird auf Wasser gegossen und 3 mal mit Essigsäureethylester extrahiert. Die vereinigten organischen Phasen werden mit Wasser, gesättigter Natrium bicarbonat Lösung (2 mal) und Sole gewaschen und nach Trocknen über Natriumsulfat unter vermindertem Druck eingeengt. Die Titelverbindung wird durch Säulenchromatographie gereinigt ($SiO_2$, Hexan/Essigsäureethylester: 1/1); DC $R_f$ (A)= 0,3; $t_{Ret}$(IV) = 25,3 min; FAB-MS (M+H$^+$)= 955.

**Referenz-Beispiel 68: 5(S)-(Boc-amino)-4(S)-hydroxy-6-cyclohexyl-2(R)-(4-hydroxyphenyl-methyl)-hexanoyl-(L)-Val-(L)-Phe-morpholin-4-ylamid**

Analog Referenz-Beispiel 1 werden 0,69 g (0,797 mMol) 5(S)-(Boc-amino)-4(S)-(tert-butyldimethylsilyloxy)-6-cyclohexyl-2(R)-(4-hydroxyphenyl-methyl)-hexanoyl-(L)-Val-(L)-Phe-morpholin-4-ylamid in 5 ml DMF mit 0,5 g (1,59 mMol) TBAF-Trihydrat zur Titelverbindung umgesetzt. Die Titelverbindung wird durch Kristallisation (Ether) gereinigt. $t_{Ret}$(IV)= 15,52 min; FAB-MS (M+H$^+$)= 751.
Die Ausgangsverbindung wird folgendermassen hergestellt:

**a) 5(S)-(Boc-amino)-4(S)-(tert-butyldimethyl)silyloxy-6-cyclohexyl-2(R)-(4-hydroxyphenyl-methyl)-hexanoyl-(L)-Val-(L)-Phe-morpholin-4-ylamid**

Analog Referenz-Beispiel 45 werden 19,5 g (20,41 mMol) 5(S)-(Boc-amino)-4(S)-hydroxy-6-cyclohexyl-2(R)-(4-benzyloxyphenyl-methyl)-hexanoyl-(L)-Val-(L)-Phe-morpholin-4-ylamid (Referenzbeispiel 67 e) in 400 ml Methanol in Gegenwart von 4 g 10% Pd/C hydriert. Die nach Aufarbeiten erhaltene Titelverbindung wird ohne

zusätzliche Reinigung weiterumgesetzt; DC $R_f$ (A) 0,28; $t_{Ret}$(IV) = 21,99 min; FAB-MS (M+H$^+$)= 866.

**Referenz-Beispiel 69: 5(S)-(Boc-amino)-4(S)-hydroxy-6-cyclohexyl-2(R)-(4-methoxyphenyl-methyl)-hexanoyl-(L)-Val-(L)-Phe-morpholin-4-ylamid**

Analog Referenz-Beispiel 1 werden 3,93 g (4,469 mMol) 5(S)-(Boc-amino)-4(S)-(tert-butyldimethylsilyloxy)-6-cyclohexyl-2(R)-(4-methoxyphenyl-methyl)-hexanoyl-(L)-Val-(L)-Phe-morpholin-4-ylamid in 15 ml DMF mit 2,82 g (8,94 mMol) TBAF-Trihydrat zur Titelverbindung umgesetzt. Die Titelverbindung wird durch Fällung (Hexan) gereinigt. DC $R_f$ (B)= 0,64; $t_{Ret}$(IV)= 17,34 min; FAB-MS (M+H$^+$)= 765.
Die Ausgangsverbindung wird folgendermassen hergestellt:

**a) 5(S)-(Boc-amino)-4(S)-(tert-butyldimethyl)silyloxy-6-cyclohexyl-2(R)-(4-methoxyphenyl-methyl)-hexanoyl-(L)-Val-(L)-Phe-morpholin-4-ylamid**

Analog Referenz-Beispiel 70 a) wird eine Lösung von 4 g (4,623 mMol) 5(S)-(Boc-amino)-4(S)-(tert-butyldimethyl)silyloxy-6-cyclohexyl-2(R)-(4-hydroxyphenyl-methyl)-hexanoyl-(L)-Val-(L)-Phe-morpholin-4-ylamid in 70 ml Dioxan mit 6,02 g (18,49 mMol) Caesiumcarbonat und 9,1 ml (92,46 mMol) Methyljodid umgesetzt. Die nach Aufarbeiten erhaltene Titelverbindung wird ohne weitere Reinigung weiterverarbeitet. DC $R_f$ (I)= 0,36; $t_{Ret}$(IV)= 24 min; FAB-MS (M+H$^+$)= 880.

**Referenz-Beispiel 70: 5(S)-(Boc-amino)-4(S)-hydroxy-6-phenyl-2(R)-benzylhexanoyl-(L)-Val-(L)-(4-isobutyloxy-Phe)-morpholin-4-ylamid**

Analog Referenz-Beispiel 1 werden 201 mg (0,22 mMol) 5(S)-(Boc-amino)-4(S)-(tert-butyldimethylsilyloxy)-6-phenyl-2(R)-benzyl-hexanoyl-(L)-Val-(L)-(4-isobutyloxy-Phe)-morpholin-4-ylamid mit 139 mg (0,44 mMol) TBAF in 5 ml DMF während 18,5 h entschützt. Säulenchromatographie ($SiO_2$, Essigsäureethylester/Hexan: 4/1) liefert die Titelverbindung: DC $R_f$(B)= 0,38; $t_{Ret}$(I)= 18,3 min; FAB-MS (M+H$^+$)= 801.
Die Ausgangsverbindungen werden folgendermassen hergestellt:

**a) N-(Benzyloxycarbonyl)-(L)-Val-(L)-(4-isobutyloxy-Phe)-morpholin-4-ylamid**

Eine Lösung von 1,93 g (4 mMol) N-(Benzyloxycarbonyl)-(L)-Val-(L)-Tyr-morpholin-4-ylamid (Referenz-Beispiel 41 A) c)) in 8 ml 1/1 DMF/Dioxan wird mit 2,6 g (8 mMol) Caesiumcarbonat und 2,31 ml (20 mMol) Isobutyljodid behandelt und anschliessend auf 50°C erhitzt. Nach 1,25 h wird nochmals 2,6 g (8 mMol) Caesiumcarbonat und 2,31 ml (20 mMol) Isobutyljodid dazugegeben und noch weitere 2,31 ml (20 mMol) Isobutyljodid nach je 2,15 h und 4 h. Nach insgesamt 5,75 h Rühren bei 50 °C wird das Reaktionsgemisch auf Eis/Wasser gegossen und 3 mal mit Methylenchlorid extrahiert. Nach dem Trocknen über Natriumsulfat wird am Rotationsverdampfer eingeengt. Säulenchromatographie ($SiO_2$, Essigsäureethylester/Hexan 4/1) liefert die Titelverbindung: DC $R_f$ (B)= 0,43.

**b) H-(L)-Val-(L)-(4-isobutyloxy-Phe)-morpholin-4-ylamid**

Analog Referenz-Beispiel 10 e) werden 1,5 g (2,78 mMol) N-(Benzyloxycarbonyl)-(L)-Val-(L)(4-isobutyloxy-Phe)-morpholin-4-ylamid in 40 ml Methanol in Gegenwart von 0,2 g 10% Pd/C hydriert. Die Titelverbindung wird ohne weitere Reinigung weiterverwendet: DC $R_f$ (F)= 0,44.

**c) 5(S)-(Boc-amino)-4(S)-(tert-butyldimethylsilyloxy)-6-phenyl-2(R)-benzylhexanoyl-(L)-Val-(L)-(4-isobutyloxy-Phe)-morpholin-4-ylamid**

Analog Referenz-Beispiel 10 f) werden 118 mg (0,22 mMol) 5(S)-(Boc-amino)-4(S)-(tert-butyldimethylsilyloxy)-6-phenyl-2(R)-benzyl-hexansäure und 100 mg (0,25 mMol) H-(L)-Val-(L)-(4-isobutyloxy-Phe)-morpholin-4-ylamid in 2,19 ml NMM/$CH_3CN$ 0,25 M mit 94,8 mg (0,24 mMol) HBTU zur Titelverbindung umgesetzt. Säulenchromatographie ($SiO_2$, Essigsäureethylester/Hexan: 4/1) liefert die Titelverbindung: DC $R_f$ (B)= 0,54.

**Referenz-Beispiel 71: 5(S)(Boc-amino)-4(S)-hydroxy-6-phenyl-2(R)-benzylhexanoyl-(L)-Leu-(L)-(p-$CH_3O$-Phe)-morpholin-4-ylamid**

Analog Referenz-Beispiel 1 werden 189 mg (0,22 mMol) 5(S)-(Boc-amino)-4(S)-(tert-butyldimethylsilyloxy)-6-phenyl-2(R)-benzyl-hexanoyl-(L)-Leu-(L)-(p-$CH_3O$-Phe)-morpholin-4-ylamid mit 139 mg (0,44 mMol) TBAF in 5 ml DMF während 18 h entschützt. Säulenchromatographie ($SiO_2$, Essigsäureethylester/Hexan: 4/1) liefert die Titelverbindung: DC $R_f$(B)= 0,37; $t_{Ret}$(I)= 16,6 min; FAB-MS (M+H$^+$)= 773.
Die Ausgangsverbindungen werden folgendermassen hergestellt:

**a) N-(Benzyloxycarbonyl)-(L)-Leu-(L)-(p-$CH_3O$-Phe)-morpholinylamid**

Es werden 0,45 g (1,68 mMol) (L)-N-Benzyloxycarbonyl-leucin (Fluka, Buchs, Schweiz) und 0,444 g (1,68 mMol) H-(L)-(p-$CH_3O$-Phe)-morpholinylamid in 70 ml Methylenchlorid mit 0,347 g (1,68 mMol) DCC und

0,25 g HOBT zur Titelverbindung umgesetzt. Säulenchromatographie ($SiO_2$, Essigsäureethylester/Hexan: 4/1) liefert die Titelverbindung: DC $R_f$ (B)= 0,28.

b) H-(L)-Leu-(L)-(p-$CH_3$O-Phe)-morpholin-4-ylamid

Analog Referenz-Beispiel 10 e) werden 0.73 g (1,43 mMol) N-(Benzyloxycarbonyl)-(L)-Leu-(L)-(p-$CH_3$O-Phe)-morpholin-4-ylamid in 20 ml Methanol in Gegenwart von 0,1 g 10% Pd/C hydriert. Die Titelverbindung wird ohne weitere Reinigung weiterverwendet: DC $R_f$ (F)= 0,47.

c) 5(S)-(Boc-amino)-4(S)-(tert-butyldimethylsilyloxy)-6-phenyl-2(R)-benzylhexanoyl-(L)-Leu-(L)-(p-$CH_3$O-Phe)-morpholin-4-ylamid

Analog Referenz-Beispiel 10 f) werden 118 mg (0,22 mMol) 5(S)-(Boc-amino)-4(S)-(tert-butyldimethylsilyloxy)-6-phenyl-2(R)-benzyl-hexansäure und 91 mg (0,25 mMol) H-(L)-Leu-(L)-(p-$CH_3$O-Phe)-morpholin-4-ylamid in 2,19 ml NMM/$CH_3$CN 0,25 M mit 94,8 mg (0,24 mMol) HBTU zur Titelverbindung umgesetzt. Säulenchromatographie ($SiO_2$, Essigsäureethylester/Hexan: 4/1) liefert die Titelverbindung: DC $R_f$ (B)= 0,57.

d) Z-(L)-(p-$CH_3$O-Phe)-morpholin-4-ylamid.

Analog Referenz-Beispiel 70 a) wird eine Lösung von 2 g (5,2 mMol) Z-(L)-Tyr-morpholin-4-ylamid (Referenz-Beispiel 70 e)) in 100 ml 1:1 DMF/Dioxan mit 3,38 g (10,4 mMol) Caesiumcarbonat und 0,324 ml (5,2 mMol) Methyliodid umgesetzt. Säulenchromatographie ($SiO_2$, Essigsäureethylester/Hexan: 4/1) liefert die Titelverbindung: DC $R_f$ (Essigsäureethylester/Hexan: 4/1)= 0,34.

e) H-(L)-(p-$CH_3$O-Phe)-morpholin-4-ylamid.

Analog Beispiel 70 b) wird eine Lösung von 3,9 g (9,8 mMol) Z-(L)-(p-$CH_3$O-Phe)-morpholin-4-ylamid in 150 ml Methanol in Gegenwart von 1,2 g 10% Pd/C hydriert. Nach Aufarbeiten wird die Titelverbindung ohne zusätzliche Reinigung weiterverwendet. DC $R_f$ (F)= 0,32.

**Referenz-Beispiel 72: 5(S)-(Boc-amino)-4(S)-hydroxy-6-phenyl-2(R)-benzyl-hexanoyl-(L)-Val-(L)-(4-n-butyloxy-Phe)-morpholin-4-ylamid**

Analog Referenz-Beispiel 1 werden 201 mg (0,22 mMol) 5(S)-(Boc-amino)-4(S)-(tert-butyldimethylsilyloxy)-6-phenyl-2(R)-benzyl-hexanoyl-(L)-Val-(L)-(4-n-butyloxy-Phe)-morpholin-4-ylamid mit 139 mg (0,44 mMol) TBAF in 5 ml DMF während 16,5 h entschützt. Säulenchromatographie ($SiO_2$, Essigsäureethylester/Hexan: 4/1) liefert die Titelverbindung: DC $R_f$(B)= 0,39; $t_{Ret}$(I)= 18,2 min; FAB-MS $(M+H^+)$= 801.

Die Ausgangsverbindungen werden folgendermassen hergestellt:

a) N-(Benzyloxycarbonyl)-(L)-Val-(L)-(4-n-butyloxy-Phe)-morpholin-4-ylamid

Analog Referenz-beispiel 70 a) wird eine Lösung von 0,48 g (0,1 mMol) N-(Benzyloxycarbonyl)-(L)-Val-(L)-Tyr-morpholin-4-ylamid in 0,2 ml 1/1 DMF/Dioxan mit 65 mg (0,2 mMol) Caesiumcarbonat und 11,9 µl (0,1 mMol) n-Butyljodid behandelt. Säulenchromatographie ($SiO_2$, Essigsäureethylester/Hexan 4/1) liefert die Titelverbindung: DC $R_f$ (B)= 0,47.

b) H-(L)-Val-(L)-(4-n-butyloxy-Phe)-morpholin-4-ylamid

Analog Referenz-Beispiel 10 e) werden 1,38 g (2,6 mMol) N-(Benzyloxycarbonyl)-(L)-Val-(L)-(4-n-butyloxy-Phe)-morpholin-4-ylamid in 40 ml Methanol in Gegenwart von 0,2 g 10% Pd/C hydriert. Die Titelverbindung wird ohne weitere Reinigung weiterverwendet: DC $R_f$ (F)= 0,45.

c) 5(S)-(Boc-amino)-4(S)-(tert-butyldimethylsilyloxy)-6-phenyl-2(R)-benzylhexanoyl-(L)-Val-(L)-(4-n-butyloxy-Phe)-morpholin-4-ylamid

Analog Referenz-Beispiel 10 f) werden 118 mg (0,22 mMol) 5(S)-(Boc-amino)-4(S)-(tert-butyldimethylsilyloxy-6-phenyl-2(R)-benzyl-hexansäure und 98 mg (0,25 mMol) H-(L)-Val-(L)(4-n-butyloxy-Phe)-morpholin-4-ylamid in 2,19 ml NMM/CH3CN 0,25 M mit 94,8 mg (0,24 mMol) HBTU zur Titelverbindung umgesetzt. Die Titelverbindung, DC $R_f$ (B)= 0,57, wird ohne zusätzliche Reinigung weiterverwendet.

**Referenz-Beispiel 73: 5(S)-(Boc-amino)-4(S)-hydroxy-6-phenyl-2(R)-benzyl-hexanoyl(L)-phenylglycyl-(L)-(p-$CH_3$O-Phe)-morpholin-4-ylamid**

Analog Referenz-Beispiel 1 werden 380 mg (0,41 mMol) 5(S)-(Boc-amino)-4(S)-(tert-butyldimethylsilyloxy)-6-phenyl-2(R)-benzyl-hexanoyl-(L)-phenylglycyl-(L)-(p-$CH_3$O-Phe)-morpholin-4-ylamid mit 265 mg (0,82 mMol) TBAF in 10 ml DMF während 17 h entschützt. Säulenchromatographie ($SiO_2$, Essigsäureethylester/Hexan: 2/1 bis 4/1) liefert die Titelverbindung: DC $R_f$(B)= 0,47; $t_{Ret}$(I)= 16,4 min; FAB-MS $(M+H^+)$= 793.

Die Ausgangsverbindungen werden folgendermassen hergestellt:

a) N-(t-Butyloxycarbonyl)-(L)-phenylglycyl-(L)-(p-$CH_3$O-Phe)-morpholin-4-ylamid

0,44 g (2 mMol) N-tert-Butyloxycarbonyl-(L)-phenylglycin und 0,53 g (2 mMol) H-(L)-(p-$CH_3$O-Phe)-morpholinylamid in 80 ml Methylenchlorid werden mit 0,413 g (2 mMol) DCC und 0,297 g HOBT zur Titelver-

bindung umgesetzt. Säulenchromatographie ($SiO_2$, Essigsäureethylester/Hexan: 4/1) liefert die Titelverbindung: DC $R_f$ (Essigsäureethylester/Hexan: 4/1)= 0,31.

**b) H-(L)-Phenylglycyl-(L)-(p-$CH_3$O-Phe)-morpholin-4-ylamid**

Eine Lösung von 900 mg (1,81 mMol) N-(t-Butyloxycarbonyl)-(L)-phenylglycyl-(L)-(p-$CH_3$O-Phe)-morpholin-4-ylamid in 19 ml Ameisensäure wird nach 2 h Rühren am Rotationsverdampfer eingeengt. Nach Lösen in Essigsäureethylester wird nacheinander 4 mal mit Natriumbicarbonat, einmal mit Wasser und einmal mit Sole gewaschen. Nach Trocknen über $Na_2SO_4$ wird eingeengt. Die Titelverbindung wird ohne weitere Reinigung weiterverwendet. DC $R_f$ (B)= 0,12.

**c) 5(S)-(Boc-amino)-4(S)-(tert-butyldimethylsilyloxy)-6-phenyl-2(R)-benzylhexanoyl-(L)-phenylglycyl-(L)-(p-$CH_3$O-Phe)-morpholin-4-ylamid**

Analog Referenz-Beispiel 10 f) werden 118 mg (0,22 mMol) 5(S)-(Boc-amino)-4(S)-(tert-butyldimethylsilyloxy)-6-phenyl-2(R)-benzyl-hexansäure und 96 mg (0,25 mMol) H-(L)-Phenylglycyl-(L)-(p-$CH_3$O-Phe)-morpholin-4-ylamid in 2,19 ml NMM/$CH_3$CN 0,25 M mit 94,8 mg (0,24 mMol) HBTU zur Titelverbindung umgesetzt. Säulenchromatographie ($SiO_2$, Essigsäureethylester/Hexan: 4/1) liefert die Titelverbindung: DC $R_f$ (B)= 0,57.

**Referenz-Beispiel 74: Boc-Phe[C](3,4-dimethoxy)Phe-(L)-Val-(L)-Phe-morpholin-4-ylamid**

Analog Ref.-Bsp. 56 O) werden 141 mg 5(S)-(Boc-amino)-4(S)-(tert-butyldimethylsilyloxy)-6-phenyl-2(R)-(3,4-dimethoxyphenylmethyl)-hexanoyl-(L)-Val-(L)-Phe-morpholin-4-ylamid in 3 ml DMF mit 100,4 mg TBAF zur Titelverbindung desilyliert. Die Reinigung erfolgt durch zweimalige Chromatographie an Kieselgel (Laufmittel Essigsäureethylester/Hexan 4:1 und B). DC $R_f$(B)=0,21. FAB-MS $(M+H)^+$=789.

Das Ausgangsmaterial wird wie folgt hergestellt:

**74 a) 3,4-Dimethoxybenzylchlorid**

Analog Referenz-Beispiel 56 O) a) erhält man aus 10 g 3,4-Dimethoxybenzylalkohol (Fluka, Buchs, Schweiz), 46,2 g Diisopropylaminomethyl-polystyrol (Polyhünigbase) und 4,62 ml Thionylchlorid in 200 ml abs. Ether die Titelverbindung. DC $R_f$(C)=0,31. $^1$H-NMR (200 MHz, $CDCl_3$): 7,0-6,87 (m, 2H); 6,82 (d, 1H); 4,56 (s, 2H); 3,9 (s, 3H); 3,87 (s, 3H).

**74 b) 3,4-Dimethoxybenzyljodid**

Analog Referenz-Beispiel 56 O) b) erhält man aus 6,185 g 3,4-Dimethoxybenzylchlorid und 24,19 g Natriumjodid in 62 ml abs. Aceton die Titelverbindung. DC $R_f$(C)=0,40. $^1$H-NMR (200 MHz, $CDCl_3$): 6,95 (dxd, 1H); 6,88 (d, 1H); 6,75 (d, 1H); 4,47 (s, 2H); 3,87 (s, 3H); 3,86 (s, 3H).

**74 c) 5(S)-[1(S)-(Boc-amino)-2-phenylethyl]-3(R)-(3,4-dimethoxyphenylmethyl)dihydrofuran-2-(3H)-on**

Analog zu Referenz-Beispiel 56 O) c) werden 1 g 5(S)-[1(S)-(Boc-amino)-2-phenylethyl]-dihydrofuran-2-(3H)-on [hergestellt nach Ref.-Bsp. 2i)] in 4 ml abs. THF mit 6,42 ml Lithium-bis-(trimethylsilyl)amid (1M in THF) und unter Zugabe von 0,66 ml DMPU deprotoniert (-75°C) und mit 911 mg 3,4-Dimethoxybenzyljodid alkyliert. Chromatographie an Kieselgel (Laufmittel D, A und Hexan/Essigsäureethylester 1:2) gibt die reine Titelverbindung. DC $R_f$(A)=0,42. MS $M^+$=455.

**74 d) 5(S)-(Boc-amino)-4(S)-hydroxy-6-phenyl-2(R)-(3,4-dimethoxyphenylmethyl)hexansäure**

Analog Referenz-Beispiel 56O/d) werden 778 mg 5(S)-[1(S)-(Boc-amino)-2-phenylethyl]-3(R)-(3,4-dimethoxyphenylmethyl)-dihydrofuran-2-(3H)-on in 27,67 ml Dimethoxyethan und 13,91 ml Wasser mit 6,83 ml Lithiumhydroxyd-Lösung 1M zur Titelverbindung hydrolysiert, die direkt weiterverarbeitet wird. DC $R_f$(A)=0,07.

**74 e) 5(S)-(Boc-amino)-4(S)-(tert-butyldimethylsilyloxy)-6-phenyl-2(R)-(3,4-dimethoxyphenyl-methyl)-hexansäure**

Analog Referenz-Beispiel 56 O) e) werden 804 mg 5(S)-(Boc-amino)-4(S)-hydroxy-6-phenyl-2(R)-(3,4-dimethoxyphenylmethyl)-hexansäure in 5,94 ml DMF mit 1,162 g tert-Butyldimethylchlorsilan und 946,6 mg Imidazol silyliert. Die Reinigung der Titelverbindung erfolgt durch zweimalige Chromatographie an Kieselgel (Laufmittel: D, A, Essigsäureethylester/Hexan 2:1 und B). DC $R_f$(A)=0,27. MS $M^+$=557.

**74 f) 5(S)-(Boc-amino)-4(S)-(tert-butyldimethylsilyloxy)-6-phenyl-2-(R)-(3,4-dimethoxyphenylmethyl)-hexanoyl-(L)-Val-(L)-Phe-morpholin-4-ylamid**

Analog Ref.-Bsp. 56 O) f) werden 100 mg 5(S)-(Boc-amino)-4(S)-(tert-butyldimethylsilyloxy)-6-phenyl-2(R)-(3,4-dimethoxyphenylmethyl)-hexansäure und 62,3 mg H-(L)-Val-(L)-Phe-morpholin-4-ylamid [hergestellt nach Ref.-Bsp. 1o)] in 1,59 ml NMM/$CH_3$CN 0,25 M mit 70,9 mg HBTU zur Titelverbindung umgesetzt. DC $R_f$ (Essigsäureethylester/Hexan 2:1)=0,19. FAB-MS $(M+H)^+$=903.

**Referenz-Beispiel 75:Boc-Phe[C](3,4,5-trimethoxy)Phe-(L)-Val-(L)-Phe-morpholin-4-ylamid**

Analog Ref.-Bsp. 56 O) werden 511 mg 5(S)-(Boc-amino)-4(S)-(tert-butyldimethylsilyloxy)-6-phenyl-2(R)-(3,4,5-trimethoxyphenylmethyl)-hexanoyl-(L)-Val-(L)-Phe-morpholin-4-ylamid in 9,36 ml DMF mit 323,5 mg TBAF zur Titelverbindung desilyliert. Die Reinigung erfolgt durch Chromatographie an Kieselgel (Laufmittel B und H). DC $R_f$(B)=0,13. FAB-MS $(M+H)^+$=819.

Das Ausgangsmaterial wird wie folgt hergestellt:

**75 a) 3,4,5-Trimethoxybenzyljodid**

Analog Referenz-Beispiel 56 O) b) erhält man aus 5 g 3,4,5-Trimethoxybenzylchlorid (Fluka, Buchs, Schweiz) und 16,89 g Natriumjodid in 40 ml abs. Aceton die Titelverbindung. DC $R_f$(C)=0,27. $^1$H-NMR (360 MHz, $CDCl_3$): 6,60 (s, 2H); 4,44 (s, 2H); 3,86 (s, 6H); 3,83 (s, 3H).

**75 b) 5(S)-[1(S)-(Boc-amino)-2-phenylethyl]-3(R)-(3,4,5-trimethoxyphenylmethyl)-dihydrofuran-2-(3H)-on**

Analog zu Referenz-Beispiel 56 O) c) werden 1 g 5(S)-[1(S)-(Boc-amino)-2-phenylethyl]-dihydrofuran-2-(3H)-on [hergestellt nach Ref.-Bsp. 2i)] in 4 ml abs. THF mit 6,42 ml Lithium-bis-(trimethylsilyl)amid (1M in THF) und unter Zugabe von 0,66 ml DMPU deprotoniert (-75°C) und mit 1,008 g 3,4,5-Trimethoxybenzyljodid alkyliert. Chromatographie an Kieselgel (Laufmittel Hexan/Aceton 3:1) liefert die Titelverbindung. DC $R_f$(Hexan/Aceton 3:1)=0,22. FAB-MS $M^+$=485.

**75 c) 5(S)-(Boc-amino)-4(S)-hydroxy-6-phenyl-2(R)-(3,4,5-trimethoxyphenylmethyl)-hexansäure**

Analog Referenz-Beispiel 56 O) d) werden 1,097 g 5(S)-[1(S)-(Boc-amino)-2-phenylethyl]-3(R)-(34,5-trimethoxyphenylmethyl)-dihydrofuran-2-(3H)-on in 36,48 ml Dimethoxyethan und 18,39 ml Wasser mit 9,03 ml Lithiumhydroxyd-Lösung 1M zur Titelverbindung hydrolysiert, die ohne Reinigen weiterverarbeitet wird.

**75 d) 5(S)-(Boc-amino)-4(S)-(tert-butyldimethylsilyloxy)-6-phenyl-2(R)-(3,4,5-trimethoxyphenylmethyl)-hexansäure**

Analog Referenz-Beispiel 56 O) e) werden 1,526 g 5(S)-(Boc-amino)-4(S)-hydroxy-6-phenyl-2(R)-(3,4,5-trimethoxyphenylmethyl)-hexansäure in 15,16 ml DMF mit 2,11 g tert-Butyldimethylchlorsilan und 1,683 g Imidazol silyliert. Die Reinigung der Titelverbindung erfolgt durch zweimalige Chromatographie an Kieselgel (Lösungsmittel: Hexan, A, Essigsäureethylester/Hexan 2:1). DC $R_f$(B)=0,39. FAB-MS $(M+H)^+$=618.

**75 e) 5(S)-(Boc-amino)-4(S)-(tert-butyldimethylsilyloxy)-6-phenyl-2(R)-(3,4,5-trimethoxyphenylmethyl)-hexanoyl-(L)-Val-(L)-Phe-morpholin-4-ylamid**

Analog Ref.-Bsp. 56 O) f) werden 316,5 mg 5(S)-(Boc-amino)-4(S)-(tert-butyldimethylsilyloxy)-6-phenyl-2(R)-(3,4,5-trimethoxyphenylmethyl)-hexansäure und 171 mg H-(L)-Val-(L)-Phe-morpholin-4-ylamid [hergestellt nach Ref.-Bsp. 1o)] in 4,82 ml NMM/$CH_3CN$ 0,25 M mit 213,39 mg HBTU zur Titelverbindung umgesetzt. DC $R_f$(B)=0,45. FAB-MS $(M+H)^+$=933.

**Referenz-Beispiel 76:Boc-Phe[C](2,3,4-trimethoxy)Phe-(L)-Val-(L)-Phe-morpholin-4-y lamid**

Analog Ref.-Bsp. 56 O) werden 407 mg 5(S)-(Boc-amino)-4(S)-(tert-butyldimethylsilyloxy)-6-phenyl-2(R)-(2,3,4-trimethoxyphenylmethyl)-hexanoyl-(L)-Val-(L)-Phe-morpholin-4-ylamid in 7,41 ml DMF mit 256 mg TBAF zur Titelverbindung desilyliert. Die Reinigung erfolgt durch Chromatographie an Kieselgel (Laufmittel B). DC $R_f$(B)=0,12. FAB-MS $(M+H)^+$=819.

Das Ausgangsmaterial wird wie folgt hergestellt:

**76 a) 2,3,4-Trimethoxybenzylchlorid**

Analog Ref.-Bsp. 56 O) a) erhält man aus 10,22 g 2,3,4-Trimethoxybenzylalkohol (Aldrich, Steinheim, BRD), 33,75 g Diisopropylaminomethyl-polystyrol (Polyhünigbase) und 10,6 ml Thionylchlorid in 200 ml abs. Ether die Titelverbindung. Die Reinigung erfolgt durch Chromatographie an Kieselgel (Laufmittel: E). DC $R_f$(E)=0,47. $^1$H-NMR (360 MHz, $CDCl_3$): 7,05 (d, 1H); 6,65 (d,1H); 4,61 (s, 2H); 3,98 (s, 3H); 3,86 (s, 3H); 3,85 (s, 3H).

**76 b) 2,3,4-Trimethoxybenzyljodid**

Analog Referenz-Beispiel 56 O) b) erhält man aus 2,34 g 2,3,4-Trimethoxybenzylchlorid und 7,87 g Natriumjodid in 18,6 ml abs. Aceton die Titelverbindung, die ohne Reinigen weiterverarbeitet wird. DC Rf(Hexan/Essigsäureethylester 2,5:1)=0,44. $^1$H-NMR (200 MHz, $CDCl_3$): 7,03 (d, 1H); 6,60 (d, 1H); 4,50 (s, 2H); 4,05 (s, 3H); 3,85 (2xs, 6H).

**76 c) 5(S)-[1(S)-(Boc-amino)-2-phenylethyl]-3(R)-(2,3,4-trimethoxyphenylmethyl)-dihydrofuran-2-(3H)-on**

Analog zu Referenz-Beispiel 56 O) c) werden 1,5 g 5(S)-[1(S)-(Boc-amino)-2-phenylethyl]-dihydrofuran-2-(3H)-on [hergestellt nach Ref.-Bsp. 2i)] in 6 ml abs. THF mit 9,62 ml Lithium-bis-(trimethylsilyl)amid (1M in THF) und unter Zugabe von 0,998 ml DMPU deprotoniert (-75°C) und mit 1,51 g 2,3,4-Trimethoxybenzyljodid alkyliert. Chromatographie an Kieselgel (Laufmitte: Hexan/Essigsäureethylester 1:2) liefert die reine Titelverbindung. DC $R_f$(A)=0,53. FAB-MS $M^+$=485.

**76 d) 5(S)-(Boc-amino)-4(S)-hydroxy-6-phenyl-2(R)-(2,3,4-trimethoxyphenylmethyl)-hexansäure**

Analog Referenz-Beispiel 56 O) d) werden 1,354 g 5(S)-[1(S)-(Boc-amino)-2-phenylethyl]-3(R)-(2,3,4-trimethoxyphenylmethyl)-dihydrofuran-2-(3H)-on in 43,36 ml Dimethoxyethan und 21,86 ml Wasser mit 10,74 ml Lithiumhydroxyd-Lösung 1M zur Titelverbindung hydrolysiert, die ohne Reinigen weiterverarbeitet wird. DC $R_f$(A)=0,03. MS $M^+$-$H_2O$ = 485.

**76 e) 5(S)-(Boc-amino)-4(S)-(tert-butyldimethylsilyloxy)-6-phenyl-2(R)-(2,3,4-trimethoxyphenylmethyl)-hexansäure**

Analog Referenz-Beispiel 56 O) e) werden 1,308 g 5(S)-(Boc-amino)-4(S)-hydroxy-6-phenyl-2(R)-(2,3,4-trimethoxyphenylm ethyl)-hexansäure in 13 ml DMF mit 1,816 g tert-Butyldimethylchlorsilan und 1,443 g Imidazol silyliert. Die Reinigung der Titelverbindung erfolgt durch zweimalige Chromatographie an Kieselgel (Lösungsmittel: A und Essigsäureethylester/Hexan 1,5:1). DC $R_f$(Essigsäureethylester/Hexan 2:1)=0,02. FAB-MS $(M+H)^+$=618.

**76 f) 5(S)-(Boc-amino)-4(S)-(tert-butyldimethylsilyloxy)-6-phenyl-2(R)-(2,3,4-trimethoxyphenylmethyl)-hexanoyl-(L)-Val-(L)-Phe-morpholin-4-ylamid**

Analog Ref.-Bsp. 56 O) f) werden 250 mg 5(S)-(Boc-amino)-4(S)-(tert-butyldimethylsilyloxy)-6-phenyl-2(R)-(2,3,4-trimethoxyphenylmethyl)-hexansäure und 148,4 mg H-(L)-Val-(L)-Phe-morpholin-4-ylamid [hergestellt nach Ref.-Bsp. 1o)] in 3,807 ml NMM/$CH_3CN$ 0,25 M mit 168,8 mg HBTU zur Titelverbindung umgesetzt. DC $R_f$(B)=0,64.

**Beispiele für Verbindungen der Formel I':**

**Beispiel 1: 5(S)-(Boc-amino)-4(S)-(2-furanylcarboxy)-6-phenyl-2(R)-phenylmethylhexanoyl-(L)-Val-(L)-Phe-morpholin-4-ylamid**

Unter $N_2$-Atmosphäre wird eine Lösung von 365 mg (0,50 mMol) Boc-Phe[C]Phe-(L)-Val-(L)-Phe-morpholin-4-ylamid (Beispiel 2) und 10 mg DMAP in 3 ml Dioxan und 0,6 ml Pyridin mit 74 μl (0,75 mMol) 2-Furancarbonsäurechlorid (Fluka; Buchs/Schweiz) versetzt und 44 h bei RT gerührt. Da laut HPLC noch Edukt vorliegt, werden nochmals 0,1 ml 2-Furancarbonsäurechlorid und 1 ml Pyridin zugesetzt. Nach weiteren 2 Tagen verdünnt man das Reaktionsgemisch mit Essigsäureethylester und wäscht mit ges. $NaHCO_3$-Lösung, Wasser und Sole. Die wässrigen Phasen werden mit 2 Portionen Essigsäureethylester extrahiert, die vereinigten organischen Phasen mit $Na_2SO_4$ getrocknet und eingedampft. Fällen mit DIPE/Hexan 1:2 aus einer konzentrierten Lösung in Methylenchlorid liefert die reine Titelverbindung: DC $R_f$(I)=0,23; $t_{Ret}$(I)=17,5 min; FAB-MS $(M+H)^+$=823.

**Beispiel 2: 5(S)-(Boc-amino)-4(S)-(N,N-dimethyl-aminoacetyloxy)-6-phenyl-2(R)-phenylmethyl-hexanoyl-(L)-Val-(L)-Phe-morpholin-4-ylamid**

Eine Lösung von 300 mg (0,41 mMol) Boc-Phe[C]Phe-(L)-Val-(L)-Phe-morpholin-4-ylamid (Referenz-Beispiel 2) und wenig DMAP in 1 ml Pyridin wird unter $N_2$-Atmosphäre mit 130 mg (0,82 mMol) N,N-Dimethylacetylchlorid (als Hydrochloridsalz) [Herstellung: N.H. Krämer und H.F.G. Linde, Arch. Pharm. (Weinheim) 324, 433 (1991)] versetzt und 9 h bei 60°C gerührt. Da gemäss HPLC noch Edukt vorliegt, werden nochmals 130 mg N,N-Dimethylacetylchlorid Hydrochlorid zugesetzt. Nach weiteren 2 h bei 60°C verdünnt man das Reaktionsgemisch mit Essigsäureethylester und wäscht mit ges. $NaHCO_3$ Lösung, 2x Wasser und Sole. Die wässrigen Phasen werden mit 2 Portionen Essigsäureethylester extrahiert, die vereinigten organischen Phasen mit $Na_2SO_4$ getrocknet und eingedampft. Säulenchromatographie ($SiO_2$, Essigsäureethylester/Ethanol 97:3 → 95:5) Digerieren in DIPE im Ultraschallbad liefert die kristalline Titelverbindung: DC $R_f$(T)=0,11; $t_{Ret}$(II)=19,5 min; FAB-MS $(M+H)^+$=814.

**Beispiel 3: 5(S)-(Boc-amino)-4(S)-[4-(dimethyl-amino)-butyryloxy]-6-phenyl-2(R)-phenylmethyl-hexanoyl-(L)-Val-(L)-Phe-morpholin-4-ylamid**

Analog Beispiel 1) werden 200 mg (0,27 mMol) Boc-Phe[C]Phe-(L)-Val-(L)-Phe-morpholin-4-ylamid (Referenz-Beispiel 2) und wenig DMAP in 5 ml Dioxan/Acetonitril 4:1 und 372 μl (2,2 mMol) N-Ethyldiisopropylamin mit 161 mg (0,87 mMol) 3-(Dimethyl-amino)butyrylchlorid (als Hydrochloridsalz) umgesetzt. Digerieren des Rohproduktes aus DIPE liefert die Titelverbindung: $t_{Ret}$(I)=13,8 min; FAB-MS $(M+H)^+$=842.

Das Ausgangsmaterial wird wie folgt hergestellt:

a: 4-(Dimethyl-amino)butyrylchlorid Hydrochlorid

In 30 ml $SOCl_2$ werden 10 g (60 mMol) 4-Dimethylaminobuttersäure Hydrochlorid (Janssen; Brüggen/Deutschland) während ca. 3 h auf 65°C erhitzt. Abdampfen des $SOCl_2$ und Verrühren des Rückstandes liefert die Titelverbindung: DC einer in Methanol gelösten Probe, $R_f$(U)=0,67; DC von 4-Dimethylaminobuttersäure Hydrochlorid, $R_f$(U)=0,50.

**Beispiel 4: 5(S)-(Boc-amino)-4(S)-(N-Z-N-methyl-aminoacetyloxy)-6-phenyl-2(R)-phenylmethyl-hexanoyl-(L)-Val-(L)-Phe-morpholin-4-ylamid**

Unter Stickstoffatmosphäre werden zu 446 mg (2,00 mMol) Z-Sarcosin (Bachem; Bubendorf/Schweiz) in 10 ml Methylenchlorid bei 0°C 339 µl (2,4 mMol) 1-Chlor-N,N,2-trimethyl-1-propenamin [B. Haveaux, A. Dekoker, M. Rens, A.R. Sidani, J. Toye, L. Ghosez, M. Murakami, M. Yoshioka, and W. Nagata, Organic Syntheses **59**, 26 (1980)] gegeben. Nach 2 h bei 0°C dampft man am HV ein. Der Rückstand wird in 2 ml Dioxan aufgenommen und unter Eiskühlung mit einer Lösung von 1,093 g (1,5 mMol) Boc-Phe[C]Phe-(L)-Val-(L)-Phe-morpholin-4-ylamid (Referenz-Beispiel 2) in 1,3 ml Pyridin und 10 ml Dioxan versetzt. Da nach 60 h bei RT gemäss HPLC noch Edukt vorliegt, werden 17 mg DMAP und zusätzliche 2,0 mMol Z-Sarcosin-säurechlorid (Herstellung analog oben) in 2 ml Dioxan beigefügt. Nach 18 h bei RT wird eingedampft und chromatographiert ($SiO_2$, Essigsäureethylester/Hexan 3:1). Digerieren in Hexan liefert die reine Titelverbindung: DC $R_f$(B)=0,74; $t_{Ret}$(I)=18,6 min; FAB-MS $(M+H)^+$=934; Anal: ber. C 68,15 %, H 7,23 %, N 7,50 %; gef. C 68,15 %, H 7,54 %, N 7,50 %.

**Beispiel 5: 5(S)-(Boc-amino)-4(S)-(methylamino-acetyloxy)-6-phenyl-2(R)-phenylmethyl-hexanoyl-(L)-Val-(L)-Phe-morpholin-4-ylamid**

Hydrieren von 200 mg (0,21 mMol) 5(S)-(Boc-amino)-4(S)-(N-Z-N-methyl-aminoacetyloxy)-6-phenyl-2(R)-phenylmethyl-hexanoyl-(L)-Val-(L)-Phe-morpholin-4-ylamid (Beispiel 4) gelöst in 10 ml Essigsäureethylester in Gegenwart von 0,20 g 10 %-igem Pd/C bei RT (ca. 3 d) liefert nach Filtration durch ®Celite, Eindampfen des Filtrats und Kristallisieren des erhaltenen Öls aus Hexan (Ultraschallbad) die Titelverbindung: DC $R_f$(B)=0,45; $t_{Ret}$(I)=13,3 min; FAB-MS $(M+H)^+$=800.

**Beispiel 6: 5(S)-(Boc-amino)-4(S)-[N-(imidazol-4-methyl)-N-methyl-aminoacetyloxy]-6-phenyl-2(R)-phenylmethyl-hexanoyl-(L)-Val-(L)-Phe-morpholin-4-ylamid**

Eine Lösung von 160 mg (0,20 mMol) 5(S)-(Boc-amino)-4(S)-(methylamino-acetyloxy)-6-phenyl-2(R)-phenylmethyl-hexanoyl-(L)-Val-(L)-Phe-morpholin-4-ylamid (Beispiel 5) und 23 mg (0,24 mMol) Imidazol-4-carbaldehyd (*PhD. Stein and St.E.Hall*, US-Patent 4977174, 11. Dez. 1990) in 2 ml Methanol und 26 mg (0,44 mMol) Essigsäure wird in Gegenwart von 20 mg 5 %-igem Pd/C bei RT während ca. 3 h hydriert. Filtration durch ®Celite, Verteilen des eingedampften Filtrats zwischen 3 Portionen Essigsäureethylester, ges. $NaHCO_3$-Lösung, Wasser und Sole, gefolgt von Säulenchromatographie ($SiO_2$, Essigsäureethylester/THF 3:1 → 1:3) und Kristallisation aus Acetonitril/DIPE/Hexan liefert die Titelverbindung: DC $R_f$(U)=0,13; $t_{Ret}$(I)=12,2 min; FAB-MS $(M+H)^+$=880.

**Beispiel 7: 5(S)-(Boc-amino)-4(S)-[N-pyridin-2-methyl)-N-methyl-aminoacetyloxy]-6-phenyl-2(R)-phenylmethyl-hexanoyl-(L)-Val-(L)-Phe-morpholin-4-ylamid**

Eine Lösung von 80 mg (0,10 mMol) 5(S)-(Boc-amino)-4(S)-(methylamino-acetyloxy)-6-phenyl-2(R)-phenylmethyl-hexanoyl-(L)-Val-(L)-Phe-morpholin-4-ylamid (Beispiel 5) und 12,9 mg (0,12 mMol) frisch destilliertem Pyridin-2-carbaldehyd (Fluka; Buchs/Schweiz) in 1,5 ml Methanol und 13 mg (0,22 mMol) Essigsäure wird in Gegenwart von 15 mg 5 %-igem Pd/C bei RT hydriert. Filtration durch ®Celite und Säulenchromatographie ($SiO_2$, Essigsäureethylester → Essigsäureethylester/Ethanol/Triethylamin 90:10:1) liefert die Titelverbindung: DC $R_f$(V)=0,3; $t_{Ret}$(I=14,3 min; FAB-MS $(M+H)^+$=891.

**Beispiel 8: 5(S)-(Boc-amino)-4(S)-[3-(1-triphenylmethyl-imidazol-4-yl)-propionyloxy]-6-phenyl-2(R)-phenylmethyl-hexanoyl-(L)-Val-(L)-Phe-morpholin-4-ylamid**

Analog Beispiel 4) werden 2,0 g (5,2 mMol) 3-(1-Triphenylmethyl-imidazol-4-yl)-propionsäure gelöst in 20 ml Methylenchlorid mit 1,1 ml(7,8 mMol) 1-Chlor-N,N,2-trimethyl-1-propenamin ins Säurechlorid überführt. Den Eindampfrückstand löst man in 12 ml Dioxan und gibt ihn zu einer Lösung von 1,23 g (1,7 mMol) Boc-Phe[C]Phe-(L)-Val-(L)-Phe-morpholin-4-ylamid (Referenz-Beispiel 2) in 4 ml Pyridin. Gemäss HPLC liegt nach 18 h bei RT noch viel Edukt vor, deshalb setzt man 50 mg DMAP und weitere 3 mMol 3-(1-Triphenylmethyl-imidazol-4-yl)-propionsäure-chlorid (Herstellung wie oben beschrieben) zu. Nach weiteren 24 h bei RT dampft man das Reaktionsgemisch ein, löst den Rückstand in Essigsäureethylester und wäscht ihn mit 3x Wasser und Sole. Die wässrigen Phasen werden mit 2 Portionen Essigsäureethylester extrahiert. Säulenchromatographie ($SiO_2$, Methylenchlorid/THF 9:1 → Methylenchlorid/THF/Triethylamin 90:10:1) liefert die Titelverbindung: $t_{Ret}(I)$=17,7 min; FAB-MS $(M+H)^+$=1093.

Das Ausgangsmaterial wird wie folgt hergestellt:

**a: Imidazol-4-yl-propionsäure (Natriumsalz)**

In 10 ml 1 N wässriger NaOH-Lösung werden 1,38 g (10 mMol) Urocaninsäure (Aldrich) gelöst. Man verdünnt mit 10 ml Methanol, setzt 80 mg 5 %-igen Pd/C-Katalysator zu und hydriert während ca. 1 h bei RT. Filtration durch ®Celite und Eindampfen liefert die Titelverbindung: $^1$H-NMR (200 MHz, $C_2O$): 2,40 und 2,73 (2t, J=7 Hz, je 2 H), 6,77 und 7,56 (2s, je 1 H).

**b: 3-(1-Triphenylmethyl-imidazol-4-yl)-propionsäure**

Ein 2-phasiges Gemisch aus 10 mMol Imidazol-4-yl-propionsäure (als Natriumsalz), 4 ml Wasser, 4,6 ml (33 mMol) Triethylamin und 8 ml $^i$Propanol wird unter Rühren während 4 h portionenweise mit 3,4 g (12,2 mMol) Triphenylchlormethan versetzt. Nach weiteren 4 h Rühren wird das Reaktionsgemisch mit 10 g Kieselgel versetzt, zur Trockne eingedampft und als Pulver auf eine Kieselgelsäule (Methylenchlorid) aufgetragen. Eluieren mit Methylenchlorid/$^i$Propanol/Methanol/Triethylamin 8:3:3:1 liefert die Titelverbindung: DC $R_f$(W)=0,77; $t_{Ret}(I)$=11,8 min; FAB-MS $(M+H)^+$=383.

**Beispiel 9: 5(S)-(Boc-amino)-4(S)-[3-(4-imidazolyl)-propionyloxy]-6-phenyl-2(R)-phenylmethyl-hexanoyl-(L)-Val-(L)-Phe-morpholin-4-ylamid**

Gelöst in 20 ml Essigsäureethylester werden 0,50 mMol 5(S)-(Boc-amino)-4(S)-[3-(1-triphenylmethyl-imidazol-4-yl)-propionyloxy]-6-phenyl-2(R)-phenylmethylhexanoyl-(L)-Val-(L)-Phe-morpholin-4-ylamid (Beispiel 8) in Gegenwart von 0,3 g Palladiumhydroxid 20 %-ig auf Kohle während 8 Tagen bei 60°C und einem Wasserstoffdruck von ca. 4 atm (ca. 4,052 bar oder 0,4052 MPa) hydriert. Abfiltrieren des Katalysators, Eindampfen des Filtrates und Säulenchromatographie ($SiO_2$, Acetonitril/Essigsäureethylester/Triethylamin 50:50:1) liefert die Titelverbindung: DC $R_f$(X)=0,09; $t_{Ret}(I)$=13,3 min; FAB-MS $(M+H)^+$=851.

**Beispiel 10: 5(S)-(Boc-amino)-4(S)-(methoxy-acetyloxy)-6-(p-fluor-phenyl)-2(R)-(p-fluorphenyl-methyl)-hexanoyl-(L)-Val-(L)-Phe-morpholin-4-ylamid**

Analog Beispiel 1) werden 100 mg (0,13 mMol) Boc-(p-F)Phe[C](p-F)Phe-(L)-Val-(L)-Phe-morpholin-4-ylamid [Referenz-Beispiel 21 B) 1)] und wenig DMAP in 1 ml Dioxan und 0,16 ml Pyridin mit 36 μl (0,39 mMol) Methoyxessigsäurechlorid (Fluka; Buchs/Schweiz) umgesetzt. Digerieren in DIPE liefert die Titelverbindung: DC $R_f$(B)=0,38; $t_{Ret}(I)$=16,9 min; FAB-MS $(M+H)^+$=837.

**Beispiel 11: 5(S)-(Boc-amino)-4(S)-(2-picolinoyl)-6-(p-fluor-phenyl)-2(R)-(P-fluorphenyl-methyl)-hexanoyl-(L)-Val-(L)-Phe-morpholin-4-ylamid**

Bei 0°C werden unter $N_2$-Atmosphäre 32,2 mg (0,26 mMol) 2-Picolinsäure (Fluka; Buchs/Schweiz) in 0,5 ml Methylenchlorid mit 22 μl (0,157 mMol) 1-Chlor-N,N,2-trimethyl-1-propenamin [B. Haveaux, A. Dekoker, M. Rens, A.R. Sidani, J. Toye, L. Ghosez, M. Murakami, M. Yoshioka, and W. Nagata, Organic Syntheses **59**, 26 (1980)] ins Säurechlorid überführt. Nach 45 min gibt man 1 ml Dioxan, 0,26 ml Pyridin, 100 mg (0,13 mMol) Boc-(p-F)Phe[C](p-F)Phe-(L)-Val-(L)-Phe-morpholin-4-ylamid [Referenz-Beispiel 21 B) 1)] und 0,3 mg DMAP zu. Da gemäss HPLC die Reaktion nach 18 h bei RT nicht beendet ist, wird bis zum vollständigen Umsatz weiter Säurechlorid zugegeben. Das dunkle Reaktionsgemisch verdünnt man mit Methylenchlorid und wäscht es 2x mit ges. $NaHCO_3$-Lösung, Wasser und Sole. Die wässrigen Phasen werden mit 2 Portionen Methylenchlorid extrahiert, die organischen Phasen mit $Na_2SO_4$ getrocknet, eingedampft und chromatographiert ($SiO_2$, Essigsäureethylester). Entfärben der rot gefärbten Lösung des Produkts in Essigsäureethylester mit Aktivkohle liefert die Titelverbindung: DC $R_f$(B)=0,16; $t_{Ret}(I)$=16,9 min; FAB-MS $(M+H)^+$=870.

**Beispiel 12: 5(S)-(Boc-amino)-4(S)-(pyridin-2-carboxyl)-6-phenyl-2(R)-(p-fluorphenyl-methyl)-hexanoyl-(L)-Val-(L)-Phe-morpholin-4-ylamid**

Analog Beispiel 11) werden 49 mg (0,40 mMol) 2-Picolinsäure in 3 ml Methylenchlorid bei RT mit 62 μl

(0,44 mMol) 1-Chlor-N,N,2-trimethyl-1-propenamin ins Säurechlorid überführt (17 h). Dazu gibt man eine Lösung von 150 mg (0,20 mMol) Boc-Phe[C](p-F)Phe-(L)-Val-(L)-Phe-morpholin-4-ylamid [Referenz-Beispiel 21 D) 1)] und 1,5 mg DMAP in 2,3 ml Pyridin. Da laut HPLC nach 17 h bei RT noch Edukt vorliegt, werden nochmals 0,6 mMol 2-Picolinsäurechlorid zugefügt. Nach weiteren 17 h giesst man das Reaktionsgemisch auf ges. $NaHCO_3$-Lösung und extrahiert mit 3 Portionen Methylenchlorid. Die organischen Phasen werden mit Wasser und Sole gewaschen, mit $Na_2SO_4$ getrocknet und eingedampft. Das dunkelrote Produkt wird in Essigsäureethylester gelöst, mit Aktivkohle behandelt, filtriert und erneut eingedampft. Digerieren im Ultraschallbad in DIPE liefert die Titelverbindung: DC $R_f$(B)=0,52; $t_{Ret}$(I)=18,8 min; FAB-MS $(M+H)^+$=852.

**Beispiel 13: 5(S)-(Boc-amino)-4(S)-(methoxy-acetyloxy)-6-phenyl-2(R)-(p-fluorphenyl-methyl)-hexanoyl-(L)-Val-(L)-Phe-morpholin-4-ylamid**

Analog Beispiel 1) werden 150 mg (0,20 mMol) Boc-Phe[C](p-F)Phe-(L)-Val-(L)-Phe-morpholin-4-ylamid [Referenz-Beispiel 21 D) 1)] und 1,2 mg DMAP in 1,5 ml Dioxan und 0,24 ml Pyridin mit 27 µl (0,30 mMol) Methoyxessigsäurechlorid (Fluka; Buchs/Schweiz) umgesetzt. Extraktion mit Essigsäureethylester liefert die Titelverbindung: DC $R_f$(B)=0,7; $t_{Ret}$(I)=16,9 min; FAB-MS $(M+H)^+$=819.

**Beispiel 14: 5(S)-(Boc-amino)-4(S)-(benzyloxy-acetyloxy)-6-phenyl-2(R)-(p-fluorphenyl-methyl)-hexanoyl-(L)-Val-(L)-Phe-morpholin-4-ylamid**

Analog Beispiel 1) werden 150 mg (0,20 mMol) Boc-Phe[C](p-F)Phe-(L)-Val-(L)-Phe-morpholin-4-ylamid [Referenz-Beispiel 21 D) 1)] und 1,2 mg DMAP in 1,5 ml Dioxan und 0,24 ml Pyridin mit 2 Portionen von je 47,5 µl (0,30 mMol) Benzyloxyacetylchlorid (Fluka; Buchs/Schweiz) umgesetzt. Digerieren in Hexan/DIPE 1:4 liefert die Titelverbindung als Schaum: DC $R_f$(A)=0,24; $t_{Ret}$(I)=18,7 min; FAB-MS $(M+H)^+$=895.

**Beispiel 15: 5(S)-(Boc-amino)-4(S)-[(S)-α-methoxy-α-phenyl-acetyloxy]-6-phenyl-2(R)-(p-fluorphenyl-methyl)-hexanoyl-(L)-Val-(L)-Phe-morpholin-4-ylamid**

Unter $N_2$-Atmosphäre werden 66,5 mg (0,40 mMol) (S)-α-Methoxyα-phenylessigsäure (Fluka; Buchs/Schweiz) in 3 ml Methylenchlorid mit 62 µl (0,44 mMol) 1-Chlor-N,N,2-trimethyl-1-propenamin [B. Haveaux, A. Dekoker, M. Rens, A.R. Sidani, J. Toye, L. Ghosez, M. Murakami, M. Yoshioka, and W. Nagata, Organic Syntheses **59**, 26 (1980)] bei RT ins Säurechlorid überführt. Nach 30 min gibt man eine Lösung von 150 mg (0,20 mMol) Boc-Phe[C](p-F)Phe-(L)-Val-(L)-Phe-morpholin-4-ylamid [Referenz-Beispiel 21 D) 1)] und 1,5 mg DMAP in 2,3 ml Pyridin zu und rührt 3 h nach. Anschliessend giesst man das Reaktionsgemisch auf $NaHCO_3$-Lösung und extrahiert mit 3 Portionen Methylenchlorid. Die organischen Phasen werden mit Wasser und Sole gewaschen, mit $Na_2SO_4$ getrocknet und eingedampft. Digerieren in DIPE liefert die Titelverbindung als Schaum: DC $R_f$(B)=0,73; $t_{Ret}$(I)=18,6 min; FAB-MS $(M+H)^+$=895.

**Beispiel 16: 5(S)-(Boc-amino)-4(S)-[(R)-α-methoxy-α-phenyl-acetyloxy]-6-phenyl-2(R)-(p-fluorphenyl-methyl)-hexanoyl-(L)-Val-(L)-Phe-morpholin-4-ylamid**

Analog Beispiel 15) werden 66,5 mg (0,40 mMol) (R)-α-Methoxyα-phenylessigsäure (Fluka; Buchs/Schweiz) in 3 ml Methylenchlorid mit 62 µl (0,44 mMol) 1-Chlor-N,N,2-trimethyl-1-propenamin ins Säurechlorid überführt und mit einer Lösung von 150 mg (0,20 mMol) Boc-Phe[C](p-F)Phe-(L)-Val-(L)-Phe-morpholin-4-ylamid [Beispiel 21 D) 1)] und 1,5 mg DMAP in 2,3 ml Pyridin zur Titelverbindung umgesetzt: DC $R_f$(A)=0,30; $t_{Ret}$(I)=18,4 min; FAB-MS $(M+H)^+$=895.

**Beispiel 17: 5(S)-(Boc-amino)-4(S)-(1-pyrazolylacetyloxy)-6-phenyl-2(R)-phenylmethyl-hexanoyl-(L)-Val-(L)-Phe-morpholin-4-ylamid**

Analog Beispiel 4 werden unter Stickstoffatmosphäre zu 252 mg (2 mMol) 1-Pyrazolylessigsäure (Jones et al., J. Org. Chem. 19, 1428-32 (1954)) in 15 ml Methylenchlorid bei 0°C 340 µl (2,4 mMol) 1-Chlor-N,N,2-trimethyl-1-propenamin *[B. Haveaux, A. Dekoker, M. Rens, A.R. Sidani, J. Toye, L. Ghosez, M. Murakami,M. Yoshioka and W. Nagata,* Organic Syntheses **59**, 26, (1980)*]* gegeben. Nach 30 Minuten Rühren bei Raumtemperatur wird das Gemisch mit einer Lösung von 729 mg (1 mMol) Boc-Phe[C]Phe-(L)-Val-(L)-Phe-morpholin-4-ylamid (Beispiel 2) und 5 mg 4-Dimethylaminopyridin in 5 ml Pyridin versetzt. Nach 155 Minuten bei Raumtemperatur wird noch während weiteren 2 Stunden bei 40°C gerührt. Da nach DC noch Edukt vorliegt werden zusätzliche 2 mMol 1-Pyrazolyl-essigsäurechlorid (Darstellung siehe oben) beigefügt. Nach weiteren 1,5 Stunden wird das Reaktionsgemisch auf 150 ml eines 2: 1-Gemisches (v/v) Wasser/gesättigte wässrige Natriumbicarbonat-Lösung gegossen und dreimal mit Methylenchlorid extrahiert. Die vereinten organischen Phasen werden nacheinander mit Wasser und Sole gewaschen und nach Trocknen über Natriumsulfat eingeengt. Nach Trocknen am HV wird chromatographiert ($SiO_2$, Methylenchlorid/Methanol von 100:0 bis 97:3). Digerieren in Diisopropylether und eine kurze Behandlung im Ultraschallbad liefert die reine Titelverbindung: DC $R_f$(Essigsäureethylester)= 0,34. $t_{Ret}$(I)= 16,8 min.

**Beispiel 18: 5(S)-(Boc-amino)-4(S)-(isochinolin-3-carbonyloxy)-6-phenyl-2(R)-phenylmethyl-hexanoyl-(L)-Val-(L)-Phe-morpholin-4-ylamid**

Analog Beispiel 4 wird ausgehend von 173 mg (1mMol) Isochinolin-3-carbonsäure (Aldrich, Bundesrepublik Deutschland) mit 250 μl 1-Chlor-N,N,2-trimethyl-1-propenamin das entsprechende Säurechlorid hergestellt. Letzteres wird im nachfolgenden Schritt mit einer Lösung von 365 mg (0,5 mMol) Boc-Phe[C]Phe-(L)-Val-(L)-Phe-morpholin-4-ylamid (Referenz-Beispiel 2) und 5 mg 4-Dimethylaminopyridin in 3 ml Pyridin versetzt. Nach Aufarbeiten analog Beispiel 17 wird chromatographiert ($SiO_2$, Essigester/Hexan von 4:1 bis 100:0). Digerieren in Diisopropylether liefert die reine Titelverbindung: DC $R_f$(Essigsäureethylester)= 0,28; $t_{Ret}$(I)= 17,2 min.

**Beispiel 19: 5(S)-(Boc-amino)-4(S)-(pyrazincarbonyloxy)-6-phenyl-2(R)-phenyl-methyl-hexanoyl-(L)-Val-(L)-Phe-morpholin-4-ylamid**

Analog Beispiel 4 wird ausgehend von 248 mg (2 mMol) Pyrazincarbonsäure (Fluka, Schweiz) mit 340 μl 1-Chlor-N,N,2-trimethyl-1-propenamin das entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) und 5 mg 4-Dimethylaminopyridin in 5 ml Pyridin versetzt. Nach Aufarbeiten analog Beispiel 17 wird chromatographiert ($SiO_2$, Essigester/Hexan von 4:1 bis 100:0). Digerieren in Diisopropylether liefert die reine Titelverbindung: DC $R_f$(Essigsäureethylester)= 0,31; $t_{Ret}$(I)= 16,4 min.

**Beispiel 20: 5(S)-(Boc-amino)-4(S)-(4-α-chlormethyl-benzoyloxy)-6-phenyl-2(R)-phenylmethyl-hexanoyl-(L)-Val-(L)-Phe-morpholin-4-ylamid**

Analog Beispiel 4 wird ausgehend von 680 mg (4 mMol) α-Chor-p-toluylsäure (Fluka, Schweiz) mit 800 μl 1-Chlor-N,N,2-trimethyl-1-propenamin das entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) in 5 ml Pyridin versetzt. Nach Aufarbeiten analog Beispiel 17 wird chromatographiert ($SiO_2$, Essigester). Digerieren in Diisopropylether liefert die reine Titelverbindung: DC $R_f$(Essigsäureethylester)= 0,5; $t_{Ret}$(II)= 28,2 min.

**Beispiel 21: 5(S)-(Boc-amino)-4(S)-[4-(4-morpholino)methyl-benzoyloxy]-6-phenyl-2(R)-phenylmethyl-hexanoyl-(L)-Val-(L)-Phe-morpholin-4-ylamid**

Analog Beispiel 4 wird ausgehend von 258 mg (1 mMol) α-(4-Morpholino)-p-toluylsäure (p-(Morpholin-4-ylmethyl)benzoesäure; Herstellung gemäss US-Patent 4,623,486 vom 18.11.1986 (Lombardino et al.)) mit 350 μl 1-Chlor-N,N,2-trimethyl-1-propenamin das entsprechende Säurechlorid hergestellt. Letzteres wird im nachfolgenden Schritt mit einer Lösung von 365 mg (0,5 mMol) Boc-Phe[C]Phe-(L)-Val-(L)-Phe-morpholin-4-ylamid (Referenz-Beispiel 2) in 2,5 ml Pyridin versetzt. Nach Aufarbeiten analog Beispiel 17 liefert eine Säulenchromatographie ($SiO_2$, Essigester dann Methylenchlorid/Methanol 100:0 bis 96:4) die reine Titelverbindung: DC $R_f$ (Essigsäureethylester)= 0,28; $t_{Ret}$(II)= 20,1 min;

**Beispiel 22: 5(S)-(Boc-amino)-4(S)-(isonicotinoyloxy)-6-phenyl-2(R)-phenylmethyl-hexanoyl-(L)-Val-(L)-Phe-morpholin-4-ylamid.**

Analog Beispiel 4 wird ausgehend von 260 mg (2 mMol) Isonicotinsäure (Fluka, Schweiz) mit 340 μl 1-Chlor-N,N,2-trimethyl-1-propenamin das entsprechende Säurechlorid hergestellt. Letzteres wird nachfolgend mit einer Lösung von 729 mg (1 mMol) Boc-Phe[C]Phe-(L)-Val-(L)-Phe-morpholin-4-ylamid (Referenz-Beispiel 2) und 5 mg 4-N,N-Dimethylaminopyridin in 5 ml Pyridin versetzt. Nach Aufarbeiten analog Beispiel 17 liefert eine Säulenchromatographie ($SiO_2$, Essigester) die reine Titelverbindung: DC $R_f$ (Essigsäureethylester)= 0,27; $t_{Ret}$(I)= 15,3 min; FAB-MS $(M+H^+)$= 834.

**Beispiel 23: 5(S)-(Boc-amino)-4(S)-(nicotinoyloxy)-6-phenyl-2(R)-phenylmethylhexanoyl-(L)-Val-(L)-Phe-morpholin-4-ylamid.**

Analog Beispiel 4 wird ausgehend von 740 mg (6 mMol) Nicotinsäure (Fluka, Schweiz) mit 1200 µl 1-Chlor-N,N,2-trimethyl-1-propenamin das entsprechende Säurechlorid hergestellt Letzteres wird im nachfolgenden Schritt mit einer Lösung von 1100 mg (1,5 mMol) Boc-Phe[C]Phe-(L)-Val-(L)-Phe-morpholin-4-ylamid (Referenz-Beispiel 2) und 10 mg 4-N,N-Dimethylaminopyridin in 5,5 ml Pyridin versetzt. Nach Aufarbeiten analog Beispiel 17 liefert eine Säulenchromatographie ($SiO_2$, Essigester) die reine Titelverbindung: DC $R_f$(Essigsäureethylester)= 0,33; $t_{Ret}$(II)= 22,4 min.

**Beispiel 24: 5(S)-(Boc-amino)-4(S)-(2-picolinoyloxy)-6-phenyl-2(R)-phenylmethyl-hexanoyl-(L)-Val-(L)-Phe-morpholin-4-ylamid.**

Analog Beispiel 41 wird ausgehend von 985 mg (8 mMol) 2-Picolinsäure (Fluka, Schweiz) mit 1600 µl 1-Chlor-N,N,2-trimethyl-1-propenamin das entsprechende Säurechlorid hergestellt. Letzteres wird im nachfolgenden Schritt mit einer Lösung von 1450 mg (2 mMol) Boc-Phe[C]Phe-(L)-Val-(L)-Phe-morpholin-4-ylamid (Referenz-Beispiel 2) und 10 mg 4-N,N-Dimethylaminopyridin in 7,5 ml Pyridin versetzt. Nach Aufarbeiten analog Beispiel 17 liefert eine Säulenchromatographie ($SiO_2$, Hexan/Essigester 1/1 bis Essigester) die reine Titelverbindung: DC $R_f$(Essigsäureethylester)= 0,23; $t_{Ret}$(II)= 28,4 min.

**Beispiel 25: 5(S)-(Boc-amino)-4(S)-(3-methoxypropanoyloxy)-6-phenyl-2(R)-phenyl-methyl-hexanoyl-(L)-Val-(L)-Phe-morpholin-4-ylamid.**

Analog Beispiel 4 wird ausgehend von 387 µl (4 mMol) 3-Methoxypropionsäure (Fluka, Schweiz) mit 680 µl 1-Chlor-N,N,2-trimethyl-1-propenamin das entsprechende Säurechlorid hergestellt. Letzteres wird im nachfolgenden Schritt mit einer Lösung von 1450 mg (2 mMol) Boc-Phe[C]Phe-(L)-Val-(L)-Phe-morpholin-4-ylamid (Referenz-Beispiel 2) und 10 mg 4-N,N-Dimethylaminopyridin in 10 ml Pyridin versetzt. Nach Aufarbeiten analog Beispiel 17 liefert eine Säulenchromatographie ($SiO_2$, Essigester) die reine Titelverbindung: DC $R_f$(Essigsäureethylester)= 0,37; $t_{Ret}$(I)= 17,3 min; FAB-MS $(M+H^+)$= 815.

**Beispiel 26: 5(S)-(Boc-amino)-4(S)-[(4-chlorphenoxy)methoxyacetyloxy)]-6-phenyl-2(R)-phenylmethyl-hexanoyl-(L)-Val-(L)-Phe-morpholin-4-ylamid**

Analog Beispiel 4 wird ausgehend von 217 mg (1 mMol) (4-Chlorphenoxy)methoxyessigsäure (Cretin et al., Phytochemistry 22(12), 2661-64 (1983)) mit 170 µl 1-Chlor-N,N,2-trimethyl-1-propenamin das entsprechende Säurechlorid hergestellt. Letzteres wird im nachfolgenden Schritt mit einer Lösung von 365 mg (0,5 mMol) Boc-Phe[C]Phe-(L)-Val-(L)-Phe-morpholin-4-ylamid (Referenz-Beispiel 2) und 5 mg 4-N,N-Dimethylaminopyridin in 2,5 ml Pyridin versetzt. Nach Aufarbeiten analog Beispiel 17 liefert eine Säulenchromatographie ($SiO_2$, Essigester) die reine Titelverbindung: DC $R_f$(Essigsäureethylester)= 0,5; $t_{Ret}$(I)= 19,1 min; FAB-MS $(M+H^+)$= 927.

**Beispiel 27: 5(S)-(Boc-amino)-4(S)-[2-(2-methoxyethoxy)acetyloxy)]-6-phenyl-2(R)-phenylmethyl-hexanoyl-(L)-Val-(L)-Phe-morpholin-4-ylamid.**

Analog Beispiel 4 wird ausgehend von 240 µl (2 mMol) 2-(2-Methoxyethoxy)essigsäure (Fluka, Schweiz) mit 340 µl 1-Chlor-N,N,2-trimethyl-1-propenamin das entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) und 5 mg 4-N,N-Dimethylaminopyridin in 5 ml Pyridin versetzt. Nach Aufarbeiten analog Beispiel 17 liefert eine Säulenchromatographie ($SiO_2$, Methylenchlorid/Methanol: 100/0 bis 97/3) die reine Titelverbindung: DC $R_f$(Essigsäureethylester)= 0,33; $t_{Ret}$(I)= 16,9 min; FAB-MS $(M+H^+)$= 845.

**Beispiel 28: 5(S)-(Boc-amino)-4(S)-(butyloxyacetyloxy)-6-phenyl-2(R)-phenylmethyl-hexanoyl-(L)-Val-(L)-Phe-morpholin-4-ylamid.**

Analog Beispiel 4 wird ausgehend von 265 mg (2 mMol) 2-(n-Butoxy)essigsäure (Janssen, Niederlande) mit 340 µl 1-Chlor-N,N,2-trimethyl-1-propenamin *das* entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) und 5 mg 4-N,N-Dimethylaminopyridin in 5 ml Pyridin *versetzt.* Nach Aufarbeiten analog Beispiel 17 liefert eine Säulenchromatographie ($SiO_2$, Essigester) die reine Titelverbindung: DC $R_f$(Essigsäureethylester)= 0,44; $t_{Ret}$(I)= 18,7 min; FAB-MS ($M+H^+$)= 843.

**Beispiel 29: 5(S)-(Boc-amino)-4(S)-[2-[2-(2-methoxyethoxy)ethoxy]acetyloxy)]-6-phenyl-2(R)-phenylmethyl-hexanoyl-(L)-Val-(L)-Phe-morpholin-4-ylamid.**

Analog Beispiel 4 wird ausgehend von 314 µl (2 mMol) 2-[2-(2-Methoxyethoxy)ethoxy]essigsäure (Fluka, Schweiz) mit 340 µl 1-Chlor-N,N,2-trimethyl-1-propenamin das entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) und 5 mg 4-N,N-Dimethylaminopyridin in 5 ml Pyridin versetzt. Nach Aufarbeiten analog Beispiel 17 liefert eine Säulenchromatographie ($SiO_2$, Methylenchlorid/Methanol: 100/0 bis 97/3) die reine Titelverbindung: DC $R_f$(Essigsäureethylester)= 0,27; $t_{Ret}$(I)= 16,7 min; FAB-MS ($M+H^+$)= 889.

**Beispiel 30: 5(S)-(Boc-amino)-4(S)-(methoxyacetyloxy)-6-phenyl-2(R)-phenyl-methyl-hexanoyl-(L)-Val-(L)-Phe-morpholin-4-ylamid**

Analog Beispiel 35 werden 729 mg (1mMol) Boc-Phe[C]Phe-(L)-Val-(L)-Phe-morpholin-4-ylamid (Referenz-Beispiel 2) mit 100 µl (1,1 mMol) Methoxyessigsäurechlorid (Fluka, Schweiz) in die Titelverbindung überführt, die durch Säulenchromatographie ($SiO_2$, Essigester/Hexan: 4/1) gereinigt wird. DC $R_f$(Essigester)= 0,43; $t_{Ret}$(I)= 16,8 min; FAB-MS ($M+H^+$)= 801.

**Beispiel 31: 5(S)-(Boc-amino)-4(S)-(phenoxyacetyloxy)-6-phenyl-2(R)-phenylmethyl-hexanoyl-(L)-Val-(L)-Phe-morpholin-4-ylamid**

Analog Beispiel 35 werden 1450 mg (2 mMol) Boc-Phe[C]Phe-(L)-Val-(L)-Phe-morpholin-4-ylamid (Referenz-Beispiel 2) mit 360 µl (2,6 mMol) Phenoxyacetylchlorid (Fluka, Schweiz) in die Titelverbindung überführt, die durch Säulenchromatographie ($SiO_2$, Essigester/Hexan: 4/1) gereinigt wird. DC $R_f$(Essigester/Hexan: 4/1)= 0,37; $t_{Ret}$(I)= 18,5 min; FAB-MS ($M+H^+$)= 863.

**Beispiel 32: 5(S)-(Boc-amino)-4(S)-[(S)-α-methoxy-α-phenylacetyloxy)-6-phenyl2(R)-phenylmethyl-hexanoyl-(L)-Val-(L)-Phe-morpholin-4-ylamid.**

Analog Beispiel 4 wird ausgehend von 332.4 mg (2 mMol) (S)-α-Methoxy-α-phenylessigsäure (Fluka, Schweiz) mit 340 µl 1-Chlor-N,N,2-trimethyl-1-propenamin das entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) und 5 mg 4-N,N-Dimethylaminopyridin in 5 ml Pyridin versetzt. Nach Aufarbeiten analog Beispiel 17 liefert eine Säulenchromatographie ($SiO_2$, Essigester) die reine Titelverbindung: DC $R_f$(Essigsäureethylester)= 0,43; $t_{Ret}$(I)= 18,5 min; FAB-MS ($M+H^+$)= 877.

**Beispiel 33: 5(S)-(Boc-amino)-4(S)-[(R)-α-methoxy-α-phenylacetyloxy)]-6-phenyl-2(R)-phenylmethyl-hexanoyl-(L)-Val-(L)-Phe-morpholin-4-ylamid.**

Analog Beispiel 4 wird ausgehend von 333 mg (2 mMol) (S)-α-Methoxy-α-phenylessigsäure (Fluka, Schweiz) mit 340 µl 1-Chlor-N,N,2-trimethyl-1-propenamin das entsprechende Säurechlorid hergestellt. Letzteres wird im nachfolgenden Schritt mit einer Lösung von 729 mg (1 mMol) Boc-Phe[C]Phe-(L)-Val-(L)-Phe-morpholin-4-ylamid (Referenz-Beispiel 2) und 5 mg 4-N,N-Dimethylaminopyridin in 5 ml Pyridin versetzt. Nach Aufarbeiten analog Beispiel 17 liefert eine Säulenchromatographie ($SiO_2$, Essigester) die reine Titelverbindung: DC $R_f$(Essigsäureethylester)= 0,5; $t_{Ret}$(I)= 18,3 min; FAB-MS ($M+H^+$)= 877.

**Beispiel 34: 5(S)-(Boc-amino)-4(S)-(valeroyloxy)-6-phenyl-2(R)-phenylmethyl-hexanoyl-(L)-Val-(L)-Phe-morpholin-4-ylamid**

Analog Beispiel 35 werden 729 mg (1mMol) Boc-Phe[C]Phe-(L)-Val-(L)-Phe-morpholin-4-ylamid (Referenz-Beispiel 2) mit 181 µl (1,5 mMol) Valeroylchlorid (Fluka, Schweiz) in die Titelverbindung überführt, die durch Säulenchromatographie ($SiO_2$, Essigester/Hexan: 4/1) gereinigt wird. DC $R_f$(Essigester/Hexan: 4/1)= 0,33; $t_{Ret}$(I)= 18,9 min; **FAB-MS ($M+H^+$)= 813.**

**Beispiel 35: 5(S)-(Boc-amino)-4(S)-(pivaloyloxy)-6-phenyl-2(R)-phenylmethylhexanoyl-(L)-Val-(L)-Phe-morpholin-4-ylamid**

Unter Argon-Atmosphäre wird eine Lösung von 510 mg (0.7 mMol) Boc-Phe[C]Phe-(L)-Val-(L)-Phe-morpholin-4-ylamid (Referenz-Beispiel 2) in 4 ml Pyridin über 10 Minuten mit einer Lösung von 130 µl (1,05 mMol) Pivaloylchlorid in 0,4 ml Pyridin versetzt. Nach weiterer zweimaliger Zugabe von 150 µl Pivaloylchlorid und 5 mg 4-N,N-Dimethylaminopyridin bei einer Gesamtreaktionszeit von 3,75 Stunden bei 50°C kann kein Edukt im DC mehr nachgewiesen werden. Das Reaktionsgemisch wird auf ca. 40 ml wässriger gesättigter Natriumbicarbonat-Lösung gegossen und dreimal mit Methylenchlorid extrahiert. Die vereinigten Extrakte werden mit Sole gewaschen, über Natriumsulfat getrocknet und eingeengt. Die anschliessende Säulenchromatographie (SiO2, Methylenchlorid/Methanol: 100/0 bis 98,5/1,5) liefert die reine Titelverbindung. DC $R_f$(Methylenchlorid/Methanol: 95/5)= 0,6; $t_{Ret}$(II)= 28,3 min; FAB-MS ($M+H^+$)= 813.

**Beispiel 36: 5(S)-(Boc-amino)-4(S)-(palmitoyloxy)-6-phenyl-2(R)-phenylmethylhexanoyl-(L)-Val-(L)-Phe-morpholin-4-ylamid**

Analog Beispiel 35 werden 729 mg (1mMol) Boc-Phe[C]Phe-(L)-Val-(L)-Phe-morpholin-4-ylamid (Referenz-Beispiel 2) mit 456 µl (1,5 mMol) Palmitinsäurechlorid (Fluka, Schweiz) in die Titelverbindung überführt, die durch Säulenchromatographie ($SiO_2$, Essigester/Hexan: 1/1) gereinigt wird. DC $R_f$(Essigester/Hexan: 4/1)= 0,43; $t_{Ret}$(100% Acetonitril)= 13,8 min; FAB-MS ($M+H^+$)= 967.

**Beispiel 37: 5(S)-(Boc-amino)-4(S)-(butyroyloxy)-6-phenyl-2(R)-phenylmethylhexanoyl-(L)-Val-(L)-Phe-morpholin-4-ylamid**

Analog Beispiel 35 werden 729 mg (1mMol) Boc-Phe[C]Phe-(L)-Val-(L)-Phe-morpholin-4-ylamid (Referenz-Beispiel 2) mit 156 µl (1,5 mMol) Buttersäurechlorid (Fluka, Schweiz) in die Titelverbindung überführt, die durch Säulenchromatographie ($SiO_2$, Essigester/Hexan: 4/1) gereinigt wird. DC $R_f$(Essigester)= 0,43; $t_{Ret}$(I)= 18,3 min; FAB-MS ($M+H^+$)= 799.

**Beispiel 38: 5(S)-(Boc-amino)-4(S)-(propanoyloxy)-6-phenyl-2(R)-phenylmethylhexanoyl-(L)-Val-(L)-Phe-morpholin-4-ylamid**

Analog Beispiel 35 werden 729 mg (1mMol) Boc-Phe[C]Phe-(L)-Val-(L)-Phe-morpholin-4-ylamid (Referenz-Beispiel 2) mit 131 µl (1,5 mMol) Propionylchlorid (Fluka, Schweiz) in die Titelverbindung überführt, die durch Säulenchromatographie ($SiO_2$, Essigester/Hexan: 4/1) gereinigt wird. DC $R_f$(Essigester)= 0,43; $t_{Ret}$(I)= 17,6 min; FAB-MS **($M+H^+$)= 785.**

**Beispiel 39: 5(S)-(Boc-amino)-4(S)-(benzoyloxy)-6-phenyl-2(R)-phenylmethylhexanoyl-(L)-Val-(L)-Phe-morpholin-4-ylamid**

Analog Beispiel 35 werden 365 mg (1mMol) Boc-Phe[C]Phe-(L)-Val-(L)-Phe-morpholin-4-ylamid (Referenz-Beispiel 2) mit 64 µl (1,1 mMol) Benzoylchlorid (Aldrich, Bundesrepublik Deutschland) in die Titelverbindung überführt, die durch Säulenchromatographie ($SiO_2$, Essigester) gereinigt wird. DC $R_f$(Essigester)= 0,5; $t_{Ret}$(II)= 27,6 min; FAB-MS ($M+H^+$)= 833.

**Beispiel 40: 5(S)-(Boc-amino)-4(S)-(methylpropionyloxy)-6-phenyl-2(R)-phenylmethyl-hexanoyl-(L)-Val-(L)-Phe-morpholin-4-ylamid**

Analog Beispiel 2) wird Boc-Phe[C]Phe-(L)-Val-(L)-Phe-morpholin-4-ylamid (Referenz-Beispiel 2) mit einem Überschuss an Isobuttersäurechlorid (Fluka; Buchs/Schweiz) umgesetzt: $t_{Ret}$(I)=18,2 min; FAB-MS $(M+H)^+$=799.

Beispiel 41: Analog einem der vorgenannten Verfahren werden die folgenden Verbindungen hergestellt:

A) 5(S)-(Boc-amino)-4(S)-[3-(dimethyl-amino)-propionyloxy]-6-phenyl-2(R)-phenylmethyl-hexanoyl-(L)-Val-(L)-Phe-morpholin-4-ylamid

B)5(S)-(Boc-amino)-4(S)-[3-(N-Z-N-methyl-amino)-propionyloxy]-6-phenyl-2(R)-phenylmethyl-hexanoyl-(L)-Val-(L)-Phe-morpholin-4-ylamid

C) 5(S)-(Boc-amino)-4(S)-(3-methyl-amino-propionyloxy)-6-phenyl-2(R)-phenylmethyl-hexanoyl-(L)-Val-(L)-Phe-morpholin-4-ylamid

D) 5(S)-(Boc-amino)-4(S)-[(dimethylaminoethoxy)-acetyloxy]-6-phenyl-2(R)-(p-fluorphenyl-methyl)-hexanoyl-(L)-Val-(L)-Phe-morpholin-4-ylamid

E) 5(S)-(Boc-amino)-4(S)-[(2-pyridylmethoxy)-acetyloxy]-6-phenyl-2(R)-(p-fluorphenyl-methyl)-hexanoyl-(L)-Val-(L)-Phe-morpholin-4-ylamid

F) 5(S)-(Boc-amino)-4(S)-(methoxy-acetyloxy)-6-phenyl-2(R)-(p-methoxyphenyl-methyl)-hexanoyl-(L)-Val-(L)-(p-$CH_3O$-Phe)-morpholin-4-ylamid

G) 5(S)-(Boc-amino)-4(S)-(pyridin-2-carboxyl)-6-phenyl-2(R)-(p-methoxyphenyl-methyl)-hexanoyl-(L)-Val-(L)-(p-$CH_3O$-Phe)-morpholin-4-ylamid

**H): 5(S)-(Boc-amino)-4(S)-(2-methylthio)acetyloxy-6-phenyl-2(R)-benzylhexanoyl-(L)-Val-(L)-Phe-morpholin-4-ylamid**

Analog Beispiel 4 wird eine Lösung von 546 mg (0,75 mMol) 5(S)-(Boc-amino)-4(S)-hydroxy-6-phenyl-2(R)-benzyl-hexanoyl-(L)-Val-(L)-Phe-morpholin-4-ylamid und 5 mg DMAP in 5 ml Pyridin zu einem Gemisch von 0,174 ml (2 mMol) 2-Methylthioessigsäure (Fluka, Buchs, Schweiz) und 0,34 ml (2,4 mMol) 1-Chlor-N,N,2-trimethyl-1-propenamin in 15 ml Methylenchlorid zugegeben. Nach Aufarbeiten und Säulenchromatographie ($SiO_2$, Essigsäureethylester/Hexan: 4/1) wird die Titelverbindung isoliert: DC $R_f$(B)= 0,41; $t_{Ret}$(I)= 17,9 min; FAB-MS $(M+H^+)$= 817

I) 5(S)-(Boc-amino)-4(S)-(benzylthioacetyloxy)-6-phenyl-2(R)-phenylmethyl-hexanoyl-(L)-Val-(L)-Phe-morpholin-4-ylamid

**J): 5(S)-(Boc-amino)-4(S)-[(L)-(prolyl)oxy]-6-phenyl-2(R)-phenylmethyl-hexanoyl-(L)-Val-(L)-Phe-morpholin-4-ylamid**

In Gegenwart von 120 mg 10 % Pd/C werden 1,12 g (1,166 mMol) 5(S)-(Boc-amino)-4(S)-[(L)-(N-Z-prolyl)oxy]-6-phenyl-2(R)-phenylmethyl-hexanoyl-(L)-Val-(L)-Phe-morpholin-4-ylamid (Beispiel 41 L)) in 20 ml Essigsäureethylester hydriert. Filtration, Eindampfen des Filtrats und Digerieren aus DIPE liefert die Titelverbindung: $t_{Ret}$(I)=13,8 min; FAB-MS $(M+H)^+$=826.

K) **5(S)-(Boc-amino)-4(S)-[(D)-(prolyl)oxy]-6-phenyl-2(R)-phenylmethyl-hexanoyl-(L)-Val-(L)-Phe-morpholin-4-ylamid**

In Gegenwart von 0,20 g 10 % Pd/C werden 950 mg (0,989 mMol) 5(S)-(Boc-amino)-4(S)-[(D)-(N-Z-prolyl)oxy]-6-phenyl-2(R)-phenylmethyl-hexanoyl-(L)-Val-(L)-Phe-morpholin-4-ylamid (Beispiel 41 M) in 20 ml Essigsäureethylester hydriert. Filtration, Eindampfen des Filtrats, Säulenchromatographie ($SiO_2$, THF/Ether 1:1) und Digerieren aus DIPE liefert die Titelverbindung: $t_{Ret}$(I)=13,5 min; FAB-MS $(M+H)^+$=826.

*L)* **5(S)-(Boc-amino)-4(S)-[(L)-(N-Z-prolyl)oxy]-6-phenyl-2(R)-phenylmethyl-hexano-yl-(L)-Val-(L)-Phe-morpholin-4-ylamid**

Unter $N_2$-Atmosphäre werden 683 mg (2,74 mMol) Z-(L)-Prolin in 5 ml Methylenchlorid bei RT mit 232 µl (1,644 mMol) 1-Chlor-N,N,2-trimethyl-1-propenamin [B. Haveaux, A. Dekoker, M. Rens, A.R. Sidani, J. Toye, L. Ghosez, M. Murakami, M. Yoshioka, and W. Nagata, Organic Syntheses **59**, 26 (1980)] umgesetzt. Nach 30 min gibt man dazu 2,7 ml Pyridin, 1,00 g (1,37 mMol) Boc-Phe[C]Phe-(L)-Val-(L)-Phe-morpholin-4-ylamid (Referenz-Beispiel 2) und 3 mg DMAP. Da gemäss HPLC nach 17 h bei RT noch Edukt vorliegt, werden nochmals 1,3 mMol Z-(L)-Prolin mit 0,8 mMol 1-Chlor-N,N,2-trimethyl-1-propenamin aktiviert und zugefügt. Nach weiteren 17 h giesst man das Reaktionsgemisch auf ges. $NaHCO_3$-Lösung und extrahiert mit 3 Portionen Methylenchlorid. Die organischen Phasen werden mit Wasser und Sole gewaschen, mit $Na_2SO_4$ getrocknet und eingedampft. Säulenchromatographie ($SiO_2$, Essigsäureethylester) liefert die Titelverbindung: DC $R_f$(B)=0,50; $t_{Ret}$(I)=20,0 min; FAB-MS $(M+H)^+$=961.

M) **5(S)-(Boc-amino)-4(S)-[(D)-(N-Z-prolyl)oxy]-6-phenyl-2(R)-phenylmethylhexanoyl-(L)-Val-(L)-Phe-morpholin-4-ylamid**

Unter $N_2$-Atmosphäre werden 683 mg (2,74 mMol) Z-(D)-Prolin in 5 ml Methylenchlorid bei RT mit 232 µl (1,644 mMol) 1-Chlor-N,N,2-trimethyl-1-propenamin umgesetzt..Nach 30 min gibt man dazu 2,7 ml Pyridin, 1,00 g (1,37 mMol) Boc-Phe[C]Phe-(L)-Val-(L)-Phe-morpholin-4-ylamid (Referenz-Beispiel 2) und 3 mg DMAP. Da gemäss HPLC nach 17 h bei RT noch Edukt vorliegt, werden nochmals 683 mg Z-(D)-Prolin mit 232 µl 1-Chlor-N,N,2-trimethyl-1-propenamin aktiviert und zugefügt. Nach weiteren 17 h arbeitet man analog Beispiel 41L) auf: DC $R_f$(B)=0,47; $t_{Ret}$(I)=19,3 min; FAB-MS $(M+H)^+$=960.

- Als Ausgangsprodukt für eine Reihe von Verbindungen dient 5(S)-(Boc-amino)-4(S)-(chlor-acetyloxy)-6-phenyl-2(R)-(p-fluorphenyl-methyl)-hexanoyl-(L)-Val-(L)-Phe-morpholin-4-ylamid (herstellbar durch Umsetzung von Chloressigsäure mit 5(S)-(Boc-amino)-4(S)-hydroxy)-6-phenyl-2(R)-(p-fluorphenyl-methyl)-hexanoyl-(L)-Val-(L)-Phe-morpholin-4-ylamid analog Beispiel 4), in welchem das Chloratom durch nukleophile Substitution in ein substituiertes N-, S- oder O-Atom überführt wird, beispielsweise bei der Herstellung der Verbindungen D) mit Dimethylaminoethanol, E) mit 2-Pyridylmethanol.

Beispiel 42:

Analog einem der in den Beispielen 1 bis 41 beschriebenen Verfahren werden, soweit nicht schon in den vorgenannten Beispielen explizit beschrieben, die Verbindungen der Formel I der Referenzbeispiele 1 bis 22 und 33 bis 56 unter Ersatz der 4(S)-Hydroxygruppe im zentralen nicht hydrolysierbaren Peptidbaustein in die entsprechenden 4(S)-Acyloxyverbindungen überführt, worin anstelle der 4(S)-Hydroxygruppe jeweils einer der folgenden Reste steht:

A) 4(S)-(2-Furanylcarboxy)-;
B) 4(S)-[4-(Dimethyl-amino)-butyryloxy];
C) 4(S)-(N-Z-N-Methyl-aminoacetyloxy);
D) 4(S)-(Methylamino-acetyloxy);
E) 4(S)-[N-(Imidazol-4-methyl)-N-methyl-aminoacetyloxy]; F) 4(S)-[3-(1-Triphenylmethyl-imidazol-4-yl)-propionyloxy];
G) 4(S)-[3-(4-Imidazolyl)-propionyloxy];
H) 4(S)-(Methoxy-acetyloxy);
I) 4(S)-((2-picolinoyl);
J) 4(S)-(Benzyloxy-acetyloxy);
K) 4(S)-[(S)-α-Methoxy-α-phenyl-acetyloxy];
L) 4(S)-[(R)-α-Methoxy-α-phenyl-acetyloxy];
M) 4(S)-(1-Pyrazolylacetyloxy);
N) 4(S)-(Isochinolin-3-carbonyloxy);
O) 4(S)-(Pyrazincarbonyloxy);
P) 4(S)-(4-α-Chlormethyl-benzoyloxy);
Q) 4(S)-[4-(4-Morpholino)methyl-benzoyloxy];
R) 4(S)-(Isonicotinoyloxy);
S) 4(S)-(Nicotinoyloxy);
T) 4(S)-(3-Methoxypropanoyloxy)
U) 4(S)-[(4-Chlorphenoxy)methoxyacetyloxy)];
V) 4(S)-[2-(2-Methoxyethoxy)acetyloxy)];
W) 4(S)-(Butyloxyacetyloxy);
X) 4(S)-[2-[2-(2-Methoxyethoxy)ethoxy]acetyloxy)];
Y) 4(S)-(Methoxyacetyloxy);
Z) 4(S)-(Phenoxyacetyloxy);
AA) 4(S)-[(S)-α-Methoxy-α-phenylacetyloxy);
AB) 4(S)-[(R)-α-methoxy-α-phenylacetyloxy)];
AC) (N,N-Dimethyl-aminoacetyloxy);
AD) 4(S)-[N-(Pyridin-2-methyl)-N-methyl-aminoacetyloxy].

Beispiel 43:

Analog einem der in den Beispielen 1 bis 41 beschriebenen Verfahren werden, soweit nicht schon in den vorgenannten Beispielen explizit beschrieben, die Verbindungen der Formel I der Referenzbeispiele 1 bis 22 und 33 bis 56 unter Ersatz der 4(S)-Hydroxygruppe im zentralen nicht hydrolysierbaren Peptidbaustein in die entsprechenden 4(S)-Acyloxyverbindungen überführt, worin anstelle der 4(S)-Hydroxygruppe jeweils einer der folgenden Reste steht:

A) 4(S)-[3-(Dimethyl-amino)-propionyloxy];
B) 4(S)-[3-(N-Z-N-Methyl-amino)-propionyloxy];
C) 4(S)-(3-Methyl-amino-propionyloxy);
D) 4(S)-[(Dimethylaminoethoxy)-acetyloxy];
E) 4(S)-[(2-Pyridylmethoxy)-acetyloxy];
F) 4(S)-(Methoxy-acetyloxy);
G) 4(S)-(Pyridin-2-carboxyl);
H) 4(S)-(Methylthioacetyloxy);
I) 4(S)-(Benzylthioacetyloxy);
J) 4(S)-((L)-Prolyloxy);
K) 4(S)-((D)-Prolyloxy);
L) 4(S)-((L)-(N-Z-Prolyl)oxy);
M) 4(S)-((D)-(N-Z-Prolyl)oxy).

**Beispiel 44: 5(S)-(Boc-amino)-4(S)-[3-(N-Z-amino)-propionyloxy]-6-phenyl-2(R)-phenylmethyl-hexanoyl-(L)-Val-(L)-Phe-morpholin-4-ylamid**

Unter $N_2$-Atmosphäre werden 612 mg (2,74 mMol) Z-β-Alanin in 5 ml Methylenchlorid bei RT mit 232 μl (1,644 mMol) 1-Chlor-N,N,2-trimethyl-1-propenamin umgesetzt. Nach 40 min gibt man dazu 2,7 ml Pyridin, 1,00 g (1,37 mMol) Boc-Phe[C]Phe-(L)-Val-(L)-Phe-morpholin-4-ylamid (Referenz-Beispiel 2) und 3 mg DMAP. Da gemäss HPLC nach 17 h bei RT noch Edukt vorliegt, werden nochmals 612 mg Z-β-Alanin mit 232 μl 1-Chlor-N,N,2-trimethyl-1-propenamin aktiviert und zugefügt. Nach weiteren 17 h arbeitet man analog Beispiel 41L) auf: DC $R_f$(B)=0,38; $t_{Ret}$(I)=18,3 min; FAB-MS $(M+H)^+$=934.

**Beispiel 45: 5(S)-(Boc-amino)-4(S)-(3-amino-propionyloxy)-6-phenyl-2(R)-phenyl-methyl-hexanoyl-(L)-Val-(L)-Phe-morpholin-4-ylamid**

Hydrieren von 1,00 g (1,07 mMol) 5(S)-(Boc-amino)-4(S)-[3-(N-Z-amino)-propionyloxy]-6-phenyl-2(R)-phenylmethyl-hexanoyl-(L)-Val-(L)-Phe-morpholin-4-ylamid in 20 ml Essigsäureethylester in Gegenwart von 0,2 g 10 %-igem Pd/C liefert nach Filtration, Eindampfen des Filtrats und Digerieren aus DIPE die Titelverbindung: $t_{Ret}$(I)=13,1 min; FAB-MS $(M+H)^+$=800.

**Beispiel 46: 5(S)-(Boc-amino)-4(S)-[(3-dimethylamino-propoxy)-acetyloxy]-6-phenyl-2(R)-phenylmethyl-hexanoyl-(L)-Val-(L)-Phe-morpholin-4-ylamid**

Zu 13 mg (0,124 mMol) 3-Dimethylamino-propanol (Fluka; Buchs, Schweiz) gibt man eine Lösung von 0,124 mMol 5(S)-(Boc-amino)-4(S)-(jod-acetyloxy)-6-phenyl-2(R)-phenylmethyl-hexanoyl-(L)-Val-(L)-Phe-morpholin-4-ylamid in 0,3 ml Aceton, spült 2x mit je 0,3 ml Aceton nach und rührt nach Zugabe von 15,6 mg (0,187 mMol) $NaHCO_3$ 17h bei RT (HPLC: vollständig umgesetzt). Das Lösungsmittel wird im $N_2$-Strom abgeblasen, der Rückstand in wenig Methylenchlorid aufgeschlämmt und auf ein Gemisch von Eiswasser und Methylenchlorid gegossen. Man trennt die organische Phase ab, wäscht sie mit Wasser und Sole und extrahiert die wässrigen Phasen 2x mit Methylenchlorid. Trocknen der organischen Phasen mit $Na_2SO_4$, Eindampfen und Fällen mit DIPE aus einer konzentrierten Lösung in Aceton ergibt die Titelverbindung: $t_{Ret}$(I)=13,5 min; FAB-MS $(M+H)^+$=872.

Das Ausgangsmaterial wird wie folgt hergestellt:

**a) 5(S)-(Boc-amino)-4(S)-(jodacetyloxy)-6-phenyl-2(R)-phenylmethyl-hexanoyl-(L)-Val-(L)-Phe-morpholin-4-ylamid**

Eine Lösung von 100 mg (0,124 mMol) 5(S)-(Boc-amino)-4(S)-(chloracetyloxy)-6-phenyl-2(R)-phenylmethyl-hexanoyl-(L)-Val-(L)-Phe-morpholin-4-ylamid in 0,3 ml Aceton wird mit 90,5 mg (0,604 mMol) Natriumjodid versetzt und 1,5 h bei RT gerührt, dabei scheidet sich ein Niederschlag aus. Da ein HPLC nach 1 h bereits vollständigen Umsatz zur Titelverbindung anzeigt, wird die überstehende Lösung abpipettiert und sofort in obiger Stufe eingesetzt: $t_{Ret}$(I)=18,2 min.

**b) 5(S)-(Boc-amino)-4(S)-(chloracetyloxy)-6-phenyl-2(R)-phenylmethyl-hexanoyl-(L)-Val-(L)-Phe-morpholin-4-ylamid**

Unter $N_2$-Atmosphäre werden 2,00 g (2,74 mMol) Boc-Phe[C]Phe-(L)-Val-(L)-Phe-morpholin-4-ylamid (Referenz-Beispiel 2) in 20 ml Acetonitril und 12 ml Dioxan vorgelegt, mit 1,3 ml Pyridin, wenig DMAP und schliesslich 1,17 g (6,85 mMol) Chloressigsäure-anhydrid versetzt. Nach 24 h Rühren bei RT wird auf Eiswasser gegossen und 3x mit Essigsäureethylester extrahiert. Die organischen Phasen werden mit 2 Portionen ges. $NaHCO_3$ Lösung, Wasser und Sole gewaschen, mit $Na_2SO_4$ getrocknet und eingedampft. Eine Säulenchromatographie ($SiO_2$, Methylenchlorid/Ether 1:1) führt zur Titelvebindung: DC $R_f$(A')=0,16; $t_{Ret}$(I)=17,7 min; FAB-MS $(M+H)^+$=805.

**Beispiel 47: 5(S)-(Boc-amino)-4(S)-[2-(2-dimethylamino-ethoxy)-ethoxy-acetyloxy]-6-phenyl-2(R)-phenylmethyl-hexanoyl-(L)-Val-(L)-Phe-morpholin-4-ylamid**

Zu 37,9 mg (0,285 mMol) 2-(2-Dimethylamino-ethoxy)-ethanol (BASF; Ludwigshafen, Deutschland) gibt man eine Lösung von 0,285 mMol 5(S)-(Boc-amino)-4(S)-(jod-acetyloxy)-6-phenyl-2(R)-phenylmethyl-hexanoyl-(L)-Val-(L)-Phe-morpholin-4-ylamid in Aceton (Beispiel 46a), spült 2x mit je 0,7 ml Aceton nach, gibt 36 mg (0,43 mMol) $NaHCO_3$ zu und rührt 20 h bei RT aus. Aufarbeitung analog zu Beispiel 46 und Verrühren des Rohproduktes aus DIPE liefert die Titelverbindung: $t_{Ret}$(I)=13,5 min; FAB-MS $(M+H)^+$=902.

**Beispiel 48: 5(S)-(Boc-amino)-4(S)-[(4-dimethylamino-butoxy)-acetyloxy]-6-phenyl-2(R)-phenylmethyl-hexanoyl-(L)-Val-(L)-Phe-morpholin-4-ylamid**

Zu 14,5 mg (0,124 mMol) 4-Dimethylamino-1-butanol (BASF; Ludwigshafen, Deutschland) gibt man eine Lösung von 0,124 mMol 5(S)-(Boc-amino)-4(S)-(jod-acetyloxy)-6-phenyl-2(R)-phenylmethyl-hexanoyl-(L)-Val-(L)-Phe-morpholin-4-ylamid in Aceton (Beispiel 46a), spült 2x mit je 0,3 ml Aceton nach und rührt nach Zugabe von 26 mg (0,187 mMol) $K_2CO_3$ 18 h bei RT aus. Da gemäss HPLC noch Jod-acetat vorliegt, werden noch 0,25 Äquivalente 4-Dimethylamino- 1-butanol zugesetzt. Aufarbeitung nach 17 h analog zu Beispiel 46 liefert die Titelverbindung: $t_{Ret}$(I)=13,5 min; FAB-MS $(M+H)^+$=886.

**Beispiel 49: 5(S)-(Boc-amino)-4(S)-[2-(2-methoxy-ethoxy)-acetyloxy]-6-phenyl-2(R)-(p-methoxy-phenyl)methyl-hexanoyl-(L)-Val-(L)-Phe-morpholin-4-ylamid**

Unter $N_2$-Atmosphäre werden 75 mg (≈ 60 %-ig; 0,296 mMol) 2-(2-Methoxy-ethoxy)-essigsäure-chlorid in 1,5 ml Dioxan mit 0,25 ml Pyridin, 150 mg (0,198 mMol) Boc-Phe[C](p-$CH_3O$)Phe-(L)-Val-(L)-Phe-morpholin-4-ylamid (Referenz-Beispiel 21 F) 1)) und wenig DMAP versetzt. Nach 17 h bei RT giesst man das Reaktionsgemisch auf ges. $NaHCO_3$ Lösung, extrahiert 3x mit Methylenchlorid, wäscht die organischen Phasen mit ges. $NaHCO_3$ Lösung, Wasser und Sole, trocknet sie mit $Na_2SO_4$ und dampft sie ein. Säulenchromatographie ($SiO_2$, Methylenchlorid/THF 4:1) und Verrühren aus Hexan liefert die Titelverbindung: DC $R_f$(B')=0,37; $t_{Ret}$(I)=16,7 min; FAB-MS $(M+H)^+$=875.

Das Ausgangsmaterial wird wie folgt hergestellt:

**a) 2-(2-Methoxy-ethoxy)-essigsäurechlorid**

Unter $N_2$-Atmosphäre gibt man zu 1,69 ml (14,9 mMol) 2-(2-Methoxy-ethoxy)-essigsäure (Fluka; Buchs/Schweiz) in 75 ml Methylenchlorid 2,56 ml (29,8 mMol) Oxalylchlorid und 2 Tropfen DMF. Nach 17 h bei RT wird das Reaktionsgemisch am RV unter Feuchtigkeitsausschluss vorsichtig eingedampft. Ein $^1$H-NMR-Spektrum des Rückstandes zeigt die Signale der Titelverbindung neben Signalen von ≈40 % Lösungsmittel (vor allem Methylenchlorid): $^1$H-NMR (200 MHz, $CDCl_3$): 3,37 (s, MeO), 3,58 und 3,77 (2d, J=5 Hz, $CH_2$-$CH_2$), 4,48 (s, $CH_2$). Dieses Gemisch wird oben eingesetzt.

**Beispiel 50: 5(S)-(Boc-amino)-4(S)-(2-picolinoyloxy)-6-phenyl-2(R)-(p-methoxyphenyl)methyl-hexanoyl-(L)-Val-(L)-Phe-morpholin-4-ylamid**

Unter $N_2$-Atmosphäre werden 48,6 mg (0,395 mMol) 2-Picolinsäure in 0,78 ml Methylenchlorid bei 0°C mit 33,5 µl (0,237 mMol) 1-Chlor-N,N,2-trimethyl-1-propenamin umgesetzt. Nach 30 min gibt man dazu 0,39 ml Pyridin, 150 mg (0,198 mMol) Boc-Phe[C](p-$CH_3O$)Phe-(L)-Val-(L)-Phe-morpholin-4-ylamid (Referenz-Beispiel 21 F) 1)) und 0,4 mg DMAP. Da gemäss HPLC nach 17 h bei RT noch Boc-Phe[C](p-$CH_3O$)Phe-(L)-Val-(L)-Phe-morpholin-4-ylamid vorliegt, werden Portionen von jeweils 48,6 mg 2-Picolinsäure mit jeweils 33,5 µl 1-Chlor-N,N,2-trimethyl-1-propenamin aktiviert und zugefügt bis nach jeweils 16 h Reaktionszeit alles Edukt umgesetzt ist. Aufarbeitung analog Beispiel 41L) und Säulenchromatographie ($SiO_2$, Methylenchlorid/THF 4:1) führt zur Titelverbindung: DC $R_f$(B')=0,30; $t_{Ret}$(I)=16,5 min; FAB-MS $(M+H)^+$=865.

**Beispiel 51: 5(S)-(Boc-amino)-4(S)-{2-[2-(2-methoxy-ethoxy)ethoxy]-acetyloxy}-6-phenyl-2(R)-(p-methoxy-phenyl)methyl-hexanoyl-(L)-Val-(L)-(p-$CH_3O$-Phe)-morpholin-4-ylamid**

Unter $N_2$-Atmosphäre werden 58 µl (0,38 mMol) 2-[2-(2-methoxy-ethoxy)ethoxy]-essigsäure (Fluka; Buchs/Schweiz) in 0,7 ml Methylenchlorid bei 0°C mit 32 µl (0,228 mMol) 1-Chlor-N,N,2-trimethyl-1-propenamin umgesetzt. Nach 30 min gibt man dazu 0,37 ml Pyridin, 150 mg (0,190 mMol) Boc-Phe[C](p-$CH_3O$)Phe-(L)-Val-(L)-(p-$CH_3O$-Phe)-morpholin-4-ylamid (Referenz-Beispiel 21 F) 3)) und wenig DMAP. Da gemäss HPLC nach 19 h bei RT noch Boc-Phe[C](p-$CH_3O$)Phe-(L)-Val-(L)-(p-$CH_3O$-Phe)-morpholin-4-ylamid vorliegt, werden nochmals 58 µl 2-[2-(2-methoxy-ethoxy)ethoxy]-essigsäure mit 32 µl 1-Chlor-N,N,2-trimethyl-1-propenamin aktiviert und zugefügt. Aufarbeitung nach 17 h analog Beispiel 41L), Säulenchromatographie ($SiO_2$, Methylenchlorid/THF 4:1) und Verrühren in DIPE liefert die Titelverbindung: DC $R_f$(B')=0,23; $t_{Ret}$(I)=16,5 min; FAB-MS $(M+H)^+$=949.

**Beispiel 52: 5(S)-(Boc-amino)-4(S)-(2-picolinoyloxy)-6-phenyl-2(R)-(p-methoxy-phenyl)methyl-hexanoyl-(L)-Val-(L)-(p-$CH_3$O-Phe)-morpholin-4-ylamid**

Unter $N_2$-Atmosphäre werden 46,8 mg (0,380 mMol) 2-Picolinsäure in 0,75 ml Methylenchlorid bei 0°C mit 32 µl (0,228 mMol) 1-Chlor-N,N,2-trimethyl-1-propenamin umgesetzt. Nach 30 min gibt man dazu 150 mg (0,190 mMol) Boc-Phe[C](p-$CH_3$O)Phe-(L)-Val-(L)-(p-$CH_3$O-Phe)-morpholin-4-ylamid (Referenz-Beispiel 21 F) 3)) in 0,38 ml Pyridin und 0,4 mg DMAP. Da gemäss HPLC nach 17 h bei RT noch Boc-Phe[C](p-$CH_3$O)Phe-(L)-Val-(L)-(p-$CH_3$O-Phe)-morpholin-4-ylamid vorliegt, werden nochmals 46,8 mg 2-Picolinsäure mit 32 µl 1-Chlor-N,N,2-trimethyl-1-propenamin aktiviert und zugefügt. Aufarbeitung nach 18 h analog Beispiel 41L), Säulenchromatographie ($SiO_2$, Methylenchlorid/THF 4:1) und Verrühren aus Hexan führt zur Titelverbindung: DC $R_f$(B')=0,37; $t_{Ret}$(I)=16,4 min; FAB-MS $(M+H)^+$=894.

**Beispiel 53: 5(S)-(Boc-amino)-4(S)-(2-benzyloxy)acetyloxy-6-phenyl-2(R)-benzyl-hexanoyl-(L)-Val-(L)-Phe-morpholin-4-ylamid**

Analog Beispiel 1 werden 730 mg (1 mMol) 5(S)-(Boc-amino)-4(S)-hydroxy-6-phenyl-2(R)-benzyl-hexanoyl-(L)-Val-(L)-Phe-morpholin-4-ylamid mit 0,237 ml (1,5 mMol) Benzyloxyacetylchlorid (Fluka, Buchs, Schweiz) und 5 mg DMAP in 5 ml Pyridin zur Titelverbindung umgesetzt. Säulenchromatographie ($SiO_2$, Essigsäureethylester) liefert die Titelverbindung: DC $R_f$(B)= 0,5; $t_{Ret}$(I)= 19 min; FAB-MS $(M+H^+)$= 877.

**Beispiel 54: 5(S)-(Boc-amino)-4(S)-(2-acetyloxy)acetyloxy-6-phenyl-2(R)-benzyl-hexanoyl-(L)-Val-(L)-Phe-morpholin-4-ylamid**

Analog Beispiel 1 werden 730 mg (1 mMol) 5(S)-(Boc-amino)-4(S)-hydroxy-6-phenyl-2(R)-benzyl-hexanoyl-(L)-Val-(L)-Phe-morpholin-4-ylamid mit 0,166 ml (1,5 mMol) Acetyloxyacetylchlorid (Aldrich, Steinheim, BRD) und 5 mg DMAP in 5 ml Pyridin zur Titelverbindung umgesetzt. Säulenchromatographie ($SiO_2$, Essigsäureethylester) liefert die Titelverbindung: DC $R_f$(B)= 0,41; $t_{Ret}$(I) = 17,3 min; FAB-MS $(M+H^+)$= 829.

**Beispiel 55: 5(S)-(Boc-amino)-4(S)-[2-(4-tetrahydropyranyloxy)acetyloxy-6-phenyl2(R)-benzyl-hexanoyl-(L)-Val-(L)-Phe-morpholin-4-ylamid**

Analog Beispiel 4 wird eine Lösung von 365 m g (0,5 mMol) 5(S)-(Boc-amino)-4(S)-hydroxy-6-phenyl-2(R)-benzyl-hexanoyl-(L)-Val-(L)-Phe-morpholin-4-ylamid und 5 mg DMAP in 4 ml Pyridin einem Gemisch von 160 mg (1 mMol) 4-Tetrahydropyranylessigsäure (Chemical Abstracts-Registry No. 85064-61-5) und 0,17 ml (1,2 mMol) 1-Chlor-N,N,2-trimethyl-1-propenamin in 10 ml Methylenchlorid zugegeben. Nach Aufarbeiten und Säulenchromatographie ($SiO_2$, Essigsäureethylester/Hexan: 4/1) wird die Titelverbindung isoliert: DC $R_f$ (Methylenchlorid/Methanol: 4/1)= 0,57; $t_{Ret}$(I) = 16,9 min; FAB-MS $(M+H^+)$= 871.

**Beispiel 56: 5(S)-(Boc-amino)-4(S)-[2(R)-(4-tetrahydropyranyloxy)]-propionyloxy-6-phenyl-2(R)-benzyl-hexanoyl-(L)-Val-(L)-Phe-morpholin-4-ylamid**

Analog Beispiel 4 wird eine Lösung von 219 mg (0,3 mMol) 5(S)-(Boc-amino)-4(S)-hydroxy-6-phenyl-2(R)-benzyl-hexanoyl-(L)-Val-(L)-Phe-morpholin-4-ylamid und 5 mg DMAP in 1,5 ml Pyridin einem Gemisch von 105 mg (0,6 mMol) 2(R)-(4-Tetrahydropyranyl)-propionsäure (Beilstein E III/IV, Vol. 18, p 3851) und 0,11 ml (0,72 mMol) 1-Chlor-N,N,2-trimethyl-1-propenamin in 5 ml Methylenchlorid zugegeben. Nach Aufarbeiten und Säulenchromatographie ($SiO_2$, Essigsäureethylester/Hexan: 4/1) wird die Titelverbindung isoliert: DC $R_f$(B)= 0,37; $t_{Ret}$(I)= 17,3 min; FAB-MS $(M+H^+)$= 885.

**Beispiel 57: 5(S)-(Boc-amino)-4(S)-[2-(2-amino-ethoxy)ethoxy-acetyloxy)]-6-phenyl-2(R)-benzyl-hexanoyl-(L)-Val-(L)-Phe-morpholin-4-ylamid**

Analog Beispiel 45 werden 0,83 g (0,82 mMol) 5(S)-(Boc-amino)-4(S)-[2-(2-benzyloxycarbonylamino-ethoxy)ethoxy-acetyloxy)]-6-phenyl-2(R)-benzyl-hexanoyl-(L)-Val-(L)-Phe-morpholin-4-ylamid (Beispiel 58) in 20 ml Methanol in Gegenwart von 0,1 g 10% Pd/C hydriert. Säulenchromatographie ($SiO_2$, Methylenchlorid/Methanol: 95/5 bis 80/20) liefert die Titelverbindung: DC $R_f$ (Methylenchlorid/Methanol:9/1 )= 0,15;$t_{Ret}$(I) = 13,1 min; FAB-MS $(M+H^+)$= 874.

**Beispiel 58: 5(S)-(Boc-amino)-4(S)-[2-(2-benzyloxycarbonylamino-ethoxy)ethoxy-acetyloxy)]-6-phenyl-2(R)-benzyl-hexanoyl-(L)-Val-(L)-Phe-morpholin-4-ylamid**

Analog Beispiel 4 wird eine Lösung von 1,09 g (1,5 mMol) 5(S)-(Boc-amino)-4(S)-hydroxy-6-phenyl-2(R)-benzyl-hexanoyl-(L)-Val-(L)-Phe-morpholin-4-ylamid und 10 mg DMAP in 7,5 ml Pyridin einem Gemisch von 0,9 g (3 mMol) 2-(2-Benzyloxycarbonylamino)-ethoxy)-ethoxyessigsäure und 0,51 ml (3,6 mMol) 1-Chlor-N,N,2-trimethyl- 1-propenamin in 20 ml Methylenchlorid zugegeben. Nach Aufarbeiten und Säulenchromatographie ($SiO_2$, Methylenchlorid bis Methylenchlorid/Methanol: 98/2) wird die Titelverbindung isoliert: DC $R_f$(B)= 0,41; $t_{Ret}$(I)= 17,95 min; FAB-MS ($M+H^+$)= 1008.

Die Ausgangsverbindungen werden folgendermassen hergestellt:

**a) 2-[2-(Phthalimidoethoxy)ethoxy]-essigsäureethylester**

Eine Lösung von 10,5 g (50 mMol) 2-[2-(Chlorethoxy)ethoxy]-essigsäure-ethylester (Chemical Abstracts-Registry No. 82227-25-6) in 100 ml DMF wird mit 9,25 g (50 mMol) Kaliumphthalimid (Fluka, Buchs, Schweiz) und 50 mg 18-Krone-6-Ether (1,4,7,10,13,16-Hexaoxacyclooctadecan, Fluka, Buchs, Schweiz) umgesetzt und während 2,5 h bei 100°C gerührt. Nach Abkühlen des Reaktionsgemisches wird unter vermindertem Druck eingeengt. Der Rückstand wird in Ether aufgenommen und je 2 mal mit Wasser und Sole gewaschen. Nach Trocknen und Einengen wird die Titelverbindung durch Säulenchromatographie gewonnen ($SiO_2$, Essigsäureethylester/Hexan: 1/1); DC $R_f$ (Essigsäureethylester/Hexan: 4/1)= 0,45;

**b) 2-[2-(Aminoethoxy)ethoxy]-essigsäure** (siehe auch E.P. 0 410 280 A1)

Eine Lösung von 9,6 g (29,9 mMol) 2-[2-(Phthalimidoethoxy)ethoxy]-essigsäure-ethylester in 195 ml 20% wässriger HCl wird während 1,5 h am Rückfluss gerührt. Nach Abkühlen wird am Rotationsverdampfer eingeengt und der erhaltene Rückstand in 45 ml Wasser aufgenommen. Nach Abfiltrieren des unlöslichen Materials wird das Filtrat 3 mal mit Essigsäureethylester extrahiert. Die vereinigten organischen Phasen werden mit Wasser und Sole gewaschen, getrocknet und eingeengt. Das erhaltene weisse kristalline Material wird in 110 ml 6N HCl während 17,5 h gerührt. Nach Abkühlen wird eingeengt und der Rückstand wird in 20 ml Wasser aufgenommen. Nach 3 maligem Waschen der wässrigen Phase mit Essigsäureethylester wird die wässrige Phase am Hochvakuum lyophilisiert. DC $R_f$(Methylethylketon/Essigsäure/Wasser: 3/1/1) = 0,45.

**c) 2-[2-(Benzyloxycarbonyl-aminoethoxy)ethoxy]-essigsäure**

Eine Suspension von 6,7 g (12,5 mMol) 2-[2-(Aminoethoxy)ethoxy]-essigsäure in 30 ml THF und 10 ml Wasser wird mit 2N NaOH auf pH 10 gestellt und tropfenweise mit 1,95 ml Chlorameisensäure-benzylester (Fluka, Buchs, Schweiz) umgesetzt. Während dem 2,5 h Rühren wird der pH mittels 2N NaOH zwischen 9 und 9,5 gehalten. Nach Einengen des Reaktionsgemisches unter vermindertem Druck wird die erhaltene wässrige Phase 2 mal mit Essigsäureethylester gewaschen, mit 2N Schwefelsäure auf pH 1,8 gestellt und 3 mal mit Essigsäureethylester extrahiert. Die vereinigten organischen Phasen werden anschliessend mit Wasser und Sole gewaschen, getrocknet und unter vermindertem Druck eingeengt. Die so erhaltene Titelverbindung wird ohne weitere Reinigung weiterverwendet. DC $R_f$ (Essigsäureethylester/Essigsäure: 9/1) = 0,39.

**Beispiel 59: 5(S)-(Boc-amino)-4(S)-[(methoxycarbonyl-methoxy)-acetyloxy]-6-phenyl-2(R)-benzyl-hexanoyl-(L)-Val-(L)-Phe-morpholin-4-ylamid**

Analog Beispiel 4 wird eine Lösung von 219 mg (0,3 mMol) 5(S)-(Boc-amino)-4(S)-hydroxy-6-phenyl-2(R)-benzyl-hexanoyl-(L)-Val-(L)-Phe-morpholin-4-ylamid und 5 mg DMAP in 1,5 ml Pyridin einem Gemisch von 89 mg (0,6 mMol) Diglykolsäure-monomethylester (Chem. Ber. 55, 670 (1922) und 0,11 ml (0,72 mMol) 1-Chlor-N,N,2-trimethyl-1-propenamin in 5 ml Methylenchlorid zugegeben. Nach Aufarbeiten und Säulenchromatographie ($SiO_2$, Essigsäureethylester/Hexan: 4/1) wird die Titelverbindung isoliert. DC $R_f$(B)= 0,35; $t_{Ret}$(*IV*)= 16,84 min; FAB-MS ($M+H^+$)= 859.

**Beispiel 60: 5(S)-(Boc-amino)-4(S)-[(methoxycarbonyl-methylthio)-acetyloxy]-6-phenyl-2(R)-benzyl-hexanoyl-(L)-Val-(L)-Phe-morpholin-4-ylamid**

Analog Beispiel 4 wird eine Lösung von 219 mg (0,3 mMol) 5(S)-(Boc-amino)-4(S)-hydroxy-6-phenyl-2(R)-benzyl-hexanoyl-(L)-Val-(L)-Phe-morpholin-4-ylamid und 5 mg DMAP in 1,5 ml Pyridin einem Gemisch von 98 mg (0,6 mMol) Thiodiglykolsäure-monomethylester (Indian J. Chem. 25B, 880 (1986)) und 0,11 ml (0,72 mMol) 1-Chlor-N,N,2-trimethyl-1-propenamin in 5 ml Methylenchlorid zugegeben. Aufarbeiten und Säulenchromatographie ($SiO_2$, Essigsäureethylester/Hexan: 4/1) ergibt die Titelverbindung.

**Beispiel 61: 5(S)-(Boc-amino)-4(S)-[(methoxycarbonyl-methylsulfo)-acetyloxy]-6-phenyl-2(R)-benzyl-hexanoyl-(L)-Val-(L)-Phe-morpholin-4-ylamid**

Analog Beispiel 4 wird eine Lösung von 219 mg (0,3 mMol) 5(S)-(Boc-amino)-4(S)-hydroxy-6-phenyl-2(R)-benzyl-hexanoyl-(L)-Val-(L)-Phe-morpholin-4-ylamid und 5 mg DMAP in 1,5 ml Pyridin einem Gemisch von 117 mg (0,6 mMol) S,S-Dioxo-thiodiglykolsäure-monomethylester und 0,11 ml (0,72 mMol) 1-Chlor-N,N,2-trimethyl-1-propenamin in 5 ml Methylenchlorid zugegeben. Nach Aufarbeiten und Säulenchromatographie ($SiO_2$, Essigsäureethylester/Hexan: 4/1) wird die Titelverbindung isoliert.

Die Ausgangsverbindung wird folgendermassen hergestellt:

a) **S,S-Dioxo-thiodiglykolsäure-monomethylester**

Thiodiglykolsäure-monomethylester (Indian J. Chem. 25B, 880 (1986)) wird in einem Methanol/Wassergemisch bei ca. 25 °C mit Kaliumperoxomonosulfat zur Titelverbindung oxidiert, welche weiterverwendet wird.

**Beispiel 62: 5(S)-(Boc-amino)-4(S)-[(pivaloyloxymethoxycarbonyl-methoxy)-acetyloxy]-6-phenyl-2(R)-benzyl-hexanoyl-(L)-Val-(L)-Phe-morpholin-4-ylamid**

5(S)-(Boc-amino)-4(S)-[(carboxy-methoxy)-acetyloxy]-6-phenyl-2(R)-benzyl-hexanoyl-(L)-Val-(L)-Phe-m orpholin- 4-ylamid wird mit Jodmethylpivalat (Chem. Abstr. Registry No. 53064-79-2) versetzt. Die anschliessende Säulenchromatographie liefert die Titelverbindung.

**Beispiel 63: 5(S)-(Boc-amino)-4(S)-[(carboxymethoxy)-acetyloxy]-6-phenyl-2(R)-benzyl-hexanoyl-(L)-Val-(L)-Phe-morpholin-4-ylamid**

Eine Lösung von 219 mg (0,3 mMol) 5(S)-(Boc-amino)-4(S)-hydroxy-6-phenyl-2(R)-benzyl-hexanoyl-(L)-Val-(L)-Phe-morpholin-4-ylamid in Pyridin wird mit Diglykolsäureanhydrid (Fluka, Buchs, Schweiz) versetzt. Nach Aufarbeiten und Säulenchromatographie ($SiO_2$, Essigsäureethylester) wird die Titelverbindung isoliert.

**Beispiel 64: (S)-(Boc-amino)-4(S)-[(acetoxymethoxycarbonyl-methoxy)-acetyloxy]-6- phenyl-2(R)-benzyl-hexanoyl-(L)-Val-(L)-Phe-morpholin-4-ylamid**

Analog Beispiel 62 wird 5(S)-(Boc-amino)-4(S)-[(carboxy-methoxy)-acetyloxy]-6-phenyl-2(R)-benzyl-hexanoyl-(L)-Val-(L)-Phe-morpholin- 4-ylamid mit Brommethylacetat (Aldrich, Steinheim, BRD) umgesetzt. Die anschliessende Säulenchromatographie liefert die Titelverbindung.

**Beispiel 65:**

Analog einem der in den Beispielen 1 bis 64 beschriebenen Verfahren werden, soweit nicht schon in den vorgenannten Beispielen explizit beschrieben, die Verbindungen der Formel I der Referenzbeispiele 57 bis 64 unter Ersatz der 4(S)-Hydroxygruppe im zentralen nicht hydrolysierbaren Peptidbaustein in die entsprechenden 4(S)-Acyloxyverbindungen überführt, worin anstelle der 4(S)-Hydroxygruppe jeweils einer der folgenden Reste steht:

A) 4(S)-(2-Furanylcarboxy)-;
B) 4(S)-[4-(Dimethyl-amino)-butyryloxy];
C) 4(S)-(N-Z-N-Methyl-aminoacetyloxy);
D) 4(S)-(Methylamino-acetyloxy);
E) 4(S)-[N-(Imidazol-4-methyl)-N-methyl-aminoacetyloxy]; F) 4(S)-[3-(1-Triphenylmethyl-imidazol-4-yl)-propionyloxy];
G) 4(S)-[3-(4-Imidazolyl)-propionyloxy];
H) 4(S)-(Methoxy-acetyloxy);
I) 4(S)-((2-picolinoyl);
J) 4(S)-(Benzyloxy-acetyloxy);
K) 4(S)-[(S)-α-Methoxy-α-phenyl-acetyloxy];
L) 4(S)-[(R)-α-Methoxy-α-phenyl-acetyloxy];
M) 4(S)-(1-Pyrazolylacetyloxy);
N) 4(S)-(Isochinolin-3-carbonyloxy);
O) 4(S)-(Pyrazincarbonyloxy);
P) 4(S)-(4-α-Chlormethyl-benzoyloxy);
Q) 4(S)-[4-(4-Morpholino)methyl-benzoyloxy];

R) 4(S)-(Isonicotinoyloxy);
S) 4(S)-(Nicotinoyloxy);
T) 4(S)-(3-Methoxypropanoyloxy)
U) 4(S)-[(4-Chlorphenoxy)methoxyacetyloxy)];
V) 4(S)-[2-(2-Methoxyethoxy)acetyloxy)];
W) 4(S)-(Butyloxyacetyloxy);
X) 4(S)-[2-[2-(2-Methoxyethoxy)ethoxy]acetyloxy)];
Y) 4(S)-(Methoxyacetyloxy);
Z) 4(S)-(Phenoxyacetyloxy);
AA) 4(S)-[(S)-α-Methoxy-α-phenylacetyloxy);
AB) 4(S)-[(R)-α-methoxy-α-phenylacetyloxy)];
AC) (N,N-Dimethyl-aminoacetyloxy);
AD) 4(S)-[N-(Pyridin-2-methyl)-N-methyl-aminoacetyloxy];
AE) 4(S)-[3-(Dimethyl-amino)-propionyloxy];
AF) 4(S)-[3-(N-Z-N-Methyl-amino)-propionyloxy];
AG) 4(S)-(3-Methyl-amino-propionyloxy);
AH) 4(S)-[(Dimethylaminoethoxy)-acetyloxy];
AI) 4(S)-[(2-Pyridylmethoxy)-acetyloxy];
AJ) 4(S)-(Methoxy-acetyloxy);
AK) 4(S)-(Pyridin-2-carboxyl);
AL) 4(S)-(Methylthioacetyloxy);
AM) 4(S)-(Benzylthioacetyloxy);
AN) 4(S)-((L)-Prolyloxy);
AO) 4(S)-((D)-Prolyloxy);
AP) 4(S)-((L)-(N-Z-Prolyl)oxy);
AQ) 4(S)-((D)-(N-Z-Prolyl)oxy).

**Beispiel 66:**

Analog einem der in den Beispielen 1 bis 64 beschriebenen Verfahren werden, soweit nicht schon in den vorgenannten Beispielen explizit beschrieben, die Verbindungen der Formel I der Referenzbeispiele 1 bis 22 und 33 bis 64 unter Ersatz der 4(S)-Hydroxygruppe im zentralen nicht hydrolysierbaren Peptidbaustein in die entsprechenden 4(S)-Acyloxyverbindungen überführt, worin anstelle der 4(S)-Hydroxygruppe jeweils einer der folgenden Reste steht:
3-(N-Z-Amino)-propionyloxy;
3-Amino-propionyloxy;
(3-Dimethylamino-propoxy)-acetyloxy;
2-(2-Dimethylamino-ethoxy)-ethoxy-acetyloxy;
(4-Dimethylamino-butoxy)-acetyloxy;
(2-Benzyloxy)acetyloxy;
2-Acetyloxy)acetyloxy;
2-(4-Tetrahydropyranyloxy)acetyloxy;
2(R)-(4-Tetrahydropyranyloxy)]-propionyloxy;
2-(2-Amino-ethoxy)ethoxy-acetyloxy);
2-(2-Benzyloxycarbonylamino-ethoxy)ethoxy-acetyloxy);
(Methoxycarbonyl-methoxy)-acetyloxy;
(Methoxycarbonyl-methylthio)-acetyloxy;
(Methoxycarbonyl-methylsulfo)-acetyloxy;
(Pivaloyloxymethoxycarbonyl-methoxy;
(Carboxymethoxy)-acetyloxy;
(Acetoxymethoxycarbonyl-methoxy)-acetyloxy.

**Beispiel 67: Gelatine-Lösung**

Eine sterilfiltrierte wässrige Lösung eines der in den vorausgehenden Beispielen 1 bis 43 und 44 bis 66 genannten Wirkstoffe der Formel I' oder von den Referenz-Beispielen 42 bis 76 der Formel I, die zusätzlich 20 % Cyclodextrin enthält, und eine sterile, mit Phenol konservierte Gelatinelösung werden unter Erwärmen unter aseptischen Bedingungen so vermischt, dass 1,0 ml Lösung folgender Zusammensetzung resultiert:

| | |
|---|---|
| Wirkstoff | 3 mg |
| Gelatine | 150,0 mg |
| Phenol | 4,7 mg |
| dest. Wasser mit 20 % Cyclodextrinen | 1,0 ml |

**Beispiel 68: Sterile Trockensubstanz zur Injektion**

Man löst 5 mg einer der in den vorausgehenden Beispielen 1 bis 43 und 44 bis 66 genannten Wirkstoffe der Formel I' oder von den Referenz-Beispielen 42 bis 76 der Formel I als Wirkstoff in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

**Beispiel 69: Nasenspray**

In einer Mischung von 3,5 ml Myglyol 812® und 0,08 g Benzylalkohol werden 500 mg fein gemahlenes (<5,0 μm) Pulver einer der in den vorausgehenden Beispielen 1 bis 43 und 44 bis 66 genannten Verbindungen der Formel I' oder von den Referenz-Beispielen 42 bis 76 der Formel I als Wirkstoff suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

**Beispiel 70: Lacktabletten**

Für die Herstellung von 10 000 Tabletten enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen 1 bis 43 und 44 bis 66 genannten Verbindungen der Formel I' oder von den Referenz-Beispielen 42 bis 76 der Formel I als Wirkstoff, 50 g Maisstärke und kolloidaler Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 Min. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

**Beispiel 71: Oral verabreichbare Dispersion 1**

625 mg einer der in den vorausgehenden Beispielen 1 bis 43 und 44 bis 66 genannten Verbindungen der Formel I' oder von den Referenz-Beispielen 42 bis 76 der Formel I als Wirkstoff und 625 mg POPC (1-Palmitoyl-2-oleoyl-phosphatidylcholin = 1-Hexadecanoyl-2-(9-cis-octadecenoyl)-3-sn-phosphatidylcholin) werden in 25 ml Ethanol gelöst. Die Lösung wird mit der zehnfachen Menge Wasser verdünnt. Hierzu wird die ethanolische Lösung bei Raumtemperatur mit einer Rate von 10 ml/min zu der vorgelegten Menge Wasser zugetropft. Das Ethanol wird aus der Mischung durch tangentiale Dialyse ("Cross Flow Filtration") gegen 1750 ml Wasser (System: Minitan®, 700 $cm^2$- Polyethersulphon-Membran mit einer Ausschlussgrenze von 100 kD, der Firma Millipore (USA)) entfernt. Die Mischung wird unter Verwendung desselben Systems auf 15 mg Wirkstoff konzentriert durch Ultrafiltration. Nach Zugabe von 1,24 mg/ml Zitronensäure und 1,24 mg/ml Dinatriumhydrogenphosphat·2 $H_2O$ zur Einstellung auf pH 4,2 und von 1 mg/ml Sorbinsäure als antimikrobiellem Konservierungsmittel wird die Dispersion erneut auf 15 mg/ml aufkonzentriert und in Gläschen, beispielsweise mit 20 ml Inhalt, abgefüllt. Die Dispersionspartikel haben einen Durchmesser von 0,1 - 2 µm. Sie sind bei +2 bis 8 °C mindestens ein halbes Jahr lang stabil und geegnet zur oralen Verabreichung.

**Beispiel 72: Oral verabreichbare Dispersion 2**

Die Herstellung erfolgt analog Beispiel 71, jedoch unter Verwendung von 25 mg Wirkstoff und 50 mg POPC zur Herstellung der ethanolischen Lösung.

**Beispiel 73: Oral verabreichbare Dispersion 3**

Die Herstellung erfolgt analog Beispiel 71, jedoch unter Verwendung von 25 mg Wirkstoff und 125 mg POPC zur Herstellung der ethanolischen Lösung.

**Beispiel 74: Oral verabreichbare Dispersion 4**

Die Herstellung erfolgt analog Beispiel 71, jedoch unter Verwendung von 50 mg Wirkstoff und 50 mg POPC zur Herstellung der ethanolischen Lösung.

**Beispiel 75: Oral verabreichbare Dispersion 5**

Die Herstellung erfolgt analog einem der Beispiele 71 bis 74, jedoch unter Verwendung von Wirkstoff und Phosphatidylcholin aus Soja oder Phosphatidylcholin aus Eigelb (70 - 100 % rein) anstelle von POPC zur Herstellung der ethanolischen Lösung. Gewünschtenfalls wird ein Antioxidans, wie Ascorbinsäure, in einer Konzentration von 5 mg/ml zugegeben.

## Patentansprüche

1. Verbindungen der Formel I',

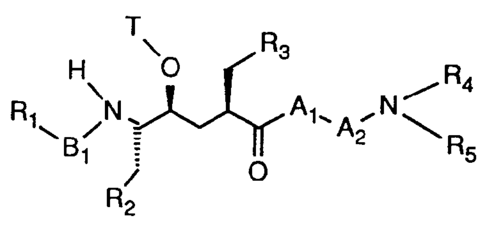

(I'),

worin T ein Acylradikal der Formel Z,

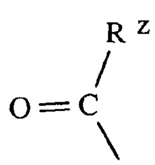

(Z)

worin

$R^z$ unsubstituiertes oder substituiertes Hydrocarbyl, in dem mindestens ein Kohlenstoffatom durch

ein Heteroatom ersetzt ist mit der Massgabe, dass ein Heteroatom nicht direkt an das Carbonyl gebunden ist, an das der Rest $R^z$ gebunden ist, Alkyl mit 2 oder mehr Kohlenstoffatomen, Niederalkenyl, Niederalkinyl, Aryl oder unsubstituiertes oder substituiertes Amino bedeutet, und worin

$R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet,

$B_1$ eine Bindung oder ein bivalentes Radikal einer $\alpha$-Aminosäure bedeutet, welches N-terminal mit $R_1$ und C-terminal mit der Aminogruppe am $R_2$-$CH_2$- tragenden Kohlenstoffatom verbunden ist,

$R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten,

$A_1$ eine Bindung zwischen -C=O und $A_2$ oder ein bivalentes Radikal einer $\alpha$-Aminosäure bedeutet, welches N-terminal mit der Gruppe -C=O und C-terminal mit $A_2$ verbunden ist,

$A_2$ ein bivalentes Radikal einer $\alpha$-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und

$R_4$ und $R_5$ gemeinsam mit dem bindenden Stickstoffatom unsubstituiertes oder substituiertes Thiomorpholino oder Morpholino bedeuten, oder Salze dieser Verbindungen, sofern salzbildende Gruppen vorliegen.

2. Eine Verbindung der Formel I' gemäss Anspruch 1, worin $B_1$ eine Bindung bedeutet und die übrigen Reste die genannten Bedeutungen haben, oder Salze davon, sofern salzbildende Gruppen vorliegen.

3. Verbindungen der Formel I' gemäss Anspruch 1, worin T ein Acylradikal der in Anspruch 1 gezeigten Formel Z bedeutet, worin $R^z$ über ein Ringkohlenstoffatom gebundenes Heterocyclyl ausgewählt aus durch bis zu drei unabhängig voneinander aus Niederalkyl, Phenylniederalkyl, Diphenylniederalkyl, Triphenylniederalkyl, Niederalkanoyl, Hydroxy, Niederalkoxy, Phenylniederalkoxy, Hydroxyniederalkyl, Halogen, Cyano, Niederalkoxycarbonyl, Phenylniederalkoxycarbonyl und Haloniederalkyl ausgewählte Reste substituiertem oder unsubstituiertem Pyrrolyl, 2,5-Dihydropyrrolyl, Indolyl, Indolizinyl, Isoindolyl, Pyrrolidinyl, Hydroxypyrrolidinyl, Furyl, Tetrahydrofuryl, Thienyl, Cyclohepta[b]pyrrolyl, Imidazolyl, N-Triphenylniederalkyl-imidazolyl, Pyrazolyl, Oxazolyl, Isoxazolyl, Thiazolyl, Isothiazolyl, Triazolyl, Tetrazolyl, Pyridyl, Chinolyl, Isochinolyl, Piperidyl, $\gamma$-Pyranyl, 4,5-Dihydropyranyl, 4H-Chromenyl, Chromanyl, $\gamma$-Thiopyranyl, Pyridazinyl, Cinnolyl, Phthalazinyl, Chinazolinyl, Chinoxalinyl, Pyrimidinyl, Pyrazinyl, Phenyzinyl, Phenoxazinyl, Phenothiazinyl, Morpholinyl, Thiazinyl und Tetrahydropyranyl, welche über ein Ringkohlenstoffatom gebunden sind; bedeutet; oder

durch mindestens einen aus Niederalkoxy, oder durch einen oder zwei Substituenten Aryl, Niederalkoxy, Niederalkylthio, Ethylthio, Niederalkoxyniederalkoxy, Niederalkylthioniederalkoxy, Aryloxy oder Arylthio, Amino, N-Niederalkylamino, N,N-Diniederalkylamino, oder Heterocyclyl, welches wie zuletzt für über ein Ringkohlenstoffatom gebundenes Heterocyclyl $R^z$ definiert ist, substituiertes Niederalkoxy; Aryloxy; Niederalkylthio, durch einen oder zwei Substituenten Aryl substituiertes Niederalkylthio; Arylthio; durch Amino oder durch einen oder zwei Reste ausgewählt aus Niederalkyl, Heterocyclylniederalkyl, worin Heterocyclyl wie für über ein Ringkohlenstoffatom gebundenes Heterocyclyl $R^z$ definiert ist, Arylniederalkyl, Niederalkanoyl, Niederalkoxycarbonyl oder Arylniederalkoxycarbonyl substituiertes Amino; Heterocyclyl, wie oben für über ein Ringkohlenstoffatom gebundenes Heterocyclyl $R^z$ definiert; Heterocyclylniederalkyl, worin Heterocyclyl wie oben für über ein Ringkohlenstoffatom gebundenes Heterocyclyl $R^z$ definiert ist, jedoch auch über ein Ringstickstoffatom gebunden sein kann; Niederalkoxycarbonyl, Niederalkanoyloxyniederalkoxycarbonyl, Carboxy, Phenylniederalkoxycarbonylaminoniederalkoxy, Aminoniederalkoxy, Diniederalkylaminoniederalkoxy, N,N-Diniederalkylaminoniederalkoxy, Niederalkoxycarbonylniederalkylsulfo, Niederalkanoyloxy oder Tetrahydropyranyloxy

ausgewählten Rest substituiertes Niederalkyl, welches einen weiteren Substituenten Aryl enthalten kann, bedeutet; oder

Aryl bedeutet

wobei Aryl in den genannten Definitionen in erster Linie Phenyl, Napthtyl, oder Fluorenyl bedeutet, welches unsubstituiert oder substituiert durch bis zu drei unabhängig voneinander aus Niederalkyl,

Niederalkanoyl, Hydroxy, Niederalkoxy, Phenylniederalkoxy, Hydroxyniederalkyl, Halogen, Cyano, Niederalkoxycarbonyl, Phenylniederalkoxycarbonyl, Haloniederalkyl, Piperidinomethyl, Piperazin-1-ylmethyl, 4-Niederalkyl-piperazin-1-yl-methyl, Morpholinomethyl, Thiomorpholinomethyl und Nitro ausgewählte Reste, welche unabhängig voneinander vorliegen können, substituiert ist; bedeutet; $R_1$ Wasserstoff, tert-Butoxycarbonyl, Isobutyloxycarbonyl, Ethoxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl, γ-Pyranyl, Furanyl und einem ganz oder teilweise gesättigten Derivat dieser Reste, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, bedeutet, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Benzyloxy, Fluor, Sulfo, Niederalkylsulfonyl, Trifluormethyl, Cyano, Isobutyloxy und n-Butyloxy ausgewählte Reste substituiert sind, bedeuten, $A_1$ ein bivalentes Radikal einer hydrophoben α-Aminosäure bedeutet, welches N-terminal mit der Gruppe -C=O und C-terminal mit $A_2$ verbunden ist, $A_2$ ein bivalentes Radikal einer hydrophoben a-Aminosäure, bedeutet, welches N-terminal mit $A_1$ verbunden ist und C-terminal mit dem Rest $NR_4R_5$ verbunden ist, oder $A_1$ und $A_2$ zusammen ein bivalentes Radikal eines Dipeptides aus zwei hydrophoben α-Aminosäuren bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino, 2,6-Dimethylmorpholino oder Morpholino bedeuten; und die Verbindungen der Formel I, worin $R_1$ Morpholinosulfonyl oder N-(2-Pyridylmethyl)-N-methyl-aminocarbonyl bedeutet und die übrigen Reste die genannten Bedeutungen haben; und pharmazeutisch verwendbare Salze dieser Verbindungen, sofern salzbildende Gruppen vorliegen.

**4.** Verbindungen der Formel I' gemäss Anspruch 1, worin T ein Acylradikal der in Anspruch 1 gezeigten Formel Z bedeutet, worin $R^z$ über ein Ringkohlenstoffatom gebundenes Heterocyclyl ausgewählt aus durch bis zu drei unabhängig voneinander aus Niederalkyl, Phenylniederalkyl, Diphenylniederalkyl, Triphenylniederalkyl, Niederalkanoyl, Hydroxy, Niederalkoxy, Phenylniederalkoxy, Hydroxyniederalkyl, Halogen, Cyano, Niederalkoxycarbonyl, Phenylniederalkoxycarbonyl und Haloniederalkyl ausgewählte Reste substituiertem oder unsubstituiertem Pyrrolyl, 2,5-Dihydropyrrolyl, Indolyl, Indolizinyl, Isoindolyl, Pyrrolidinyl, Hydroxypyrrolidinyl, Furyl, Tetrahydrofuryl, Thienyl, Cyclohepta[b]pyrrolyl, Imidazolyl, N-Triphenylniederalkyl-imidazolyl, Pyrazolyl, Oxazolyl, Isoxazolyl, Thiazolyl, Isothiazolyl, Triazolyl, Tetrazolyl, Pyridyl, Chinolyl, Isochinolyl, Piperidyl, γ-Pyranyl, 4,5-Dihydropyranyl, 4H-Chromenyl, Chromanyl, γ-Thiopyranyl, Pyridazinyl, Cinnolyl, Phthalazinyl, Chinazolinyl, Chinoxalinyl, Pyrimidinyl, Pyrazinyl, Phenazinyl, Phenoxazinyl, Phenothiazinyl, Morpholinyl und Thiazinyl, welche über ein Ringkohlenstoffatom gebunden sind; bedeutet; oder

durch mindestens einen aus Niederalkoxy; durch einen oder zwei Substituenten Aryl, Niederalkoxy, Niederalkylthio, Ethylthio, Niederalkoxyniederalkoxy, Niederalkylthioniederalkoxy, Aryloxy oder Arylthio, Amino, N-Niederalkylamino, N,N-Diniederalkylamino, oder Heterocyclyl, welches wie zuletzt für über ein Ringkohlenstoffatom gebundenes Heterocyclyl $R^z$ definiert ist, substituiertes Niederalkoxy; Aryloxy, Niederalkylthio, durch einen oder zwei Substituenten Aryl substituiertes Niederalkylthio, Arylthio; durch Amino oder durch einen oder zwei Reste ausgewählt aus Niederalkyl, Heterocyclylniederalkyl, worin Heterocyclyl wie für über ein Ringkohlenstoffatom gebundenes Heterocyclyl $R^z$ definiert ist, Arylniederalkyl, Niederalkanoyl, Niederalkoxycarbonyl oder Arylniederalkoxycarbonyl substituiertes Amino; Heterocyclyl, wie oben für über ein Ringkohlenstoffatom gebundenes Heterocyclyl $R^z$ definiert, und Heterocyclylniederalkyl, worin Heterocyclyl wie oben für über ein Ringkohlenstoffatom gebundenes Heterocyclyl $R^z$ definiert ist, jedoch auch über ein Ringstickstoffatom gebunden sein kann;

ausgewählten Rest substituiertes Niederalkyl, welches einen weiteren Substituenten Aryl tragen kann, bedeutet; oder

Aryl bedeutet

wobei Aryl in den genannten Definitionen in erster Linie Phenyl, Napthtyl, oder Fluorenyl bedeutet, welches unsubstituiert oder substituiert durch bis zu drei unabhängig voneinander aus Niederalkyl, Niederalkanoyl, Hydroxy, Niederalkoxy, Phenylniederalkoxy, Hydroxyniederalkyl, Halogen, Cyano, Niederalkoxycarbonyl, Phenylniederalkoxycarbonyl, Haloniederalkyl, Piperidinomethyl, Piperazin- 1-ylmethyl, 4-Niederalkyl-piperazin-1-yl-methyl, Morpholinomethyl, Thiomorpholinomethyl und Nitro ausge-

wählte Reste, welche unabhängig voneinander vorliegen können, substituiert ist; $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem ganz oder teilweise gesättigten Derivat dieser Reste, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, bedeutet, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Benzyloxy, Fluor, Sulfo, Niederalkylsulfonyl, Trifluormethyl und Cyano ausgewählte Reste substituiert sind, bedeuten, $A_1$ ein bivalentes Radikal einer hydrophoben α-Aminosäurebedeutet, welches N-terminal mit der Gruppe -C=O und C-terminal mit $A_2$ verbunden ist, $A_2$ ein bivalentes Radikal einer hydrophoben α-Aminosäure, bedeutet, welches N-terminal mit $A_1$ verbunden ist und C-terminal mit dem Rest $NR_4R_5$ verbunden ist, oder $A_1$ und $A_2$ zusammen ein bivalentes Radikal eines Dipeptides aus zwei hydrophoben α-Aminosäuren bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino bedeuten; und die Verbindungen der Formel I, worin $R_1$ Morpholinosulfonyl oder N-(2-Pyridylmethyl)-N-methyl-aminocarbonyl bedeutet und die übrigen Reste die genannten Bedeutungen haben; und pharmazeutisch verwendbare Salze dieser Verbindungen, sofern salzbildende Gruppen vorliegen.

5. Verbindungen der Formel I' gemäss Anspruch 1, worin T Pyrrolidin-2-ylcarbonyl oder -3-ylcarbonyl, Furan-3- oder Furan-2-ylcarbonyl, Pyridyl-4-, Pyridyl-3- oder Pyridyl-2-ylcarbonyl, Isochinolin-1- oder Isochinolin-3-ylcarbonyl, Pyrazin-2-ylcarbonyl, Niederalkoxyniederalkylcarbonyl,Phenyl- oder Naphthylniederalkoxyniederalkylcarbonyl, α-Niederalkoxy-α-phenylniederalkylcarbonyl, Niederalkoxyniederalkoxyniederalkylcarbonyl, Niederalkoxyniederalkoxyniederalkoxyniederalkylcarbonyl, o-, m- oder p-Chlorphenyloxy-niederalkoxy-niederalkylcarbonyl, N,N-Diniederalkylaminoniederalkoxyniederalkylcarbonyl, 2-, 3- oder 4-Pyridylniederalkyloxyniederalkylcarbonyl, Phenyloxyniederalkylcarbonyl, Niederalkylthioniederal kylcarbonyl, Phenylniederalkylthioniederalkylcarbonyl, N,N-Diniederalkyl-aminoniederalkylcarbonyl, N-Niederalkylaminoniederalkylcarbonyl, N-Imidazol(-2-, -4- oder -5-)ylmethyl-N-niederalkylaminoniederalkylcarbonyl, N-(Imidazol-4-ylmethyl)-N-methylaminoacetyl, N-Pyridin(-2-, -3- oder -4-)ylmethyl-N-niederalkylaminoniederalkylcarbonyl, N-Phenylniederalkoxycarbonyl-N-niederalkylaminoniederalkylcarbonyl, Imidazol(-1-, -2-, -4- oder -5-)ylniederalkylcarbonyl, N-Triphenylniederalkylimidazol(-4- oder -5-)ylniederalkylcarbonyl, Pyrazol(-1-, -3-, -4- oder -5-)ylniederalkylcarbonyl, wie Pyrazol-1-ylacetyl, Haloniederalkylbenzoyl, p-(Morpholino- oder Thiomorpholinomethyl)benzoyl, Benzoyl, Niederalkanoyloxyniederalkylcarbonyl, Niederalkoxycarbonylniederalkoxy-niederalkylcarbonyl, Niederalkoxycarbonylniederalkylthio-niederalkylcarbonyl, Niederalkoxycarbonylniederalkylsulfo-niederalkylcarbonyl, Niederalkanoyloxyniederalkoxycarbonylniederalkoxy-niederalkylcarbonyl,Carboxyniederalkoxyniederalkylcarbonyl, N-Phenylniederalkoxycarbonylamino-niederalkylcarbonyl, Aminoniederalkylcarbonyl, Diniederalkyl- aminoniederalkoxyniederalkoxy-niederalkylcarbonyl, N-Phenylniederalkoxycarbonyl -aminoniederalkoxyniederalkoxy-niederalkylcarbonyl, Aminoniederalkoxyniederalkoxy-niederalkylcarbonyl oder Tetrahy- dropyranyloxy-niederalkylcarbonyl bedeutet; $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl, Ethoxycarbonyl, 4-Tetrahydropyranyloxycarbonyl, Tetrahydrofuran-2(R oder S)-yloxycarbonyl, Morpholinosulfonyl oder N-(2-Pyridylmethyl)-N-methyl-aminocarbonyl bedeutet; $B_1$ eine Bindung oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis drei, unabhängig voneinander aus Hydroxy, Methoxy, Benzyloxy, Fluor, Sulfo, Niederalkylsulfonyl, Cyano, Trifluormethyl, Isobutyloxy und n-Butyloxy ausgewählte Reste substituiert sind, $A_1$ ein bivalentes Radikal einer der α-Aminosäuren Glycin, Valin, Isoleucin, Leucin oder Phenylglycin bedeutet, welches N-terminal mit der Gruppe -C=O und C-terminal mit $A_2$ verbunden ist, $A_2$ ein bivalentes Radikal einer der α-Aminosäuren Glycin, Alanin, Valin, Leucin, Isoleucin, Phenylalanin, Tyrosin, Cyclohexylalanin, p-Methoxy-phenylalanin, p-Benzyloxyphenylalanin, p-Fluorphenylalanin, p-(Isobutyloxy)-phenylalanin oder p-(n-Butyloxy)phenylalanin bedeutet, welches N-terminal mit $A_1$ und C-terminal mit der Gruppe $NR_4R_5$ verbunden ist, oder ferner $A_1$ und $A_2$ zusammen ein bivalentes Radikal eines Dipeptides mit reduzierter zentraler Peptidbindung

bedeuten, welches aus einem N-terminalen Aminosäureradikal ausgewählt aus Gly(red), Val(red) oder Ile(red) und einem C-terminalen Aminosäureradikal ausgewählt aus Glycin, Phenylalanin, Cyclohexylalanin, Tyrosin, p-Methoxy-phenylalanin oder p-Fluorphenylalanin besteht, und welches N-terminal mit der Gruppe -C=O und C-terminal mit der Gruppe $NR_4R_5$ verbunden ist, wie es oben für $A_1$ und $A_2$ definiert ist, und $R_4$ und $R_5$ gemeinsam mit dem bindenden Stickstoffatom Thiomorpholino, Morpholino oder 2,6-Dimethylmorpholino bedeutet, und pharmazeutisch verwendbare Salze davon, sofern salzbildende Gruppen vorliegen.

6. Eine Verbindung der Formel I' gemäss Anspruch 1, worin T Pyrrolidin-2-ylcarbonyl oder -3-ylcarbonyl, Furan-3- oder Furan-2-ylcarbonyl, Pyridyl-4-, Pyridyl-3- oder Pyridyl-2-ylcarbonyl, Isochinolin-1- oder Isochinolin-3-ylcarbonyl, Pyrazin-2-ylcarbonyl, Niederalkoxyniederalkylcarbonyl, Phenyl- oder Naphthylniederalkoxyniederalkylcarbonyl, α-Niederalkoxy-α-phenylniederalkylcarbonyl, Niederalkoxyniederalkoxyniederalkylcarbonyl, Niederalkoxyniederalkoxyniederalkoxyniederalkylcarbonyl, o-, m- oder p-Chlorphenyloxy-niederalkoxy-niederalkylcarbonyl, N,N-Diniederalkylaminoniederalkoxyniederalkylcarbonyl, 2-, 3- oder 4-Pyridylniederalkyloxyniederalkylcarbonyl,Phenyloxyniederalk ylcarbonyl, Niederalkylthioniederalkylcarbonyl, Phenylniederalkylthioniederalkylcarbonyl, N,N-Diniederalkyl-aminoniederalkylcarbonyl, N-Niederalkylaminoniederalkylcarbonyl, N-Imidazol(-2-, -4- oder -5-)ylmethyl-N-niederalkylaminoniederalkylcarbonyl, N-Pyridin(-2-, -3- oder -4-)ylmethyl-N-niederalkylaminoniederalkylcarbonyl, N-Phenylniederalkoxycarbonyl-N-niederalkylaminoniederalkylcarbonyl, Imidazol(-1-, -2-, -4- oder -5-)ylniederalkylcarbonyl, N-Triphenylniederalkylimidazol(-4- oder -5-)ylniederalkylcarbonyl, Pyrazol(-1-, -3-, -4- oder -5-)ylniederalkylcarbonyl, Pyrazol-1-ylacetyl, Haloniederalkylbenzoyl, p-(Morpholino- oder Thiomorpholinomethyl)benzoyl, Benzoyl, Niederalkanoyloxy-niederalkylcarbonyl, Niederalkoxycarbonylniederalkoxy-niederalkylcarbonyl, Niederalkoxycarbonylniederalkylthio-niederalkylcarbonyl, Niederalkoxycarbonylniederalkylsulfo-niederalkylcarbonyl, Niederalkanoyloxyniederalkoxycarbonylniederalkoxy-niederalkylcarbonyl, Carboxyniederalkoxyniedealkylcarbonyl, N-Phenylniederalkoxycarbonylamino-niederalkylcarbonyl, Aminoniederalkylcarbonyl, Diniederalkylaminoniederalkoxyniederalkoxy-niederalkylcarbony 1, N-Phenylniederalkoxycarbonylaminoniederalkoxyniederalkoxy-niederalkylcarbonyl, Aminoniederalkoxyniederalkoxy-niederalkylcarbonyl oder Tetrahydropyranyloxy-niederalkylcarbonyl bedeutet, $R_1$ tert-Butoxycarbonyl bedeutet, $R_2$ Phenyl, Cyclohexyl, p-Hydroxyphenyl, o-, m- oder p-Methoxyphenyl, p-Benzyloxyphenyl, o-, m- oder p-Fluorphenyl, p-Trifluormethylphenyl oder o-, m- oder p-Cyanophenyl bedeutet, $R_3$ unabhängig von $R_2$ eine der für $R_2$ genannten Bedeutungen hat, $A_1$ ein bivalentes Radikal einer der α-Aminosäuren Glycin, (L)-Valin oder (L)-Isoleucin bedeutet, welches N-terminal mit der Gruppe -C=O und C-terminal mit $A_2$ verbunden ist, $A_2$ ein bivalentes Radikal einer der α-Aminosäuren Glycin, Alanin, Valin, Leucin, Isoleucin, Phenylalanin, Tyrosin, Cyclohexylalanin, p-Methoxy-phenylalanin, p-Benzyloxyphenylalanin oder p-Fluorphenylalanin bedeutet, welches N-terminal mit $A_1$ und C-terminal mit der Gruppe $NR_4R_5$ verbunden ist, und der Rest $-NR_5R_6$ Morpholino bedeutet, und Salze davon, sofern salzbildende Gruppen vorliegen.

7. Eine Verbindung der Formel I' gemäss Anspruch 1, worin T Pyrrolidin-2-ylcarbonyl oder -3-ylcarbonyl, Furan-3- oder Furan-2-ylcarbonyl, Pyridyl-4-, Pyridyl-3- oder Pyridyl-2-ylcarbonyl, Isochinolin-1- oder Isochinolin-3-ylcarbonyl, Pyrazin-2-ylcarbonyl, Niederalkoxyniederalkylcarbonyl, Phenyl- oder Naphthylniederalkoxyniederalkylcarbonyl, α-Niederalkoxy-α-phenylniederalkylcarbonyl, Niederalkoxyniederalkoxyniederalkylcarbonyl, Niederalkoxyniederalkoxyniederalkoxyniederalkylcarbonyl, o-, m- oder p-Chlorphenyloxy-niederalkoxy-niederalkylcarbonyl, N,N-Diniederalkylaminoniederalkoxyniederalkylcarbonyl, 2-, 3- oder 4-Pyridylniederalkyloxyniederalkylcarbonyl,Phenyloxyniederalk ylcarbonyl, Niederalkylthioniederalkylcarbonyl, Phenylniederalkylthioniederalkylcarbonyl, N,N-Diniederalkyl-aminoniederalkylcarbonyl, N-Niederalkylaminoniederalkylcarbonyl, N-Imidazol(-2-, -4- oder -5-)ylmethyl-N-niederalkylaminoniederalkylcarbonyl, N-Pyridin(-2-, -3- oder -4-)ylmethyl-N-niederalkylaminoniederalkylcarbonyl, N-Phenylniederalkoxycarbonyl-N-niederalkylaminoniederalkylcarbonyl, Imidazol(-1-, -2-, -4- oder -5-)ylniederalkylcarbonyl, N-Triphenylniederalkylimidazol(-4- oder -5-)ylniederalkylcarbonyl, Pyrazol (-1-, -3-, -4- oder -5-)ylniederalkylcarbonyl, Pyrazol-1-ylacetyl, Haloniederalkylbenzoyl, p-(Morpholino- oder Thiomorpholinomethyl)benzoyl, Benzoyl, Niederalkanoyloxy-niederalkylcarbonyl, Niederalkoxycarbo- nylniederalkoxy-niederalkylcarbonyl,Niederalkoxycarbonylniederalkylthio-niederalkylcarbonyl, Niederalkoxycarbonylniederalkylsulfo-niederalkylcarbonyl, Niederalkanoyloxyniederalkoxy- carbonylniederalkoxy-niederalkylcarbonyl, Carboxyniederalkoxyniederal- kylcarbonyl, N-Phenylniederalkoxycarbonylamino-niederalkylcarbonyl, Aminoniederalkylcarbonyl, Diniederalkylaminoniederal- koxyniederalkoxy-niederalkylcarbonyl, N-Phenylniederalkoxycarbonyl-aminoniederalkoxyniederalkoxy-niederalkylcarbonyl, Aminoniederalkoxyniederalkoxy-niederalkylcarbonyl oder Tetrahydropyranyloxy-nie- deralkylcarbonyl bedeutet, und worin entweder $R_1$ tert-Butoxycarbonyl; $B_1$ eine Bindung; $R_2$ Phenyl; $R_3$ Phenyl; $A_1$ das

bivalente Radikal von (L)-Valin bedeutet, welches N-terminal mit der Gruppe -C=O und C-terminal mit $A_2$ verbunden ist; $A_2$ das bivalente Radikal von Phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder worin $R_1$ tert-Butoxycarbonyl; $B_1$ eine Bindung; $R_2$ Phenyl; $R_3$ p-Methoxyphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von p-Methoxyphenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder worin $R_1$ tert-Butoxycarbonyl; $B_1$ eine Bindung; $R_2$ Phenyl; $R_3$ p-Methoxyphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten;

oder Salze davon, sofern salzbildende Gruppen vorliegen.

8. Eine Verbindung der Formel I' gemäss Anspruch 1, worin T 4(S)-(2-Furanylcarboxy)-; 4(S)-[4-(Dimethylamino)-butyryloxy]; 4(S)-(N-Z-N-Methyl-aminoacetyloxy); 4(S)-(Methylamino-acetyloxy); 4(S)-[N-(Imidazol-4-methyl)-N-methyl-aminoacetyloxy]; 4(S)-[3-(1-Triphenylmethyl-imidazol-4-yl)-propionyloxy]; 4(S)-[3-(4-Imidazolyl)-propionyloxy]; 4(S)-(Methoxy-acetyloxy); 4(S)-((2-picolinoyl); 4(S)-(Benzyloxyacetyloxy); 4(S)-[(S)-α-Methoxy-α-phenyl-acetyloxy]; 4(S)-[(R)-α-Methoxy-α-phenylacetyloxy]; 4(S)-(1-Pyrazolylacetyloxy); 4(S)-(Isochinolin-3-carbonyloxy); 4(S)-(Pyrazincarbonyloxy); 4(S)-(4-α-Chlormethylbenzoyloxy); 4(S)-[4-(4-Morpholino)-methyl-benzoyloxy]; 4(S)-(Isonicotinoyloxy); 4(S)-(Nicotinoyloxy); 4(S)-(3-Methoxypropanoyloxy); 4(S)-[(4-Chlorphenoxy)methoxyacetyloxy)]; 4(S)-[2-(2-Methoxyethoxy)-acetyloxy)]; 4(S)-(Butyloxyacetyloxy); 4(S)-[2-[2-(2-Methoxyethoxy)ethoxy]acetyloxy)]; 4(S)-(Methoxyacetyloxy); 4(S)-(Phenoxyacetyloxy); 4(S)-[(S)-α-Methoxy-α-phenyl-acetyloxy); 4(S)-[(R)-α-methoxy-α-phenylacetyloxy)]; (N,N-Dimethyl-aminoacetyloxy); 4(S)-[N-(Pyridin-2-methyl)-N-methyl-aminoacetyloxy]; 4(S)-[3-(Dimethyl-amino)-propionyloxy]; 4(S)-[3-(N-Z-N-Methyl-amino)-propionyloxy]; 4(S)-(3-Methyl-amino-propionyloxy); 4(S)-[(Dimethylaminoethoxy)-acetyloxy]; 4(S)-[(2-Pyridylmethoxy)-acetyloxy]; 4(S)-(Methoxy-acetyloxy); 4(S)-(Pyridin-2-carboxyl); 4(S)-(Methylthioacetyloxy); 4(S)-(Benzylthioacetyloxy); 4(S)-((L)-Prolyloxy); 4(S)-((D)-Prolyloxy); 4(S)-((L)-(N-Z-Prolyl)oxy); 4(S)-((D)-(N-Z-Prolyl)oxy); 3-(N-Z-Amino)-propionyloxy; 3-Amino-propionyloxy; (3-Dimethylamino-propoxy)-acetyloxy; 2-(2-Dimethylamino-ethoxy)-ethoxy-acetyloxy; (4-Dimethylamino-butoxy)-acetyloxy; (2-Benzyloxy)acetyloxy; 2-Acetyloxy)acetyloxy; 2-(4-Tetrahydropyranyloxy)acetyloxy; 2(R)-(4-Tetrahydropyranyloxy)]-propionyloxy; 2-(2-Amino-ethoxy)ethoxy-acetyloxy); 2-(2-Benzyloxycarbonylamino-ethoxy)ethoxy-acetyloxy); (Methoxycarbonyl-methoxy)-acetyloxy; (Methoxycarbonyl-methylthio)--acetyloxy; (Methoxycarbonyl-methylsulfo)-acetyloxy; (Pivaloyloxymethoxycarbonyl-methoxy-acetyloxy; (Carboxymethoxy)-acetyloxy; oder (Acetoxymethoxycarbonyl-methoxy)-acetyloxy bedeutet; und
$R_1$ tert-Butoxycarbonyl; $B_1$ eine Bindung; $R_2$ Phenyl; $R_3$ Phenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von Phenylalanin, welches N-terminal mit $A_1$ und C-terminal mit der Gruppe $-NR_4R_5$ verbunden ist; und $R_4$ und $R_5$ gemeinsam mit dem bindenden Stickstoffatom Morpholino bedeuten; oder jeweils ein pharmazeutisch verwendbares Salz davon, sofern salzbildende Gruppen vorliegen.

9. Eine Verbindung der Formel I' gemäss Anspruch 1, worin T 4(S)-(2-Furanylcarboxy)-; 4(S)-[4-(Dimethylamino)-butyryloxy]; 4(S)-(N-Z-N-Methyl-aminoacetyloxy); 4(S)-(Methylamino-acetyloxy); 4(S)-[N-(Imidazol-4-methyl)-N-methyl-aminoacetyloxy]; 4(S)-[3-(1-Triphenylmethyl-imidazol-4-yl)-propionyloxy]; 4(S)-[3-(4-Imidazolyl)-propionyloxy]; 4(S)-(Methoxy-acetyloxy); 4(S)-((2-picolinoyl); 4(S)-(Benzyloxyacetyloxy); 4(S)-[(S)-α-Methoxy-α-phenyl-acetyloxy]; 4(S)-[(R)-α-Methoxy-α-phenylacetyloxy]; 4(S)-(1-Pyrazolylacetyloxy); 4(S)-(Isochinolin-3-carbonyloxy); 4(S)-(Pyrazincarbonyloxy); 4(S)-(4-α-Chlormethyl-benzoyloxy); 4(S)-[4-(4-Morpholino)-methyl-benzoyloxy]; 4(S)-(Isonicotinoyloxy); 4(S)-(Nicotinoyloxy); 4(S)-(3-Methoxypropanoyloxy); 4(S)-[(4-Chlorphenoxy)methoxyacetyloxy)]; 4(S)-[2-(2-Methoxyethoxy)-acetyloxy)]; 4(S)-(Butyloxyacetyloxy); 4(S)-[2-[2-(2-Methoxyethoxy)ethoxy]acetyloxy)]; 4(S)-(Methoxyacetyloxy); 4(S)-(Phenoxyacetyloxy); 4(S)-[(S)-α-Methoxy-α-phenylacetyloxy); 4(S)-[(R)-α-methoxy-α-phenylacetyloxy)]; (N,N-Dimethyl-aminoacetyloxy); 4(S)-[N-(Pyridin-2-methyl)-N-methyl-aminoacetyloxy]; 4(S)-[3-(Dimethyl-amino)-propionyloxy]; 4(S)-[3-(N-Z-N-Methyl-amino)-propionyloxy]; 4(S)-(3-Methyl-amino-propionyloxy); 4(S)-[(Dimethylaminoethoxy)-acetyloxy]; 4(S)-[(2-Pyridylmethoxy)-acetyloxy]; 4(S)-(Methoxy-acetyloxy); 4(S)-(Pyridin-2-carboxyl); 4(S)-(Methylthioacetyloxy); 4(S)-(Benzylthioacetyloxy); 4(S)-((L)-Proly-

loxy); 4(S)-((D)-Prolyloxy); 4(S)-((L)-(N-Z-Prolyl)oxy); 4(S)-((D)-(N-Z-Prolyl)oxy); 3-(N-Z-Amino)-propionyloxy; 3-Amino-propionyloxy; (3-Dimethylamino-propoxy)-acetyloxy; 2-(2-Dimethylamino-ethoxy)-ethoxy-acetyloxy; (4-Dimethylamino-butoxy)-acetyloxy; (2-Benzyloxy)acetyloxy; 2-Acetyloxy)acetyloxy; 2-(4-Tetrahydropyranyloxy)acetyloxy; 2(R)-(4-Tetrahydropyranyloxy)]-propionyloxy; 2-(2-Amino-ethoxy)ethoxy-acetyloxy); 2-(2-Benzyloxycarbonylamino-ethoxy)ethoxy-acetyloxy); (Methoxycarbonyl-methoxy)-acetyloxy; (Methoxycarbonyl-methylthio)-acetyloxy; (Methoxycarbonyl-methylsulfo)-acetyloxy; (Pivaloyloxymethoxycarbonyl-methoxy-acetyloxy; (Carboxymethoxy)-acetyloxy; oder (Acetoxymethoxycarbonyl-methoxy)-acetyloxy bedeutet; und

$R_1$ tert-Butoxycarbonyl; $B_1$ eine Bindung; $R_2$ Phenyl; $R_3$ p-Methoxyphenyl; $A_1$ das bivalente Radikal von (L)-Valin (-(L)-Val-) bedeutet, welches N-terminal mit der Gruppe -C=O und C-terminal mit $A_2$ verbunden ist; $A_2$ das bivalente Radikal von Phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten; oder

jeweils ein pharmazeutisch verwendbares Salz davon, sofern salzbildende Gruppen vorliegen.

**10.** Eine Verbindung der Formel I' gemäss Anspruch 1, worin T Methoxyacetyl oder Pyridin-2-ylcarbonyl bedeutet, $R_1$ tert-Butoxycarbonyl; $B_1$ eine Bindung; $R_2$ Phenyl; $R_3$ Phenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten; oder Salze davon, sofern salzbildende Gruppen vorliegen.

**11.** Eine Verbindung der Formel I' gemäss Anspruch 1, worin T Methoxyacetyl oder Pyridin-2-ylcarbonyl bedeutet, $R_1$ tert-Butoxycarbonyl; $B_1$ eine Bindung; $R_2$ Phenyl; $R_3$ p-Methoxyphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von p-Methoxy-phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten; oder Salze davon, sofern salzbildende Gruppen vorliegen.

**12.** Eine Verbindung der Formel I' gemäss Anspruch 1, worin T Methoxyacetyl oder Pyridin-2-ylcarbonyl bedeutet, $R_1$ tert-Butoxycarbonyl; $B_1$ eine Bindung; $R_2$ Phenyl; $R_3$ p-Methoxyphenyl; $A_1$ das bivalente Radikal von (L)-Valin bedeutet, welches N-terminal mit der Gruppe -C=O und C-terminal mit $A_2$ verbunden ist; $A_2$ das bivalente Radikal von Phenylalanin, welches N- terminal mit $A_1$ und C-terminal mit der Gruppe $-NR_4R_5$ verbunden ist; und $R_4$ und $R_5$ gemeinsam mit dem bindenden Stickstoffatom Morpholino bedeuten; oder Salze davon, sofern salzbildende Gruppen vorliegen.

**13.** Eine Verbindung der Formel I' gemäss Anspruch 1 mit der Bezeichnung 5(S)-(Boc-amino)-4(S)-[2-(2-methoxyethoxy)acetyloxy)]-6-phenyl-2(R)-phenylmethyl-hexanoyl-(L)-Val-(L)-Phe-morpholin-4-ylamid.

**14.** Eine Verbindung der Formel I' gemäss Anspruch 1 mit der Bezeichnung 5(S)-(Boc-amino)-4(S)-[2-[2-(2-methoxyethoxy)ethoxy]acetyloxy )]-6-phenyl-2(R)-phenylmethyl-hexanoyl-(L)-Val-(L)-Phe-morpholin -4-ylamid.

**15.** Verbindungen der Formel I,

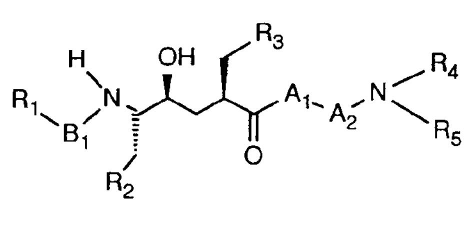

(I)

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $R_2$ Phenyl, Cyclohexyl,

Niederalkoxyphenyl, Benzyloxyphenyl, p-Fluor-phenyl, p-Trifluormethylphenyl oder p-Hydroxyphenyl bedeutet, $R_3$ Phenyl, Niederalkoxyphenyl, m- oder p-Methoxyphenyl, p-Trifluormethylphenyl, o-, m- oder p-Cyanophenyl, Benzyloxyphenyl, p-Benzyloxyphenyl, o-, m- oder p-Fluorphenyl oder Hydroxyphenyl bedeutet, $A_1$ das bivalente Radikal der Aminosäure (L)-Valin, (L)-Isoleucin oder Glyin bedeutet, welches N-terminal mit der Gruppe -C=O und C-terminal mit $A_2$ verbunden ist, $A_2$ das bivalente Radikal der Aminosäure Glycin, Alanin, Valin, Leucin, Isoleucin, Phenylalanin, Tyrosin, Cyclohexylalanin, p-Niederalkoxyphenylalanin, p-Benzyloxyphenylalanin oder p-Fluorphenylalanin, welches N-terminal mit $A_1$ und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten,
mit der Massgabe, dass mindestens entweder einer der Reste $R_2$ und $R_3$ Benzyloxyphenyl bedeutet oder $A_2$ das bivalente Radikal von p-Benzyloxyphenylalanin bedeutet, während die übrigen Reste die genannten Bedeutungen haben, wenn entweder $R_3$ eine andere Bedeutung als o- oder m-Fluorphenyl, o- oder m-Cyanophenyl oder o- oder m-Methoxyphenyl hat, oder wenn $A_2$ eine andere Bedeutung als Alanin oder Leucin hat;
oder Salze dieser Verbindungen, sofern salzbildende Gruppen vorliegen.

**16.** Eine Verbindung mit der Bezeichnung Boc-Phe[C](p-$CH_3O$)Phe-(L)-Val-(L)-Phe-morpholin-4-ylamid.

**17.** Eine Verbindung mit der Bezeichnung Boc-(p-$CH_3O$)Phe[C]Phe-(L)-Val-(L)-Phe-morpholin-4-ylamid.

**18.** Eine Verbindung oder ein pharmazeutisch verwendbares Salz davon gemäss einem der Ansprüche 1 bis 17 zur Anwendung in einem Verfahren zur diagnostischen oder therapeutischen Behandlung des menschlichen oder tierischen Körpers.

**19.** Verwendung einer Verbindung gemäss einem der Ansprüche 1 bis 17, oder eines pharmazeutisch verwendbaren Salzes davon, zur Herstellung von pharmazeutischen Präparaten zur Behandlung von AIDS.

**20.** Pharmazeutische Präparate enthaltend Verbindungen gemäss einem der Ansprüche 1 bis 17 oder ein pharmazeutisch verwendbares Salz einer solchen Verbindung mit mindestens einer salzbildenden Gruppe zusammen mit pharmazeutisch verwendbarem Trägermaterial.

**21.** Verfahren zur Herstellung von Verbindungen der Formel I' gemäss Anspruch 1, dadurch gekennzeichnet, dass man

a) zur Herstellung von Verbindungen der Formel

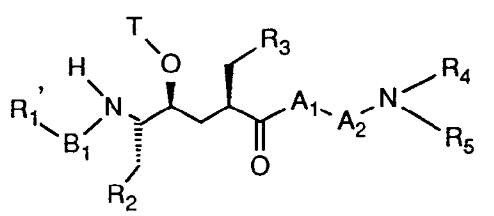

(Ib'),

worin $R_1$' die für Verbindungen der Formel I' genannten Bedeutungen von $R_1$ ausser Wasserstoff hat, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I' genannten Bedeutungen haben, eine Säure der Formel

$R_1$'-OH (II)

oder ein reaktionsfähiges Säurederivat davon, worin $R_1$' ausser Wasserstoff dieselben Bedeutungen hat wie $R_1$ in Verbindungen der Formel I', mit einer Aminoverbindung der Formel

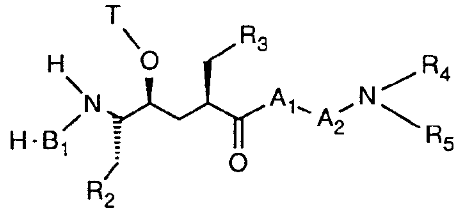

(III')

oder einem reaktionsfähigen Derivat hiervon, worin die Reste die für Verbindungen der Formel I' genannten Bedeutungen haben, kondensiert, wobei in den Ausgangsmaterialien der Formeln II und III' freie funktionelle Gruppen mit Ausnahme der an der Reaktion beteiligten erforderlichenfalls in geschützter Form vorliegen, und gewünschtenfalls vorhandene Schutzgruppen abspaltet, oder
b) zur Herstellung von Verbindungen der Formel

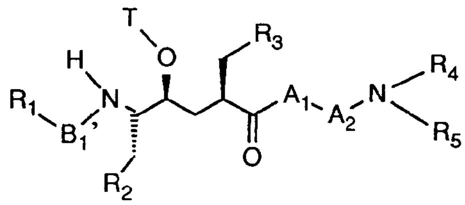

(Ic'),

worin $B_1$' dieselben Reste wie $B_1$ in Verbindungen der Formel I' ausser einer Bindung bedeuten kann, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I' genannten Bedeutungen haben, eine Carbonsäure der Formel

$R_1$-$B_1$'-OH (IV)

oder ein reaktionsfähiges Säurederivat davon, worin $R_1$ die für Verbindungen der Formel I' genannten Bedeutungen hat und $B_1$' die zuletzt genannten Bedeutungen hat, mit einer Aminoverbindung der Formel

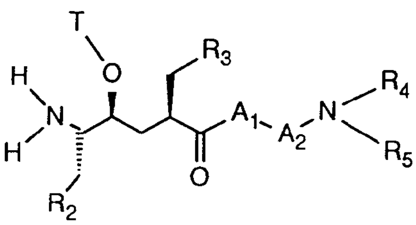

(V'),

oder einem reaktionsfähigen Derivat davon, worin die Reste die für Verbindungen der Formel I' genannten Bedeutungen haben, kondensiert, wobei freie funktionelle Gruppen in den Ausgangsmaterialien der Formeln IV und V' mit Ausnahme der an der Reaktion beteiligten erforderlichenfalls in geschützter Form vorliegen, und gewünschtenfalls vorhandene Schutzgruppen abspaltet, oder
c) eine Carbonsäure der Formel

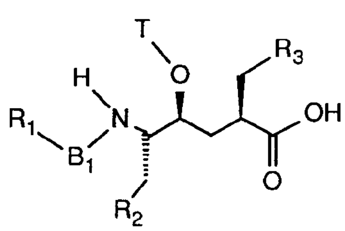

(VI'),

oder ein reaktionsfähiges Derivat davon, worin die Reste die für Verbindungen der Formel I' genannten Bedeutungen haben, mit einer Aminoverbindung der Formel

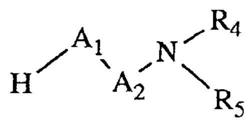

(VII)

oder einem reaktionsfähigen Derivat davon, worin die Reste die für Verbindungen der Formel I' genannten Bedeutungen haben, kondensiert, wobei freie funktionelle Gruppen in den Ausgangsmaterialien der Formeln VI' und VII mit Ausnahme der an der Reaktion beteiligten erforderlichenfalls in geschützter Form vorliegen, und gewünschtenfalls vorhandene Schutzgruppen abspaltet, oder

d) zur Herstellung einer Verbindung der Formel

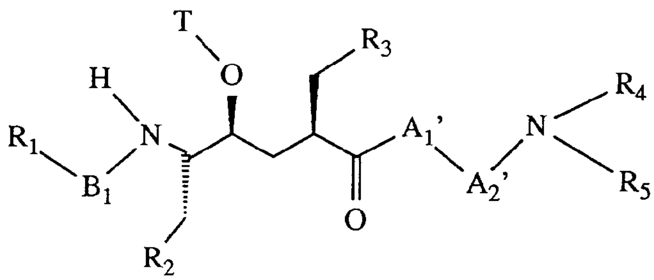

(Id'),

worin $A_1$' und $A_2$' die Bedeutungen von $A_1$ und $A_2$ in Verbindungen der Formel I' haben, wobei $A_1$' jedoch keine Bindung bedeutet und die Peptidbindung zwischen $A_1$' und $A_2$' nicht in reduzierter Form vorliegt, die Hydroxygruppe an dem Kohlenstoffatom, welches dem Kohlenstoffatom benachbart ist, das den Rest $R_2$-$CH_2$- trägt, frei oder in geschützter Form vorliegt, und die übrigen Reste die für Verbindungen der Formel I' genannten Bedeutungen haben, eine Carbonsäure der Formel

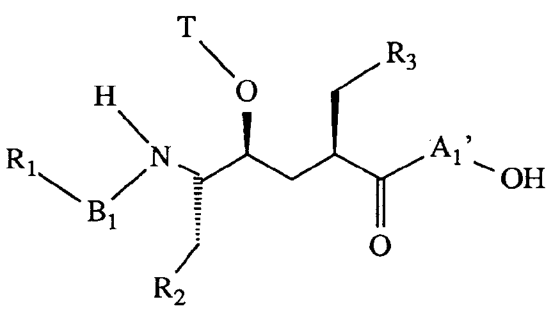

(VIII')

oder ein reaktionsfähiges Derivat davon, worin die Reste die zuletzt genannten Bedeutungen haben, mit einer Aminoverbindung der Formel

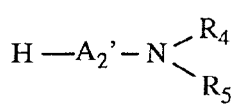

(IX)

oder einem reaktionsfähigen Derivat davon, worin die Reste die zuletzt genannten Bedeutungen haben, kondensiert, wobei freie funktionelle Gruppen in den Ausgangsmaterialien der Formeln VIII' und IX mit Ausnahme der an der Reaktion beteiligten erforderlichenfalls in geschützter Form vorliegen, und gewünschtenfalls vorhandene Schutzgruppen abspaltet, oder

e) eine Carbonsäure der Formel

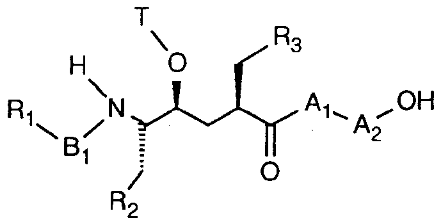

(X')

oder ein reaktionsfähiges Derivat davon, worin die Reste die für Verbindungen der Formel I' genannten Bedeutung haben, mit einer Aminoverbindung der Formel

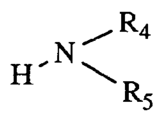

(XI)

oder einem reaktionsfähigen Derivat hiervon, worin die Reste die für Verbindungen der Formel I' genannten Bedeutungen haben, kondensiert, wobei freie funktionelle Gruppen in den Ausgangsmaterialien der Formeln X' und XI mit Ausnahme der an der Reaktion beteiligten erforderlichenfalls in geschützter Form vorliegen, und gewünschtenfalls vorhandene Schutzgruppen abspaltet, oder

f) in einer Verbindung der Formel I', worin die Substituenten die oben genannten Bedeutungen haben mit der Massgabe, dass in der betreffenden Verbindung der Formel I' mindestens eine funktionelle Gruppe durch Schutzgruppen geschützt ist, vorhandene Schutzgruppen abspaltet, oder

g) eine Verbindung der Formel I,

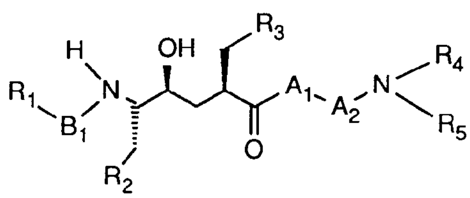

(I)

worin die Reste die für Verbindungen der Formel I' genannten Bedeutungen haben, mit einer Carbonsäure der Formel XXV,

T-OH (XXV)

worin T die bei der Definition von Verbindungen der Formel I' genannten Bedeutungen hat, oder einem reaktionsfähigen Säurederivat davon umsetzt, wobei freie funktionelle Gruppen in den Ausgangsmaterialien der Formeln XXV und I mit Ausnahme der an der Reaktion beteiligten erforderlichenfalls in geschützter Form vorliegen, und

gewünschtenfalls vorhandene Schutzgruppen abspaltet, oder

h) zur Herstellung einer Verbindung der Formel I', worin T einen Rest der Formel Z bedeutet, worin $R^z$ ein durch verethertes oder verestertes Hydroxy oder Mercapto, unsubstituiertes oder substituierte Amino oder über Stickstoff gebundenes Heterocyclyl substituiertes Niederalkyl bedeutet und die übrigen Reste die für Verbindungen der Formel I' genannten Bedeutungen haben, eine Verbindung der Formel

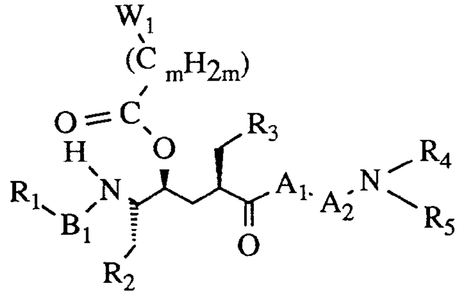

(XXVI)

worin $W_1$ eine Abgangsgruppe bedeutet, $-(C_mH_{2m})-$ einen Niederalkylenrest (m liegt zwischen 1 und 7) bedeutet und die übrigen Reste die für Verbindungen der Formel I' genannten Bedeutungen haben, mit einer Verbindung der Formel

L-H (XXVII),

worin L verethertes oder verestertes Hydroxy oder Mercapto, unsubstituiertes oder substituierte Amino oder über Stickstoff gebundenes Heterocyclyl bedeutet, oder einen reaktionsfähigen Derivat davon umsetzt, wobei freie funktionelle Gruppen in den Ausgangsmaterialien der Formeln XXVI und XXVII mit Ausnahme der an der Reaktion beteiligten erforderlichenfalls in geschützter Form vorliegen, und gewünschtenfalls vorhandene Schutzgruppen abspaltet,
und/oder gewünschtenfalls eine nach einem der vorstehenden Verfahren a) bis h) erhaltene Verbindung der Formel I' mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I' auftrennt und/oder eine erfindungsgemässe Verbindung der Formel I' in eine andere erfindungsgemässe Verbindung der Formel I' umwandelt.